# EUROPÄISCHE PATENTSCHRIFT

(11) **EP 1 352 061 B1**
(45) Veröffentlichungstag und Bekanntmachung des Hinweises auf die Patenterteilung: **31.05.2006**
(21) Anmeldenummer: 02710786.1
(22) Anmeldetag: 09.01.2002
(51) Int. Cl.: C12N 15/11

(54) **VERFAHREN ZUR HEMMUNG DER EXPRESSION EINE ZIELGENS**
METHOD FOR INHIBITING THE EXPRESSION OF A TARGET GENE
PROCEDE POUR INHIBER L'EXPRESSION D'UN GENE CIBLE

(30) Priorität: 09.01.2001 DE 10100586; 26.10.2001 DE 10155280; 29.11.2001 DE 10158411; 07.12.2001 DE 10160151
(43) Veröffentlichungstag der Anmeldung: 15.10.2003
(73) Patentinhaber: Alnylam Europe AG, 95326 Kulmbach (DE)
(72) Erfinder: KREUTZER, Roland, 95466 Weidenberg (DE); LIMMER, Stephan, 95326 Kulmbach (DE); LIMMER, Sylvia, 95326 Kulmbach (DE); HADWIGER, Philipp, 96264 Altenkunstadt (DE)
(74) Vertreter: Gassner, Wolfgang
(86) Internationale Anmeldenummer: PCT/EP2002/000152
(87) Internationale Veröffentlichungsnummer: WO 2002/055693

(56) Entgegenhaltungen:
- WO-A-00/44895
- WO-A-00/44914
- WO-A-01/75164
- WO-A-02/44321
- WO-A-94/01550
- WO-A-98/05770
- WO-A-99/32619
- ZAMORE PHILLIP D ET AL: "RNAi: Double-stranded RNA directs the ATP-dependent cleavage of mRNA at 21 to 23 nucleotide intervals" CELL, CELL PRESS, CAMBRIDGE, NA, US, Bd. 101, Nr. 1, 31. März 2000 (2000-03-31), Seiten 25-33, XP002208683 ISSN: 0092-8674
- BASS BRENDA L: "Double-stranded RNA as a template for gene silencing" CELL, CELL PRESS, CAMBRIDGE, NA, US, Bd. 101, Nr. 3, 28. April 2000 (2000-04-28), Seiten 235-238, XP002194756 ISSN: 0092-8674
- UHLMANN E ET AL: "ANTISENSE OLIGONUCLEOTIDES: A NEW THERAPEUTIC PRINCIPLE" CHEMICAL REVIEWS, AMERICAN CHEMICAL SOCIETY. EASTON, US, Bd. 90, Nr. 4, 1. Juni 1990 (1990-06-01), Seiten 543-584, XP000141412 ISSN: 0009-2665
- PARRISH S., FLEENOR J., ET AL.: "Functional Anatomy of a dsRNA trigger: differential requirement for the two trigger strands in RNA interference." MOL. CELL, Bd. 6, November 2000 (2000-11), Seiten 1077-187, XP002226361
- AMBROS VICTOR: "Dicing up RNAs" SCIENCE, AMERICAN ASSOCIATION FOR THE ADVANCEMENT OF SCIENCE,, US, Bd. 293, Nr. 5531, 3. August 2001 (2001-08-03), Seiten 811-813, XP002183122 ISSN: 0036-8075
- ELBASHIR SAYDA M ET AL: "RNA interference is mediated by 21- and 22-nucleotide RNAs" GENES AND DEVELOPMENT, COLD SPRING HARBOR LABORATORY PRESS, NEW YORK, US, Bd. 15, Nr. 2, 15. Januar 2001 (2001-01-15), Seiten 188-200, XP002204651 ISSN: 0890-9369

## Beschreibung

Die Erfindung betrifft ein Verfahren, eine Verwendung und ein Medikament zur Hemmung der Expression eines Zielgens.

Aus der WO 99/32619 sowie der WO 00/44895 sind Verfahren zur Hemmung der Expression von medizinisch oder biotechnologisch interessanten Genen mit Hilfe einer doppelsträngigen Ribonukleinsäure (dsRNA) bekannt. Die bekannten Verfahren sind zwar hoch effektiv. Es besteht gleichwohl das Bedürfnis, deren Effizienz weiter zu steigern.

Aufgabe der vorliegenden Erfindung ist es, die Nachteile nach dem Stand der Technik zu beseitigen. Es sollen insbesondere ein Verfahren, eine Verwendung und ein Medikament angegeben werden, mit denen eine noch effizientere Hemmung der Expresion eines Zielgens erreichbar ist.

Diese Aufgabe wird durch die Merkmale der Ansprüche 1, 41 und 81 gelöst. Vorteilhafte Ausgestaltungen ergeben sich aus den Merkmalen der Ansprüche 2 bis 40, 42 bis 80 und 82 bis 103.

Mit den erfindungsgemäß beanspruchten Merkmalen wird überraschenderweise eine drastische Erhöhung der Effektivität der Hemmung der Expression eines Zielgens in vitro und in vivo erreicht. Durch die besondere Ausbildung der Enden der dsRNA kann sowohl deren Effizienz bei der Vermittlung der hemmenden Wirkung auf die Expression eines Zielgens als auch deren Stabilität gezielt beeinflusst werden. Durch die Vergößerung der Stabilität wird die wirksame Konzentration in der Zelle erhöht.

Unter einem "Zielgen" im Sinne der Erfindung wird der DNA-Strang der doppelsträngigen DNA in der Zelle verstanden, welcher koplementär zu einem bei der Transkription als Matritze dienenden DNA-Strang einschließlich aller transkibierten Bereiche ist. Bei dem "Zielgen" handelt es sich also im allgemeienen um den Sinnstrang. Der eine Strang bzw. Antisinnstrang (as1) kann komplementär zu einem bei der Expression des Zielgens gebildeten RNA-Transkipt oder deren Prozessierungsprodukt, z .B. eine mRNA, sein. Unter "Einführen" wird die Aufnahme in die Zelle verstanden. Die Aufnahme kann durch die Zelle selbst erfolgen; sie kann auch durch Hilfsstoffe oder Hilfsmittel vermittelt werden. Unter einem "Überhang" wird ein endständiger einzelsträngiger Überstand verstanden, welcher nicht nach Matson & Crick gepaarte Nukleotide aufweist. Unter einer "doppelsträngigen Struktur" wird eine Struktur verstanden, bei der die Nukleotide der Einzelstränge im wesentlichen nach Watson & Crick gepaart sind. Im Rahmen der vorliegenden Erfindung kann eine doppelsträngige Struktur auch einzelne Fehlpaarungen ("Mismatches") aufweisen.

Nach einer besonderes vorteilhaften Ausgestaltung weist die dsRNA I den Überhang am 3'-Ende des einen Strangs bzw. Antisinnstrangs as1 auf. Die dsRNA I kann auch an einem Ende glatt ausgebildet sein. In diesem Fall befindet sich das glatte Ende vorteilhafterweise auf der Seite der dsRNA 1, die das 5'-Ende des einen Strangs (Antsinnstrang; as1). In dieser Ausbildung zeigt die dsRNA I einerseits eine sehr gute Effektivität und andererseits eine hohe Stabilität im lebenden Organismus. Die Effektivität insgesamt in vivo ist hervorragend. Der Überhang ist zweckmäßigerweise aus 1 bis 4 Nukleotiden, vorzugsweise aus 1 oder 2 Nukleotiden, gebildet.

Nach einem weiteren Ausgestaltungsmerkmal kann die Effektivität des Verfahrens weiter erhöht werden, wenn zumindest eine entsprechend der erfindungsgemäßen dsRNA I ausgebildete weitere dsRNA II in die Zelle eingeführt wird, wobei der eine Strang oder zumindest ein Abschnitt des einen Strangs der doppelsträngigen Struktur der dsRNA I komplementär zu einem ersten Bereich des Sinnstrangs des Zielgens ist, und wobei ein weiterer Strang oder zumindest ein Abschnitt des weiteren Strangs der doppelsträngigen Struktur der weiteren dsRNA II komplementär zu einem zweiten Bereich des Sinnstrangs des Zielgens ist. Die Hemmung der Expression des Zielgens ist in diesem Fall deutlich gesteigert. Der erste und der zweite Bereich können abschnittsweise überlappen, aneinander grenzen oder auch voneinander beabstandet sein.

Es hat sich weiter als vorteilhaft erwiesen, wenn die dsRNA I und/oder die weitere dsRNA II eine Länge von weniger als 25 aufeinander folgenden Nukleotidpaaren aufweisen. Als besonders effektiv hat sich eine Länge im Bereich zwischen 19 und 23 Nukleotidpaaren erwiesen. Die Effizienz kann weiter gesteigert werden, wenn an den vorzugsweise aus 19 bis 23 Nukleotidpaaren gebildeten Doppelsträngen einzelsträngige Überhänge von 1 bis 4 Nukleotiden vorhanden sind.

Das Zielgen kann nach einem weiteren Ausgestaltungsmerkmal eine der in dem anhängenden Sequenzprotokoll wiedergegebenen Sequenzen SQ001 bis SQ140 aufweisen. Es kann auch aus der folgenden Gruppe ausgewählt sein: Onkogen, Cytokin-Gen, Id-Protein-Gen, Priongen, Gene zur Expression von Angiogenese induzierenden Molekülen, von Adhäsions-Molekülen und Zelloberflächenrezeptoren, Gene von Proteinen, die an metastasierenden und/oder invasiven Prozessen beteiligt sind, Gene von Proteinasen sowie Apoptose- und Zellzyklusregulierende Molekülen sowie Gene zur Expression des EGF-Rezeptors. Beim Zielgen kann es sich insbesondere um das MDR1-Gen handeln. Es kann in diesem Zusammenhang eine der Sequenzen SQ141 - 173 bestehende bzw. ein aus jeweils zusammengehörenden Antisinn (as)- und Sinnsequenzen (ss) kombinierte dsRNA I/II verwendet werden.

Nach einem weiteren vorteilhaften Ausgestaltungsmerkmal wird die Expression nach dem Prinzip der RNA-Interferenz gehemmt.

Das Zielgen wird zweckmäßigerweise in pathogenen Organismen, vorzugsweise in Plasmodien, exprimiert. Es kann Bestandteil eines Virus oder Viroids, insbesondere eines humanpathogenen Virus oder Viroids, sein. Das Virus oder Viroid kann auch ein tier- oder pflanzenpathogenes Virus oder Viroid sein.

Nach einem weiteren Ausgestaltungsmerkmal ist vorgesehen, dass die ungepaarten Nukleotide durch Nukleosidthiophosphate substituiert sind.

Zumindest ein Ende der dsRNA I/II kann modifiziert werden, um einem Abbau in der Zelle oder einer Dissoziation in die Einzelstränge entgegenzuwirken. Vorteilhafterweise wird dazu der durch die komplementären Nukleotidpaare bewirkte Zusammenhalt der doppelsträngigen Struktur durch mindestens eine chemische Verknüpfung erhöht. Die chemische Verknüpfung kann durch eine kovalente oder ionische Bindung, eine Wasserstoffbrückenbindung, hydrophobe Wechselwirkungen, vorzugsweise van-der-Waals- oder Stapelungswechelwirkungen, oder durch Metall-Ionenkoordination gebildet werden. Es hat sich weiter als zweckmäßig und die Stabilität erhöhend erwiesen, wenn die chemische Verknüpfung in der Nähe des einen Endes gebildet ist. Weitere vorteilhafte Ausgestaltungen hinsichtlich der chemischen Verknüpfung können den Merkmalen der Ansprüche 24 bis 30 entnommen werden, ohne dass es dafür einer näheren Erläuterung bedarf.

Die dsRNA I/II kann dann besonders einfach in die Zelle eingeschleust werden, wenn sie in micellare Strukturen, vorteilhafterweise in Liposomen, eingeschlossen wird. Zum Transport der dsRNA I/II in die Zelle hat es sich auch als vorteilhaft erwiesen, dass diese an mindestens ein von einem Virus stammendes, davon abgeleitetes oder ein synthetisch hergestelltes virales Hüllprotein gebunden, damit assoziiert oder davon umgeben werden. Das Hüllprotein kann vom Polyomavirus abgeleitet sein. Das Hüllprotein kann insbesondere das Virus-Protein 1 und/oder das Virus-Protein 2 des Polyomavirus enthalten. Nach einer weiteren Ausgestaltung ist vorgesehen, dass bei Bildung eines Kapsids oder kapsidartigen Gebildes aus dem Hüllprotein die eine Seite zum Inneren des Kapsids oder kapsidartigen Gebildes gewandt ist. Ferner ist es von Vorteil, dass der eine Strang der dsRNA I/II (as1/2) zum primären oder prozessierten RNA-Transkript des Zielgens komplementär ist. Die Zelle kann eine Vertebratenzelle oder eine menschliche Zelle sein.

Weiterhin hat es sich gezeigt, dass die dsRNA I/II vorteilhafterweise bereits in einer Menge von höchstens 5 mg/kg Körpergewicht pro Tag einem Säugetier, vorzugsweise einem Menschen, verabreicht werden kann. Bereits in dieser geringen Dosis wird eine ausgezeichnete Effektivität erzielt.

Überraschenderweise hat sich gezeigt, dass die dsRNA I/II zur Applikation in eine Pufferlösung aufgenommen und dann oral oder mittels Injektion oder Infusion intravenös, intratumoral, inhalativ, intraperitoneal verabreicht werden kann.

Erfindungsgemäß ist weiterhin die Verwendung einer doppelsträngigen Ribonukleinsäure (dsRNA I) zur Hemmung der Expression eines Zielgens in einer Zelle vorgesehen, wobei die dsRNA I eine doppelsträngige aus höchstens 49 aufeinander folgenden Nukleotidpaaren gebildete Struktur aufweist, und wobei ein Strang (Antisinnstrang; as1) oder zumindest ein Abschnitt des einen Strangs (as1) der doppelsträngigen Struktur komplementär zum Sinnstrang des Zielgens ist, und wobei die dsRNA I zumindest an einem Ende einen aus 1 bis 4 Nukleotiden gebildeten Überhang aufweist.

Nach weiterer Maßgabe der Erfindung ist ein Medikament zur Hemmung der Expression eines Zielgens in einer Zelle vorgesehen, enthaltend eine doppelsträngige Ribonukleinsäure (dsRNA I) in einer zur Hemmung der Expression des Zielgens ausreichenden Menge, wobei die dsRNA I eine doppelsträngige aus höchstens 49 aufeinander folgenden Nukleotidpaaren Struktur aufweist, und wobei ein Strang (as1) oder zumindest ein Abschnitt des einen Strangs (as1) der doppelsträngigen Struktur komplementär zum Sinnstrang des Zielgens ist, und wobei die dsRNA I zumindest an einem Ende einen aus 1 bis 4 Nukleotiden gebildeten Überhang aufweist.

Wegen der weiteren vorteilhaften Ausgestaltung der dsRNA I/II wird auf die vorangegangenen Ausführungen verwiesen.

Die Erfindung wird nachfolgend anhand der Zeichnungen und Ausführungsbeispiele beispielhaft erläutert. Es zeigen:
- Fig. 1a, b: schematisch eine erste und zweite doppelsträngige RNA und
- Fig. 2: schematisch ein Zielgen,
- Fig. 3: relative YFP-Fluoreszenz nach Applikation verschiedener dsRNA in NIH/3T3-Zellen (erstes Experiment),
- Fig. 4: relative YFP-Fluoreszenz nach Applikation verschiedener dsRNA in NIH/3T3-Zellen (zweites Experiment),
- Fig. 5: relative YFP-Fluoreszenz nach Applikation verschiedener dsRNA in NIH/3T3-Zellen (drittes Experiment),
- Fig. 6: relative YFP-Fluoreszenz nach Applikation verschiedener dsRNA in NIH/3T3-Zellen (viertes Experiment),
- Fig. 7: relative YFP-Fluoreszenz nach Applikation verschiedener dsRNA in HeLa-S3-Zellen (fünftes Experiment),
- Fig. 8: fluoreszenzmikroskopische Aufnahmen von NIH/3T3-Zellen nach Transfektion mit pcDNA-YFP bzw nach Kotransfektion mit pcDNA-YFP und verschiedenen dsRNAs,
- Fig. 9: fluoreszenzmikroskopische Aufnahmen von HeLa-S3-Zellen nach Transfektion mit pcDNA-YFP bzw. nach Kotransfektion mit pcDNA-YFP und verschiedenen dsRNAs,
- Fig. 10: gelelektrophoretische Auftrennung von S1 nach Inkubation in Maus-Serum,
- Fig. 11: gelelektrophoretische Auftrennung von S1 nach Inkubation in humanem Serum,
- Fig. 12: gelelektrophoretische Auftrennung von S7 nach Inkubation in Maus-Serum,
- Fig. 13: gelelektrophoretische Auftrennung von S7 nach Inkubation in humanem Serum,
- Fig. 14: gelelektrophoretische Auftrennung von K3 nach Inkubation in Maus-Serum,
- Fig. 15: gelelektrophoretische Auftrennung von PKC1/2 nach Inkubation in Maus-Serum,
- Fig. 16: gelelektrophoretische Auftrennung von S1A/S4B nach Inkubation in humanem Serum,
- Fig. 17: gelelektrophoretische Auftrennung von K2 nach Inkubation in humanem Serum und
- Fig. 18: GFP-spezifische Immunoperoxidase-Färbung an Nieren-Paraffinschnitten transgener GFP-Mäuse,
- Fig. 19: GFP-spezifische Immunoperoxidase-Färbung an Herz-Paraffinschnitten transgener GFP-Mäuse,
- Fig. 20: GFP-spezifische Immunoperoxidase-Färbung an Pankreas-Paraffinschritten transgener GFP-Mäuse,
- Fig. 21: Western-Blot-Analyse der GFP-Expression im Plasma,
- Fig. 22: Western-Blot-Analyse der GFP-Expression in der Niere,
- Fig. 23: Western-Blot-Analyse der GFP-Expression im Herz,
- Fgi. 24: Western-Blot-Analyse der EGFR-Expression in U-87 MG Glioblastom-Zellen,
- Fig. 25a: Northern-Blot-Analyse des MDRI mRNA-Niveaus in der Kolonkarzinom-Zelllinie LS174T, wobei die Zellen nach 74 Stunden geerntet wurden,
- Fig. 25b: Quantifizierung der Banden nach Fig. 25a, wobei die Mittelwerte aus zwei Werten dargestellt sind,
- Fig. 26a: Northern-Blot-Analyse des MDRI mRNA-Niveaus in der Kolonkarzinom-Zelllinie LS174T, wobei die Zellen nach 48 Stunden geerntet wurden,
- Fig. 26b: Quantifizierung der Banden nach Fig. 26a, wobei die Mittelwerte aus zwei Werten dargestellt sind,
- Fig. 27: vergleichende Darstellung einer durchlicht- und fluoreszenzmikroskopischen Aufnahme einer Transfektion mit 175 nM dsRNA (Sequenz R1 in Tabelle 4).

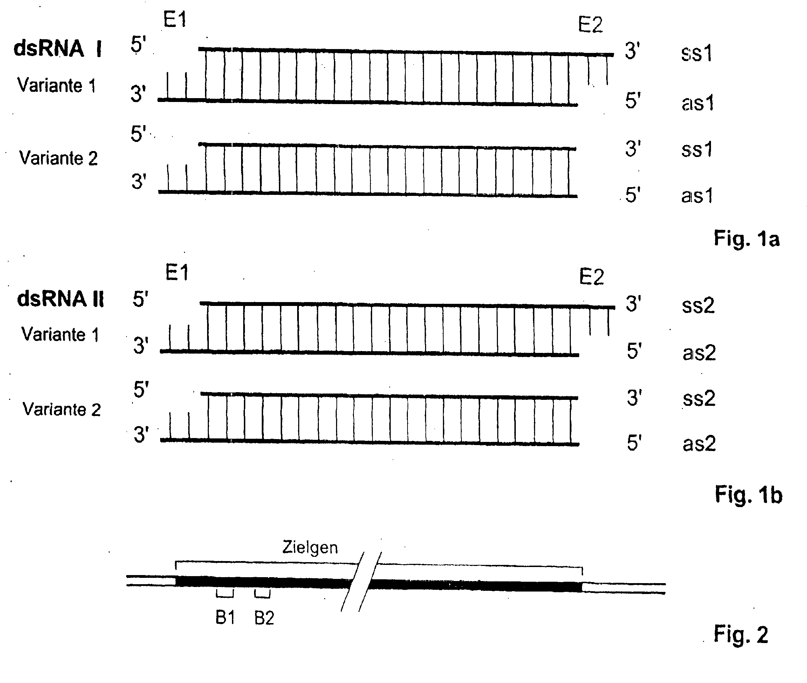

Die in den Fig. 1a und 1b schematisch gezeigten doppelsträngigen Ribonukleinsäuren dsRNA I und dsRNA II weisen jeweils ein ersces Ende E1 und ein zweites Ende E2 auf. Die erste und die zweite Ribonukleinsäure dsRNA I/dsRNAII weisen an ihren beiden Enden E1 und E2 einzelsträngige, aus etwa 1 bis 4 ungepaarten Nukleotiden gebildete Abschnitts auf. Es sind zwei mögliche Varianten dargestellt (Variante 1 und 2), wobei Variante 2 ein glattes Ende (E2) aufweist. Das glatte Ende kann jedoch auch in einer weiteren Variante am anderen Ende (E1) liegen.

In Fig. 2 ist schematisch ein auf einer DNA befindliches Zielgen gezeigt. Das Zielgen ist durch einen schwarzen Balken kenntlich gemacht. Es weist einen ersten Bereich B1 und einen zweiten Bereich B2 auf.

Jeweils der eine Strang der ersten dsRNA I (as1) bzw. der zweiten dsRNA II (as2) ist komplementär zum entsprechenden Bereich B1 bzw. B2 auf dem Zielgen.

Die Expression des Zielgens wird dann besonders wirkungsvoll gehemmt, wenn die dsRNA I/dsRNA II an ihren Enden E1, E2 einzelsträngige Abschnitte aufweist. Die einzelsträngigen Abschnitte können sowohl am Strang as1 oder as2 als auch am Gegenstrang (ss1 bzw. ss2) oder am Strang as1, as2 und am Gegenstrang ausgebildet sein.

Die Bereiche B1 und B2 können, wie in Fig. 2 gezeigt, von einander beabstandet sein. Sie können aber auch aneinander grenzen oder überlappen.

### I. Hemmung der Expression des YFP-Gens in Fibroblasten:

Es wurden aus Sequenzen des Yellow Fluorescent Proteine (YFP), einer Variante des GFP (Grün-fluoreszierendes Protein) der Alge *Aequoria victoria* abgeleitete doppelsträngige RNAs (dsRNAs) hergestellt und zusammen mit einem YFP-kodierenden Plasmid in Fibroblasten mikroinjiziert. Anschließend wurde die Fluoreszenzabnahme gegenüber Zellen ohne dsRNA ausgewertet.

### Versuchsprotokoll:

Mittels eines RNA-Synthesizer (Typ Expedite 8909, Applied Biosystems, Weiterstadt, Deutschland) und herkömmlicher chemischer Verfahren wurden die aus den Sequenzprotokollen SQ148, 149 und SQ159 ersichtlichen RNA-Einzelstränge und die zu ihnen komplementären Einzelstränge synthetisiert. Anschließend erfolgte die Reinigung mit Hilfe der HPLC. Die Hybridisierung der Einzelstränge zum Doppelstrang erfolgte durch Erhitzen des stöchiometrischen Gemischs der Einzelstränge in 10 mM Natriumphosphatpuffer, pH 6,8, 100 mM NaCl, auf 90°C und nachfolgendes langsames Abkühlen über 6 Stunden auf Raumtemperatur. Die so erhaltenen dsRNAs wurden in die Testzellen mikroinjiziert.

Als Testsystem für diese Zellkultur-Experimente diente die murine Fibroblasten-Zellinie NIH/3T3, ECACC No. 93061524 (European Collection of Animal Cell Culture). Für die Mikroinjektionen wurde das Plasmid pcDNA-YFP verwendet, das ein 800bp großes Bam HI/Eco RI-YFP-Fragment in den entsprechenden Restriktionsschnittstellen des Vectors pcDNA3 enthält. Die Expression des YFP wurde unter dem Einfluß gleichzeitig mittransfizierter sequenzhomologer dsRNA untersucht. Die Auswertung unter dem Fluoreszenzmikroskop erfolgte frühestens 3 Stunden nach Injektion anhand der grünen Fluoreszenz.

### Vorbereitung der Zellkulturen:

Die Kultivierung der Zellen erfolgte in DMEM mit 4,5 g/l Glucose, 10 % fötalem Kälberserum (FCS), 2 mM L-Glutamin, Penicillin/Streptomycin (100 IE/100 µg/ml, Biochrom) im Brutschrank unter 5 % CO₂-Atmosphäre bei 37°C. Die Zellen wurden alle 3 Tage passagiert, um sie in der exponentiellen Wachstumsphase zu halten. Einen Tag vor der Durchführung der Transfektion wurden die Zellen trypsiniert (10x Trypsin/TEDTA, Biochrom) und mit einer Zelldichte von 0,3 x 10⁵ Zellen in beschichteten Petrischalen (CORNING® Cell Culture Dish, 35 mm, Corning Inc., Corning, USA) ausgesät. Die Petrischalen wurden mit 0,2 % Gelatine (Biochrom) für mindestens 30 Minuten bei 37°C inkubiert, einmal mit PBS gewaschen und sofort für die Aussaat der Zellen verwendet. Um ein Wiederfinden individueller Zellen zu ermöglichen, wurden CELLocate Coverslips der Fa. Eppendorf (Square size 55 µm) verwendet.

### Mikroinjektion:

Zur Durchführung der Mikroinjektion wurden die Petrischalen ca. 10 Minuten aus dem Brutschrank genommen. Pro Schale und Ansatz wurden ca. 50 Zellen mikroinjiziert (FemtoJet; Mikromanipulator 5171, Eppendorf). Für die Mikroinjektion wurden Glaskapillaren (FemtoTip) der Firma Eppendorf mit einem Spitzeninnendurchmesser von 0,5 µm verwendet. Die Injektionsdauer betrug 0,8 Sekunden und der Druck 30 hPa. Durchgeführt wurden die Mikroinjektionen an einem Olympus IX50 Mikroskop mit Fluoreszenzeinrichtung. Als Injektionspuffer wurde 14 mM NaCl, 3 mM KCl, 10 mM KH₂PO₄, pH 7,0 verwendet, der 0,01 µg/µl pcDNA-YFP enthielt. Zur Überprüfung einer erfolgreichen Mikroinjektion wurde der Injektionslösung jeweils 0,08% (w/v) an Dextran-70000 gekoppeltes Texas-Rot (Molecular Probes, Leiden, Niederlande) zugesetzt. Um die Inhibition der YFP-Expression mit spezifischer dsRNA zu untersuchen, wurden der Injektionslösung dsRNAs zugegeben: Ansatz 1: 0,1 µM dsRNA (Sequenzprotokoll SQ148/149); Ansatz 2: 0,1 µM dsRNA (Sequenzprotokoll SQ148/159); Ansatz 3: ohne RNA. Nach der Mikroinjektion wurden die Zellen für mindestens drei weitere Stunden im Brutschrank inkubiert. Danach wurden die intrazelluläre YFP-Fluoreszenz am Mikroskop ausgewertet: gleichzeitig rot und grün-fluoreszierende Zellen: Mikroinjektion war erfolgreich, es wird keine Inhibition der YFP-Expression durch dsRNA beobachtet; bzw. es handelt sich um Kontrollzellen, in die keine dsRNA injiziert wurde; nur rot-fluoreszierende Zellen: Mikroinjektion war erfolgreich, die dsRNA inhibiert YFP-Expression.

### Ergebnisse:

Bei einer dsRNA-Konzentration von 0,1 *µ*M konnte beim Einsatz der dsRNA mit den an beiden 3'-Enden um je zwei Nukleotide überstehenden Einzelstrangbereichen (Sequenzprotokoll SQ148/159) eine merklich erhöhte Hemmung der Expression des YFP-Gens in Fibroblasten beobachtet werden im Vergleich zur dsRNA ohne überstehende Einzelstrangenden (Tabelle 1).

Die Verwendung von kurzen, 19-25 Basenpaare enthaltenden, dsRNA-Molekülen mit Überhängen aus wenigen, vorzugsweise 1 bis 3 nicht-basengepaarten, einzelsträngigen Nukleotiden ermöglicht somit eine vergleichsweise stärkere Hemmung der Genexpression in Säugerzellen als die Verwendung von dsRNAs mit derselben Anzahl von Basenpaaren ohne die entsprechenden Einzelstrangüberhänge bei jeweils gleichen RNA-Konzentrationen.

**Tabelle 1: Die Symbole geben den relativen Anteil an nicht oder schwach grün-fluoreszierenden Zellen an (+++ > 90%; ++ 60-90%; + 30-60%; - < 10%).**

| **Ansatz** | **Name** | **Sequenzprotokoll-Nr.** | **0.1 µM** |
|---|---|---|---|
| 1 | **S1A/** | SQ148 | + |
| | **S1B** | SQ149 | |
| **2** | **S1A/** | SQ148 (überstehende Enden) | +++ |
| | **S4B** | SQ159 | |
| **3** | | ohne RNA | - |

### II. Hemmung der Genexpression eines Zielgens in kultivierten HELA-S3-Zellen und Mausfibroblasten durch dsRNA:

Die Effektivität der Inhibition der YFP-Expression nach transienter Transfektion eines YFP-codierenden Plasmids auf der Basis der RNA-Interferenz mit dsRNAs läßt sich durch Gestaltung der 3'-Enden und der Länge des basengepaarten Bereichs modulieren.

### Ausführungsbeispiel:

Zum Wirksamkeitsnachweis der dsRNA bei der spezifischen Inhibition der Genexpression wurden transient transfizierte NIH/3T3-Zellen (Fibroblasten aus NIH Swiss Mausembryo, ECCAC (European collection of animal cell culture) Nr. 93061524) und HELA-S3 (humane cervikale Karzinomzellen, DSMZ (Deutsche Sammlung von Mikroorganismen und Zellkulturen) Nr. ACC 161) verwendet. Für die Transfektion wurde das Plasmid pcDNA-YFP verwendet, das ein 800 bp großes Bam HI /Eco RI-YFP-Fragment in den entsprechenden Schnittstellen des Vektors pcDNA3 enthält. Aus der Sequenz des gelb-fluoreszierenden Proteins (YFP) abgeleitete doppelsträngige RNAs (dsRNAs) wurden hergestellt und zusammen mit dem Plasmid pcDNA-YFP transient in die Fibroblasten transfiziert (Die verwendeten spezifischen dsRNAs sind in ihren Antisinn-Strängen komplementär zu entsprechenden Abschnitten der Gensequenzen von sowohl YFP als auch GFP). Nach 48 Stunden wurde die Fluoreszenzabnahme quantifiziert. Als Kontrollen fungierten Zellen, die entweder nur mit pcDNA-YFP oder mit pcDNA-YFP und einer Kontroll-dsRNA (nicht aus der YFP-Sequenz abgeleitet) transfiziert wurden.

### Versuchsprotokoll:

### dsRNA-Synthese:

Mittels eines RNA-Synchesizers (Typ Expedite 8909, Applied Biosystems, Meiterstadt, Deutschland) und herkömmlicher chemischer Verfahren wurden die aus den Sequenzprotokollen ersichtlichen RNA-Eintelstränge und die zu ihnen komplementären Einzelstränge synthetisiert. Anschließend erfolgte die Reinigung der rohen Syntheseprodukte mit Hilfe der HPLC. Verwendet wurde die Säule NucleoPac PA-100, 9x250 mm, der Fa. Dionex; als Niedersalz-Puffer 20 mM Tris, 10 mM NaClO₄, pH 6,8, 10% Acetonitril und als Hochsalz-Puffer 20 mM Tris, 400 mM NaClO₄, pH 6,8, 10% Acetonitril. Der Fluß betrug 3 ml/ Minute. Die Hybridisierung der Einzelstränge zum Doppelstrang erfolgte durch Erhitzen des stöchiometrischen Gemischs der Einzelstränge in 10 mM Natriumphosphatpuffer, pH 6,8, 100 mM NaCl, auf 80-90°C und nachfolgendes langsames Abkühlen über 6 Stunden auf Raumtemperatur.

### Aussaat der Zellen:

Alle Zellkulturarbeiten wurden unter sterilen Bedingungen in einer entsprechenden Werkbank (HS18, Hera Safe, Kendro, Heraeus) durchgeführt. Die Kultivierung der NIH/3T3-Zellen und der HELA-S3 erfolgte im Brutschrank (CO₂-Inkubator T20, Hera cell, Kendro, Heraeus) bei 37°C, 5% CO₂ und gesättigter Luftfeuchtigkeit in DMEM (Dulbecco's modified eagle medium, Biochrom), für die Mausfibroblasten, und Ham's F12 für die HELA-Zellen mit 10% FCS (fetal calf serum, Biochrom), 2 mM L-Glutamin (Biochrom) und Penicillin/Streptomycin (100 IE/100 µg/ml, Biochrom). Um die Zellen in der exponentiellen Wachstumsphase zu halten, wurden die Zellen alle 3 Tage passagiert. 24 Stunden vor der Durchführung der Transfektion wurden die Zellen trypsiniert (10x Trypsin/EDTA, Biochrom, Deutschland) und mit einer Zelldichte von 1,0 x 10⁴ Zellen/Vertiefung in einer 96-Loch-Platte (Multiwell Schalen 96-Well Flachboden, Labor Schubert & Weiss GmbH) in 150 µl Wachstumsmedium ausgesät.

### Durchführung der transienten Transfektion:

Die Transfektion wurde mit Lipofectamine Plus^{™} Reagent (Life Technologies) gemäß den Angaben des Herstellers durchgeführt. Pro Well wurden 0,15 µg pcDNA-YFP-Plasmid eingesetzt. Das Gesamt-Transfektionsvolumen betrug 60 *µ*l. Es wurden jeweils3-fach-Proben angesetzt. Die Plasmid-DNA wurde zuerst zusammen mit der dsRNA komplexiert. Dazu wurde die Plasmid-DNA und die dsRNA in serumfreiem Medium verdünnt und pro 0,1 *µ*g Plasmid-DNA 1 *µ*l PLUS Reagent eingesetzt (in einem Volumen von 10 *µ*l) und nach dem Mischen für 15 Minuten bei Raumtemperatur inkubiert. Während der Inkubation wurde pro 0,1 *µ*g Plasmid-DNA 0,5 *µ*l Lipofectamine in insgesamt 10 *µ*l serumfreiem Medium verdünnt, gut gemischt, zu dem Plasmid/dsRNA/PLUS-Gemisch zugegeben und nochmals 15 Minuten inkubiert. Während der Inkubation wurde ein Mediumwechsel durchgeführt. Die Zellen wurden dazu 1 x mit 200 *µ*l serumfreiem Medium gewaschen und danach mit 40 *µ*l serumfreiem Medium bis zur Zugabe von DNA/dsRNA/PLUS/Lipofectamine weiter im Brutschrank inkubiert. Nach der Zugabe von 20 *µ*l DNA/dsRNA/PLUS/Lipofectamine pro Well wurden die Zellen für 2,5 Stunden im Brutschrank inkubiert. Anschließend wurden die Zellen nach der Inkubation 1 x mit 200 µl Wachstumsmedium gewaschen und für 24 Stunden bis zur Detektion der Fluoreszenz in 200 *µ*l Wachstumsmedium im Brutschrank inkubiert.

### Detektion der Fluoreszenz:

24 Stunden nach dem letzten Mediumwechsel wurde die Fluoreszenz der Zellen am Fluoreszenz-Mikroskop (IX50-S8F2, Fluoreszenz-Einheit U-ULS100Hg, Brenner U-RFL-T200, Olympus) mit einer USH-I02D-Quecksilber-Lampe (USHIO Inc., Tokyo, Japan), ausgestattet mit einem WIB-Fluoreszenz-Würfel und einer digitalen CCD-Kamera (Orca IIIm, Hamamatsu) und C4742-95 Kamera-Controller) photographiert. Die Auswertung der Fluoreszenzaufnahmen erfolgte mit der analysis-Software 3.1 (Soft Imaging Sytem GmbH, Deutschland). Um die YFP-Fluoreszenz in Relation zur Zelldichte zu setzen, wurde eine Zellkernfärbung (Hoechst-Staining) durchgeführt- Dazu wurden die Zellen in 100 *µ*l Methylcarnoy (75% Methanol, 25% Eisessig) zuerst für 5 und danach nochmals für 10 Minuten in Methylcarnoy fixiert. Nach dem Lufttrocknen wurden die fixierten Zellen für 30 Minuten im Dunkeln mit 100 *µ*l pro Well Hoechst-Farbstoff (75 ng/ml) inkubiert. Nach 2maligem Waschen mit PBS (PBS Dulbecco w/o Ca ²⁺, Mg ²⁺, Biochrom) wurden die Hoechst-gefärbten Zellen unter dem Fluoreszenz-Mikroskop (Olympus, WU-Fluoreszenz-Würfel für Hoechst) photographiert.

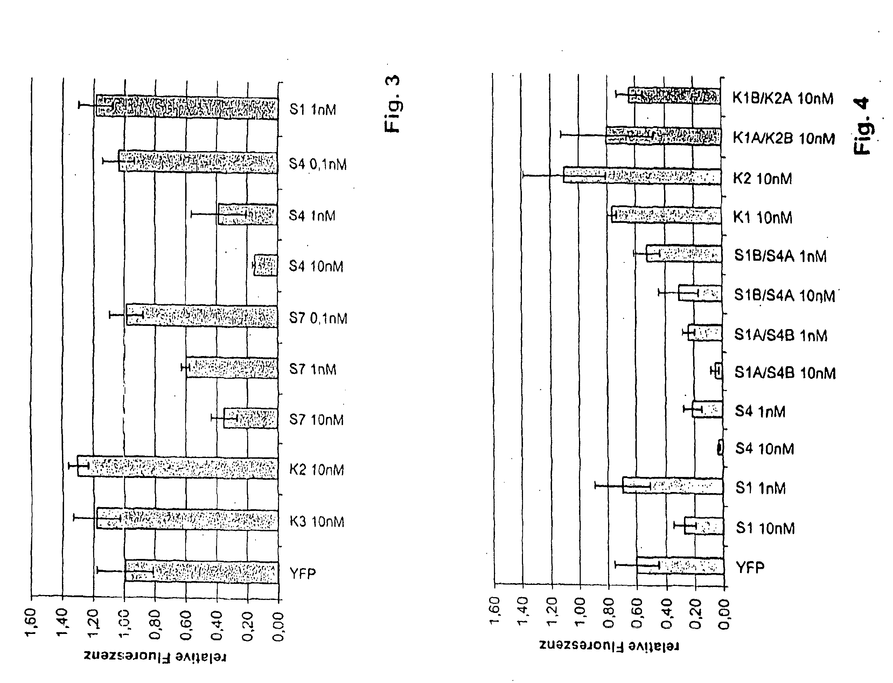

In den Fig. 3 bis 9 sind die Ergebnisse zur Inhibition der YFP-Expression durch dsRNA in kultivierten Zellen zusammengefasst:

In Fig. 3, 4, 5 und 6 sind die Effekte von YFP-spezifischen dsRNAs und von Kontroll-dsRNAs auf die YFP-Expression in NIH/3T3-Mausfibroblasten nach transienter Transfektion zusammengefasst. Die Experimente wurden wie im Versuchsprotokoll beschrieben durchgeführt. Die Konzentration der dsRNA bezieht sich auf die Konzentration im Medium während der Transfektionsreaktion. Die Bezeichnungen für die dsRNAs sind der Tabelle 2 zu entnehmen. Dargestellt ist die relative Fluoreszenz pro Bildausschnitt in Flächenprozent. Pro Well wurden 3 verschiedene Bildausschnitte ausgewertet. Die Mittelwerte ergeben sich aus den 3-fach-Ansätzen.

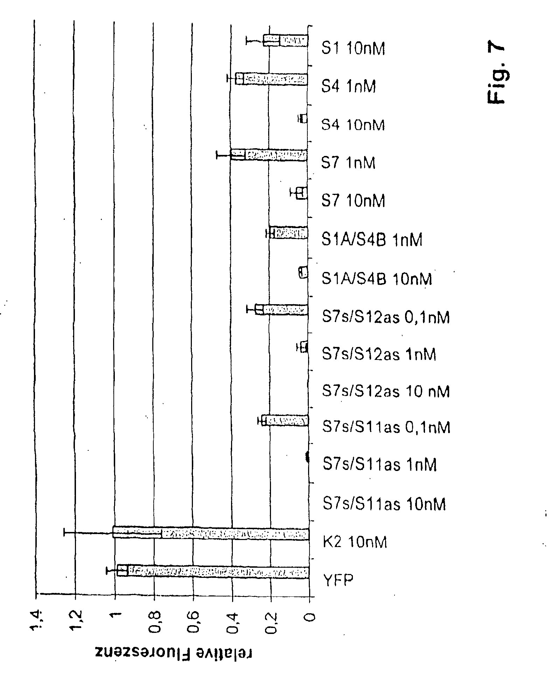

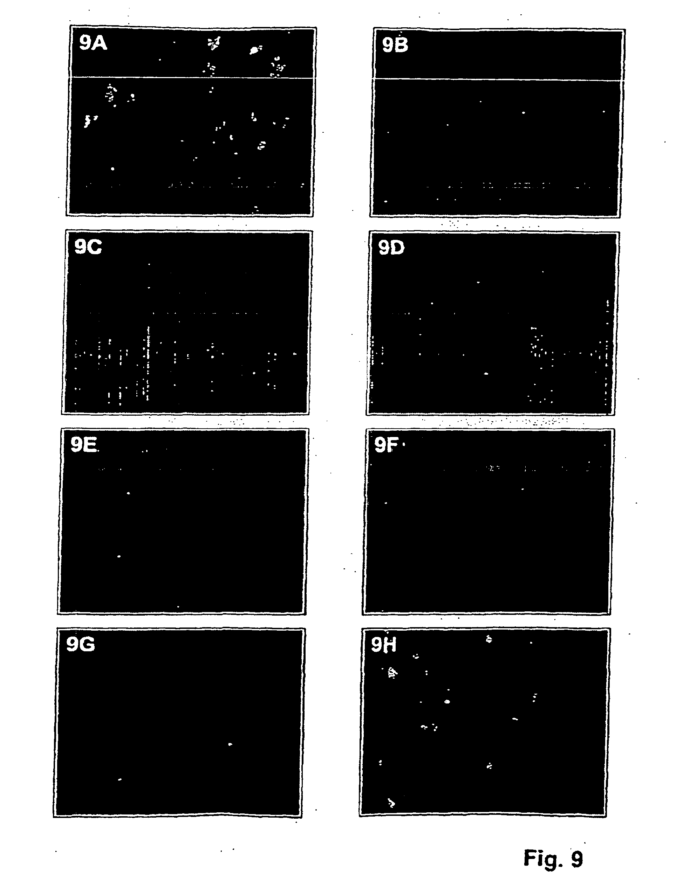

In den Fig. 7 und 9 ist die spezifische Inhibition der YFP-Genexpression durch dsRNAs in HELA-S3-Zellen dargestellt.
In Fig. 7 ist die hemmende Wirkung unterschiedlich gestalteter dsRNA-Konstrukte (Tabelle 2) in verschiedenen Konzentrationen auf die Expression von YFP in EieLa-Zellen dargestellt.

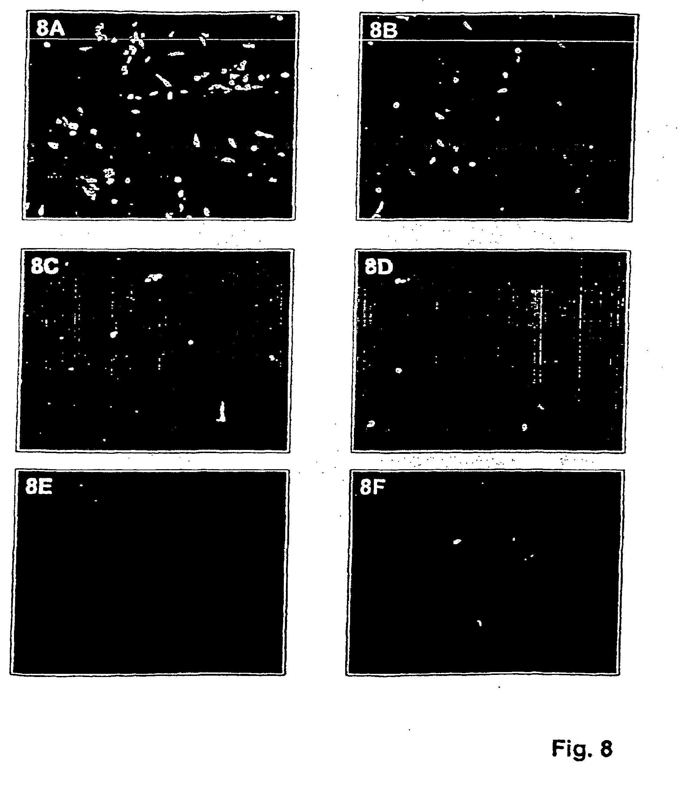

Fig. 8 zeigt repräsentative fluoreszenzmikroskopische Aufnahmen von transient mit YFP transfizierten NIH/3T3-Mausfibroblasten ohne dsRNA und mit spezifisch gegen YFP gerichteten dsRNAs (x 100 Vergrößerung).
8A: YFP-Kontrolle
8B: S1, 10 nM
8C: S4, 10 nM
8D: S7, 10 nM
8E: S7/S11, 1 nM
8F: S7/S12, 1 nM

Fig. 9 zeigt repräsentative fluoreszenzmikroskopische Aufnahmen von transient mit YFP transfizierten HELA-3S-Zellen ohne dsRNA und mit spezifisch gegen YFP gerichteten dsRNAs (x 100 Vergrößerung).
9A: K2-Kontrolle, 10 nM
9B : S1, 10 nM
9C: S4, 10 nM
9D: S7, 10 nM
9E: S7/11, 1 nM
9F: S7/12, 1 nM
9G: S1A/S4B, 10 nM
9H: YFP-Kontrolle

### Ergebnisse:

Fig. 3 zeigt, dass die YFP-Expression nach transienter Kotransfektion von Mausfibroblasten mit dem YFP-Plasmid und spezifisch gegen die YFP-Sequenz gerichteten dsRNAs dann besonders wirkungsvoll gehemmt wird, wenn die 3'-Enden der 22 und 19 Basenpaare enthaltenden Bereiche der dsRNAs einzelsträngige Abschnitte von 2 Nukleotiden (nt) aufweisen. Während die dsRNA S1 mit glatten 3'-Enden bei einer Konzentration von 1 nM (bezogen auf die Konzentration im Zellkultur-Medium während der Durchführung der Transfektion) keine inhibitorischen Effekte auf die YFP-Expression zeigt, inhibieren die dsRNAs S7 (19 Nukleotidpaare) und S4 (22 Nukleotidpaare) mit jeweils 2nt Überhängen an beiden 3'-Enden die YFP-Expression um 50 bzw. um 70% im Vergleich zu den entsprechenden Kontroll-dsRNAs K3 und K2. Bei einer Konzentration von 10 nM inhibiert die als S1 bezeichnete dsRNA mit glatten Enden die YFP-Expression um ~65%, während die Inhibition der YFP-Expression durch die S4 dsRNA ~93% beträgt (Fig. 4). Der inhibitorische Effekt der mit S4 und S7 bezeichneten dsRNAs ist konzentrationsabhängig (Fig. 3 und 4, siehe auch Fig. 7).

Fig. 4 zeigt, dass für die effiziente Unterdrückung der YFP-Genexpression die einzelsträngige Ausbildung nicht an beiden 3'-Enden (auf Sinn- und Antisinn-Strang) notwendig ist. Um eine möglichst effektive Inhibition der YFP-Expression zu erreichen, ist lediglich der 2nt-Überhang am 3'-Ende auf dem Antisinn-Strang notwendig. So liegt die Inhibition der YFP-Expression bei einer Konzentration von 1 nM bei den beiden dsRNAs S4 (mit 2nt-Überhängen auf beiden 3'-Enden) und S1A/S4B (mit einem 2nt-Überhang auf dem 3'-Ende des Antisinn-Stranges) bei ~70%. Befindet sich dagegen der 2nt-Überhang auf dem 3'-Ende des Sinn-Stranges (und das 3'-Ende des Antisinn-Stranges trägt keinen einzelsträngigen Bereich), so liegt die Inhibition der YFP-Genexpression lediglich bei 50%. Analog ist die Inhibition bei höheren Konzentrationen deutlich besser, wenn mindestens das 3'-Ende des Antisinn-Stranges einen 2nt-Überhang trägt.

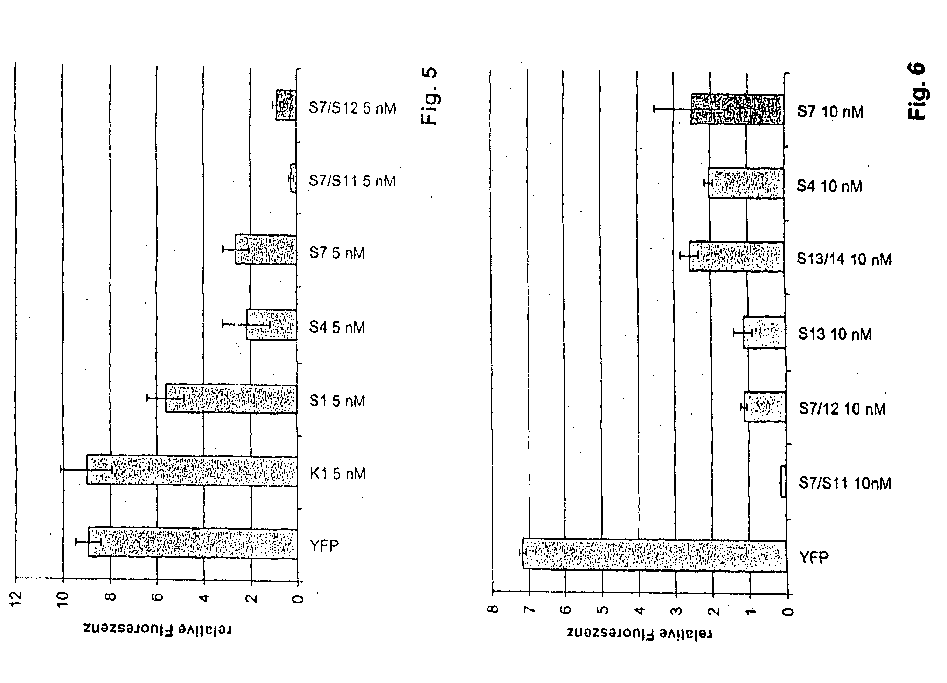

Eine deutlichere Hemmung der YFP-Expression wird erreicht, wenn der basengepaarte Bereich 21 Nukleotid-Paare statt 22 (S1 und S4), 20 (S13 bzw. S13/14) oder 19 (S7) umfasst (Fig. 5, 6 und 7). So beträgt die Inhibition der YFP-Expression durch S1 (22 Basenpaarungen mit glatten Enden) in einer Konzentration von 5 nM ~40%, während die Inhibition durch S7/S12 (21 Basenpaarungen mit glatten Enden), ebenfalls mit 5 nM bei ~92% liegt. Weist die dsRNA mit 21 Basenpaarungen noch einen 2nt-Überhang am Antisinnstrang-3'-Ende (S7/S11) auf, so liegt die Inhibition bei ~ 97% (verglichen mit ~73% Inhibition durch S4 und ~70% Inhibition durch S7).

### III. Untersuchung der Serumstabilität der doppelsträngigen RNA (dsRNA):

Ziel ist es, die in den Zellkulturen gefundene Effektivität der durch dsRNAs vermittelten Hemmung der Genexpression von Zielgenen für den Einsatz *in vivo* zu steigern. Dies wird durch eine verbesserte Stabilität der dsRNAs im Serum und durch eine daraus resultierende verlängerte Verweilzeit des Moleküls im Kreislauf bzw. die damit verbundenen erhöhtewirksame- Konzentration des funktionellen Moleküls erreicht.

### Ausführungsbeispiel:

Die Serumstabilität der die GFP-Expression hemmenden dsRNAs wurde *ex vivo* in murinem und humanem Serum getestet.

### Versuchsprotokoll:

Die Inkubation mit humanem bzw. murinem Serum mit der entsprechenden dsRNA erfolgte bei 37°C. Es wurden je 85 µl Serum mit 15 µl 10*µ*M dsRNA inkubiert. Nach bestimmten Inkubationszeiten (30 min, 1h, 2h, 4h, 8h, 12h, 24h) wurden die Proben bei -80°C eingefroren. Als Kontrolle wurde dsRNA ohne Serum (+85 µl ddH₂O) und dsRNA mit Serum zum Zeitpunkt 0 verwendet.

Für die Isolierung der dsRNA aus dem Inkubationsansatz, die auf Eis erfolgte, wurden jeweils 400 µl 0,1% SDS zu den Ansätzen gegeben und diese einer Phenolextraktion unterzogen: Pro Ansatz wurden 500 µl Phenol : Chloroform : Isoamylalkohol (IAA, 25:24:1, Roti® -Phenol, Roth, Karlsruhe) zugegeben und für 30 sec auf höchster Stufe gevorcext (Vortex Genie-2; Sciencific Industries). Nach 10minütiger Inkubation auf Eis erfolgte die Phasentrennung durch Zentrifugation bei 12.000xg, 4°C, für 10 min (Sigma 3K30, Rotor 12131-H). Die obere wässrige Phase (ca. 200 *µ*l) wurde abgenommen und zuerst einem DNase I- und danach einem Proteinase K - Verdau unterzogen: Zugabe von 20 *µ*l 10xfach DNaseI-Puffer (100 mM Tris, pH 7,5, 25 mM MgCl₂, 1 mM CaCl₂) und 10 U DNase I (D7291, Sigma-Aldrich), 30 min Inkubation bei 37°C, erneute Zugabe von 6 U DNase I und Inkubation für weitere 20 min bei 37°C, Zugabe von 5 *µ*l Proteinase K (20 mg/ml, 04-1075, Peqlab, Deutschland) und 30 min Inkubation bei 37°C. Danach wurde eine Phenolextraktion durchgeführt. Dazu wurde 500 µl Phenol : Chloroform : IAA (25:24:1) zugegeben, 30 sec auf höchster Stufe gevortext, 10 min bei 12.000xg, 4°C, zentrifugiert, der Überstand abgenommen und nacheinander mit 40 µl 3 M Na-Ac (Natriumacetat), pH 5,2, und 1 ml 100% EtOH versetzt, dazwischen gut gemischt und für mindestens 1 h bei -80°C gefällt. Das Präzipitat wurde durch Zentrifugation bei 12.000xg für 30 min und 4°C pelletiert, mit 70% EtOH gewaschen und erneut zentrifugiert (10 min, 12.000xg, 4°C). Das luftgetrocknete Pellet wurde in 30 *µ*l RNA-Gelauftragspuffer (7 M Harnstoff, 1 x TBE (0,09 M Tris-Borat, 0,002 M EDTA (Ethylendiamintetraacetat), 0,02% (w/v) Bromphenolblau, 0,02% (w/v) Xylencyanol) aufgenommen und bis zum Gelauftrag bei -20°C gelagert.

Zur Charakterisierung der dsRNA wurde eine analytische, denaturierende Polyacrylamid-Gelelektrophorese (analytische PAGE) durchgeführt. Die Harnstoffgele wurden kurz vor dem Lauf hergestellt: 7M Harnstoff (21g) wurde in 25 ml 40% wässrige Acrylamid/Bisacrylamid Stammlösung (Rotiphorese-Gel, A515.1, Roth) und 5 ml 10 x TBE (108 g Tris, 55 g Borsäure, 9,3 g EDTA pro L Aqua dest.) unter Rühren gelöst und auf 50 ml mit Aqua desto aufgefüllt. Kurz vor dem Gießen wurden 50 µl TEMED (N,N,N',N'-Tetramethylethylendiamin) und 500 µl 10% APS (Ammoniumperoxidisulfat) zugesetzt. Nach dem Auspolymerisieren wurde das Gel in eine vertikale Elektrophorese-Apparatur (Merck, Darmstadt) eingesetzt und ein Vorlauf für 30 min bei konstant 40 mA Stromstärke durchgeführt. Als Laufpuffer wurde 1 x TBE-Puffer verwendet. Vor dem Auftrag auf das Gel wurden die RNA-Proben für 5 min bei 100°C erhitzt, auf Eis abgekühlt und für 20 sec in einer Tischzentrifuge (Eppendorf, minispin) abzentrifugiert. Es wurden je 15 µl auf das Gel aufgetragen. Der Lauf erfolgte für ca. 2h bei einem konstanten Stromfluß von 40 mA. Nach dem Lauf wurde das Gel 30 min bei RT (Raumtemperatur) mit Stains all-Färbelösung (20 ml Stains all Stammlösung (200 mg Stains all in 200 ml Formamid gelöst) mit 200 ml Aqua dest. und 180 ml Formamid versetzt) gefärbt und die Hintergrundfärbung danach durch Spülen in Aqua dest. für 45 min entfernt. Die Gele wurden mit dem Photodokumentationssystem Image Master VDS von Pharmacia photographiert.

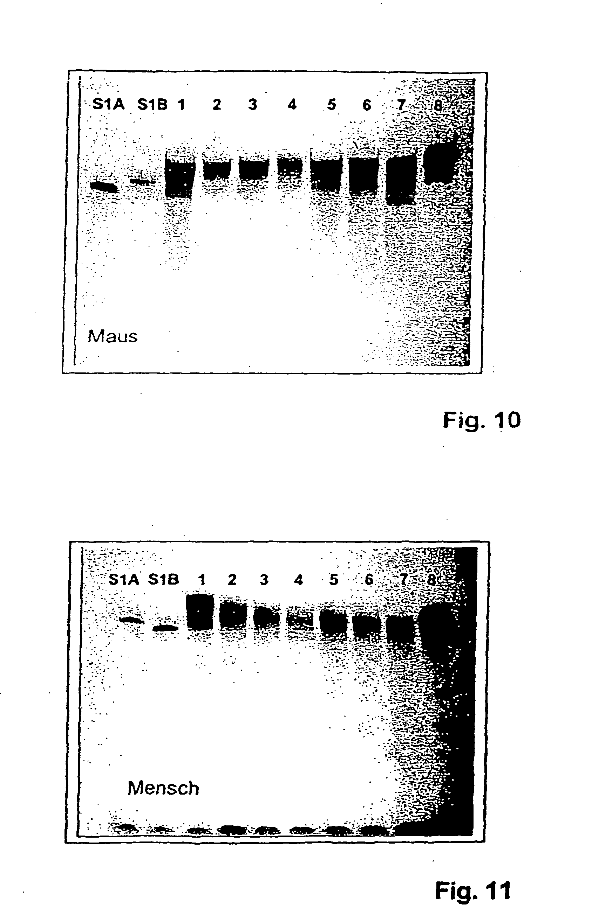

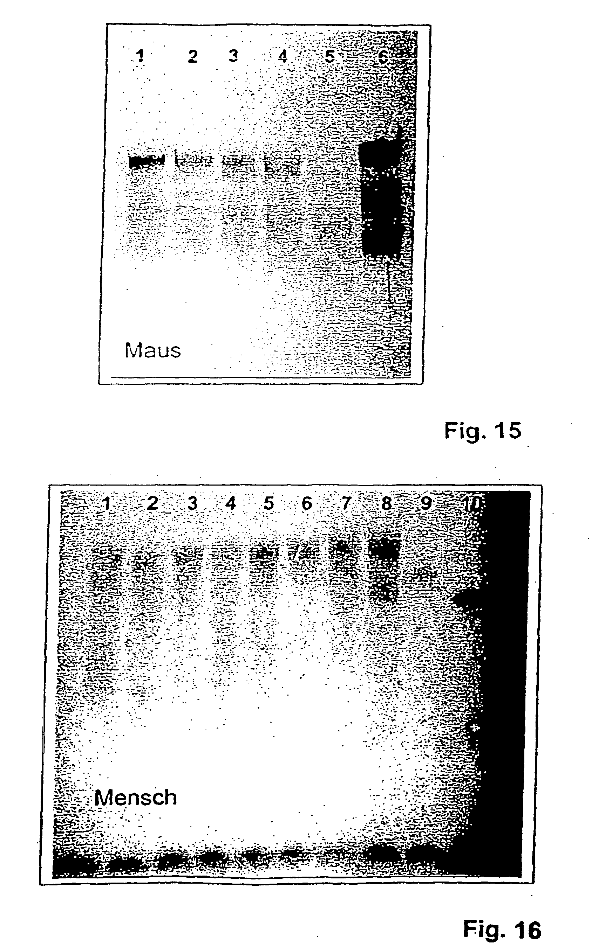

Die Fig. 10 bis 17 zeigen die Serumstabilität der dsRNA nach Inkubation mit humanem bzw. murinem Serum und nachfolgender elektrophoretischer Auftrennung im 20%igem 7M Harnstoffgel.
**Fig. 10: Inkubation von S1 (0-22-0) in Maus-Serum**
1. zum Zeitpunkt 0 (ohne Serum)
2. zum Zeitpunkt 0
3. für 30 Minuten
4. für 1 Stunde
5. für 2 Stunden
6. für 4 Stunden
7. für 12 Stunden
8. 2 µl 100 µM S1 ohne Inkubation S1A) Sinnstrang S1 (10 µl 20 µM S1A) S1B) Antisinnstrang S1 (10 µl 20 µM S13)
**Fig. 11: Inkubation von S1 (0-22-0) in humanem Serum**
1. 2 µl 100 µM S1 unbehandelt (ohne Inkubation)
2. für 30 Minuten
3. für 2 Stunden
4. für 4 Stunden
5. für 6 Stunden
6. für 8 Stunden
7. für 12 Stunden
8. für 24 Stunden S1A) Sinnstrang S1 (10 µl 20 *µ*M S1A) S1B) Antisinnstrang S1 (10 µl 20 µM S1B)

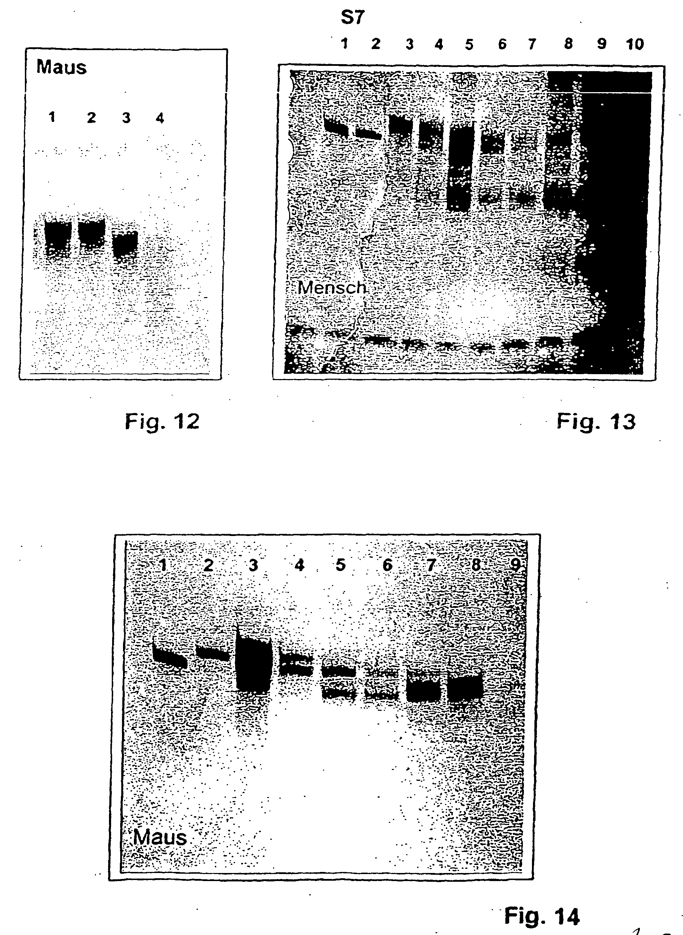

**Fig. 12: Inkubation von S7 (2-19-2) in Maus-Serum**
1. zum Zeitpunkt 0 (ohne Serum)
2. für 30 Minuten
3. für 4 Stunden
4. für 12 Stunden
**Fig. 13: Inkubation von S7 (2-19-2) in humanem Serum**
1. Sinnstrang S7 (10 µl 20 µM S7A)
2. Antisinnstrang S7 (10 µl 20 µM S7B)
3. für 30 Minuten
4. für 1 Stunde
5. für 2 Stunden
6. für 4 Stunden
7. für 6 Stunden
8. für 12 Stunden
9. für 24 Stunden
10. zum Zeitpunkt 0 (ohne Serum)
**Fig. 14: Inkubation von K3 (2-19-2) in Maus-Serum**
1. Sinnstrang K3 (10 µl 20 µM K3A)
2. Antisinnstrang K3 (10 µl 20 µM K3B)
3. zum Zeitpunkt 0 (ohne Serum)
4. zum Zeitpunkt 0 (mit Serum)
5. für 30 Minuten
6. für 1 Stunde
7. für 2 Stunden
8. für 4 Stunden
9. für 12 Stunden
**Fig. 15: Inkubation von PKC1/2 (0-22-2) in Maus-Serum**
1. für 30 Minuten
2. für 1 Stunde
3. für 2 Stunden
4. für 4 Stunden
5. für 12 Stunden
6. 2 *µ*l 100 *µ*M PKC1/2 (unbehandelt)
**Fig. 16: Inkubation von S1A/S4B (0-22-2) in humanem Serum**
1. zum Zeitpunkt 0 (ohne Serum)
2. für 24 Stunden
3. für 12 Stunden
4. für 8 Stunden
5. für 6 Stunden
6. für 4 Stunden
7. für 2 Stunden
8. für 30 Minuten
9. Sinnstrang S1A (10 µl 20 µM S1A)
10. Antisinnstrang S4B (10 µl 20 uM S4B)

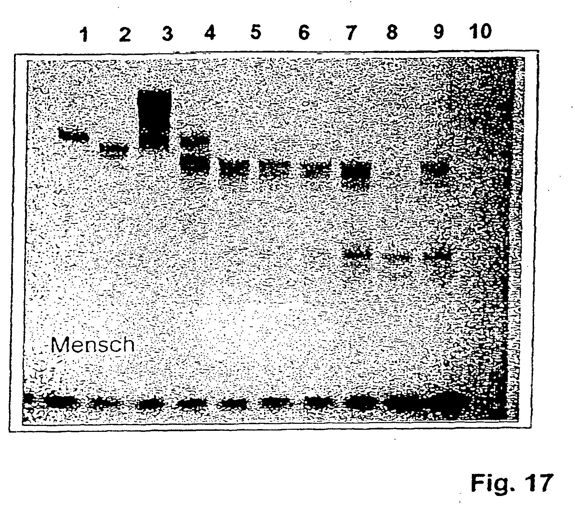

**Fig. 17: Inkubation von K2 (2-22-2) in humanem Serum**
1. Sinnstrang K2 (10 µl 20 µM K2A)
2. Antisinnstrang K2 (10 µl 20 µM K2B)
3. zum Zeitpunkt 0 (ohne Serum)
4. für 30 Minuten
5. für 2 Stunden
6. für 4 Stunden
7. für 6 Stunden
8. für 8 Stunden
9. für 12 Stunden
10. für 24 Stunden

### Ergebnisse:

dsRNAs ohne einzelsträngige Bereiche an den 3'-Enden sind im Serum sowohl von Mensch und Maus wesentlich stabiler als dsRNAs mit einzelsträngigen 2nt-Überhängen an den 3'-Enden (Fig. 10 bis 14 und 17). Nach 12 bzw. 24 Stunden Inkubation von S1 in murinem bzw. humanem Serum ist noch immer eine Bande in der ursprünglichen Größe fast vollständig erhalten. Dagegen nimmt bei dsRNAs mit 2nt-Überhängen an beiden 3'-Enden die Stabilität in humanem als auch im murinen Serum deutlich ab. Bereits nach 4 Stunden Inkubation von S7 (Fig. 12 und 13) oder K3 (Fig. 14) ist keine Bande in der Originalgröße mehr detektierbar.

Um die Stabilität von dsRNA im Serum zu erhöhen, ist es ausreichend, wenn die dsRNA ein glattes Ende besitzt. Im Maus-Serum ist nach 4 Stunden Inkubation (Fig. 15, Bahn 4) die Bande in der Originalgröße kaum abgebaut im Vergleich zu S7 (nach 4 Stunden vollständiger Abbau; Fig. 12, Bahn 3).

Als optimaler Kompromiß hinsichtlich der biologischen Wirksamkeit von dsRNA kann die Verwendung von dsRNA mit einem glattem Ende und einem einzelsträngigem Bereich von 2 Nukleotiden angesehen werden, wobei sich der einzelsträngige Überhang am 3'-Ende des Antisinn-Stranges befinden sollte.

Die hier verwendeten Sequenzen sind aus der nachstehenden Tabelle 2 und den Sequenzprotokollen GQ148-151- und 153-167 ersichtlich.

**Tabelle 2**

| **Name** | **Sequenzprotokoll-Nr.** | **dsRNA-Sequenz** | | |
|---|---|---|---|---|
| **S1** | SQ148 | (A) | 5'-CCACAUGAAGCAGCACGACUUC-3' | **0-22-0** |
| | SQ149 | (B) | 3'-GGUGUACUUCGUCGUGCUGAAG-5' | |
| **S7** | SQ150 | (A) | 5'-CCACAUGAAGCAGCACGACUU-3' | **2-19-2** |
| | SQ151 | (B) | 3'-CUGGUGUACUUCGUCGUGCUG-5' | |
| **K1** | SQ153 | (A) | 5'-ACAGGAUGAGGAUCGUUUCGCA-3' | **0-22-0** |
| | SQ154 | (B) | 3'-UGUCCUACUCCUAGCAAAGCGU-5' | |
| **K3** | SQ155 | (A) | 5'-GAUGAGGAUCGUUUCGCAUGA-3' | **2-19-2** |
| | SQ156 | (B) | 3'-UCCUACUCCUAGCAAAGCGUA-5' | |
| **K2** | SQ157 | (A) | 5'-ACAGGAUGAGGAUCGUUUCGCAUG-3' | **2-22-2** |
| | SQ158 | (B) | 3'-UCUGUCCUACUCCUAGCAAAGCGU-5' | |
| **S1A/** | SQ148 | (A) | 5'-CCACAUGAAGCAGCACGACUUC -3' | **0-22-2** |
| **S4B** | SQ159 | (B) | 3'-CUGGUGUACUUCGUCGUGCUGAAG -5' | |
| **PKC 1/2** | SQ160 | (A) | 5'-CUUCUCCGCCUCACACCGCUGCAA-3' | **2-22-0** |
| | SQ161 | (B) | 3'-GAAGAGGCGGAGUGUGGCGACG-5' | |
| **S7/S12** | SQ150 | (A) | 5'-CCACAUGAAGCAGCACGACUU-3' | **0-21-0** |
| | SQ162 | (B) | 3'-GGUGUACUUCGUCGUGCUGAA-5' | |
| **S7/S11** | SQ150 | (A) | 5'-CCACAUGAAGCAGCACGACUU-3' | **0-21-2** |
| | SQ163 | (B) | 3'-CUGGUGUACUUCGUCGUGCUGAA-5' | |
| **S13** | SQ164 | (A) | 5'-CCACAUGAAGCAGCACGACU-3' | **0-20-2** |
| | SQ165 | (B) | 3'-CUGGUGUACUUCGUCGUGCUGA-5' | |
| **S13/14** | SQ164 | (A) | 5'-CCACAUGAAGCAGCACGACU-3' | **0-20-0** |
| | SQ166 | (B) | 3'-GGUGUACUUCGUCGUGCUGA-5' | |
| **S4** | SQ167 | (A) | 5'-CCACAUGAAGCAGCACGACUUCUU-3' | **2-22-2** |
| | SQ159 | (B) | 3'-CUGGUGUACUUCGUCGUGCUGAAG-5' | |
| **K1A/** | SQ153 | (A) | 5'-ACAGGAUGAGGAUCGUUUCGCA-3' | **0-22-2** |
| **K2B** | SQ158 | (B) | 3'-UCUGUCCUACUCCUAGCAAAGCGU-5' | |
| **K1B/** | SQ154 | (A) | 5'-ACAGGAUGAGGAUCGUUUCGCAUG-3' | **2-22-0** |
| **K2A** | SQ157 | (B) | 3'-UGUCCUACUCCUAGCAAAGCGU-5' | |
| **S1B/** | SQ149 | (A) | 5'-CCACAUGAAGCAGCACGACUUCUU -3' | **2-22-0** |
| **S4A** | SQ167 | (B) | 3'-GGUGUACUUCGUCGUGCUGAAG -5' | |

### IV. In vivo-Studie:

Es wurde "GFP-Labormäusen", die das Grün-fluoreszierende Protein (GFP) in allen Proteinbiosynthese betreibenden Zellen exprimieren, doppelsträngige RNA (dsRNA), die aus der GFP-Sequenz abgeleitet wurde, bzw. unspezifische dsRNA intravenös in die Schwanzvene injiziert. Am Versuchsende wurden die Tiere getötet und die GFP-Expression in Gewebeschnitten und im Plasma analysiert.

### Versuchsprotokoll:

### Synthese der dsRNA:

Mittels eines RNA-Synthesizers (Typ Expedite 8909, Applied Biosystems, Weiterstadt, Deutschland) und herkömmlicher chemischer Verfahren wurden die aus den Sequenzprotokollen ersichtlichen RNA-Einzelstränge und die zu ihnen komplementären Einzelstränge synthetisiert. Anschließend erfolgte die Reinigung der rohen Syntheseprodukte mit Hilfe der HPLC. Als Säulen wurden NucleoPac PA-100, 9x250 mm der Fa. Dionex, verwendet; als Niedersalz-Puffer 20 m.N Tris, 10 mM NaClO₄, pH 6,8, 10% Acetonitril und als Hochsalz-Puffer 20 mM Tris, 400 mM NaClO₄, pH 6,8, 10% Acetonitril. Der Fluß betrug 3 ml/Minute. Die Hybridisierung der Einzelstränge zum Doppelstrang erfolgte durch Erhitzen des stöchiometrischen Gemischs der Einzelstränge in 10 mM Natriumphosphatpuffer, pH 6, 8, 100 mM NaCl, auf 80-90°C und nachfolgendes langsames Abkühlen über 6 Stunden auf Raumtemperatur.

### Versuchstierhaltung und Versuchsdurchführung:

Es wurde der transgene Labormausstamm TgN(GFPU)5Nagy (The Jackson Laboratory, Bar Harbor, ME, USA) verwendet, der GFP (mit einem beta-Aktin-Promotor und einem CMV intermediate early enhancer) in allen bisher untersuchten Zellen exprimiert (Hadjantonakis AK et al., 1993, Mech. Dev. 76: 79-90; Hadjantonakis AK et al., 1998 Nature Genetics 19: 220-222). GFP-transgene Mäuse lassen sich eindeutig anhand der Fluoreszenz (mit einer UV-Handlampe) von den entsprechenden Wildtypen (WT) unterscheiden. Für die Zucht wurde jeweils der entsprechende WT mit einem heterozygotem GFP-Typ verpaart.

Die Versuchsdurchführung erfolgte gemäß den deutschen Tierschutzbestimmungen. Die Tiere wurden unter kontrollierten Umweltbedingungen in Gruppen von 3-5 Tieren in Typ III Makrolon-Käfigen der Fa. Ehret, Emmendingen, bei einer konstanten Temperatur von 22°C und einem Hell-Dunkel-Rhythmus von 12h gehalten. Als Sägemehleinstreu wurde Weichholzgranulat 8/15 der Fa. Altromin, Lage, verwendet. Die Tiere erhielten Leitungswasser und Standardfutter Altromin 1324 pelletiert (Altromin) ad libitum.

Für die Versuchsdurchführung wurden die heterozygoten GFP-Tiere zu je 3 Tieren gruppenweise in Käfigen wie oben beschrieben gehalten. Die Injektionen der dsRNA-Lösung erfolgten intravenös (i.v.) in die Schwanzvene im 12h-Turnus (zwischen 5³⁰ und 7⁰⁰ sowie zwischen 17³⁰ und 19⁰⁰ Uhr) über 5 Tage hinweg. Das Injektionsvolumen betrug 60 µl pro 10 g Körpergewicht und die Dosis betrug 2,5 mg dsRNA bzw. 50 µg pro kg Körpergewicht. Die Einteilung in die Gruppen war wie folgt:
- Gruppe A:: PBS (phosphate buffered saline) je 60 µl pro 10 g Körpergewicht,
- Gruppe B:: 2,5 mg pro kg Körpergewicht einer unspezifischen Kontroll-dsRNA (K1-Kontrolle mit glatten Enden und einem Doppelstrangbereich von 22 Nukleotidpaaren),
- Gruppe C:: 2,5 mg pro kg Körpergewicht einer weiteren unspezifischen Kontroll-dsRNA (K3-Kontrolle mit 2nt-Überhängen an beiden 3'-Enden und einem Doppelstrangbereich von 19 Nukleotidpaaren),
- Gruppe D:: 2,5 mg pro kg Körpergewicht dsRNA (spezifisch gegen GFP gerichtet, im weiteren als S1 be- zeichnet, mit glatten Enden und einem Doppelstrangbereich von 22 Nukleotidpaaren),
- Gruppe E:: 2,5 mg dsRNA pro kg Körpergewicht (spezifisch gegen GFP gerichtet, im Weiteren als S7 bezeichnet, mit 2nt-Überhängen an den 3'-Enden beider Stränge und einem Doppelstrangbereich von 19 Nukleotidpaaren)
- Gruppe F:: 50 µg S1-dsRNA pro kg Körpergewicht (also 1/50 der Dosis der Gruppe D).

Nach der letzten Injektion von insgesamt 10 Injektionen wurden die Tiere nach 14-20h getötet und Organe und Blut wie beschrieben entnommen.

### Organentnahme:

Sofort nach dem Töten der Tiere durch CO₂-Inhalation wurden Blut und verschiedene Organe entnommen (Thymus, Lunge, Herz, Milz, Magen, Darm, Pankreas, Gehirn, Niere und Leber). Die Organe wurden kurz in kaltem, sterilem PBS gespült und mit einem sterilen Skalpell zerteilt. Ein Teil wurde für immunhistochemische Färbungen in Methyl Carnoys (MC, 60% Methanol, 30% Chloroform, 10% Eisessig) für 24h fixiert, ein Teil für Gefrierschnitte und für Proteinisolierungen sofort in flüssigem Stickstoff schockgefroren und bei -80°C gelagert und ein weiterer, kleinerer Teil wurde für RNA-Isolierungen in RNAeasy-Protect (Qiagen) bei -80°C eingefroren. Das Blut wurde sofort nach der Entnahme 30 min auf Eis gehalten, gemixt, 5 min bei 2000 rpm (Mini spin, Eppendorf) zentrifugiert, der Überstand abgenommen und bei -80°C gelagert (hier als Plasma bezeichnet).

### Prozessieren der Biopsien:

Nach 24h Fixierung der Gewebe in MC wurden die Gewebestücke in einer aufsteigenden Alkoholreihe bei RT (Raumtemperatur) dehydriert: je 40 min 70% Methanol, 80% Methanol, 2 x 96% Methanol und 3 x 100% Isopropanol. Danach wurden die Gewebe in 100% Isopropanol auf 60°C im Brutschrank erwärmt, nachfolgend für 1h in einem Isopropanol/Paraffin-Gemisch bei 60°C und 3 x für 2h in Paraffin inkubiert und sodann in Paraffin eingebettet. Für Immunperoxidase-Färbungen wurden mit einem Rotationsmikrotom (Leica) Gewebeschnitte von 3 µm Schnittdikke angefertigt, auf Objektträger (Superfrost, Vogel) aufgezogen und für 30 min bei 60°C im Brutschrank inkubiert.

### Immunperoxidase-Färbung gegen GFP:

Die Schniefe wurden 3 x 5 min in Xylol deparaffiniert, in einer absteigenden Alkoholreihe (3 x 3 min 100% Ethanol, 2 x 2 min 95% Ethanol) rehydriert und danach 20 min in 3% H₂O₂/Methanol zum Blocken endogener Peroxidasen inkubiert Alle Inkubationsschritte wurden im Folgenden in einer feuchten Kammer durchgeführt. Nach 3 x 3 min Waschen mit PBS wurde mit dem 1. Antikörper (goat anti-GFP, sc-5384, Santa Cruz Biotechnology) 1:500 in 1% BSA/PBS über Nacht bei 4°C inkubiert. Die Inkubation mit dem biotinyliertem Sekundärantikörper (donkey anti-goat; Santa Cruz Biotechnology; 1:2000 Verdünnung) erfolgte für 30 min bei RT, danach wurde für 30 min mit Avidin D Peroxidase (1:2000-Verdünnung, Vector Laboratories) inkubiert. Nach jeder Antikörperinkubation wurden die Schnitte 3 x 3 min in PBS gewaschen und Pufferreste mit Zellstoff von den Schnitten entfernt. Alle Antikörper wurden in 1% Rinderserumalbumin (BSA)/PBS verdünnt. Die Färbung mit 3,3'-Diaminobenzidin (DAB) wurde mit dem DAB Substrat Kit (Vector Laboratories) nach Herstellerangaben durchgeführt. Als nukleäre Gegenfärbung wurde Hämatoxylin III nach Gill (Merck) verwendet. Nach der Dehydrierung in einer aufsteigenden Alkoholreihe und 3 x 5 min Xylol wurden die Schnitte mit Entellan (Merck) eingedeckt. Die mikroskopische Auswertung der Färbung erfolgte mit dem IX50 Mikroskop von Olympus, ausgestattet mit einer CCD-Camera (Hamamatsu).

### Proteinisolierung aus Gewebestücken:

Zu den noch gefrorenen Gewebestücken wurden jeweils 800 *µ*l Isolierungspuifer (50 mM HEPES, pH 7,5; 150 mM NaCl; 1 mM EDTA; 2,5 mM EGTA; 10% Glycerol; 0,1% Tween; 1 mM DTT; 10 mM β-Glycerol-Phosohat; 1 mM NaF; 0, 1 mM Na₃VO₄ mit einer Protease-Inhibitor-Tablette "Complete" von Roche) zugegeben und 2 x 30 Sekunden mit einem Ultraturrax (DIAX 900, Dispergierwerkzeug 6G, Heidolph) homogenisiert, dazwischen auf Eis abgekühlt. Nach 30 Minuten Inkubation auf Eis wurde gemischt und für 20 Minuten bei 10.000xg, 4 °C, zentrifugiert (3K30, Sigma). Der Überstand wurde erneut 10 Minuten auf Eis inkubiert, gemischt und 20 Minuten bei 15.000xg, 4°C, zentrifugiert. Mit dem Überstand wurde eine Proteinbestimmung nach Bradford, 1976, modifiziert nach Zor & Selinger, 1996, mit dem Roti-Nanoquant-System von Roth nach den Angaben des Herstellers durchgeführt. Für die Protein-Eichgerade wurde BSA (bovines Serumalbumin) in Konzentrationen von 10 bis 100 µg/ml eingesetzt.

### SDS-Gelelektrophorese:

Die elektrophoretische Auftrennung der Proteine erfolgte in einer Multigel-Long Elektrophoresekammer von Biometra mit einer denaturierenden, diskontinuierlichen 15% SDS-PAGE (Polyacrylamid Gelelektrophorese) nach Lämmli (Nature 277: 680-685, 1970). Dazu wurde zunächst ein Trenngel mit 1,5 mm Dicke gegossen: 7,5 ml Acrylamid/Bisacrylamid (30%, 0,9%), 3,8 ml 1,5 M Tris/HCl, pH 8,4, 150 µl 10% SDS, 3,3 ml Aqua bidest., 250 µl Ammoniumpersulfat (10%), 9 µl TEMED (N,N,N',N'-Tetramethylendiamin) und bis zum Auspolymerisieren mit 0,1% SDS überschichtet. Danach wurde das Sammelgel gegossen: 0,83 µl Acrylamid/Bisacrylamid (30%/0,9%), 630 µl 1 M Tris/HCl, pH 6,8, 3,4 ml Aqua bidest. , 50 µl 10% SDS, 50 µl 10% Ammoniumpersulfat, 5 µl TEMED.

Vor dem Auftrag auf das Gel wurden die Proteine mit einer entsprechenden Menge an 4fach Probenpuffer (200 mM Tris, pH 6,8, 4% SDS, 100 mM DTT (Dithiotreithol), 0,02% Bromphenolblau, 20% Glycerin) versetzt, für 5 min im Heizblock bei 100°C denaturiert, nach dem Abkühlen auf Eis kurz abzentrifugiert und auf das Gel aufgetragen. Pro Bahn wurde die gleichen Plasma- bzw. Proteinmengen eingesetzt (je 3µl Plasma bzw. 25 µg Gesamtprotein). Die Elektrophorese erfolgte wassergekühlt bei RT und konstant 50 V. Als Längenstandard wurde der Proteingelmarker von Bio-Rad (Kaleidoscope Prestained Standard) verwendet.

### Western Blot und Immundetektion:

Der Transfer der Proteine vom SDS-PAGE auf eine PVDF (Polyvenyldifluorid)-Membran (Hybond-P, Amersham) erfolgte im semidry Verfahren nach Kyhse-Anderson (J. Biochem. Biophys. Methods 10: 203-210, 1984) bei RT und einer konstanten Stromstärke von 0, 8 mA/cm² für 1,5 h. Als Transferpuffer wurde ein Tris/Glycin-Puffer eingesetzt (39 mM Glycin, 46 mM Tris, 0,1 % SDS und 20% Methanol). Zum Überprüfen des elektrophoretischen Transfers wurden sowohl die Gele nach dem Blotten als auch die Blotmembranen nach der Immundetektion mit Coomassie gefärbt (0,1% Coomassie G250, 45% Methanol, 10% Eisessig). Zum Absättigen unspezifischer Bindungen wurde die Blotmembran nach dem Transfer in 1% Magermilchpulver/PBS für 1h bei RT inkubiert. Danach wurde je dreimal für 3 min mit 0,1% Tween-20/PBS gewaschen. Alle nachfolgenden Antikörperinkubationen und Waschschritte erfolgten in 0,1% Tween-20/ PBS. Die Inkubation mit dem Primärantikörper (goat anti-GFP, sc-5384, Santa Cruz Biotechnology) in einer Verdünnung von 1:1000 erfolgte für 1h bei RT. Danach wurde 3 x 5 min gewaschen und für 1h bei RT mit dem Sekundärantikörper (donkey anti-goat IgG Hoseradish Peroxidase gelabelt, Santa Cruz Biotechnology)in einer Verdünnung von 1 : 10.000 inkubiert. Die Detektion erfolgte mit dem ECL-System von Amersham nach den Angaben des Herstellers.

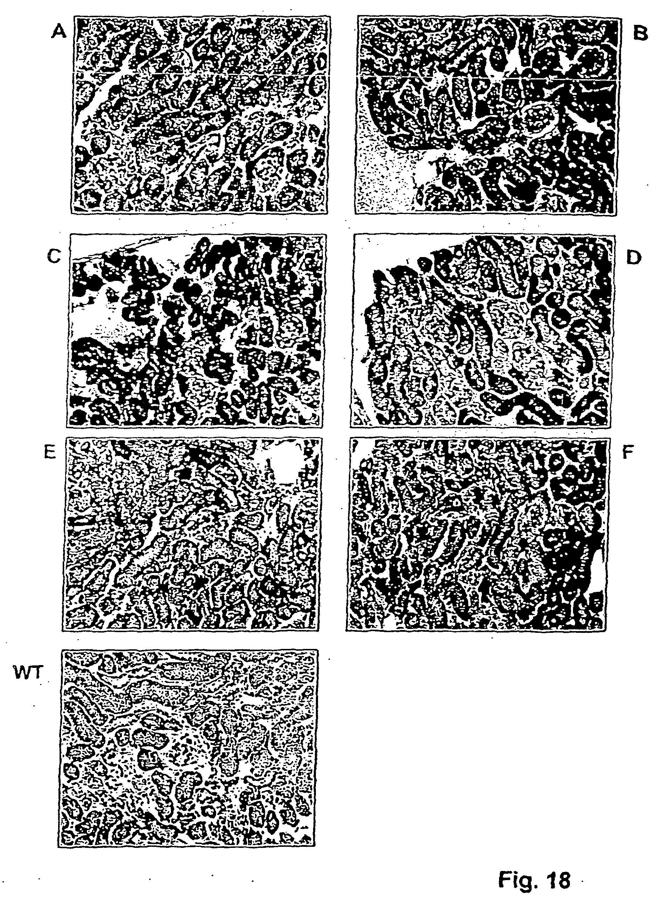

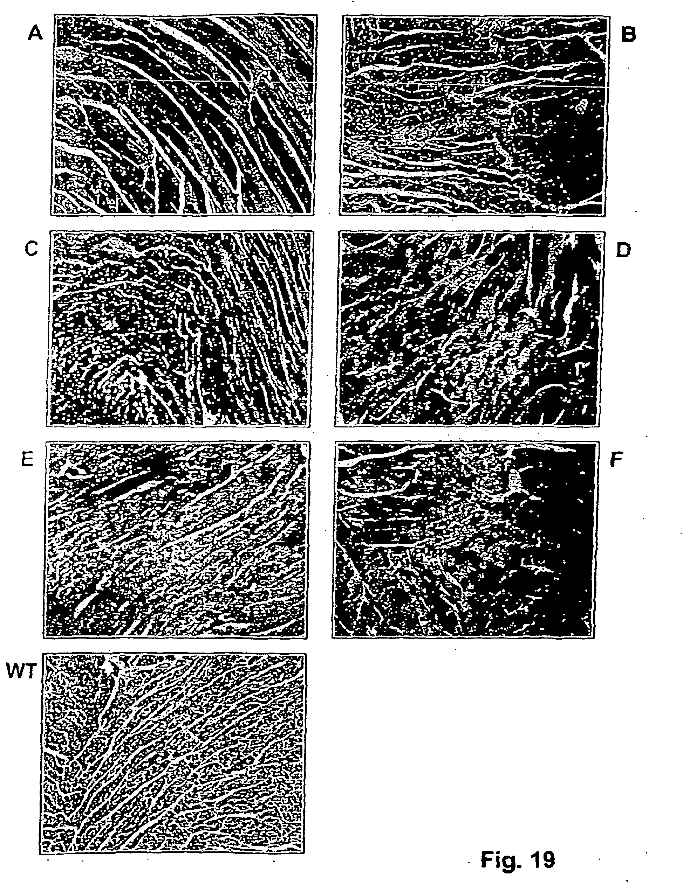

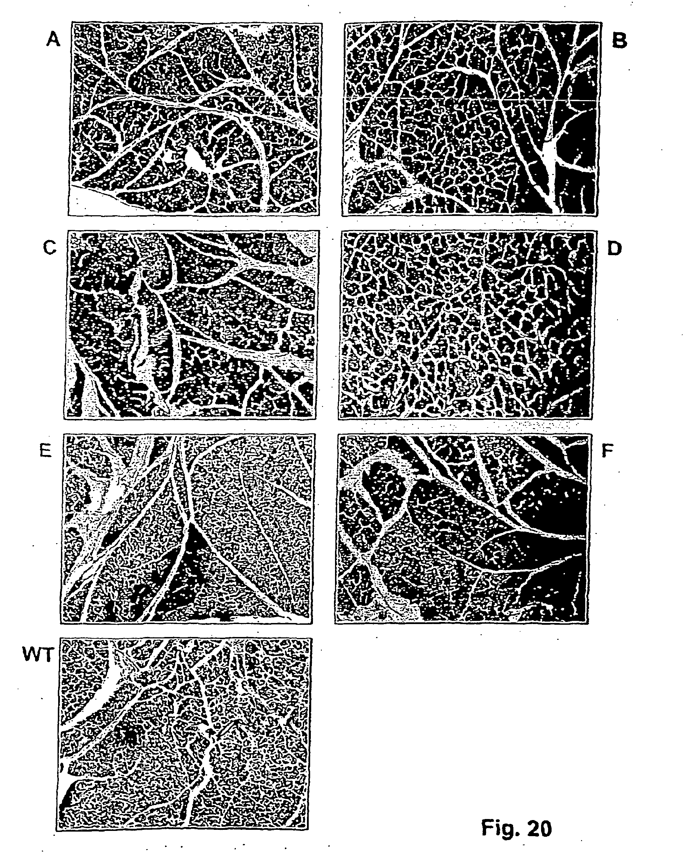

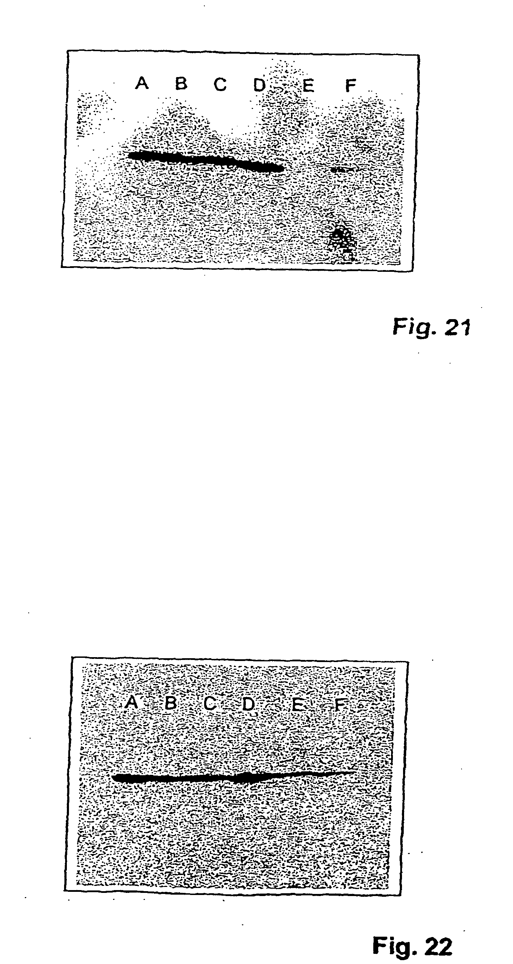

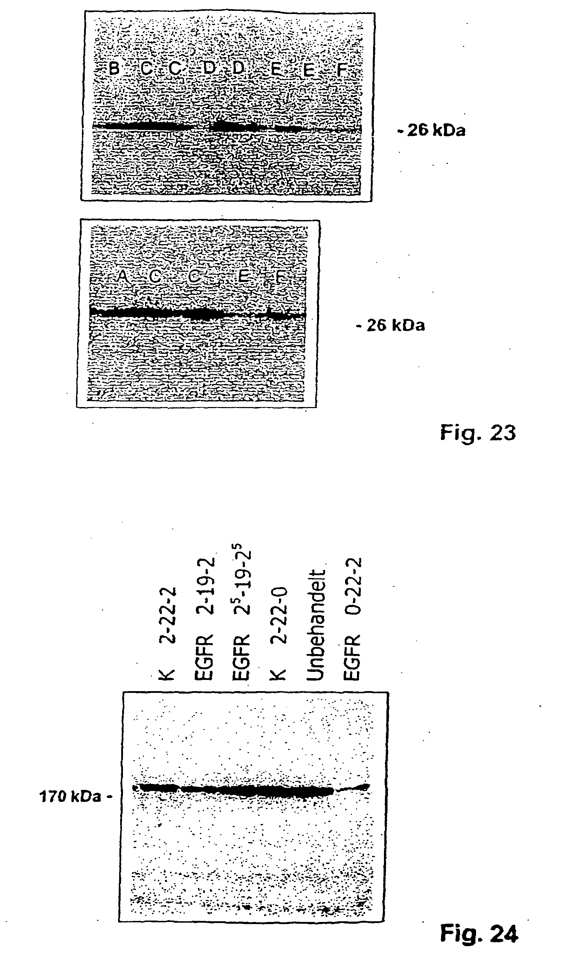

In den Fig. 18 bis 20 ist die Inhibition der GFP-Expression nach intravenöser Injektion von spezifisch gegen GFP gerichteter dsRNA mit Immunperoxidase-Färbungen gegen GFP an 3 µm Paraffinschnitten dargestellt. Im Versuchsverlauf wurde gegen GFP gerichtete dsRNA mit einem doppelsträngigen Bereich von 22 Nukleotid-(nt) paaren ohne Überhänge an den 3'-Enden (D) und die entsprechende unspezifische Kontroll-dsRNA (B) sowie spezifisch gegen GFP gerichtete dsRNA mit einem 19 Nukleotidpaare umfassenden Doppelstrangbereich mit 2nt-Überhängen an den 3'-Enden (E) und die entsprechende unspezifische Kontroll-dsRNA (C) im 12 Stunden-Turnus über 5 Tage hinweg appliziert. (F) erhielt 1/50 der Dosis von Gruppe D. Als weitere Kontrolle wurden Tiere ohne dsRNA-Gabe (A) bzw. WT-Tiere untersucht. Die Fig. 18 zeigt die Inhibition der GFP-Expression in Nierenschnitten, Fig. 19 in Herz- und Fig. 20 in Pankreasgewebe. In den Fig. 21 bis 23 sind Western Blot-Analysen der GFP-Expression in Plasma und Geweben dargestellt. In der Fig. 21 ist die Inhibition der GFP-Expression im Plasma, in Fig. 22 in der Niere und in Fig. 23 in Herz gezeigt. In Fig. 23 sind Gesamtproteinisolate aus verschiedenen Tieren aufgetragen. Es wurden jeweils gleiche Gesamtproteinmengen pro Bahn aufgetragen. In den Tieren, denen unspezifische Kontroll-dsRNA verabreicht wurde (Tiere der Gruppen B und C), ist die GFP-Expression gegenüber Tieren, die keinerlei dsRNA erhielten, nicht reduziert. Tiere, die spezifisch gegen GFP gerichtete dsRNA mit 2nt-Überhängen an den 3'-Enden beider Stränge und einen 19 Nukleotidpaare umfassenden Doppelstrangbereich erhielten, zeigten eine signifikant inhibierte GFP-Expression in den untersuchten Geweben (Herz, Niere, Pankreas und Blut), verglichen mit unbehandelten Tieren (Fig. 18 bis 23). Bei den Tieren der Gruppen D und F, denen spezifisch gegen GFP gerichtete dsRNA mit glatten Enden und einem 22 Nukleotidpaare umfassenden Doppelstrangbereich appliziert wurde, zeigten nur jene Tiere, die die dsRNA in einer Dosis von 50 µg/kg Körpergewicht pro Tag erhielten, eine spezifische Inhibition der GFP-Expression, die allerdings weniger deutlich ausgeprägt war als die der Tiere in Gruppe E.

Die zusammenfassende Auswertung von GFP-Inhibition in den Gewebeschnitten und im Western Blot ergibt, dass die Inhibition der GFP-Expression im Blut und in der Niere am stärksten ist (Fig. 18, 21 und 22).

### V. Hemmung der Genexpression des EGF-Rezeptors mit dsRNA als therapeutischer Ansatz bei Krebsformen mit EGFR-Überexpression oder EGFR-induzierter Proliferation:

Der Epidermal Growth Factor (=EGF))-Rezeptor (=EGFR) gehört zu den Rezeptor-Tyrosinkinasen, transmembranen Proteinen mit einer intrinsischen Tyrosinkinase-Aktivität, die an der Kontrolle einer Reihe von zellulären Prozessen wie Zellwachstum, Zelldifferenzierungen, migratorischen Prozessen oder der Zellvitalität beteiligt sind (Übersicht in: Van der Geer et al. 1994). Die Familie der EGFR besteht aus 4 Mitgliedern, EGFR (ErbB1), HER2 (ErbB2), HER3 (ErbB3) und HER4 (ErbB4) mit einer transmembranen Domäne, einer cysteinreichen extrazellulären Domäne und einer intrazellullären katalytischen Domäne. Die Sequenz des EGFR, einem 170 kDa Protein, ist seit 1984 bekannt (Ullrich et al., 1984).

Aktiviert wird der EGFR durch Peptid-Wachstumsfaktoren wie EGF, TGFα (transforming growth factor), Amphiregulin, Betacellulin, HB-EGF (heparin-binding EGF-like growth factor) und Neureguline. Ligandenbindung induziert die Bildung von Homo- oder Heterodimeren mit nachfolgender Autophosphorylierung zytoplasmatischer Tyrosine (Ullrich & Schlessinger, 1990; Alroy & Yarden, 1997). Die phosphorylierten Aminosäuren bilden die Bindungsstellen für eine Vielzahl von Proteinen, die an den proximalen Schritten der Signalweiterleitung in einem komplexen Netzwerk beteiligt sind. Der EGFR isc an den verschiedensten Tumorerkrankungen beteiligt und damit ein geeignetes Target für therapeutische Ansätze (Huang & Harari, 1999). Die Mechanismen, die zu einer aberranten EGFR-Aktivierung führen, können auf Überexpression, Amplifikation, konstitutiver Aktivierung mutanter Rezeptor-Formen oder autokrinen Loops beruhen (Voldborg et al., 1997). Eine Überexpression des EGFR wurde für eine Reihe von Tumoren beschrieben, wie z.B. Brustkrebs (Walker & Dearing, 1999), Nicht-Klein-Lungenkarzinom (Fontanini et al., 1998), Pankreaskarzinomen, Kolonkarzinom (Salomon et al., 1995) und Glioblastomen (Rieske et al., 1998). Insbesondere für maligne Glioblastome sind bisher keine effizienten und spezifischen Therapeutika verfügbar.

### Ausführungsbeispiel:

Zum Nachweis der Wirksamkeit der dsRNA bei der spezifischen Inhibition der EGFR-Genexpression wurden U-87 MG-Zellen (humane Glioblastomzellen), ECCAC (European collection of animal cell culture) Nr. 89081402, verwendet, die mit spezifisch gegen den EGF-Rezeptor (Sequenzprotokoll SQ 51) gerichteten dsRNA transfiziert wurden. Nach ca. 72 Stunden Inkubation wurden die Zellen geerntet, Protein isoliert und im Western Blot verfahren die EGFR-Expression untersucht.

### Versuchsprotokoll:

### dsRNA-Synthese:

Mittels eines RNA-Synthesizers (Typ Expedite 8909, Applied Biosystems, Weiterstadt, Deutschland) und herkömmlicher chemischer Verfahren wurden die aus den Sequenzprotokollen ersichtlichen RNA-Einzelstränge und die zu ihnen komplementären Einzelstränge synthetisiert. Anschließend erfolgte die Reinigung der rohen Syntheseprodukte mit Hilfe der HPLC. Verwendet wurde die Säule NucleoPac PA-100, 9x250 mm, der Fa. Dionex; als Niedersalz-Puffer 20 mM Tris, 10 mM NaClO₄, pH 6,8, 10% Acetonitril 1 und als Hochsalz-Puffer 20 mM Tris, 400 mM NaClO₄, pH 6,8, 10% Acetonitril. Der Fluß betrug 3 ml/Minute. Die Hybridisierung der Einzelstränge zum Doppelstrang erfolgte durch Erhitzen des stöchiometrischen Gemischs der Einzelstränge in 10 mM Natriumpnosphatpuffer, pH 6,8, 100 mM NaCl, auf 80-90°C und nachfolgendes langsames Abkühlen über 6 Stunden auf Raumtemperatur.

### Aussaat der Zellen:

Alle Zellkulturarbeiten wurden unter sterilen Bedingungen in einer entsprechenden werkbank (HS18, Hera Safe, Kendro, Heraeus) durchgeführt. Die Kultivierung der U-87 MG-Zellen erfolgte im Brutschrank (CO₂-Inkubator T20, Hera cell, Kendro, Heraeus) bei 37°C, 5% CO₂ und gesättigter Luftfeuchtigkeit in DMEM (Dulbecco's modified eagle medium, Biochrom) mit 10% FCS (fetal calf serum, Biochrom), 2 mM L-Glutamin (Biochrom), 1 mM Natrium-Pyruvat (Biochrom), 1xNEAA (Nonessetial Aminoacids, Biochrom) und Penicillin/Streptomycin (100 IE/100 µg/ml, Biochrom). Um die Zellen in der exponentiellen Wachstumsphase zu halten, wurden die Zellen alle 3 Tage passagiert. 24 Stunden vor der Applikation der dsRNA mittels Transfektion wurden die Zellen trypsiniert (10x Trypsin/EDTA, Biochrom, Deutschland) und mit einer Zelldichte von 5 x 10⁵ Zellen/Vertiefung in einer 6-Well-Platte (6-Well Schalen, Labor Schubert & Weiss GmbH) in 1,5 ml Wachstumsmedium ausgesät.

### Applikation der dsRNA in kultivierte U-87 MG-Zellen:

Die Applikation der dsRNA erfolgte mittels Transfektion mit dem OLIGOFECTAMINE^{™} Reagent (Life Technologies) gemäß den Angaben des Herstellers. Das Gesamt-Transfektionsvolumen betrug 1 ml. Zuerst wurde die dsRNA in serumfreiem Medium verdünnt: Dazu wurden pro Well 0,5 µl einer 20 µM Stammlösung spezifisch gegen EGFR gerichteten dsRNA und 9,5 µl einer 20 µM Stammlösung unspezifischer dsRNA (K1A/K2B) mit 175 µl serumfreiem Medium verdünnt (200 nM dsRNA im Transfektionsansatz bzw. 10 nM spezifische EGFR-dsRNA). Das OLIGOFECTAMINE^{™} Reagent wurde ebenfalls in serumfreien Medium verdünnt: pro Well 3 µl mit 12 µl Medium und danach 10 min bei Raumtemperatur inkubiert. Danach wurde das verdünnte OLIGOFECTAMINE^{™} Reagent zu den in Medium verdünnten dsRNAs gegeben, gemischt und für weitere 20 min bei RT inkubiert. Während der Inkubation wurde ein Mediumwechsel durchgeführt. Die Zellen wurden dazu 1 x mit 1 ml serumfreiem Medium gewaschen und mit 800 µl serumfreiem Medium bis zur Zugabe von dsRNA/OLlGOFECTAMINE^{™} Reagent weiter im Brutschrank inkubiert. Nach der Zugabe von 200 µl dsRNA/OLIGOFECTAMINE^{™} Reagent pro Well wurden die Zellen bis zur Proteinisolierung weiter im Brutschrank inkubiert.

### Proteinisolierung:

Ca. 72 Stunden nach der Transfektion wurden die Zellen geerntet und eine Proteinisolierung durchgeführt. Dazu wurde das Medium abgenommen und das Zellmonolayer 1 x mit PBS gewaschen. Nach Zugabe von 200 µl Proteinisolierungspuffer (1x Protease-Inhibitor "Complete", Roche, 50 mM HEPES, pH 7,5, 150 mM NaCl, 1 mM EDTA, 2,5 mM EGTA, 10% Glyzerin, 0,1% Tween-20, 1 mM DTT, 10 mM β-Glycerinphosphat, 1 mM NaF, 0,1 mM Na₃VO₄) wurden die Zellen mit Hilfe eines Zellschabers abgelöst, 10 min auf Eis inkubiert, in ein Eppendorf-Reaktionsgefäß überführt und bei -80°C für mindestens 30 min gelagert. Nach dem Auftauen wurde das Lysat für 10 sec mit einem Dispergierer (DIAX 900, Dispergierwerkzeug 6G, Heidolph-Instruments GmbH & Co KG, Schwabach) auf Stufe 3 homogenisiert, für 10 min auf Eis inkubiert und für 15 min bei 14.000xg, 4°C (3K30, Sigma) zentrifugiert. Mit dem Überstand wurde eine Proteinbestimmung nach Bradford mit dem Roti®-Nanoquant-System von Roth (Roth GmbH & Co., Karlsruhe) nach angeben des Herstellers durchgeführt. Dazu wurden je 200 µl Proteinlösung in geeigneter Verdünnung mit 800 µl 1x Arbeitslösung gemischt und die Extinktion in Halbmikroküvetten bei 450 und 590 nm gegen Aqua dest. in einem Beckman-Spektralphotometer (DU 250) gemessen. Für die Eichgerade wurden entsprechende BSA-Verdünnungen verwendet (perliertes BSA, Sigma).

### SDS-Gelelektrophorese:

Die elektrophoretische Auftrennung der Proteine erfolgte in einer Multigel-Long Elektrophoresekammer von Biometra mit einer denaturierenden, diskontinuierlichen 7,5% SDS-PAGE (Polyacrylamid Gelelektrophorese) nach Lämmli (Nature 277: 680-685, 19970). Dazu wurde zunächst ein Trenngel mit 1,5 mm Dikke gegossen: 3,75 ml Acrylamid/Bisaacrylamid (30%, 0,9%), 3,8 ml 1 M Tris/HCl, pH 8,4, 150 µl 10% SDS, 7,15 ml Aqua bidest., 150 µl Ammoniumpersulfat (10%), 9 µl TEMED (N, N, N', N'-Tetramethylendiamin) und bis zum Auspolymerisieren mit 0,1% SDS überschichtet. Danach wurde das Sammelgel gegossen: 0,83 ml Acrylamid/Bisacrylamid (30%/0,9%), 630 µl 1 M Tris/HCl, pH 6,8, 3,4 ml Aqua bidest., 50 µl 10% SDS, 50 µl 10% Ammoniumpersulfat, 5 µl TEMED.

Für den Auftrag auf das Gel wurden die Proteinproben 1:3 mit 4x Probenpuffer (200 mM Tris, pH 6,8, 4% SDS, 100 mM DTT (Dithiotreithol), 0,02% Bromphenolblau, 20% Glycerin) versetzt, für 5 min bei 100°C denaturiert, nach dem Abkühlen auf Eis kurz abzentrifugiert und auf das Gel aufgetragen. Pro Bahn wurden 35 µg Gesamtprotein aufgetragen. Der Gelauf erfolgte wassergekühlt bei RT und konstant 50 V. Als Längenstandard wurde der Kaleidoskop-Proteingelmarker (BioRad)) verwendet.

### Western Blot und Immundetektion:

Der Transfer der Proteine vom SDS-PAGE auf eine PVDF (Polyvenyldifluorid) -Membran (Hybond-P, Amersham) erfolgte im semidry Verfahren nach Kyhse-Anderson (J. Biochem. Biophys. Methods 10: 203-210, 1984) bei RT und einer konstanten Stromstärke von 0, 5 mA/cm² für 1,5 h. Als Transferpuffer wurden verwendet: Kathodenpuffer (30 mM Tris, 40 mM Glycin, 10% Methanol, 0,01% SDS; pH 9,4), Anodenpuffer I (300 mM Tris, pH 10,4, 10% Methanol) und Anodenpuffer II (30 mM Tris, pH 10,4, 10% Methanol). Vor dem Zusammensetzen des Blotstapels mit 3MM Whatman-Papier (Schleicher & Schüll) wurden das Gel in Kathodenpuffer und die PVDF-Membran (zuvor 30 sec in 100% Methanol) in Anodenpuffer II inkubiert (5 min): 2 Lagen 3MM-Papier (Anodenpuffer I), 1 Lage 3MM-Papier (Anodenpuffer II), PVDF-Membran, Gel, 3 Lagen 3MM-Papier (Kathodenpuffer). Zum Überprüfen des elektrophoretischen Transfers wurden sowohl die Gele nach dem Blotten als auch die Blotmembranen nach der Immundetektion mit Coomassie gefärbt (0,1% Coomassie G250, 45% Methanol, 10% Eisessig).

Die Blotmembran wurde nach dem Transfer in 1% Magermilchpulver/PBS/0,1% Tween-20 für 1h bei RT inkubiert. Danach wurde dreimal für 3 min mit 0,1% Tween-20/PBS gewaschen. Alle nachfolgenden Antiköperinkubationen und Waschschritte erfolgten in 0,1% Tween-20/ PBS. Die Inkubation mit dem Primärantikörper (human EGFR extracellular domain, specific goat IgG, Cat-Nr. AF231, R&D Systems) erfolgte auf einem Schüttler für 2h bei RT in einer Konzentration von 1,5 *µ*g/ml. Danach wurde 3 x 5 min gewaschen und für 1h bei RT mit dem Sekundärantikörper (donkey anti-goat IgG Horseradish Peroxidase gelabelt, Santa Cruz Biotechnology) inkubiert (1:10.000 verdünnt). Nach dem Waschen (3 x 3min in PBS/0,1% Tween-20) erfolgte sofort die Detektion mittels ECL-Reaktion (enhanced chemiluminescence): Zu 18 ml Aqua dest. wurden 200 µl Lösung A (250 mM Luminol, Roth, gelöst in DMSO), 89 µl Lösung B (90 mM p-Coumarsäure, Sigma, gelöst in DMSO) und 2 ml 30% H₂O₂-Lösung pipettiert. Je nach Membrangröße wurden 4-6 ml direkt auf die Membran pipettiert, 1 min bei RT inkubiert und danach sofort ein Röntgenfilm (Biomax MS, Kodak) aufgelegt.

Die hier verwendeten Sequenzen sind in der nachstehenden Tabelle 3 sowie in den Sequenzprotokollen SQ153, 157, 158, 168-173 wiedergegeben.

**Tabelle 3**

| | | | | |
|---|---|---|---|---|
| **ES-7** | SQ168 | (A) | 5'-AACACCGCAGCAUGUCAAGAU-3' | **2-19-2** |
| | SQ169 | (B) | 3'-UUUUGUGGCGUCGUACAGUUC-5' | |
| **ES-8** | SQ170 | (A) | 5'-AAGUUAAAAUUCCCGUCGCUAU-3' | **2**^{**5**}**-19-2**^{**5**} |
| | SQ171 | (B) | 3'-CAAUUUUAAGGGCAGCGAUAGU-5' | |
| **ES2A/ ES5B** | SQ172 | (A) | 5'-AGUGUGAUCCAAGCUGUCCCAA-3' | **0-22-2** |
| | SQ173 | (B) | 3'-UUUCACACUAGGUUCGACAGGGUU-5' | |
| **K2** | SQ157 | (A) | 5'-ACAGGAUGAGGAUCGUUUCGCAUG-3' | **2-22-2** |
| | SQ158 | (B) | 3'-UCUGUCCUACUCCUAGCAAAGCGU -5' | |
| **K1A/ K2B** | SQ153 | (A) | 5'-ACAGGAUGAGGAUCGUUUCGCA -3' | **0-22-2** |
| | SQ158 | (B) | 3'-UCUGUCCUACUCCUAGCAAAGCGU -5' | |

### Inhibition der EGFR-Expression in U-87 MG Glioblastom-Zellen:

24 Stunden nach dem Aussäen der Zellen wurden diese mit 10 nM dsRNA wie angegeben (Oligofectamine) transfiziert. Nach 72 Stunden wurden die Zellen geerntet und Protein isoliert. Die Auftrennung der Proteine erfolgte im 7,5% SDS-PAGE. Pro Bahn wurden je 35 µg Gesamtprotein aufgetragen. In Fig. 24 ist die entsprechende Western Blot-Analyse gezeigt, aus der hervorgeht, dass sich mit der spezifisch gegen das EGFR-Gen gerichteten dsRNA mit einem 2nt-Überhang am 3'-Ende des AntisinnStrangs die EGFR-Expression nach Transfektion in U-87 MG-Zellen signifikant gegenüber den entsprechenden Kontrollen inhibieren lässt. Diese Inhibition der Expression eines endogenen Gens durch spezifische dsRNA bestätigt somit die in Ausführungsbeispiel II angeführten Ergebnisse zur Inhibition der Expression eines nach transienter Transfektion in die Zelle eingebrachten artifiziellen Gens. Die durch ES-7 bzw. ES-8 vermittelte Inhibition der EGFR-Expression ist deutlich geringer. Die in Fig. 24 verwendeten dsRNAs sind Tabelle 3 zu entnehmen.

### VI. Hemmung der Expression des Multidrug resistance Gens 1(MDR1):

### Versuchsprotokoll:

Der *in vitro* Nachweis für das Blockieren der MDR1-Expression wurde in der Kolonkarzinom-Zellinie LS174T (ATCC - *American Type Culture Collection*; Tom et al., 1976) durchgeführt. Von dieser Zellinie ist bekannt, daß die Expression von MDR1 durch Zugabe von Rifampicin zum Kulturmedium induzierbar ist (Geick et al., 2001). Transfektionen wurden mit verschiedenen käuflichen Transfektions-Kits (Lipofectamine, Oligofectamine, beide Invitrogen; TransMessenger, Qiagen) durchgeführt, wobei der TransMessenger Transfektions-Kit sich als für diese Zellinie am geeignetsten herausstellte.

Zur Durchführung der RNA-Interferenz-Experimente wurden 4 kurze doppelsträngige Ribonukleinsäuren R1-R4 eingesetzt, deren Sequenzen in Tabelle 4) gezeigt sind. Die Ribonukleinsäuren sind mit Abschnitten der kodierenden Sequenz von MDR1 (Sequenzprotokoll SQ 30)homolog. Die Sequenzen R1 - R3 bestehen aus einem 22-mer Sinn- und einem 24-mer Antisinn-Strang, wobei der entstehende Doppelscrang am 3'-Ende des Antisinn-Stranges einen 2-Nukleotid-Überhang aufweist (0-22-2). Die Sequenz R4 entspricht R1, jedoch besteht sie aus einem 19-mer Doppelscrang mit je 2-Nukleotid-Überhängen an jedem 3'-Ende (2-19-2).

**Tabelle 4**

| **Name** | **Sequenzprotokoll-Nr.** | **Sequenz** | **Position in Datenbank-# AF016535** |
|---|---|---|---|
| Seq R1 | SQ141 | 5'- CCA UCU CGA AAA GAA GUU AAG A-3' | 1320-1342 |
| | SQ142 | 3'-UG GGU AGA GCU UUU CUU CAA UUC U-5' | 1335-1318 |
| Seq R2 | SQ143 | 5'- UAU AGG UUC CAG GCU UGC UGU A-3' | 2599-2621 |
| | SQ152 | 3'-CG AUA UCC AAG GUC CGA ACG ACA U-5' | 2621-2597 |
| Seq R3 | SQ144 | 5'- CCA GAG AAG GCC GCA CCU GCA U-3' | 3778-3799 |
| | SQ145 | 3'-UC GGU CUC UUC CGG CGU GGA CGU A-5' | 3799-3776 |
| Seq R4 | SQ146 | 5'- CCA UCU CGA AAA GAA GUU AAG-3' | 1320-1341 |
| | SQ147 | 3'-UG GGU AGA GCU UUU CUU CAA U -5' | 1339-1318 |

| | | | **Position in Datenbank-# AF402779** |
|---|---|---|---|
| K1A/ K2B | SQ153 | 5'- ACA GGA UGA GGA UCG UUU CGC A-3' | 2829-2808 |
| | SQ158 | 3'-UC UGU CCU ACU CCU AGC AAA GCG U-5' | 2808-2831 |

Die in Tabelle 4 gezeigten Sequenzen sind nochmals im Sequenzprotokoll als Sequenzen SQ141-147, 152, 153, 158 wiedergegeben. Die dsRNAs wurden in einer Konzentration von 175 nM jeweils als doppelte Ansätze in die Zellen transfiziert, welche am Tag zuvor in 12-Loch-Platten à 3,8 x 10⁵ Zellen/Vertiefung ausgesät wurden. Dazu wurden pro Transfektionsansatz 93,3 µl EC-R-Puffer (TransMessenger Kit, Qiagen, Hilden) mit 3,2 µl Enhancer-R vermengt und danach 3,5 µl der jeweiligen 20 µM dsRNA zugegeben, gut gemischt und 5 Minuten bei Raumtemperatur inkubiert. Nach Zugabe von jeweils 6 µl TransMessenger Transfection Reagent wurden die Transfektionsansätze 10 Sekunden kräftig gemischt und 10 Minuten bei Raumtemperatur inkubiert. In der Zwischenzeit wurde das Medium von den Zellen abgesaugt, einmal mit PBS (Phosphate buffered saline) gewaschen und 200 µl frisches Medium ohne FCS pro Vertiefung auf die Zellen gegeben. Nach Ablauf der 10-minütigen Inkubationszeit wurden je 100 µl FCS-freies Medium zu den Transfektionsansätzen pipettiert, gemischt, und die Mischung tropfenweise zu den Zellen pipettiert (die dsRNA-Konzentration von 175 µM bzieht sich auf 400 µl Medium Gesamtvolumen). Die dsRNA/Trans-Messenger-Komplexe wurden 4 Stunden bei 37°C mit den Zellen in FCS-freiem Medium inkubiert. Danach wurde ein Mediumwechsel durchgeführt, wobei das frische Medium 10 µM Rifampicin und 10% FCS enthielt. Als Kontrolle wurde eine unspezifische dsRNA-Sequenz, die keinerlei Homologie mit der MDR1-Gensequenz aufweist,eingesetzt (K) und eine MOCK-Transfektion durchgeführt, die alle Reagenzien außer dsRNA enthielt.

Die Zellen wurden nach 24, 48 und 72 Stunden geerntet und die Gesamt-RNA mit dem RNeasy-Mini-Kit von Qiagen extrahiert. 10 µg Gesamt-RNA jeder Probe wurden auf einem 1%igen Agarose-Formaldehyd-Gel elektrophoretisch aufgetrennt, auf eine Nylon-Membran geblottet und mit 5'-α³²P-dCTP random-markierten, spezifischen Sonden zuerst gegen MDR1 und nach dem Strippen des Blots gegen GAPDH als interne Kontrolle hybridisiert und auf Röntgenfilmen exponiert.

Die Röntgenfilme wurden digitalisiert (Image Master, VDS Pharmacia) und mit der Image-Quant-Software quantifiziert. Dabei wurde ein Abgleich der MDR1-spezifischen Banden mit den entsprechenden GAPDH-Banden durchgeführt.

### Ergebnisse:

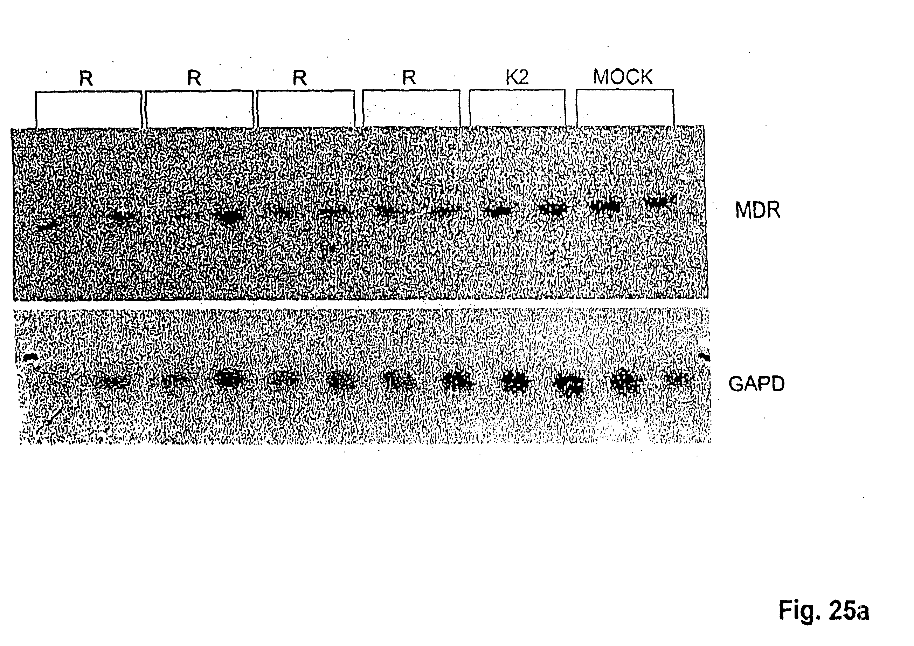

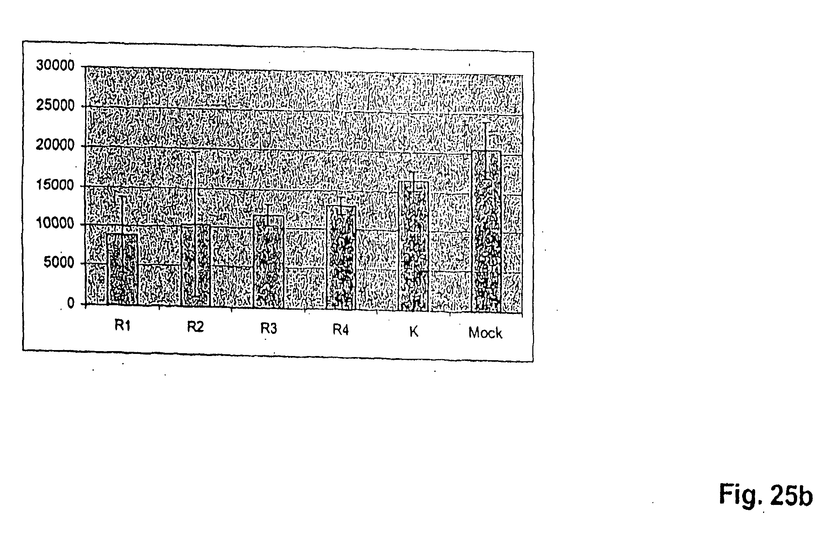

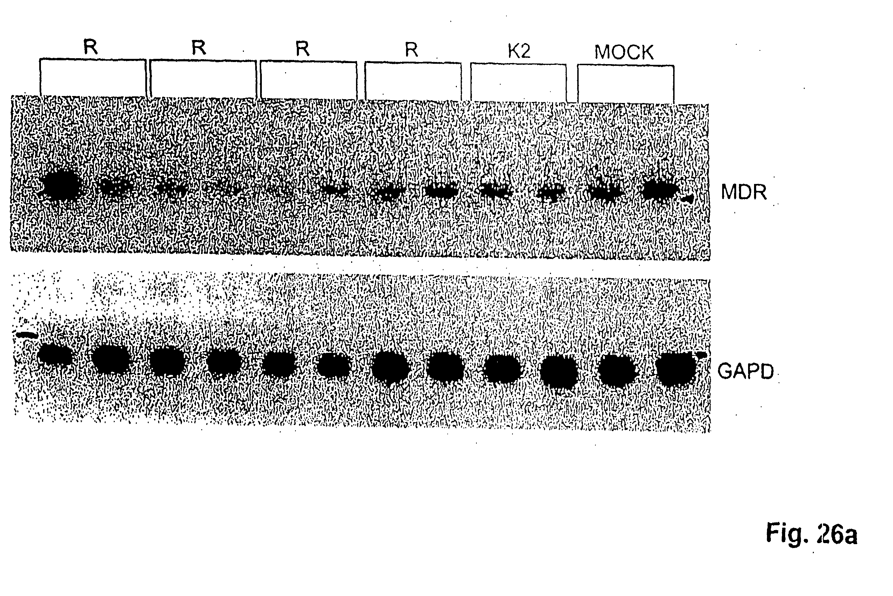

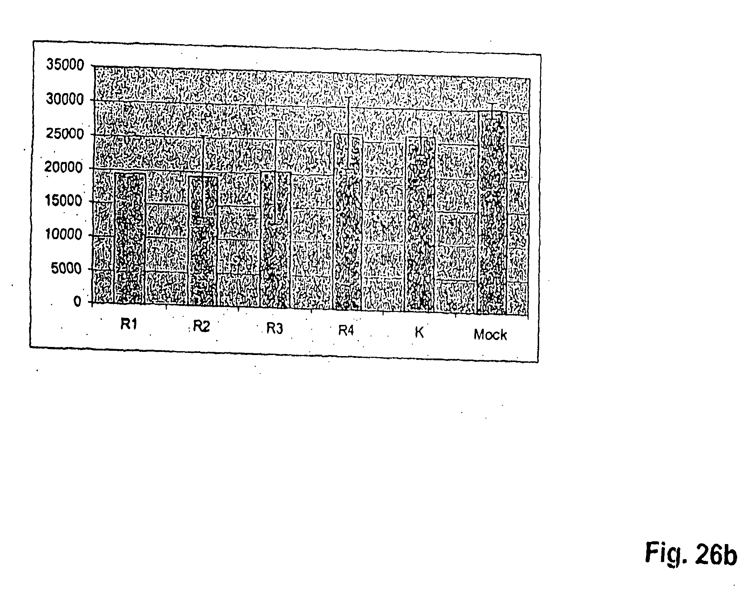

Die Fig. 25 und 26 zeigen Northern-Blots (Fig. 25a, 26a) mit quantitativer Auswertung der MDR1-spezifischen Banden nach Abgleich mit den entsprechenden GAPDH-Werten (Fig. 25b, 26b). Es konnte eine Reduktion der MDR1-mRNA um bis zu 55 % im Vergleich zur MOCK-Transfektion und um bis zu 45 % im Vergleich zur unspezifischen Kontroll-Transfektion beobachtet werden. Nach 48 h ist eine signifikante Reduktion des MDR1-mRNA-Niveaus mit den als R1, R2, R3 (Tabelle 4) bezeichneten dsRNA-Konstrukten erreicht worden. Mit den R4-dsRNA-Konstrukten wurde nach 48 h keine signifikante Reduktion gegenüber den Kontrollen beobachtet (Fig. 26a und 26b).
Nach 74 h war eine deutlich stärkere Reduktion des MDR1-mRNA-Levels mit R1, R2 und R3 gegenüber den Kontrollen im Vergleich zu den 48 h-Werten zu beobachten (Fig. 25a und 25b).

Mit R4 konnte konnte zu diesem Zeitpunkt ebenfalls eine siginifikante Verringerung des MDR1-mRNA-Niveaus erzielt werden. Somit reduzieren die Konstrukte mit einem 2nt-Überhang am 3'-Ende des Antisinnstrangs und einem doppelsträngigen Bereich aus 22 Nukleotidpaaren, relativ unabhängig von dem jeweiligen zum MDR1-Gen homologen Sequenzbereich (nach 48 h; Fig. 26b) das MDR1-mRNA-Level effizienter als die Konstrukte mit mit 2nt-Überhängen an den 3'-Enden beider Stränge (Antisinn- und Sinnstrang) und einem Doppelstrangbereich von 19 Nukleotidpaaren. Die Ergebnisse bekräftigen damit die in Ausführungsbeispiel IV beschriebene Inhibition der EGFR-Genexpression durch spezifische dsRNAs nach Transfektion in U-87 MG-Zellen.

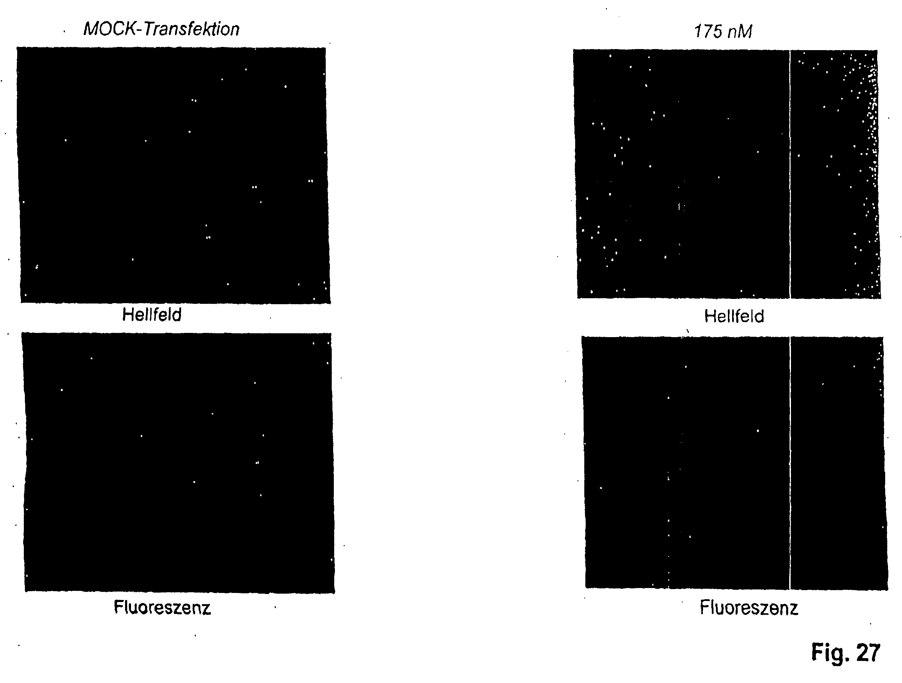

Die Transfektionserfizienz wurde in einem getrennten Experiment mit Hilfe eines Texas-Red-markierten DNA-Oligonukleotids (TexRed-A(GATC)₅T; ebenfalls 175 nM transfiziert) ermittelt (Fig. 27a, 27b; 400fache Vergrößerung, 48h nach Transfektion). Sie betrug etwa 50% auf der Grundlage der rot fluoreszierenden Zellen im Vergleich zur Gesamtzellzahl. Berücksichtigt man die Transfektionsrate der Zellen von etwa 50%, so legt die beobachtete Verringerung des MDR1-mRNA-Niveaus um ca. 45-55% liegt (verglichen mit den Kontrollen), den Schluss nahe, dass in allen Zellen, die mit spezifischer dsRNA erfolgreich transfiziert werden konnten, die MDR1-mRNA nahezu vollständig und spezifisch abgebaut wurde.

### Literatur:

Alroy I & Yarden Y (1997) : The Erb signalling network in embryogenesis and oncogenesis: signal deversification through combinatorial ligand-receptor interactions. FEBS Letters 410: 83-86.
Bass,B.L., 2000. Double-stranded RNA as a template for gene silencing. Cell 101, 235-238.
Bosher,J.M. and Labouesse,M., 2000. RNA interference: genetic wand and genetic watchdog. Nature Cell Biology 2, E31-E36.
Bradford MM (1975): Rapid and sensitive method for the quantitation of microgram quantities of protein utilizing the principle of protein-dye binding. Anal. Biochem. 72: 243-254.
Caplen,N.J., Fleenor,J., Fire,A., and Morgan,R.A., 2000. dsRNA-mediated gene silencing in cultured *Drosophila* cells: a tissue culture model for the analysis of RNA interference. Gene 252, 95-105.
Clemens,J.C., Worby, C.A., Simonson-Leff,N., Muda, M., Maehama,T., Hemmings,B.A., and Dixon,J.E., 2000. Use of double-stranded RNA interference in *Drosophila* cell lines to dissect signal transduction pathways. *Proc.Natl.Acad.Sci.USA* 97, 6499-6503.
Cobleigh MA, Vogel CL, Tripathy D, Robert NJ, Scholl S, Fehrenbacher L, Wolter JM, Paton V, Shak S, Liebermann G & Slamon DJ (1999): Multinational study of the efficacy and safety of humanized anti-HER2 monoclonal antibody in women who have HER2-overexpressing metastatic breast cancer that has progressed after chemotherapy for metastatic disease. Journal of Clinical Oncology 17: 2639-2648.
Ding,S.w. , 2000. RNA silencing. Curr. Opin. Biotechnol. 11, 152-156.
Fire,A., Xu,S., Montgomery,M.K., Kostas,S.A., Driver, S.E., and Mello,C.C., 1998. Potent and specific genetic interference by double-stranded RNA in *Caenorhabditis elegans.* Nature 391, 806-811.
Fire, A., 1999. RNA-triggered gene silencing. Trends Genet. 15, 358-363.
Freier, S.M., Kierzek, R., Jaeger, J.A-, Sugimoto, N., Caruthers, M.H., Neilson, T., and Turner,D.H., 1986. Improved free-energy parameters for prediction of RNA duplex stability. Proc. Natl. Acad. Sci. USA 83, 9373-9377
Geick, A., Eichelbaum, M., Burk, O. (2001). Nuclear receptor response elements mediate induction of intestinal MDR1 by rifampin. J. Biol. Chem. 276 (18), 14581-14587.
Fontanini G, De Laurentiis M, Vignati S, Chine S, Lucchi M, Silvestri V, Mussi A, De Placido S, Tortora G, Bianco AR, Gullick W, Angeletti CA, Bevilaqua G & Ciardiello F (1998): Evaluation of epidermal growth factor-related growth factors and receptors and of neoangiogenesis in completely resected stage I-IIIA non-small-cell lung cancer: amphiregulin and microvessel count are independent prognostic factors of survival. Clinical Cancer Research 4: 241-249.
Hammond,S.M., Bernstein,E., Beach,D., and Hannon,G.J., 2000. An RNA-directed nuclease mediates post-transcriptional gene silencing in *Drosophila* cells. Nature 404, 293-296.
Higgins, C.F. (1995). The ABC of channel regulation. Cell, 82, 693-696.
Hadjantonakis AK, Gertsenstein M, Ikawa M, Okabe M & Nagy A (1993): Generating green fluorescent mice by germline transmission of green fluorescent ES cells. Mech. Dev. 76: 79-90.
hadjantonakis AK, Gertsenstein M, Ikawa M, Okabe M & Nagy A (1998): Non-invasive sexing of preimplantation mammalian embryos. Nature Genetics 19: 220-222.
Kyhse-Anderson J (1984) : Electroblotting of multiple gels: A simple apparatus without buffer tank for rapid transfer of proteins from polyacrylamide to nitrocellulose. J. Biochem. Biophys. Methods 10: 203-210.
Lämmli UK (1970) : Cleavage of structural proteins during the assembly of the head of bacteriophage T4. Nature 277: 680-685.
Loo, T.W., and Clarke, D.M. (1999) *Biochem. Cell Biol.* 77, 11-23.
Huang SM & Harari PM (1999): Epidermal growth factor receptor inhibition in cancer therapy: biology, rationale and preliminary clinical results. Investigational New Drugs 17: 259-269.
Limmer,S., Hofmann,H.-P., Ott,G., and Sprinzl,M., 1993. The 3'-terminal end (NCCA) of tRNA determines the structure and stability of the aminoacyl acceptor stem. Proc. Natl. Acad. Sci. USA 90 , 6199-6202.
Montgomery, M.K. and Fire, A., 1998. Double-stranded RNA as a mediator in sequence-specific genetic silencing and cosuppression. Trends Genet. 14, 255-258.
Montgomery, M. K., Xu, S., and Fire, A., 1998. RNA as a carget of double-stranded RNA-mediated genetic interference in *Caenorhabditis elegans.* Proc. Natl. Acad. Sci. USA 95, 15502-15507.
Rieske P, Kordek R, Bartkowiak J, Debiec-Rychter M, Bienhat W & Liberski PP (1998): A comparative study of epidermal growth factor (EGFR) and mdm2 gene amplification and protein immunoreactivity in human glioblastomas. Polish Journal of Pathology 49: 145-149.
Robert:, J. (1999). Multidrug resistance in oncology: diagnostic and therapeutic approaches. Europ J Clin Invest 29, 536-545.
Stavrovskaya, A.A. (2000) Biochemistry (Moscow) 65 (1), 95-106.
Salomon DS, Brandt R, Ciardiello F & Normanno N (1995): Epidermal growth factor related peptides and their receptors in human malignancies: Critical Reviews in Oncology and Haematology 19: 183-232.
Tom, B.H., Rutzky, L.P., Jakstys, M.M., Oyasu, R., Kaye, C.I., Kahan, B.D. (1976), In vitro, 12, 180-191.
Tsuruo, T., Iida, H., Tsukagoshi, S., Sakurai, Y. (1981). Overcoming of vincristine resistance in P388 leukemia in vivo and in vitro through enhanced cytotoxicity of vincristine and vinblastine by verapamil. *Cancer Res*, 41, 1367-72.
Ui-Tei, K., Zenno,S., Miyata, Y., and Saigo, K., 2000. Sensitive assay of RNA interference in *Drosophila* and Chinese hamster cultured cells using firefly luciferase gene as target. FEBS Lett. 479, 79-82.
Ullrich A, Coussens L, Hayflick JS, Dull TJ, Gray A, Tam AW, Lee J, Yarden Y, Liebermann TA, Schlessinger J et al. (1984) : Human epidermal growth factor receptor cDMA sequences and aberrant expression of the amplified gene in A431 epidermoid carcinoma cells. Nature 309: 418-425.
Ullrich A & Schlessinger J (1990): Signal transduction by receptors with tyrosine kinase activity. Cell 61: 203-212.
Van der Geer P, Hunter T & Linberg RA (1994) : Receptor protein-tyrosine kinases and their signal transduction pathways. Annual review in Cell Biology 10: 251-337.
Voldborg BR, Damstrup L, Spang-Thopmsen M & Poulsen HS (1997): Epidermal growth factor Receptor (EGFR) and EGFR mutations, function and possible role in clinical trials. Annuals of Oncology 8: 1197-1206.
Walker RA & Dearing SJ (1999): Expression of epidermal growth factor receptor mRNA and protein in primary breast carcinomas. Breast Cancer Research Treatment 53: 167-176.
Zamore,P.D., Tuschl, T., Sharp, P.A., and Bartel,D.P., 2000. RNAi: double-stranded RNA directs the ATP-dependent cleavage of mRNA at 21 to 23 nucleotide intervals. Cell 101 , 25-33.
Zor T & Selinger Z (1996) : Linearization of the Bradford protein assay increases its sensitivity: theoretical and experimental studies. Anal. Biochem. 236: 302-308.

## Patentansprüche

1. Verfahren zur Hemmung der Expression eines Zielgens in einer Zelle umfassend die folgenden Schritte:
Einführen mindestens einer doppelsträngigen Ribonukleinsäure (dsRNA I) in einer zur Hemmung der Expression des Zielgens ausreichenden Menge,
wobei die dsRNA I eine doppelsträngige aus höchstens 49 aufeinander folgenden Nukleotidpaaren gebildete Struktur aufweist, und wobei ein Strang (as1) oder zumindest ein Abschnitt des einen Strangs (as1) der doppelsträngigen Struktur komplementär zum Zielgen ist,
wobei die dsRNA I einen aus 1 bis 4 Nukleotiden gebildeten Überhang am 3'-Ende des einen Strangs (as1) aufweist und wobei die dsRNA I an einem das 5'-Ende des einen Strangs (as1) enthaltenden Ende (E1, E2) glatt ausgebildet ist, wobei das Zielgen das MDR1-Gen ist oder eine der Sequenzen SQ001 bis SQ140 aufweist,
wobei jedes verfahren zur chirurgischen oder therapeutischen Behandlung des menschlichen oder tierischen Körpers und jedes Diagnostizierverfahren, welches am menschlichen oder tierischen Körper vorgenommen wird, ausgenommen ist.

2. Verfahren nach Anspruch 1, wobei der Überhang aus 1 oder 2 Nukleotiden gebildet ist.

3. Verfahren nach einem der vorhergehenden Ansprüche, wobei zumindest eine entsprechend der dsRNA I nach einem der vorhergehenden Ansprüche ausgebildete weitere doppelsträngige Ribonukleinesäure (dsRNA II) in die Zelle eingeführt wird, wobei der eine Strang (as1) oder zumindest ein Abschnitt des einen Strangs (as1) der dsRNA I komplementär zu einem ersten Bereich (B1) des Zielgens ist, und wobei ein weiterer Strang (as2) oder zumindest ein Abschnitt des weiteren Strangs (as2) der dsRNA II komplementär zu einem zweiten Bereich (B2) des Zielgens ist.

4. Verfahren nach einem der vorhergehenden Ansprüche, wobei die dSRNA I und/oder die dsRNA II eine Länge von weniger als 25, vorzugsweise 19 bis 23, aufeinander folgenden Nukleotidpaaren aufweist/en.

5. Verfahren nach einem der vorhergehenden Ansprüche, wobei der erste (B1) und der zweite Bereich (B2) abschnittsweise überlappen oder aneinander grenzen.

6. Verfahren nach einem der vorhergehenden Ansprüche, wobei der erste (B1) und der zweite Bereich (B2) voneinander beabstandet sind.

7. Verfahren nach einem der vorhergehenden Ansprüche, wobei das Zielgen aus der folgenden Gruppe ausgewählt ist: Onkogen, Cytokin-Gen, Id-Protein-Gen, Priongen, Gene von Angiogenese induzierenden Molekülen, von Adhäsions-Molekülen und von Zelloberflächenrezeptoren, Gene von Proteinen, die an metastasierenden und/oder invasiven Prozessen beteiligt sind, Gene von Proteinasen sowie Apoptose- und Zellzyklusregulierenden Molekülen.

8. Verfahren nach einem der vorhergehenden Ansprüche, wobei als dsRNA I/II eine der Sequenzen SQ141 -173 bzw. ein aus zwei jeweils zusamanengehörenden Antisinn- (as1/2) und Sinnsequenzen (ss1/2) kombiniertes dsRNA-Konstrukt der Sequenzen SQ141 - 173 verwendet wird.

9. Verfahren nach einem der vorhergehenden Ansprüche, wobei die Expression nach dem Prinzip der RNA-Interferenz gehemmt wird.

10. Verfahren nach einem der vorhergehenden Ansprüche, wobei das Zielgen in pathogenen Organismen exprimiert wird.

11. Verfahren nach einem der vorhergehenden Ansprüche, wobei das Zielgen Bestandteil eines Virus oder Viroids ist.

12. Verfahren nach Anspruch 11, wobei das Virus ein humanpathogenes Virus oder Viroid ist.

13. Verfahren nach Anspruch 11, wobei das Virus oder Viroid ein tierpathogenes Virus oder Viroid ist.

14. Verfahren nach einem der vorhergehenden Ansprüche, wobei ungepaarte Nukleotide durch Nukleosidthiophosphate substituiert sind.

15. Verfahren nach einem der vorhergehenden Ansprüche, wobei zumindest ein Ende (E1, E2) der dsRNA I/II modifiziert wird, um einem Abbau in der Zelle oder einer Dissoziation in die Einzelstränge entgegenzuwirken.

16. Verfahren nach einem der vorhergehenden Ansprüche, wobei der durch die komplementären Nukleotidpaare bewirkte Zusammenhalt der doppelsträngigen Struktur durch mindestens eine chemische Verknüpfung erhöht wird.

17. Verfahren nach einem der vorhergehenden Ansprüche, wobei die chemische Verknüpfung durch eine kovalente oder ionische Bindung, eine Wasserstoffbrückenbindung, hydrophobe Wechselwirkungen, vorzugsweise van-der-Waals- oder Stapelungswechselwirkungen, oder durch Metall-Ionenkoordination gebildet wird.

18. Verfahren nach einem der vorhergehenden Ansprüche, wobei die chemische Verknüpfung in der Nähe des einen Endes (E1, E2) gebildet ist.

19. Verfahren nach einem der vorhergehenden Ansprüche, wobei die chemische Verknüpfung mittels einer oder mehrerer Verbindungsgruppen gebildet wird, wobei die Verbindungsgruppen vorzugsweise Poly-(oxyphosphinicooxy-1,3-propandiol)- und/oder Oligothylenglycol-Ketten sind.

20. Verfahren nach einem der vorhergehenden Ansprüche, wobei die chemische Verknüpfung durch anstelle von Nukleotiden benutzte verzweigte Nukleotidanaloga gebildet wird.

21. Verfahren nach einem der vorhergehenden Ansprüche, wobei die chemische Verknüpfung durch Purinanaloga gebildet wird.

22. Verfahren nach einem der vorhergehenden Ansprüche, wobei die chemische Verknüpfung durch Azabenzoleinheiten gebildet wird.

23. Verfahren nach einem der vorhergehenden Ansprüche, wobei zur Herstellung der chemischen Verknüpfung mindestens eine der folgenden Gruppen benutzt wird: Methylenblau; bifunktionelle Gruppen, vorzugsweise Bis-(2-chlorethyl)-amin; N-acetyl-N'-(p-glyoxyl-benzoyl)-cystamin; 4-Thiouracil; Psoralen.

24. Verfahren nach einem der vorhergehenden Ansprüche, wobei die chemische Verknüpfung durch in der Nähe der Enden (E1, E2) des doppelsträngigen Bereichs angebrachte Thiophosphoryl-Gruppen gebildet wird.

25. Verfahren nach einem der vorhergehenden Ansprüche, wobei die chemische Verknüpfung durch in der Nähe der Enden (E1, E2) befindliche Tripelhelix-Bindungen hergestellt wird.

26. Verfahren nach einem der vorhergehenden Ansprüche, wobei die dsRNA I/II in micellare Strukturen, vorteilhafterweise in Liposomen, eingeschlossen wird.

27. Verfahren nach einem der vorhergehenden Ansprüche, wobei die dsRNA I/II an mindestens ein von einem Virus stammendes, davon abgeleitetes oder ein synthetisch hergestelltes virales Hüllprotein gebunden, damit assoziiert oder davon umgeben wird/werden.

28. Verfahren nach einem der vorhergehenden Ansprüche, wobei das Hüllprotein vom Polyomavirus abgeleitet ist.

29. Verfahren nach einem der vorhergehenden Ansprüche, wobei das Hüllprotein das Virus-Protein 1 (VP1) und/oder das Virus-Protein 2 (VP2) des Polyomavirus enthält.

30. Verfahren nach einem der vorhergehenden Ansprüche, wobei bei Bildung eines Kapsids oder kapsidartigen Gebildes aus dem Hüllprotein die eine Seite zum Inneren des Kapsids oder kapsidartigen Gebildes gewandt ist.

31. Verfahren nach einem der vorhergehenden Ansprüche, wobei der eine Strang (as1, as2) der dsRNA I/II zum primären oder prozessierten RNA-Transkript des Zielgens komplementär ist.

32. verfahren nach einem der vorhergehenden Ansprüche, wobei die Zelle eine Vertebratenzelle oder eine menschliche Zelle ist.

33. Verfahren nach einem der vorhergehenden Ansprüche, wobei die dsRNA I/II zur Applikation in einer Pufferlösung aufgenommen ist.

34. Verwendung einer doppelsträngigen Ribonukleinsäure (dsRNA I) zur Herstellung eines Medikaments,
wobei die dsRNA I eine doppelsträngige aus höchstens 49 aufeinander folgenden Nukleotidpaaren gebildete Struktur aufweist, und wobei ein Strang (as1) oder zumindest ein Abschnittdes einen Strangs (as1) der doppelsträngigen Struktur komplementär zu einem Zielgen in einer Zelle ist, wobei die dsRNA I einen aus 1 bis 4 Nukleotiden gebildeten Überhang am 3'-Ende des einen Strangs (as1) aufweist und wobei die dsRNA I an einem das 5'-Ende des einen Strangs (as1) enthaltenden Ende (E1, E2) glatt ausgebildet ist, wobei das Zielgen das MDR1-Gen ist oder eine der Sequenzen SQ001 bis SQ140 aufweist.

35. Verwendung nach Anspruch 34, wobei der Überhang aus 1 oder 2 Nukleotiden gebildet ist.

36. Verwendung nach Anspruch 34 oder 35, wobei zumindest eine weitere entsprechend der dsRNA I nach einem der Ansprüche 34 oder 35 ausgebildete doppelsträngige Ribonukleinesäure (dsRNA II) zur Herstellung des Medikaments verwendet wird, wobei der eine Strang (as1) oder zumindest ein Abschnitt des einen Strangs (as1) der dsRNA I komplementär zu einem ersten Bereich (B1) des Sinn-Strangs des Zielgens ist, und wobei der weitere Strang (as2) oder zumindest ein Abschnitt des weiteren Strangs (as2) der dsRNA II komplementär zu einem zweiten Bereich (B2) des Zielgens ist.

37. Verwendung nach einem der Ansprüche 34 bis 36, wobei die dsRNA I und/oder die dsRNA II eine Länge von weniger als 25, vorzugsweise 19 bis 23, aufeinander folgenden Nukleotidpaaren aufweist/en.

38. Verwendung nach einem der Ansprüche 34 bis 37, wobei der erste (B1) und der zweite Bereich (B2) abschnittsweise überlappen oder aneinander grenzen.

39. Verwendung nach einem der Ansprüche 34 bis 38, wobei der erste (B1) und der zweite Bereich (B2) voneinander beabstandet sind.

40. Verwendung nach einem der Ansprüche 34 bis 39, wobei das Zielgen aus der folgenden Gruppe ausgewählt ist: Onkogen, Cytokin-Gen, Id-Protein-Gen, Priongen, Gene von Angiogenese induzierenden Molekülen, von Adhäsions-Molekülen und von Zelloberflächenrezeptoren, Gene von Proteinen,die an metastasierenden und/oder invasiven Prozessen beteiligt sind, Gene von Proteinasen sowie von Apoptose- und Zellzyklusregulierende Molekülen.

41. Verwendung nach einem der Ansprüche 34 bis 40, wobei als dsRNA I/II eine der Sequenzen SQ141 -173 bzw. ein aus zwei jeweils zusammengehörenden Antisinn- (as1/2) und Sinnsequenzen (ss1/2) kombiniertes dsRNA-Konstrukt der Sequenzen SQ141 - 173 verwendet wird.

42. Verwendung nach einem der Ansprüche 34 bis 41, wobei die Expression nach dem Prinzip der RNA-Interferenz gehemmt wird.

43. Verwendung nach einem der Ansprüche 34 bis 42, wobei das Zielgen in pathogenen Organismen exprimiert wird.

44. Verwendung nach einem der Ansprüche 34 bis 43, wobei das Zielgen Bestandteil eines Virus oder Viroids ist.

45. Verwendung nach Anspruch 44, wobei das Virus ein humanpathogenes Virus oder Viroid ist.

46. Verwendung nach Anspruch 44, wobei das Virus oder Viroid ein tierpathogenes Virus oder Viroid ist.

47. Verwendung nach einem der Ansprüche 34 bis 46, wobei ungepaarte Nukleotide durch Nukleosidthiophosphate substituiert sind.

48. Verwendung nach einem der Ansprüche 34 bis 47, wobei zumindest ein Ende (E1, E2) der dsRNA modifiziert wird, um einem Abbau in der Zelle oder einer Dissoziation in die Einzelstränge entgegenzuwirken.

49. Verwendung nach einem der Ansprüche 34 bis 48, wobei der durch die komplementären Nukleotidpaare bewirkte Zusammenhalt der doppelsträngigen Struktur durch mindestens eine chemische Verknüpfung erhöht wird.

50. Verwendung nach einem der Ansprüche 34 bis 49, wobei die chemische Verknüpfung durch eine kovalente oder ionische Bindung, eine Wasserstoffbrückenbindung, hydrophobe Wechselwirkungen, vorzugsweise van-der-Waals- oder Stapelungswechselwirkungen, oder durch Metall-Ionenkoordination gebildet wird.

51. Verwendung nach einem der Ansprüche 34 bis 50, wobei die chemische Verknüpfung in der Nähe des einen Endes (E1, E2) gebildet ist.

52. Verwendung nach einem der Ansprüche 34 bis 51, wobei die chemische Verknüpfung mittels einer oder mehrerer Verbindungsgruppen gebildet wird, wobei die Verbindungsgruppen vorzugsweise Poly- (oxyphosphinicooxy-1,3-propandiol)- und/oder Oligoethylenglycol-Ketten sind.

53. Verwendung nach einem der Ansprüche 34 bis 52, wobei die chemische Verknüpfung durch anstelle von Nukleotiden benutzte verzweigte Nukleotidanaloga gebildet wird.

54. Verwendung nach einem der Ansprüche 34 bis 53, wobei die chemische Verknüpfung durch Purinanaloga gebildet wird.

55. Verwendung nach einem der Ansprüche 34 bis 54, wobei die chemische Verknüpfung durch Azabenzoleinheiten gebildet wird.

56. Verwendung nach einem der Ansprüche 34 bis 55, wobei zur Herstellung der chemischen Verknüpfung mindestens eine der folgenden Gruppen benutzt wird: Methylenblau; bifunktionelle Gruppen, vorzugsweise Bis-(2-chlorethyl)-amin; N-acetyl-N'-(p-glyoxyl-benzoyl)-cystamin; 4-Thiouracil; Psoralen.

57. Verwendung nach einem der Ansprüche 34 bis 56, wobei die chemische Verknüpfung durch in der Nähe der Enden (E1, E2) des doppelsträngigen Bereichs angebrachte Thiophosphoryl-Gruppen gebildet wird.

58. Verwendung nach einem der Ansprüche 34 bis 57, wobei die chemische Verknüpfung durch in der Nähe der Enden (E1, E2) befindliche Tripelhelix-Bindungen hergestellt wird.

59. Verwendung nach einem der Ansprüche 34 bis 58, wobei die dsRNA I/II in micellare Strukturen, vorteilhafterweise in Liposomen, eingeschlossen wird.

60. Verwendung nach einem der Ansprüche 34 bis 59, wobei die dsRNA I/II an mindestens ein von einem Virus stammendes, davon abgeleitetes oder ein synthetisch hergestelltes virales Hüllprotein gebunden, damit assoziiert oder davon umgeben wird/werden.

61. Verwendung nach einem der Ansprüche 34 bis 60, wobei das Hüllprotein vom Polyomavirus abgeleitet ist.

62. Verwendung nach einem der Ansprüche 34 bis 61, wobei das Hüllprotein das Virus-Protein 1 (VP1) und/oder das Virus-Protein 2 (VP2) des Polyomavirus enthält.

63. Verwendung nach einem der Ansprüche 34 bis 62, wobei bei Bildung eines Kapsids oder kapsidartigen Gebildes aus dem Hüllprotein die eine Seite zum Inneren des Kapsids oder kapsidartigen Gebildes gewandt ist.

64. Verwendung nach einem der Ansprüche 34 bis 63, wobei der eine Strang (as1, as2) der dsRNA I/II zum primären oder prozessierten RNA-Transkript des Zielgens komplementär ist.

65. Verwendung nach einem der Ansprüche 34 bis 64, wobei die Zelle eine Vertebratenzelle oder eine menschliche Zelle ist.

66. Verwendung nach einem der Ansprüche 34 bis 65, wobei das Medikament dazu geeignet ist, die dsRNA I/II in einer Menge von höchstens 5 mg je Kilogramm Körpergewicht pro Tag einem Säugetier, vorzugsweise einem Menschen, zu verabreichen.

67. Verwendung nach einem der Ansprüche 34 bis 66, wobei die dsRNA I/II zur Applikation in einer Pufferlösung aufgenommen ist.

68. Verwendung nach einem der Ansprüche 34 bis 67, wobei das Medikament dazu geeignet ist, die dsRNA I/II oral oder mittels Injektion oder Infusion intravenös, intratumoral, inhalativ, intraperitoneal zu verabreichten.

69. Medikament zur Hemmung der Expression eines Zielgens in einer Zelle enthaltend eine doppelsträngige Ribonukleinsäure (dsRNA I) in einer zur Hemmung der Expression des Zielgens ausreichenden Menge,
wobei die dsRNA I eine doppelsträngige aus höchstens 49 aufeinander folgenden Nukleotidpaaren gebildete Struktur aufweist,
wobei ein Strang (as1) oder zumindest ein Abschnitt des einen Strangs (as1) der doppelsträngigen Struktur komplementär zum Zielgen ist,
wobei die dsRNA I einen aus 1 bis 4 Nukleotiden gebildeten Überhang am 3'-Ende des einen Strangs (as1) aufweist und wobei die dsRNA I an einem das 5'-Ende des einen Strangs (as1) enthaltenden Ende (E1, E2) glatt ausgebildet ist, wobei das Zielgen das MDR1-Gen ist oder eine der Sequenzen SQ001 bis SQ140 aufweist.

70. Medikament nach Anspruch 69, wobei der Überhang aus 1 oder 2 Nukleotiden gebildet ist.

71. Medikament nach Anspruch 69 oder 70, enthaltend zumindest eine weitere entsprechend der dsRNA I nach einem der Ansprüche 69 oder 70 ausgebildete doppelsträngige Ribonukleinesäure (dsRNA II), wobei der eine Strang (as1) oder zumindest ein Abschnitt des einen Strangs (as1) der dsRNA I komplementär zu einem ersten Bereich (B1) des Zielgens ist, und wobei der weitere Strang (as2) oder zumindest ein Abschnitt des weiteren Strangs (as2) der dsRNA II komplementär zu einem zweiten Bereich (B2) des Zielgens ist.

72. Medikament nach einem der Ansprüche 69 bis 71, wobei die dsRNA I und/oder die dsRNA II eine Länge von weniger als 25, vorzugsweise 19 bis 23. aufeinander folgenden Nukleotidpaaren aufweist/en.

73. Medikament nach einem der Ansprüche 69 bis 72, wobei der erste (B1) und der zweite Bereich (B2) abschnittsweise überlappen oder aneinander grenzen.

74. Medikament nach einem der Ansprüche 69 bis 73, wobei das Zielgen aus der folgenden Gruppe ausgewählt ist: Onkogen, Cytokin-Gen, Id-Protein-Gen, Priongen, Gene von Angiogenese induzierenden Molekülen, von Adhäsions-Molekülen und von Zelloberflächenrezeptoren, Gene von Proteinen, die an metastasierenden und/oder invasiven Prozessen beteiligt sind, Gene von Proteinasen sowie von Apoptose- und Zellzyklusregulierende Molekülen.

75. Medikament nach einem der Ansprüche 69 bis 74, wobei als dsRNA eine der Sequenzen SQ141 -173 bzw. ein aus zwei jeweils zusammengehörenden Antisinn- (as1/2) und Sinnsequenzen (ss1/2) kombiniertes dsRNA-Konstrukt der Sequenzen SQ141 - 173 verwendet wird.

76. Medikament nach einem der Ansprüche 69 bis 75, wobei die Expression nach dem Prinzip der RNA-Interferenz gehemmt wird.

77. Medikament nach einem der Ansprüche 69 bis 76, wobei das Zielgen in pathogenen Organismen exprimierbar ist.

78. Medikament nach einem der Ansprüche 69 bis 77, wobei das Zielgen Bestandteil eines Virus oder Viroids ist.

79. Medikament nach Anspruch 78, wobei das Virus ein humanpathogenes Virus oder Viroid ist.

80. Medikament nach Anspruch 78, wobei das Virus oder Viroid ein tierpathogenes Virus oder Viroid ist.

81. Medikament nach einem der Ansprüche 69 bis 80, wobei ungepaarte Nukleotide durch Nukleosidthiophosphate substituiert sind.

82. Medikament nach einem der Ansprüche 69 bis 81, wobei zumindest ein Ende (E1, E2) der dsRNA modifiziert ist, um einem Abbau in der Zelle oder einer Dissoziation in die Einzelstränge entgegenzuwirken.

83. Medikament nach einem der Ansprüche 69 bis 82, wobei der durch die komplementären Nukleotidpaare bewirkte Zusammenhalt der doppelsträngigen Struktur durch mindestens eine chemische Verknüpfung erhöht ist.

84. Medikament nach einem der Ansprüche 69 bis 83, wobei die chemische Verknüpfung durch eine kovalente oder ionische Bindung, eine Wasserstoffbrückenbindung, hydrophobe Wechselwirkungen, vorzugsweise van-der-Waals- oder Stapelungswechselwirkungen, oder durch Metall-Ionenkoordination gebildet ist.

85. Medikament nach einem der Ansprüche 69 bis 84, wobei die chemische Verknüpfung in der Nähe des einen Endes (E1, E2) gebildet ist.

86. Medikament nach einem der Ansprüche 69 bis 85, wobei die chemische Verknüpfung mittels einer oder mehrerer Verbindungsgruppen gebildet wird, wobei die Verbindungsgruppen vorzugsweise Poly-(oxyphosphinicooxy-1,3-propandiol)- und/oder Oligoechylenglycol-Ketten sind.

87. Medikament nach einem der Ansprüche 69 bis 86, wobei die chemische Verknüpfung durch anstelle von Nukleotiden benutzte verzweigte Nukleotidanaloga gebildet ist.

88. Medikament nach einem der Ansprüche 69 bis 87, wobei die chemische Verknüpfung durch Purinanaloga gebildet ist.

89. Medikament nach einem der Ansprüche 69 bis 88, wobei die chemische Verknüpfung durch Azabenzoleinheiten gebildet ist.

90. Medikament nach einem der Ansprüche 69 bis 89, wobei zur Herstellung der chemischen Verknüpfung mindestens eine der folgenden Gruppen benutzt wird: Methylenblau; bifunktionelle Gruppen, vorzugsweise Bis-(2-chlorethyl)-amin; N-acetyl-N'-(p-glyoxyl-benzoyl)-cystamin; 4-Thiouracil; Psoralen.

91. Medikament nach einem der Ansprüche 69 bis 90, wobei die chemische Verknüpfung durch in der Nähe der Enden (E1, E2) des doppelsträngigen Bereichs angebrachte Thiophosphoryl-Gruppen gebildet ist.

92. Medikament nach einem der Ansprüche 69 bis 91, wobei die chemische Verknüpfung durch in der Nähe der Enden (E1, E2) befindliche Tripelhelix-Bindungen hergestellt ist.

93. Medikament nach einem der Ansprüche 69 bis 92, wobei die dsRNA I/II in micellare Strukturen, vorteilhafterweise in Liposomen, eingeschlossen ist.

94. Medikament nach einem der Ansprüche 69 bis 93, wobei die dsRNA I an mindestens ein von einem Virus stammendes, davon abgeleitetes oder ein synthetisch hergestelltes virales Hüllprotein gebunden, damit assoziiert oder davon umgeben ist/sind.

95. Medikament nach einem der Ansprüche 69 bis 94, wobei das Hüllprotein vom Polyomavirus abgeleitet ist.

96. Medikament nach einem der Ansprüche 69 bis 95, wobei das Hüllprotein das Virus-Protein 1 (VP1) und/oder das Virus-Protein 2 (VP2) des Polyomavirus enthält.

97. Medikament nach einem der Ansprüche 69 bis 96, wobei bei Bildung eines Kapsids oder kapsidartigen Gebildes aus dem Hüllprotein die eine Seite zum Inneren des Kapsids oder kapsidartigen Gebildes gewandt ist.

98. Medikament nach einem der Ansprüche 69 bis 97, wobei der eine Strang (as1, as2) der dsRNA I zum primären oder prozessierten RNA-Transkript des Zielgens komplementär ist.

99. Medikament nach einem der Ansprüche 69 bis 98, wobei die Zelle eine Vertebratenzelle oder eine menschliche Zelle ist.

100. Medikament nach einem der Ansprüche 69 bis 99, wobei der erste (B1) und der zweite Bereich (B2) voneinander beabstandet sind.

101. Medikament nach einem der Ansprüche 69 bis 100, wobei die dsRNA in einer Menge von höchstens 5 mg pro Verabreichungseinheit enthalten ist.

102. Medikament nach einem der Ansprüche 69 bis 101, wobei die dsRNA in einer Pufferlösung aufgenommen ist.

103. Medikament nach einem der Ansprüche 69 bis 102, wobei die dsRNA oral oder mittels Injektion oder Infusion intravenös, intractumoral, inhalativ, intraperitoneal verabreichbar ist.

## Claims

1. Method for inhibiting the expression of a target gene in a cell, comprising the following steps:
Introduction of at least one double-stranded ribonucleic acid (dsRNA I) in a quantity sufficient to inhibit expression of the target gene,
wherein dsRNA I exhibits a double-stranded structure made up from a maximum of 49 successive nucleotide pairs, and wherein one strand (as1) or at least one segment of said one strand (as1) of the double-stranded structure is complementary to the target gene,
wherein the dsRNA I exhibits an overhang consisting of 1 to 4 nucleotides at the 3'-end of said one strand (as1) and wherein the dsRNA I is blunt ended at an end (E1, E2) containing the 5'-end of said one strand (as1), wherein the target gene is the MDR1 gene or exhibits one of the sequences SQ001 to SQ140,
and wherein any method for the surgical or therapeutic treatment of a human or animal body, and any diagnostic method which is performed on the human or animal body, is excluded.

2. Method in accordance with claim 1, wherein the overhang is made up from 1 or 2 nucleotides.

3. Method in accordance with any one of the preceding claims, wherein at least one further double-stranded ribonucleic acid (dsRNA II) having a configuration according to the dsRNA I as defined in the preceding claims, is introduced into the cell, wherein said one strand (as1) or at least one segment of said one strand (as1) of dsRNA I is complementary to a first region (B1) of the target gene, and wherein another strand (as2) or at least one segment of the other strand (as2) of dsRNA II is complementary to a second region (B2) of the target gene.

4. Method in accordance with any one of the preceding claims, wherein dsRNA I and/or dsRNA II exhibit/s a length of fewer than 25, preferably 19 to 23 successive nucleotide pairs.

5. Method in accordance with claim 3 or 4, wherein the first (B1) and the second region (B2) overlap segmentally or adjoin each other.

6. Method in accordance with claim 3 or 4, wherein the first (B1) and the second region (B2) are separated from each other.

7. Method in accordance with any one of the preceding claims, wherein the target gene is selected from the following group: oncogene, cytokine gene, Id protein gene, prion gene; genes of angiogenesis-inducing molecules, of adhesion molecules, and of cell-surface receptors; genes of proteins involved in metastatic and/or invasive processes; genes of proteinases as well as of molecules that regulate apoptosis and the cell cycle.

8. Method in accordance with any one of the preceding claims, wherein a dsRNA construct being combined of antisense- (as1/2) and sense sequences (ss1/2) of the sequences SQ141-173 belonging together each are used as the dsRNA I/II.

9. Method in accordance with any one of the preceding claims, wherein expression is inhibited according to the principle of RNA interference.

10. Method in accordance with any one of the preceding claims, wherein the target gene is expressed in pathogenic organisms.

11. Method in accordance with any one of the preceding claims, wherein the target gene is a component of a virus or viroid.

12. Method in accordance with claim 11, wherein the virus is a human pathogenic virus or viroid.

13. Method in accordance with claim 11, wherein the virus or viroid is a virus or viroid that is pathogenic in animals.

14. Method in accordance with any one of the preceding claims, wherein unpaired nucleotides are substituted by nucleoside thiophosphates.

15. Method in accordance with any one of the preceding claims, wherein at least one end (E1, E2) of dsRNA I/II is modified in order to counter degradation in the cell or dissociation in the individual strands.

16. Method in accordance with any one of the preceding claims, wherein the cohesion of the double-stranded structure effected by the complementary nucleotide pairs is increased by at least one chemical linkage.

17. Method in accordance with claim 16, wherein the chemical linkage is formed either by a covalent or ionic bond, a hydrogen bond, hydrophobic interactions, preferably by means of van der Waals or stacking interactions, or by means of metal-ion coordination.

18. Method in accordance with claims 16 or 17, wherein the chemical linkage is formed in the vicinity of said one end (E1, E2).

19. Method in accordance with any one of claims 16 to 18, wherein the chemical linkage is created by one or several linkage groups, wherein the linkage groups are preferably poly-(oxyphosphinico-oxy-1,3-propandiol) and/or oligoethyleneglycol chains.

20. Method in accordance with any one of claims 16 to 18, wherein the chemical linkage is formed by using branched nucleotide analogs instead of nucleotides.

21. Method in accordance with any one of claims 16 to 18, wherein the chemical linkage is formed by purine analogs.

22. Method in accordance with any one of claims 16 to 18, wherein the chemical linkage is formed by azabenzene units.

23. Method in accordance with any one of claims 16 to 18, wherein at least one of the following groups is used in creating the chemical linkage: methylene blue; bifunctional groups, preferably bis-(2-chlorethyl)-amine; N-acetyl-N'-(p-glyoxyl-benzoyl)-cystamine; 4-thiouracil; psoralen.

24. Method in accordance with any one of claims 16 to 18, wherein the chemical linkage is formed by thiophosphoryl groups that are attached in the vicinity of the ends (E1, E2) of the double-stranded region.

25. Method in accordance with any one of claims 16 to 18, wherein the chemical linkage is generated by triple helix bonds that are present in the vicinity of the ends (E1, E2).

26. Method in accordance with any one of the preceding claims, wherein the dsRNA I/II is enclosed in micellar structures, most advantageously in liposomes.

27. Method in accordance with any one of the preceding claims, wherein the dsRNA I/II is bound to, associated with, or enclosed by at least one viral coat protein that stems from a virus, is derived from it, or is synthetically generated.

28. Method in accordance with claim 27, wherein the coat protein is derived from polyomavirus.

29. Method in accordance with claim 27 or 28, wherein the coat protein contains Virus Protein 1 (VP1) and/or Virus Protein 2 (VP2) of the polyomavirus.

30. Method in accordance with any one of claims 27 to 29, wherein at the formation of a capsid or capsid-like structure from the coat protein, the one side is turned toward the inside of the capsid or capsid-like structure.

31. Method in accordance with any one of the preceding claims, wherein the one strand (as1, as2) of dsRNA I/II is complementary to the primary or processed RNA transcript of the target gene.

32. Method in accordance with any one of the preceding claims, wherein the cell is a vertebrate cell or a human cell.

33. Method in accordance with any one of the preceding claims, wherein the dsRNA I/II is taken up in a buffer solution for application.

34. Use of a double-stranded ribonucleic acid (dsRNA I) for the manufacture of a medicament,
wherein dsRNA I exhibits a double-stranded structure made up from a maximum of 49 successive nucleotide pairs, and wherein one strand (as1) or at least one segment of said one strand (as1) of the double-stranded structure is complementary to the target gene,
wherein the dsRNA I exhibits an overhang consisting of 1 to 4 nucleotides at the 3'-end of said one strand (as1) and wherein the dsRNA I is blunt ended at an end (E2) containing the 5'-end of said one strand (as1), wherein the target gene is the MDR1 gene or exhibits one of the sequences SQ001 to SQ140.

35. Use in accordance with claim 34, wherein the overhang is made up from 1 or 2 nucleotides.

36. Use in accordance with claim 34 or 35, wherein at least one further double-stranded ribonucleic acid (dsRNA II) having a configuration according to dsRNA I as defined in claim 34 or 35, is used for the manufacture of the medicament, wherein said one strand (as1) or at least one segment of said one strand (as1) of the dsRNA I is complementary to a first region (B1) of the sense strand of the target gene, and wherein another strand (as2) or at least one segment of the other strand (as2) of dsRNA II is complementary to a second region (B2) of the target gene.

37. Use in accordance with any one of claims 34 to 36, wherein the dsRNA I and/or dsRNA II exhibit/s a length of fewer than 25, preferably 19 to 23, successive nucleotide pairs.

38. Use in accordance with claim 36 or 37, wherein the first (B1) and the second region (B2) overlap segmentally or adjoin each other.

39. Use in accordance with claim 36 or 37, wherein the first (B1) and the second region (B2) are separated from each other.

40. Use in accordance with any one of claims 34 to 39, wherein the target gene is selected from the following group: oncogene, cytokine gene, Id protein gene, prion gene; genes of angiogenesis-inducing molecules, of adhesion molecules, and of cell-surface receptors; genes of proteins involved in metastatic and/or invasive processes; genes of proteinases as well as of molecules that regulate apoptosis and the cell cycle.

41. Use in accordance with any one of claims 34 to 40, wherein a dsRNA construct being combined of antisense-(as1/2) and sense sequences (ssl/2) of the sequences SQ141-173 belonging together each are used as the dsRNA I/II.

42. Use in accordance with any one of claims 34 to 41, wherein expression is inhibited according to the principle of RNA interference.

43. Use in accordance with any one of claims 34 to 42, wherein the target gene is expressed in pathogenic organisms.

44. Use in accordance with any one of claims 34 to 43, wherein the target gene is a component of a virus or viroid.

45. Use in accordance with claim 44, wherein the virus is a human pathogenic virus or viroid.

46. Use in accordance with claim 44, wherein the virus or viroid is a virus or viroid that is pathogenic in animals.

47. Use in accordance with any one of claims 34 to 46, wherein unpaired nucleotides are substituted by nucleoside thiophosphates.

48. Use in accordance with any one of claims 34 to 47, wherein at least one end (E1, E2) of the dsRNA is modified in order to counter degradation in the cell or dissociation in the individual strands.

49. Use in accordance with any one of claims 34 to 48, wherein the cohesion of the double-stranded structure effected by the complementary nucleotide pairs is increased by at least one chemical linkage.

50. Use in accordance with claim 49, wherein the chemical linkage is formed either by a covalent or ionic bond, a hydrogen bond, hydrophobic interactions, preferably by means of van der Waals or stacking interactions, or by means of metal-ion coordination.

51. Use in accordance with claim 49 or 50, wherein the chemical linkage is formed in the vicinity of said one end (E1, E2).

52. Use in accordance with any one of claims 49 to 51, wherein the chemical linkage is created by one or several linkage groups, wherein the linkage groups are preferably poly-(oxyphosphinico-oxy-1,3-propandiol) and/or oligoethyleneglycol chains.

53. Use in accordance with any one of claims 49 to 51, wherein the chemical linkage is formed by using branched nucleotide analogs instead of nucleotides.

54. Use in accordance with any one of claims 49 to 51, wherein the chemical linkage is formed by purine analogs.

55. Use in accordance with any one of claims 49 to 51, wherein the chemical linkage is formed by azabenzene units.

56. Use in accordance with any one of claims 49 to 51, wherein at least one of the following groups is used in creating the chemical linkage: methylene blue; bifunctional groups, preferably bis-(2-chlorethyl)-amine; N-acetyl-N'-(p-glyoxyl-benzoyl)-cystamine; 4-thiouracil; psoralen.

57. Use in accordance with any one of claims 49 to 51, wherein the chemical linkage is formed by thiophosphoryl groups that are attached in the vicinity of the ends (E1, E2) of the double-stranded region.

58. Use in accordance with any one of claims 49 to 51, wherein the chemical linkage is generated by triple helix bonds that are present in the vicinity of the ends (E1, E2).

59. Use in accordance with any one of claims 34 to 58, wherein the dsRNA I/II is enclosed in micellar structures, most advantageously in liposomes.

60. Use in accordance with any one of claims 34 to 59, wherein the dsRNA I/II is bound to, associated with, or enclosed by at least one viral coat protein that stems from a virus, is derived from it, or is synthetically generated.

61. Use in accordance with claim 60, wherein the coat protein is derived from polyomavirus.

62. Use in accordance with claims 60 or 61, wherein the coat protein contains Virus Protein 1 (VP1) and/or Virus Protein 2 (VP2) of the polyomavirus.

63. Use in accordance with any one of claims 60 to 62, wherein at the formation of a capsid or capsid-like structure from the coat protein, the one side is turned toward the inside of the capsid or capsid-like structure.

64. Use in accordance with any one of claims 34 to 63, wherein the one strand (as1, as2) of dsRNA I/II is complementary to the primary or processed RNA transcript of the target gene.

65. Use in accordance with any one of claims 34 to 64, wherein the cell is a vertebrate cell or a human cell.

66. Use in accordance with any one of claims 34 to 65, wherein the medicament is suitable for the delivery of a dose of at most 5 mg per kg body weight per day of dsRNA I/II to a mammal, preferably a human.

67. Use in accordance with any one of claims 34 to 66, wherein the dsRNA I/II is taken up in a buffer solution for application.

68. Use in accordance with any one of claims 34 to 67, wherein the medicament is suitable for application of dsRNA I/II orally, by inhalation, or by injection or infusion intravenously, intratumorally, or intraperitoneally.

69. Medicament for inhibiting the expression of a target gene in a cell, containing a double-stranded ribonucleic acid (dsRNA I) in a dosage sufficient to inhibit the expression of the target gene,
wherein dsRNA I exhibits a double-stranded structure made up from a maximum of 49 successive nucleotide pairs, and wherein one strand (as1) or at least one segment of said one strand (as1) of the double-stranded structure is complementary to the target gene,
wherein the dsRNA I exhibits an overhang consisting of 1 to 4 nucleotides at the 3'-end of said one strand (as1) and wherein the dsRNA I is blunt ended at an end (E2) containing the 5'-end of said one strand (as1), wherein the target gene is the MDR1 gene or exhibits one of the sequences SQ001 to SQ140.

70. Medicament in accordance with claim 69, wherein the overhang is made up from 1 or 2 nucleotides.

71. Medicament in accordance with any one of claims 69 or 70 containing at least one further double-stranded ribonucleic acid (dsRNA II) having a configuration according to the dsRNA I as defined in claims 69 or 70, wherein said one strand (as1) or at least one segment of said one strand (as1) of dsRNA I is complementary to a first region (31) of the target gene, and wherein another strand (as2) or at least one segment of the other strand (as2) of dsRNA II is complementary to a second region (B2) of the target gene.

72. Medicament in accordance with any one of claims 69 to 71, wherein the dsRNA I and/or dsRNA II exhibit/s a length of fewer than 25, preferably in 19 to 23, successive nucleotide pairs.

73. Medicament in accordance with claim 71 or 72, wherein the first (B1) and the second region (B2) overlap segmentally or adjoin each other.

74. Medicament in accordance with any one of claims 69 to 73, wherein the target gene is selected from the following group: oncogene, cytokine gene, Id protein gene, prion gene; genes of angiogenesis-inducing molecules, of adhesion molecules, and of cell-surface receptors; genes of proteins involved in metastatic and/or invasive processes; genes of proteinases as well as of molecules that regulate apoptosis and the cell cycle.

75. Medicament in accordance with any one of claims 69 to 74, wherein a dsRNA construct being combined of antisense-(as1/2) and sense sequences (ss1/2) of the sequences SQ141-173 belonging together each are used as the dsRNA.

76. Medicament in accordance with any one of claims 69 to 75, wherein expression is inhibited according to the principle of RNA interference.

77. Medicament in accordance with any one of claims 69 to 76, wherein the target gene can be expressed in pathogenic organisms.

78. Medicament in accordance with any one of claims 69 to 77, wherein the target gene is a component of a virus or viroid.

79. Medicament in accordance with claim 78, wherein the virus is a human pathogenic virus or viroid.

80. Medicament in accordance with claim 78, wherein the virus or viroid is a virus or viroid that is pathogenic in animals.

81. Medicament in accordance with any one of claims 69 to 80, wherein unpaired nucleotides are substituted by nucleoside thiophosphates.

82. Medicament in accordance with any one of claims 69 to 81, wherein at least one end (E1, E2) of the dsRNA is modified in order to counter degradation in the cell or dissociation in the individual strands.

83. Medicament in accordance with any one of claims 69 to 82, wherein the cohesion of the double-stranded structure effected by the complementary nucleotide pairs is increased by at least one chemical linkage.

84. Medicament in accordance with claim 83, wherein the chemical linkage is formed either by a covalent or ionic bond, a hydrogen bond, hydrophobic interactions, preferably by means of van der Waals or stacking interactions, or by means of metal-ion coordination.

85. Medicament in accordance with claim 83 or 84, wherein the chemical linkage is formed in the vicinity of said one end (E1, E2).

86. Medicament in accordance with any one of claims 83 to 85, wherein the chemical linkage is created by one or several linkage groups, wherein the linkage groups are preferably poly-(oxyphosphinico-oxy-1,3-propandiol) and/or oligoethyleneglycol chains.

87. Medicament in accordance with any one of claims 83 to 85, wherein the chemical linkage is formed by using branched nucleotide analogs instead of nucleotides.

88. Medicament in accordance with any one of claims 83 to 85, wherein the chemical linkage is formed by purine analogs.

89. Medicament in accordance with any one of claims 83 to 85, wherein the chemical linkage is formed by azabenzene units.

90. Medicament in accordance with any one of claims 83 to 85, wherein at least one of the following groups is used in creating the chemical linkage: methylene blue; bifunctional groups, preferably bis-(2-chlorethyl)-amine; N-acetyl-N'-(p-glyoxyl-benzoyl)-cystamine; 4-thiouracil; psoralen.

91. Medicament in accordance with any one of claims 83 to 85, wherein the chemical linkage is formed by thiophosphoryl groups that are attached in the vicinity of the ends (E1, E2) of the double-stranded region.

92. Medicament in accordance with any one of claims 83 to 85, wherein the chemical linkage is generated by triple helix bonds that are present in the vicinity of the ends (E1, E2).

93. Medicament in accordance with any one of claims 69 to 92, wherein the dsRNA I/II is enclosed in micellar structures, most advantageously in liposomes.

94. Medicament in accordance with any one of claims 69 to 93, wherein the dsRNA I is bound to, associated with, or enclosed by at least one viral coat protein that stems from a virus, is derived from it, or is synthetically generated.

95. Medicament in accordance with claim 94, wherein the coat protein is derived from polyomavirus.

96. Medicament in accordance with claims 94 or 95, wherein the coat protein contains Virus Protein 1 (VP1) and/or Virus Protein 2 (VP2) of the polyomavirus.

97. Medicament in accordance with any one of claims 94 to 96, wherein at the formation of a capsid or capsid-like structure from the coat protein, the one side is turned toward the inside of the capsid or capsid-like structure.

98. Medicament in accordance with any one of claims 69 to 97, wherein the one strand (as1, as2) of dsRNA I is complementary to the primary or processed RNA transcript of the target gene.

99. Medicament in accordance with any one of claims 69 to 98, wherein the cell is a vertebrate cell or a human cell.

100. Medicament in accordance with any one of claims 71, 72 or 74 to 99, wherein the first (B1) and the second region (B2) are separated from each other.

101. Medicament in accordance with any one of claims 69 to 100, wherein a maximum amount of 5 mg of the dsRNA is contained in each dosing unit.

102. Medicament in accordance with any one of claims 69 to 101, wherein the dsRNA is taken up in a buffer solution.

103. Medicament in accordance with any one of claims 69 to 102, wherein the dsRNA is suitable to be administered orally, by inhalation, or by injection or infusion intravenously, intratumorally, or intraperitoneally.

## Revendications

1. Procédé d'inhibition de l'expression d'un gène cible dans une cellule, comportant les étapes suivantes :
introduction d'au moins un acide ribonucléique double brin (ARNdb I) dans une quantité suffisante pour inhiber l'expression du gène cible,
l'ARNdb I possédant une structure double brin formée d'au plus 49 paires de nucléotides successives, et un brin (as1) ou au moins un segment d'un brin (as1) de la structure double brin étant complémentaire du gène cible,
l'ARNdb I présentant une extension formée de 1 à 4 nucléotides au niveau de l'extrémité 3' d'un brin (as1) et l'ARNdb I étant formé de façon lisse au niveau d'une extrémité (E1, E2) contenant l'extrémité 5' d'un brin (as1), le gène cible étant le gène MDR1 ou présentant une des séquences SQ001 à SQ140,
chaque procédé de traitement chirurgical ou thérapeutique du corps humain ou animal et chaque procédé de diagnostic qui est entrepris sur le corps humain ou animal étant exclu.

2. Procédé selon la revendication 1, dans lequel l'extension est formée de 1 ou 2 nucléotides.

3. Procédé selon l'une des revendications précédentes, dans lequel au moins un autre acide ribonucléique double brin (ARNdb II) réalisé de façon correspondante à l'ARNdb I selon l'une des revendications précédentes, est introduit dans la cellule, où un brin (as1) ou au moins un segment d'un brin (as1) de l'ARNdb I est complémentaire d'une première zone (B1) du gène cible, et où un autre brin (as2) ou au moins un segment de l'autre brin (as2) de l'ARNdb II est complémentaire d'une seconde zone (B2) du gène cible.

4. Procédé selon l'une des revendications précédentes, dans lequel l'ARNdb I et/ou l'ARNdb II présente(nt) une longueur de moins de 25, de préférence de 19 à 23, paires de nucléotides successives.

5. Procédé selon la revendication 3 ou 4, dans lequel la première (B1) et la seconde (B2) zones se chevauchent par segments ou sont contiguës.

6. Procédé selon la revendication 3 ou 4, dans lequel la première (B1) et la seconde (B2) zones sont distantes l'une de l'autre.

7. Procédé selon l'une des revendications précédentes, dans lequel le gène cible est sélectionné parmi le groupe suivant : oncogène, gène de cytokine, gène d'Id-protéine, gène de prion, gènes de molécules induisant l'angiogénèse, de molécules d'adhésion et de récepteurs de surfaces cellulaires, gènes de protéines qui participent à des processus métastasants et/ou invasifs, gènes de protéinases et de molécules régulant l'apoptose et le cycle cellulaire.

8. Procédé selon l'une des revendications précédentes, dans lequel on utilise en tant qu'ARNdb I/II un construct d'ARNdb combiné à partir des séquences antisens (as1/2) et sens (ss1/2) respectivement correspondantes des séquences SQ141 - 173.

9. Procédé selon l'une des revendications précédentes, dans lequel l'expression est inhibée selon le principe de l'interférence ARN.

10. Procédé selon l'une des revendications précédentes, dans lequel le gène cible est exprimé dans des organismes pathogènes.

11. Procédé selon l'une des revendications précédentes, dans lequel le gène cible est un constituant d'un virus ou d'un viroïde.

12. Procédé selon la revendication 11, dans lequel le virus est un virus ou un viroïde pathogène humain.

13. Procédé selon la revendication 11, dans lequel le virus ou le viroïde est un virus ou un viroïde pathogène animal.

14. Procédé selon l'une des revendications précédentes, dans lequel des nucléotides non appariés sont substitués par des thiophosphates de nucléoside,

15. Procédé selon l'une des revendications précédentes, dans lequel au moins une extrémité (E1, E2) de l'ARNdb I/II est modifiée pour contrer une dégradation dans la cellule ou une dissociation dans les simples brins.

16. Procédé selon l'une des revendications précédentes, dans lequel la cohérence de la structure double brin, générée par les paires de nucléotides complémentaires, est augmentée par au moins une liaison chimique.

17. Procédé selon la revendication 16, dans lequel la liaison chimique est formée par une liaison covalente ou ionique, une liaison par pont d'hydrogène, des interactions hydrophobes, de préférence des forces de Van der Waals ou des interactions d'empilage, ou par coordination ion-métal.

18. Procédé selon la revendication 16 ou 17, dans lequel la liaison chimique est formée à proximité d'une extrémité (E1, E2).

19. Procédé selon l'une des revendications 16 à 18, dans lequel la liaison chimique est formée au moyen d'un ou de plusieurs groupes de liaison, les groupes de liaison étant de préférence des chaînes poly-(oxyphosphinicooxy-1,3-propanediol) et/ou oligoéthylèneglycol.

20. Procédé selon l'une des revendications 16 à 18, dans lequel la liaison chimique est formée par des analogues de nucléotides ramifiés utilisés à la place des nucléotides.

21. Procédé selon l'une des revendications 16 à 18, dans lequel la liaison chimique est formée par des analogues de purine.

22. Procédé selon l'une des revendications 16 à 18, dans lequel la liaison chimique est formée par des unités d'azabenzène.

23. Procédé selon l'une des revendications 16 à 18, dans lequel, pour réaliser la liaison chimique on utilise au moins un des groupes suivants : du bleu de méthylène ; des groupes bifonctionnels, de préférence de la bis-(2-chloroéthyl)-amine ; de la N-acétyl-N'-(p-glyoxyl-benzoyl)-cystamine ; du 4-thiouracile ; du psoralène.

24. Procédé selon l'une des revendications 16 à 18, dans lequel la liaison chimique est formée par des groupes thiophosphoryle fixés à proximité des extrémités (E1, E2) de la zone double brin.

25. Procédé selon l'une des revendications 16 à 18, dans lequel la liaison chimique est réalisée par des liaisons à triple hélice se trouvant à proximité des extrémités (E1, E2).

26. Procédé selon l'une des revendications précédentes, dans lequel l'ARNdb I/II est enfermé dans des structures micellaires, de façon avantageuse dans des liposomes.

27. Procédé selon l'une des revendications précédentes, dans lequel l'ARNdb I/II est lié, associé à ou entouré d'au moins une protéine d'enveloppe virale provenant d'un virus, en dérivant ou étant fabriquée par voie de synthèse.

28. Procédé selon la revendication 27, dans lequel la protéine d'enveloppe est dérivée du polyomavirus.

29. Procédé selon la revendication 27 ou 28, dans lequel la protéine d'enveloppe contient la protéine virale 1 (VP1) et/ou la protéine virale 2 (VP2) du polyomavirus.

30. Procédé selon l'une des revendications 27 à 29, dans lequel, lors de la formation d'une capside ou d'un produit de type capside à partir de la protéine d'enveloppe, un côté est orienté vers l'intérieur de la capside ou du produit de type capside.

31. Procédé selon l'une des revendications précédentes, dans lequel un brin (as1, as2) de l'ARNdb I/II est complémentaire du transcript d'ARN primaire ou traité du gène cible,

32. Procédé selon l'une des revendications précédentes, dans lequel la cellule est une cellule de vertébré ou une cellule humaine.

33. Procédé selon l'une des revendications précédentes, dans lequel l'ARNdb L'II est placé, pour l'application, dans une solution tampon.

34. Utilisation d'un acide ribonucléique double brin (ARNdb I) pour fabriquer un médicament,
où l'ARNdb I possède une structure double brin formée d'au plus 49 paires de nucléotides successives, et où un brin (as1) ou au moins un segment d'un brin (as1) de la structure double brin est complémentaire d'un gène cible dans une cellule,
l'ARNdb I présentant une extension formée de 1 à 4 nucléotides au niveau de l'extrémité 3' d'un brin (as1) et l'ARNdb 1 étant formé de façon lisse au niveau d'une extrémité (E1, E2) contenant l'extrémité 5' d'un brin (as1), le gène cible étant le gène MDR1 ou présentant une des séquences SQ001 à SQ140.

35. Utilisation selon la revendication 34, dans laquelle l'extension est formée de 1 ou 2 nucléotides.

36. Utilisation selon la revendication 34 ou 35, dans laquelle on utilise pour fabriquer le médicament, au moins un autre acide ribonucléique double brin (ARNdb II) réalisé de façon correspondante à l'ARNdb 1 selon l'une des revendications 34 ou 35,
un brin (as1) ou au moins un segment d'un brin (as1) de l'ARN'db I étant complémentaire à une première zone (B1) du brin sens du gène cible, et
un autre brin (as2) ou au moins un segment de l'autre brin (as2) de l'ARNdb II étant complémentaire d'une seconde zone (B2) du gène cible.

37. Utilisation selon l'une des revendications 34 à 36, dans laquelle l'ARNdb I et/ou l'ARNdb II présente(nt) une longueur de moins de 25, de préférence de 19 à 23, paires de nucléotides successives.

38. Utilisation selon la revendication 36 ou 37, dans laquelle la première (B1) et la seconde (B2) zones se chevauchent par segments ou sont contiguës.

39. Utilisation selon la revendication 36 ou 37, dans laquelle la première (B1) et la seconde (B2) zones sont distantes l'une de l'autre.

40. Utilisation selon l'une des revendications 34 à 39, dans laquelle le gène cible est sélectionné parmi le groupe suivant : oncogène, gène de cytokine, gène d'Id-protéine, gène de prion, gènes de molécules induisant l'angiogénèse, de molécules d'adhésion et de récepteurs de surfaces cellulaires, gènes de protéines qui participent à des processus métastasants et/ou invasifs, gènes de protéinases et molécules régulant l'apoprose et le cycle cellulaire.

41. Utilisation selon l'une des revendications 34 à 40, dans laquelle on utilise en tant qu'ARNdb I/II un construct d'ARNdb combiné à partir de séquences antisens (as 1/2) et sens (ss1/2) respectivement correspondantes des séquences SQ141 - 173.

42. Utilisation selon l'une des revendications 34 à 41, dans laquelle l'expression est inhibée selon le principe de l'interférence ARN.

43. Utilisation selon l'une des revendications 34 à 42, dans laquelle le gène cible est exprimé dans des organismes pathogènes.

44. Utilisation selon l'une des revendications 34 à 43, dans laquelle le gène cible est un constituant d'un virus ou d'un viroïde.

45. Utilisation selon la revendication 44, dans laquelle le virus est un virus ou un viroïde pathogène humain.

46. Utilisation selon la revendication 44, dans laquelle le virus ou le viroïde est un virus ou un viroïde pathogène animal.

47. Utilisation selon l'une des revendications 34 à 46, dans laquelle des nucléotides non appariés sont substitués par des thiophosphates de nucléosides.

48. Utilisation selon l'une des revendications 34 à 47, dans laquelle au moins une extrémité (E1, E2) de l'ARNdb est modifiée pour contrer une dégradation dans la cellule ou une dissociation dans les simples brins.

49. Utilisation selon l'une des revendications 34 à 48, dans laquelle la cohérence de la structure double brin, générée par les paires de nucléotides complémentaires, est augmentée par au moins une liaison chimique,

50. Utilisation selon la revendication 49, dans laquelle la liaison chimique est formée par une liaison covalente ou ionique, une liaison par pont d'hydrogène, des interactions hydrophobes, de préférence des forces de Van der Waals ou des interactions d'empilage, ou par coordination ion-métal.

51. Utilisation selon la revendication 49 ou 50, dans laquelle la liaison chimique est formée à proximité d'une extrémité (E1, E2),

52. Utilisation selon l'une des revendications 49 à 51, dans laquelle la liaison chimique est formée au moyen d'un ou de plusieurs groupes de liaison, les groupes de liaison étant de préférence des chaînes poly-(oxyphosphinicooxy-1,3-propanediol) et/ou oligoéthylèneglycol.

53. Utilisation selon l'une des revendications 49 à 51, dans laquelle la liaison chimique est formée par des analogues de nucléotides ramifiés utilisés à la place des nucléotides.

54. Utilisation selon l'une des revendications 49 à 51, dans laquelle la liaison chimique est formée par des analogues de purine.

55. Utilisation selon l'une des revendications 49 à 51, dans laquelle la liaison chimique est formée par des unités d'azabenzène.

56. Utilisation selon l'une des revendications 49 à 51, dans laquelle, pour réaliser la liaison chimique, on utilise au moins un des groupes suivants : du bleu de méthylène ; des groupes bifonctionnels, de préférence de la bis-(2-chloroéthyl)-amine ; de la N-acétyl-N'-(p-glyoxyl-benzoyl)-cystamine ; du 4-thiouracile ; du psoralène.

57. Utilisation selon l'une des revendications 49 à 51, dans laquelle la liaison chimique est formée par des groupes thiophospheryle fixés à proximité des extrémités (E1, E2) de la zone double brin.

58. Utilisation selon l'une des revendications 49 à 51, dans laquelle la liaison chimique est réalisée par des liaisons à triple hélice se trouvant à proximité des extrémités (E1, E2).

59. Utilisation selon l'une des revendications 34 à 58, dans laquelle l'ARNdb I/II est enfermé dans des structures micellaires, de façon avantageuse dans des liposomes.

60. Utilisation selon l'une des revendications 34 à 59, dans laquelle l'ARNdb I/II est lié, associé à ou entouré d'au moins une protéine d'enveloppe virale provenant d'un virus, en dérivant ou étant fabriquée par voie de synthèse.

61. Utilisation selon la revendication 60, dans laquelle la protéine d'enveloppe est dérivée du polyomavirus.

62. Utilisation selon la revendication 60 ou 61, dans laquelle la protéine d'enveloppe contient la protéine virale 1 (VP1) et/ou la protéine virale 2 (VP2) du polyomavirus.

63. Utilisation selon l'une des revendications 60 à 62, dans laquelle, lors de la formation d'une capside ou d'un produit de type capside à partir de la protéine d'enveloppe, un côté est orienté vers l'intérieur de la capside ou du produit de type capside.

64. Utilisation selon l'une des revendications 34 à 63, dans laquelle un brin (as1, as2) de l'ARNdb I/II est complémentaire du transcript d'ARN primaire ou traité du gène cible.

65. Utilisation selon l'une des revendications 34 à 64, dans laquelle la cellule est une cellule de vertébré ou une cellule humaine.

66. Utilisation selon l'une des revendications 34 à 65, dans laquelle le médicament est approprié pour administrer l'ARNdb I/II à un mammifère, de préférence un homme, dans une quantité au plus égale à 5 mg par kg de masse corporelle et par jour.

67. Utilisation selon l'une des revendications 34 à 66, dans laquelle l'ARNdb I/II est placé, pour l'application, dans une solution tampon,

68. Utilisation selon l'une des revendications 34 à 67, dans laquelle le médicament est approprié pour administrer l'ARNdb I/II par voie orale, par inhalation, ou au moyen d'une injection ou d'une perfusion par voie intraveineuse, intratumorale ou intrapéritonéale.

69. Médicament destiné à inhiber l'expression d'un gène cible dans une cellule, contenant un acide ribonucléique double brin (ARNdb I) dans une quantité suffisante pour inhiber l'expression du gène cible,
l'ARNdb I possédant une structure double brin formée par au plus 49 paires de nucléotides successives,
un brin (as1) ou au moins un segment d'un brin (as1) de la structure double brin étant complémentaire du gène cible,
l'ARNdb I présentant une extension formée de 1 à 4 nucléotides au niveau de l'extrémité 3' d'un brin (as1) et l'ARNdb 1 étant formé de façon lisse au niveau d'une extrémité (E1, E2) contenant l'extrémité 5' d'un brin (as1), le gène cible étant le gène MDR1 ou présentant une des séquences SQ001 à SQ140.

70. Médicament selon la revendication 69, dans lequel l'extension est formée de 1 ou 2 nucléotides.

71. Médicament selon l'une des revendications 69 ou 70, contenant au moins un autre acide ribonucléique double brin (ARNdb II) réalisé de façon correspondante à l'ARNdb I selon l'une des revendications 69 ou 70, un brin (as1) ou au moins un segment d'un brin (as1) de l'ARNdb I étant complémentaire d'une première zone (B1) du gène cible, et un autre brin (as2) ou au moins un segment de l'autre brin (as2) de l'ARNdb II étant complémentaire d'une seconde zone (B2) du gène cible.

72. Médicament selon l'une des revendications 69 à 71, dans lequel l'ARNdb I et/ou l'ARNdb II présente(nt) une longueur de moins de 25, de préférence de 19 à 23, paires de nucléotides successives.

73. Médicament selon l'une des revendications 71 ou 72, dans lequel la première (B1) et la seconde (B2) zones se chevauchent par segments ou sont contiguës.

74. Médicament selon l'une des revendications 69 à 73, dans lequel le gène cible est sélectionné parmi le groupe suivant : oncogène, gène de cytokine, gène d'Id-protéine, gène de prion, gènes de molécules induisant l'angiogénèse, de molécules d'adhésion et de récepteurs de surfaces cellulaires, gènes de protéines qui participent à des processus métastasants et/ou invasifs, gènes de protéinases et molécules régulant l'apoptose et le cycle cellulaire.

75. Médicament selon l'une des revendications 69 à 74, dans lequel on utilise en tant qu'ARNdb un construct d'ARNdb combiné à partir des séquences antisens (as1/2) et sens (ss1/2) respectivement correspondantes des séquences SQ141-173.

76. Médicament selon l'une des revendications 69 à 75, dans lequel l'expression est inhibée selon le principe de l'interférence ARN.

77. Médicament selon l'une des revendications 69 à 76, dans lequel le gène cible peut être exprimé dans des organismes pathogènes.

78. Médicament selon l'une des revendications 69 à 77, dans lequel le gène cible est un constituant d'un virus ou d'un viroïde.

79. Médicament selon la revendication 78, dans lequel le virus est un virus ou un viroïde pathogène humain.

80. Médicament selon la revendication 78, dans lequel le virus ou le viroïde est un virus ou un viroïde pathogène animal.

81. Médicament selon l'une des revendications 69 à 80, dans lequel des nucléotides non appariés sont substitués par des thiophosphates de nucléosides,

82. Médicament selon l'une des revendications 69 à 81, dans lequel au moins une extrémité (E1, E2) de l'ARNdb est modifiée pour contrer une dégradation dans la cellule ou une dissociation dans les simples brins.

83. Médicament selon l'une des revendications 69 à 82, dans lequel la cohérence de la structure double brin, générée par les paires de nucléotides complémentaires, est augmentée par au moins une liaison chimique.

84. Médicament selon la revendication 83, dans lequel la liaison chimique est formée par une liaison covalente ou ionique, une liaison par pont d'hydrogène, des interactions hydrophobes, de préférence des forces de Van der Waals ou des interactions d'empilage, ou par coordination ion-métal.

85. Médicament selon la revendication 83 ou 84, dans lequel la liaison chimique est formée à proximité d'une extrémité (E1, E2),

86. Médicament selon l'une des revendications 83 à 85, dans lequel la liaison chimique est formée au moyen d'un ou de plusieurs groupes de liaison, les groupes de liaison étant de préférence des chaînes poly-(oxyphosphinicooxy-1,3-propanediol) et/ou oligoéthylèneglycol.

87. Médicament selon l'une des revendications 83 à 85, dans lequel la liaison chimique est formée par des analogues de nucléotides ramifiés utilisés à la place des nucléotides.

88. Médicament selon l'une des revendications 83 à 85, dans lequel la liaison chimique est formée par des analogues de purine.

89. Médicament selon l'une des revendications 83 à 85, dans lequel la liaison chimique est formée par des unités d'azabenzène.

90. Médicament selon l'une des revendications 83 à 85, dans lequel, pour réaliser la liaison chimique, on utilise au moins un des groupes suivants : du bleu de méthylène ; des groupes bifonctionnels, de préférence de la bis-(2-chloroéthyl)-amine ; de la N-acétyl-N'-(p-glyoxyl-benzoyl)-cystamine ; du 4-thiouracile ; du psoralène.

91. Médicament selon l'une des revendications 83 à 85, dans lequel la liaison chimique est formée par des groupes thiophosphotyle fixés à proximité des extrémités (E1, E2) de la zone double brin.

92. Médicament selon l'une des revendications 83 à 85, dans lequel la liaison chimique est réalisée par des liaisons à triple hélice se trouvant à proximité des extrémités (E1, E2).

93. Médicament selon l'une des revendications 69 à 92, dans lequel l'ARNdb I/II est enfermé dans des structures micellaires, de façon avantageuse dans des liposomes.

94. Médicament selon l'une des revendications 69 à 93, dans lequel l'ARNdb I est lié, associé à ou entouré de au moins une protéine d'enveloppe virale provenant d'un virus, en dérivant ou étant fabriquée par voie de synthèse.

95. Médicament selon la revendication 94, dans lequel la protéine d'enveloppe est dérivée d'un polyomavirus.

96. Médicament selon la revendication 94 ou 95, dans lequel la protéine d'enveloppe contient la protéine virale 1 (VP1) et/ou la protéine virale 2 (VP2) du polyomavirus.

97. Médicament selon l'une des revendications 94 à 96, dans lequel, lors de la formation d'une capside ou d'un produit de type capside à partir de la protéine d'enveloppe, un côté est orienté vers l'intérieur de la capside ou du produit de type capside.

98. Médicament selon l'une des revendications 69 à 97, dans lequel un brin (as1, as2) de l'ARNdb I est complémentaire du transcript d'ARN primaire ou traité du gène cible.

99. Médicament selon l'une des revendications 69 à 98, dans lequel la cellule est une cellule de vertébré ou une cellule humaine.

100. Médicament selon l'une des revendications 71, 72 ou 74 à 99, dans lequel la première (B1) et la seconde (B2) zones sont distantes l'une de l'autre.

101. Médicament selon l'une des revendications 69 à 100, dans lequel l'ARNdb est contenu dans une quantité au plus égale à 5 mg par dose administrable.

102. Médicament selon l'une des revendications 69 à 101, dans lequel l'ARNdb est placé dans une solution tampon.

103. Médicament selon l'une des revendications 69 à 102, dans lequel l'ARNdb peut être administré par vote orale, par inhalation, ou au moyen d'une injection ou d'une perfusion par voie intraveineuse, intratumorale ou intrapéritonéale,


### SEQUENZPROTOKOLL

<110> Ribopharma AG
<120> Verfahren zur Hemmung der Expression eines Zielgens
<130>
<140>
   <141>
<160> 142
<170> PatentIn Ver. 2.1
<210> 1
   <221> 2955
   <212> DNA
   <213> Homo sapiens
<300>
   <302> Eph A1
   <310> NM00532
<300>
   <302> ephrin A1
   <310> NM00532
<400> 1
<210> 2
   <211> 3042
   <212> DNA
   <213> Homo sapiens
<300>
   <302> ephrin A2
   <310> XM002088
<400> 2
   gaagttgcgc gcaggccggc gggcgggagc ggacaccgag gccggcgtgc aggcgtgcgg 60
   gtgtgcggga gccgggctcg gggggatcgg accgagagcg agaagcgcgg catggagctc 120
   caggcagccc gcgcctgctt cgccctgctg tggggctgtg cgctggccgc ggccgcggcg 180
   gcgcagggca aggaagtggt actgctggac tttgctgcag ctggagggga gctcggctgg 240
   ctcacacacc cgtatggcaa agggtgggac ctgatgcaga acatcatgaa tgacatgccg 300
   atctacatgt actccgtgtg caacgtgatg tctggcgacc aggacaactg gctccgcacc 360
   aactgggtgt accgaggaga ggctgagcgt atcttcattg agctcaagtt tactgtacgt 420
   gactgcaaca gcttccctgg tggcgccagc tcctgcaagg agactttcaa cctctactat 480
   gccgagtcgg acctggacta cggcaccaac ttccagaagc gcctgttcac caagattgac 540
   accattgcgc ccgatgagat caccgtcagc agcgacttcg aggcacgcca cgtgaagctg 600
   aacgtggagg agcgctccgt ggggccgctc acccgcaaag gcttctacct ggccttccag 660
   gatatcggtg cctgtgtggc gctgctctcc gtccgtgtct actacaagaa gtgccccgag 720
   ctgctgcagg gcctggccca cttccctgag accatcgccg gctctgatgc accttccctg 780
   gccactgtgg ccggcacctg tgtggaccat gccgtggtgc caccgggggg tgaagagccc 840
   cgtatgcact gtgcagtgga tggcgagtgg ctggtgccca ttgggcagtg cctgtgccag 900
   gcaggctacg agaaggtgga ggatgcctgc caggcctgct cgcctggatt ttttaagttt 960
   gaggcatctg agagcccctg cttggagtgc cctgagcaca cgctgccatc ccctgagggt 1020
   gccacctcct gcgagtgtga ggaaggcttc ttccgggcac ctcaggaccc agcgtcgatg 1080
   ccttgcacac gacccccctc cgccccacac tacctcacag ccgtgggcat gggtgccaag 1140
   gtggagctgc gctggacgcc ccctcaggac agcgggggcc gcgaggacat tgtctacagc 1200
   gtcacctgcg aacagtgctg gcccgagtct ggggaatgcg ggccgtgtga ggccagtgtg 1260
   cgctactcgg agcctcctca cggactgacc cgcaccagtg tgacagtgag cgacctggag 1320
   ccccacatga actacacctt caccgtggag gcccgcaatg gcgtctcagg cctggtaacc 1380
   agccgcagct tccgtactgc cagtgtcagc atcaaccaga cagagccccc caaggtgagg 1440
   ctggagggcc gcagcaccac ctcgcttagc gtctcctgga gcatcccccc gccgcagcag 1500
   agccgagtgt ggaagtacga ggtcacttac cgcaagaagg gagactccaa cagctacaat 1560
   gtgcgccgca ccgagggttt ctccgtgacc ctggacgacc tggccccaga caccacctac 1620
   ctggtccagg tgcaggcact gacgcaggag ggccaggggg ccggcagcaa ggtgcacgaa 1680
   ttccagacgc tgtccccgga gggatctggc aacttggcgg tgattggcgg cgtggctgtc 1740
   ggtgtggtcc tgcttctggt gctggcagga gttggcttct ttatccaccg caggaggaag 1800
   aaccagcgtg cccgccagtc cccggaggac gtttacttct ccaagtcaga acaactgaag 1860
   cccctgaaga catacgtgga cccccacaca tatgaggacc ccaaccaggc tgtgttgaag 1920
   ttcactaccg agatccatcc atcctgtgtc actcggcaga aggtgatcgg agcaggagag 1980
   tttggggagg tgtacaaggg catgctgaag acatcctcgg ggaagaagga ggtgccggtg 2040
   gccatcaaga cgctgaaagc cggctacaca gagaagcagc gagtggactt cctcggcgag 2100
   gccggcatca tgggccagtt cagccaccac aacatcatcc gcctagaggg cgtcatctcc 2160
   aaatacaagc ccatgatgat catcactgag tacatggaga atggggccct ggacaagttc 2220
   cttcgggaga aggatggcga gttcagcgtg ctgcagctgg tgggcatgct gcggggcatc 2280
   gcagctggca tgaagtacct ggccaacatg aactatgtgc accgtgacct ggctgcccgc 2340
   aacatcctcg tcaacagcaa cctggtctgc aaggtgtctg actttggcct gtcccgcgtg 2400
   ctggaggacg accccgaggc cacctacacc accagtggcg gcaagatccc catccgctgg 2460
   accgccccgg aggccatttc ctaccggaag ttcacctctg ccagcgacgt gtggagcttt 2520
   ggcattgtca tgtgggaggt gatgacctat ggcgagcggc cctactggga gttgtccaac 2580
   cacgaggtga tgaaagccat caatgatggc ttccggctcc ccacacccat ggactgcccc 2640
   tccgccatct accagctcat gatgcagtgc tggcagcagg agcgtgcccg ccgccccaag 2700
   ttcgctgaca tcgtcagcat cctggacaag ctcattcgtg cccctgactc cctcaagacc 2760
   ctggctgact ttgacccccg cgtgtctatc cggctcccca gcacgagcgg ctcggagggg 2820
   gtgcccttcc gcacggtgtc cgagtggctg gagtccatca agatgcagca gtatacggag 2880
   cacttcatgg cggccggcta cactgccatc gagaaggtgg tgcagatgac caacgacgac 2940
   atcaagagga ttggggtgcg gctgcccggc caccagaagc gcatcgccta cagcctgctg 3000
   ggactcaagg accaggtgaa cactgtgggg atccccatct ga                    3042
<210> 3
   <211> 2953
   <212> DNA
   <213> Homo sapiens
<300>
   <302> ephrin A3
   <310> NM005233
<400> 3
   atggattgtc agctctccat cctcctcctt ctcagctgct ctgttctcga cagcttcggg 60
   gaactgattc cgcagccttc caatgaagtc aatctactgg attcaaaaac aattcaaggg 120
   gagctgggct ggatctctta tccatcacat gggtgggaag agatcagtgg tgtggatgaa 180
   cattacacac ccatcaggac ttaccaggtg tgcaatgtca tggaccacag tcaaaacaat 240
   tggctgagaa caaactgggt ccccaggaac tcagctcaga agatttatgt ggagctcaag 300
   ttcactctac gagactgcaa tagcattcca ttggttttag gaacttgcaa ggagacattc 360
   aacctgtact acatggagtc tgatgatgat catggggtga aatttcgaga gcatcagttt 420
   acaaagattg acaccattgc agctgatgaa agtttcactc aaatggatct tggggaccgt 480
   attctgaagc tcaacactga gattagagaa gtaggtcctg tcaacaagaa gggattttat 540
   ttggcatttc aagatgttgg tgcttgtgtt gccttggtgt ctgtgagagt atacttcaaa 600
   aagtgcccat ttacagtgaa gaatctggct atgtttccag acacggtacc catggactcc 660
   cagtccctgg tggaggttag agggtcttgt gtcaacaatt ctaaggagga agatcctcca 720
   aggatgtact gcagtacaga aggcgaatgg cttgtaccca ttggcaagtg ttcctgcaat 780
   gctggctatg aagaaagagg ttttatgtgc caagcttgtc gaccaggttt ctacaaggca 840
   ttggatggta atatgaagtg tgctaagtgc ccgcctcaca gttctactca ggaagatggt 900
   tcaatgaact gcaggtgtga gaataattac ttccgggcag acaaagaccc tccatccatg 960
   gcttgtaccc gacctccatc ttcaccaaga aatgttatct ctaatataaa cgagacctca 1020
   gttatcctgg actggagttg gcccctggac acaggaggcc ggaaagatgt taccttcaac 1080
   atcatatgta aaaaatgtgg gtggaatata aaacagtgtg agccatgcag cccaaatgtc 1140
   cgcttcctcc ctcgacagtt tggactcacc aacaccacgg tgacagtgac agaccttctg 1200
   gcacatacta actacacctt tgagattgat gccgttaatg gggtgtcaga gctgagctcc 1260
   ccaccaagac agtttgctgc ggtcagcatc acaactaatc aggctgctcc atcacctgtc 1320
   ctgacgatta agaaagatcg gacctccaga aatagcatct ctttgtcctg gcaagaacct 1380
   gaacatccta atgggatcat attggactac gaggtcaaat actatgaaaa gcaggaacaa 1440
   gaaacaagtt ataccattct gagggcaaga ggcacaaatg ttaccatcag tagcctcaag 1500
   cctgacacta tatacgtatt ccaaatccga gcccgaacag ccgctggata tgggacgaac 1560
   agccgcaagt ttgagtttga aactagtcca gactctttct ccatctctgg tgaaagtagc 1620
   caagtggtca tgatcgccat ttcagcggca gtagcaatta ttctcctcac tgttgtcatc 1680
   tatgttttga ttgggaggtt ctgtggctat aagtcaaaac atggggcaga tgaaaaaaga 1740
   cttcattttg gcaatggcca tttaaaactt ccaggtctca ggacttatgt tgacccacat 1800
   acatatgaag accctaccca agctgttcat gagtttgcca aggaattgga tgccaccaac 1860
   atatccattg ataaagttgt tggagcaggt gaatttggag aggtgtgcag tggtcgctta 1920
   aaacttcctt caaaaaaaga gatttcagtg gccattaaaa ccctgaaagt tggctacaca 1980
   gaaaagcaga ggagagactt cctgggagaa gcaagcatta tgggacagtt tgaccacccc 2040
   aatatcattc gactggaagg agttgttacc aaaagtaagc cagttatgat tgtcacagaa 2100
   tacatggaga atggttcctt ggatagtttc ctacgtaaac acgatgccca gtttactgtc 2160
   attcagctag tggggatgct tcgagggata gcatctggca tgaagtacct gtcagacatg 2220
   ggctatgttc accgagacct cgctgctcgg aacatcttga tcaacagtaa cttggtgtgt 2280
   aaggtttctg atttcggact ttcgcgtgtc ctggaggatg acccagaagc tgcttataca 2340
   acaagaggag ggaagatccc aatcaggtgg acatcaccag aagctatagc ctaccgcaag 2400
   ttcacgtcag ccagcgatgt atggagttat gggattgttc tctgggaggt gatgtcttat 2460
   ggagagagac catactggga gatgtccaat caggatgtaa ttaaagctgt agatgagggc 2520
   tatcgactgc caccccccat ggactgccca gctgccttgt atcagctgat gctggactgc 2580
   tggcagaaag acaggaacaa cagacccaag tttgagcaga ttgttagtat tctggacaag 2640
   cttatccgga atcccggcag cctgaagatc atcaccagtg cagccgcaag gccatcaaac 2700
   cttcttctgg accaaagcaa tgtggatatc tctaccttcc gcacaacagg tgactggctt 2760
   aatggtgtcc ggacagcaca ctgcaaggaa atcttcacgg gcgtggagta cagttcttgt 2820
   gacacaatag ccaagatttc cacagatgac atgaaaaagg ttggtgtcac cgtggttggg 2880
   ccacagaaga agatcatcag tagcattaaa gctctagaaa cgcaatcaaa gaatggccca 2940
   gttcccgtgt aaa                                                    2953
<210> 4
   <211> 2784
   <212> DNA
   <213> Homo sapiens
<300>
   <302> ephrin A4
   <310> XM002578
<400> 4
   atggatgaaa aaaatacacc aatccgaacc taccaagtgt gcaatgtgat ggaacccagc 60
   cagaataact ggctacgaac tgattggatc acccgagaag gggctcagag ggtgtatatt 120
   gagattaaat tcaccttgag ggactgcaat agtcttccgg gcgtcatggg gacttgcaag 180
   gagacgttta acctgtacta ctatgaatca gacaacgaca aagagcgttt catcagagag 240
   aaccagtttg tcaaaattga caccattgct gctgatgaga gcttcaccca agtggacatt 300
   ggtgacagaa tcatgaagct gaacaccgag atccgggatg tagggccatt aagcaaaaag 360
   gggttttacc tggcttttca ggatgtgggg gcctgcatcg ccctggtatc agtccgtgtg 420
   ttctataaaa agtgtccact cacagtccgc aatctggccc agtttcctga caccatcaca 480
   ggggctgata cgtcttccct ggtggaagtt cgaggctcct gtgtcaacaa ctcagaagag 540
   aaagatgtgc caaaaatgta ctgtggggca gatggtgaat ggctggtacc cattggcaac 600
   tgcctatgca acgctgggca tgaggagcgg agcggagaat gccaagcttg caaaattgga 660
   tattacaagg ctctctccac ggatgccacc tgtgccaagt gcccacccca cagctactct 720
   gtctgggaag gagccacctc gtgcacctgt gaccgaggct ttttcagagc tgacaacgat 780
   gctgcctcta tgccctgcac ccgtccacca tctgctcccc tgaacttgat ttcaaatgtc 840
   aacgagacat ctgtgaactt ggaatggagt agccctcaga atacaggtgg ccgccaggac 900
   atttcctata atgtggtatg caagaaatgt ggagctggtg accccagcaa gtgccgaccc 960
   tgtggaagtg gggtccacta caccccacag cagaatggct tgaagaccac caaagtctcc 1020
   atcactgacc tcctagctca taccaattac acctttgaaa tctgggctgt gaatggagtg 1080
   tccaaatata accctaaccc agaccaatca gtttctgtca ctgtgaccac caaccaagca 1140
   gcaccatcat ccattgcttt ggtccaggct aaagaagtca caagatacag tgtggcactg 1200
   gcttggctgg aaccagatcg gcccaatggg gtaatcctgg aatatgaagt caagtattat 1260
   gagaaggatc agaatgagcg aagctatcgt atagttcgga cagctgccag gaacacagat 1320
   atcaaaggcc tgaaccctct cacttcctat gttttccacg tgcgagccag gacagcagct 1380
   ggctatggag acttcagtga gcccttggag gttacaacca acacagtgcc ttcccggatc 1440
   attggagatg gggctaactc cacagtcctt ctggtctctg tctcgggcag tgtggtgctg 1500
   gtggtaattc tcattgcagc ttttgtcatc agccggagac ggagtaaata cagtaaagcc 1560
   aaacaagaag cggatgaaga gaaacatttg aatcaaggtg taagaacata tgtggacccc 1620
   tttacgtacg aagatcccaa ccaagcagtg cgagagtttg ccaaagaaat tgacgcatcc 1680
   tgcattaaga ttgaaaaagt tataggagtt ggtgaatttg gtgaggtatg cagtgggcgt 1740
   ctcaaagtgc ctggcaagag agagatctgt gtggctatca agactctgaa agctggttat 1800
   acagacaaac agaggagaga cttcctgagt gaggccagca tcatgggaca gtttgaccat 1860
   ccgaacatca ttcacttgga aggcgtggtc actaaatgta aaccagtaat gatcataaca 1920
   gagtacatgg agaatggctc cttggatgca ttcctcagga aaaatgatgg cagatttaca 1980
   gtcattcagc tggtgggcat gcttcgtggc attgggtctg ggatgaagta tttatctgat 2040
   atgagctatg tgcatcgtga tctggccgca cggaacatcc tggtgaacag caacttggtc 2100
   tgcaaagtgt ctgattttgg catgtcccga gtgcttgagg atgatccgga agcagcttac 2160
   accaccaggg gtggcaagat tcctatccgg tggactgcgc cagaagcaat tgcctatcgt 2220
   aaattcacat cagcaagtga tgtatggagc tatggaatcg ttatgtggga agtgatgtcg 2280
   tacggggaga ggccctattg ggatatgtcc aatcaagatg tgattaaagc cattgaggaa 2340
   ggctatcggt taccccctcc aatggactgc cccattgcgc tccaccagct gatgctagac 2400
   tgctggcaga aggagaggag cgacaggcct aaatttgggc agattgtcaa catgttggac 2460
   aaactcatcc gcaaccccaa cagcttgaag aggacaggga cggagagctc cagacctaac 2520
   actgccttgt tggatccaag ctcccctgaa ttctctgctg tggtatcagt gggcgattgg 2580
   ctccaggcca ttaaaatgga ccggtataag gataacttca cagctgctgg ttataccaca 2640
   ctagaggctg tggtgcacgt gaaccaggag gacctggcaa gaattggtat cacagccatc 2700
   acgcaccaga ataagatttt gagcagtgtc caggcaatgc gaacccaaat gcagcagatg 2760
   cacggcagaa tggttcccgt ctga                                        2784
<210> 5
   <211> 2997
   <212> DNA
   <213> Homo sapiens
<300>
   <302> ephrin A7
   <310> XM004485
<400> 5
   atggtttttc aaactcggta cccttcatgg attattttat gctacatctg gctgctccgc 60
   tttgcacaca caggggaggc gcaggctgcg aaggaagtac tactgctgga ttctaaagca 120
   caacaaacag agttggagtg gatttcctct ccacccaatg ggtgggaaga aattagtggt 180
   ttggatgaga actatacccc gatacgaaca taccaggtgt gccaagtcat ggagcccaac 240
   caaaacaact ggctgcggac taactggatt tccaaaggca atgcacaaag gatttttgta 300
   gaattgaaat tcaccctgag ggattgtaac agtcttcctg gagtactggg aacttgcaag 360
   gaaacattta atttgtacta ttatgaaaca gactatgaca ctggcaggaa tataagagaa 420
   aacctctatg taaaaataga caccattgct gcagatgaaa gttttaccca aggtgacctt 480
   ggtgaaagaa agatgaagct taacactgag gtgagagaga ttggaccttt gtccaaaaag 540
   ggattctatc ttgcctttca ggatgtaggg gcttgcatag ctttggtttc tgtcaaagtg 600
   tactacaaga agtgctggtc cattattgag aacttagcta tctttccaga tacagtgact 660
   ggttcagaat tttcctcttt agtcgaggtt cgagggacat gtgtcagcag tgcagaggaa 720
   gaagcggaaa acgcccccag gatgcactgc agtgcagaag gagaatggtt agtgcccatt 780
   ggaaaatgta tctgcaaagc aggctaccag caaaaaggag acacttgtga accctgtggc 840
   cgtgggttct acaagtcttc ctctcaagat cttcagtgct ctcgttgtcc aactcacagt 900
   ttttctgata aagaaggctc ctccagatgt gaatgtgaag atgggtatta cagggctcca 960
   tctgacccac catacgttgc atgcacaagg cctccatctg caccacagaa cctcattttc 1020
   aacatcaacc aaaccacagt aagtttggaa tggagtcctc ctgcagacaa tgggggaaga 1080
   aacgatgtga cctacagaat attgtgtaag cggtgcagtt gggagcaggg cgaatgtgtt 1140
   ccctgtggga gtaacattgg atacatgccc cagcagactg gattagagga taactatgtc 1200
   actgtcatgg acctgctagc ccacgctaat tatacttttg aagttgaagc tgtaaatgga 1260
   gtttctgact taagccgatc ccagaggctc tttgctgctg tcagtatcac cactggtcaa 1320
   gcagctccct cgcaagtgag tggagtaatg aaggagagag tactgcagcg gagtgtcgag 1380
   ctttcctggc aggaaccaga gcatcccaat ggagtcatca cagaatatga aatcaagtat 1440
   tacgagaaag atcaaaggga acggacctac tcaacagtaa aaaccaagtc tacttcagcc 1500
   tccattaata atctgaaacc aggaacagtg tatgttttcc agattcgggc ttttactgct 1560
   gctggttatg gaaattacag tcccagactt gatgttgcta cactagagga agctacaggt 1620
   aaaatgtttg aagctacagc tgtctccagt gaacagaatc ctgttattat cattgctgtg 1680
   gttgctgtag ctgggaccat cattttggtg ttcatggtct ttggcttcat cattgggaga 1740
   aggcactgtg gttatagcaa agctgaccaa gaaggcgatg aagagcttta ctttcatttt 1800
   aaatttccag gcaccaaaac ctacattgac cctgaaacct atgaggaccc aaatagagct 1860
   gtccatcaat tcgccaagga gctagatgcc tcctgtatta aaattgagcg tgtgattggt 1920
   gcaggagaat tcggtgaagt ctgcagtggc cgtttgaaac ttccagggaa aagagatgtt 1980
   gcagtagcca taaaaaccct gaaagttggt tacacagaaa aacaaaggag agactttttg 2040
   tgtgaagcaa gcatcatggg gcagtttgac cacccaaatg ttgtccattt ggaaggggtt 2100
   gttacaagag ggaaaccagt catgatagta atagagttca tggaaaatgg agccctagat 2160
   gcatttctca ggaaacatga tgggcaattt acagtcattc agttagtagg aatgctgaga 2220
   ggaattgctg ctggaatgag atatttggct gatatgggat atgttcacag ggaccttgca 2280
   gctcgcaata ttcttgtcaa cagcaatctc gtttgtaaag tgtcagattt tggcctgtcc 2340
   cgagttatag aggatgatcc agaagctgtc tatacaacta ctggtggaaa aattccagta 2400
   aggtggacag cacccgaagc catccagtac cggaaattca catcagccag tgatgtatgg 2460
   agctatggaa tagtcatgtg ggaagttatg tcttatggag aaagacctta ttgggacatg 2520
   tcaaatcaag atgttataaa agcaatagaa gaaggttatc gtttaccagc acccatggac 2580
   tgcccagctg gccttcacca gctaatgttg gattgttggc aaaaggagcg tgctgaaagg 2640
   ccaaaatttg aacagatagt tggaattcta gacaaaatga ttcgaaaccc aaatagtctg 2700
   aaaactcccc tgggaacttg tagtaggcca ataagccctc ttctggatca aaacactcct 2760
   gatttcacta ccttttgttc agttggagaa tggctacaag ctattaagat ggaaagatat 2820
   aaagataatt tcacggcagc tggctacaat tcccttgaat cagtagccag gatgactatt 2880
   gaggatgtga tgagtttagg gatcacactg gttggtcatc aaaagaaaat catgagcagc 2940
   attcagacta tgagagcaca aatgctacat ttacatggaa ctggcattca agtgtga 2997
<210> 6
   <211> 3217
   <212> DNA
   <213> Homo sapiens
<300>
   <302> ephrin A8
   <310> XM001921
<400> 6
<210> 7
   <211> 1497
   <212> DNA
   <213> Homo sapiens
<300>
   <308> U83508
<300>
   <302> angiopoietin 2
   <310> U83508
<400> 7
   atgacagttt tcctttcctt tgctttcctc gctgccattc tgactcacat agggtgcagc 60
   aatcagcgcc gaagtccaga aaacagtggg agaagatata accggattca acatgggcaa 120
   tgtgcctaca ctttcattct tccagaacac gatggcaact gtcgtgagag tacgacagac 180
   cagtacaaca caaacgctct gcagagagat gctccacacg tggaaccgga tttctcttcc 240
   cagaaacttc aacatctgga acatgtgatg gaaaattata ctcagtggct gcaaaaactt 300
   gagaattaca ttgtggaaaa catgaagtcg gagatggccc agatacagca gaatgcagtt 360
   cagaaccaca cggctaccat gctggagata ggaaccagcc tcctctctca gactgcagag 420
   cagaccagaa agctgacaga tgttgagacc caggtactaa atcaaacttc tcgacttgag 480
   atacagctgc tggagaattc attatccacc tacaagctag agaagcaact tcttcaacag 540
   acaaatgaaa tcttgaagat ccatgaaaaa aacagtttat tagaacataa aatcttagaa 600
   atggaaggaa aacacaagga agagttggac accttaaagg aagagaaaga gaaccttcaa 660
   ggcttggtta ctcgtcaaac atatataatc caggagctgg aaaagcaatt aaacagagct 720
   accaccaaca acagtgtcct tcagaagcag caactggagc tgatggacac agtccacaac 780
   cttgtcaatc tttgcactaa agaaggtgtt ttactaaagg gaggaaaaag agaggaagag 840
   aaaccattta gagactgtgc agatgtatat caagctggtt ttaataaaag tggaatctac 900
   actatttata ttaataatat gccagaaccc aaaaaggtgt tttgcaatat ggatgtcaat 960
   gggggaggtt ggactgtaat acaacatcgt gaagatggaa gtctagattt ccaaagaggc 1020
   tggaaggaat ataaaatggg ttttggaaat ccctccggtg aatattggct ggggaatgag 1080
   tttatttttg ccattaccag tcagaggcag tacatgctaa gaattgagtt aatggactgg 1140
   gaagggaacc gagcctattc acagtatgac agattccaca taggaaatga aaagcaaaac 1200
   tataggttgt atttaaaagg tcacactggg acagcaggaa aacagagcag cctgatctta 1260
   cacggtgctg atttcagcac taaagatgct gataatgaca actgtatgtg caaatgtgcc 1320
   ctcatgttaa caggaggatg gtggtttgat gcttgtggcc cctccaatct aaatggaatg 1380
   ttctatactg cgggacaaaa ccatggaaaa ctgaatggga taaagtggca ctacttcaaa 1440
   gggcccagtt actccttacg ttccacaact atgatgattc gacctttaga tttttga 1497
<210> 8
   <211> 3417
   <212> DNA
   <213> Homo sapiens
<300>
   <310> XM001924
<300>
   <302> Tiel
<400> 8
   atggtctggc gggtgccccc tttcttgctc cccatcctct tcttggcttc tcatgtgggc 60
   gcggcggtgg acctgacgct gctggccaac ctgcggctca cggaccccca gcgcttcttc 120
   ctgacttgcg tgtctgggga ggccggggcg gggaggggct cggacgcctg ggcccgcccc 180
   ctgctgctgg agaaggacga ccgtatcgtg cgcaccccgc ccgggccacc cctgcgcctg 240
   gcgcgcaacg gttcgcacca ggtcacgctt cgcggcttct ccaagccctc ggacctcgtg 300
   ggcgtcttct cctgcgtggg cggtgctggg gcgcggcgca cgcgcgtcat ctacgtgcac 360
   aacagccctg gagcccacct gcttccagac aaggtcacac acactgtgaa caaaggtgac 420
   accgctgtac tttctgcacg tgtgcacaag gagaagcaga cagacgtgat ctggaagagc 480
   aacggatcct acttctacac cctggactgg catgaagccc aggatgggcg gttcctgctg 540
   cagctcccaa atgtgcagcc accatcgagc ggcatctaca gtgccactta cctggaagcc 600
   agccccctgg gcagcgcctt ctttcggctc atcgtgcggc gttgtggggc tgggcgctgg 660
   gggccaggct gtaccaagga gtgcccaggt tgcctacatg gaggtgtctg ccacgaccat 720
   gacggcgaat gtgtatgccc ccctggcttc actggcaccc gctgtgaaca ggcctgcaga 780
   gagggccgtt ttgggcagag ctgccaggag cagtgcccag gcatatcagg ctgccggggc 840
   ctcaccttct gcctcccaga cccctatggc tgctcttgtg gatctggctg gagaggaagc 900
   cagtgccaag aagcttgtgc ccctggtcat tttggggctg attgccgact ccagtgccag 960
   tgtcagaatg gtggcacttg tgaccggttc agtggttgtg tctgcccctc tgggtggcat 1020
   ggagtgcact gtgagaagtc agaccggatc ccccagatcc tcaacatggc ctcagaactg 1080
   gagttcaact tagagacgat gccccggatc aactgtgcag ctgcagggaa ccccttcccc 1140
   gtgcggggca gcatagagct acgcaagcca gacggcactg tgctcctgtc caccaaggcc 1200
   attgtggagc cagagaagac cacagctgag ttcgaggtgc cccgcttggt tcttgcggac 1260
   agtgggttct gggagtgccg tgtgtccaca tctggcggcc aagacagccg gcgcttcaag 1320
   gtcaatgtga aagtgccccc cgtgcccctg gctgcacctc ggctcctgac caagcagagc 1380
   cgccagcttg tggtctcccc gctggtctcg ttctctgggg atggacccat ctccactgtc 1440
   cgcctgcact accggcccca ggacagtacc atggactggt cgaccattgt ggtggacccc 1500
   agtgagaacg tgacgttaat gaacctgagg ccaaagacag gatacagtgt tcgtgtgcag 1560
   ctgagccggc caggggaagg aggagagggg gcctgggggc ctcccaccct catgaccaca 1620
   gactgtcctg agcctttgtt gcagccgtgg ttggagggct ggcatgtgga aggcactgac 1680
   cggctgcgag tgagctggtc cttgcccttg gtgcccgggc cactggtggg cgacggtttc 1740
   ctgctgcgcc tgtgggacgg gacacggggg caggagcggc gggagaacgt ctcatccccc 1800
   caggcccgca ctgccctcct gacgggactc acgcctggca cccactacca gctggatgtg 1860
   cagctctacc actgcaccct cctgggcccg gcctcgcccc ctgcacacgt gcttctgccc 1920
   cccagtgggc ctccagcccc ccgacacctc cacgcccagg ccctctcaga ctccgagatc 1980
   cagctgacat ggaagcaccc ggaggctctg cctgggccaa tatccaagta cgttgtggag 2040
   gtgcaggtgg ctgggggtgc aggagaccca ctgtggatag acgtggacag gcctgaggag 2100
   acaagcacca tcatccgtgg cctcaacgcc agcacgcgct acctcttccg catgcgggcc 2160
   agcattcagg ggctcgggga ctggagcaac acagtagaag agtccaccct gggcaacggg 2220
   ctgcaggctg agggcccagt ccaagagagc cgggcagctg aagagggcct ggatcagcag 2280
   ctgatcctgg cggtggtggg ctccgtgtct gccacctgcc tcaccatcct ggctgccctt 2340
   ttaaccctgg tgtgcatccg cagaagctgc ctgcatcgga gacgcacctt cacctaccag 2400
   tcaggctcgg gcgaggagac catcctgcag ttcagctcag ggaccttgac acttacccgg 2460
   cggccaaaac tgcagcccga gcccctgagc tacccagtgc tagagtggga ggacatcacc 2520
   tttgaggacc tcatcgggga ggggaacttc ggccaggtca tccgggccat gatcaagaag 2580
   gacgggctga agatgaacgc agccatcaaa atgctgaaag agtatgcctc tgaaaatgac 2640
   catcgtgact ttgcgggaga actggaagtt ctgtgcaaat tggggcatca ccccaacatc 2700
   atcaacctcc tgggggcctg taagaaccga ggttacttgt atatcgctat tgaatatgcc 2760
   ccctacggga acctgctaga ttttctgcgg aaaagccggg tcctagagac tgacccagct 2820
   tttgctcgag agcatgggac agcctctacc cttagctccc ggcagctgct gcgtttcgcc 2880
   agtgatgcgg ccaatggcat gcagtacctg agtgagaagc agttcatcca cagggacctg 2940
   gctgcccgga atgtgctggt cggagagaac ctggcctcca agattgcaga cttcggcctt 3000
   tctcggggag aggaggttta tgtgaagaag acgatggggc gtctccctgt gcgctggatg 3060
   gccattgagt ccctgaacta cagtgtctat accaccaaga gtgatgtctg gtcctttgga 3120
   gtccttcttt gggagatagt gagccttgga ggtacaccct actgtggcat gacctgtgcc 3180
   gagctctatg aaaagctgcc ccagggctac cgcatggagc agcctcgaaa ctgtgacgat 3240
   gaagtgtacg agctgatgcg tcagtgctgg cgggaccgtc cctatgagcg accccccttt 3300
   gcccagattg cgctacagct aggccgcatg ctggaagcca ggaaggccta tgtgaacatg 3360
   tcgctgtttg agaacttcac ttacgcgggc attgatgcca cagctgagga ggcctga    3417
<210> 9
   <211> 3375
   <212> DNA
   <213> Homo sapiens
<300>
   <302> TEK
   <310> L06139
<400> 9
   atggactctt tagccagctt agttctctgt ggagtcagct tgctcctttc tggaactgtg 60
   gaaggtgcca tggacttgat cttgatcaat tccctacctc ttgtatctga tgctgaaaca 120
   tctctcacct gcattgcctc tgggtggcgc ccccatgagc ccatcaccat aggaagggac 180
   tttgaagcct taatgaacca gcaccaggat ccgctggaag ttactcaaga tgtgaccaga 240
   gaatgggcta aaaaagttgt ttggaagaga gaaaaggcta gtaagatcaa tggtgcttat 300
   ttctgtgaag ggcgagttcg aggagaggca atcaggatac gaaccatgaa gatgcgtcaa 360
   caagcttcct tcctaccagc tactttaact atgactgtgg acaagggaga taacgtgaac 420
   atatctttca aaaaggtatt gattaaagaa gaagatgcag tgatttacaa aaatggttcc 480
   ttcatccatt cagtgccccg gcatgaagta cctgatattc tagaagtaca cctgcctcat 540
   gctcagcccc aggatgctgg agtgtactcg gccaggtata taggaggaaa cctcttcacc 600
   tcggccttca ccaggctgat agtccggaga tgtgaagccc agaagtgggg acctgaatgc 660
   aaccatctct gtactgcttg tatgaacaat ggtgtctgcc atgaagatac tggagaatgc 720
   atttgccctc ctgggtttat gggaaggacg tgtgagaagg cttgtgaact gcacacgttt 780
   ggcagaactt gtaaagaaag gtgcagtgga caagagggat gcaagtctta tgtgttctgt 840
   ctccctgacc cctatgggtg ttcctgtgcc acaggctgga agggtctgca gtgcaatgaa 900
   gcatgccacc ctggttttta cgggccagat tgtaagctta ggtgcagctg caacaatggg 960
   gagatgtgtg atcgcttcca aggatgtctc tgctctccag gatggcaggg gctccagtgt 1020
   gagagagaag gcataccgag gatgacccca aagatagtgg atttgccaga tcatatagaa 1080
   gtaaacagtg gtaaatttaa tcccatttgc aaagcttctg gctggccgct acctactaat 1140
   gaagaaatga ccctggtgaa gccggatggg acagtgctcc atccaaaaga ctttaaccat 1200
   acggatcatt tctcagtagc catattcacc atccaccgga tcctcccccc tgactcagga 1260
   gtttgggtct gcagtgtgaa cacagtggct gggatggtgg aaaagccctt caacatttct 1320
   gttaaagttc ttccaaagcc cctgaatgcc ccaaacgtga ttgacactgg acataacttt 1380
   gctgtcatca acatcagctc tgagccttac tttggggatg gaccaatcaa atccaagaag 1440
   cttctataca aacccgttaa tcactatgag gcttggcaac atattcaagt gacaaatgag 1500
   attgttacac tcaactattt ggaacctcgg acagaatatg aactctgtgt gcaactggtc 1560
   cgtcgtggag agggtgggga agggcatcct ggacctgtga gacgcttcac aacagcttct 1620
   atcggactcc ctcctccaag aggtctaaat ctcctgccta aaagtcagac cactctaaat 1680
   ttgacctggc aaccaatatt tccaagctcg gaagatgact tttatgttga agtggagaga 1740
   aggtctgtgc aaaaaagtga tcagcagaat attaaagttc caggcaactt gacttcggtg 1800
   ctacttaaca acttacatcc cagggagcag tacgtggtcc gagctagagt caacaccaag 1860
   gcccaggggg aatggagtga agatctcact gcttggaccc ttagtgacat tcttcctcct 1920
   caaccagaaa acatcaagat ttccaacatt acacactcct cggctgtgat ttcttggaca 1980
   atattggatg gctattctat ttcttctatt actatccgtt acaaggttca aggcaagaat 2040
   gaagaccagc acgttgatgt gaagataaag aatgccacca tcattcagta tcagctcaag 2100
   ggcctagagc ctgaaacagc ataccaggtg gacatttttg cagagaacaa catagggtca 2160
   agcaacccag ccttttctca tgaactggtg accctcccag aatctcaagc accagcggac 2220
   ctcggagggg ggaagatgct gcttatagcc atccttggct ctgctggaat gacctgcctg 2280
   actgtgctgt tggcctttct gatcatattg caattgaaga gggcaaatgt gcaaaggaga 2340
   atggcccaag ccttccaaaa cgtgagggaa gaaccagctg tgcagttcaa ctcagggact 2400
   ctggccctaa acaggaaggt caaaaacaac ccagatccta caatttatcc agtgcttgac 2460
   tggaatgaca tcaaatttca agatgtgatt ggggagggca attttggcca agttcttaag 2520
   gcgcgcatca agaaggatgg gttacggatg gatgctgcca tcaaaagaat gaaagaatat 2580
   gcctccaaag atgatcacag ggactttgca ggagaactgg aagttctttg taaacttgga 2640
   caccatccaa acatcatcaa tctcttagga gcatgtgaac atcgaggcta cttgtacctg 2700
   gccattgagt acgcgcccca tggaaacctt ctggacttcc ttcgcaagag ccgtgtgctg 2760
   gagacggacc cagcatttgc cattgccaat agcaccgcgt ccacactgtc ctcccagcag 2820
   ctccttcact tcgctgccga cgtggcccgg ggcatggact acttgagcca aaaacagttt 2880
   atccacaggg atctggctgc cagaaacatt ttagttggtg aaaactatgt ggcaaaaata 2940
   gcagattttg gattgtcccg aggtcaagag gtgtacgtga aaaagacaat gggaaggctc 3000
   ccagtgcgct ggatggccat cgagtcactg aattacagtg tgtacacaac caacagtgat 3060
   gtatggtcct atggtgtgtt actatgggag attgttagct taggaggcac accctactgc 3120
   gggatgactt gtgcagaact ctacgagaag ctgccccagg gctacagact ggagaagccc 3180
   ctgaactgtg atgatgaggt gtatgatcta atgagacaat gctggcggga gaagccttat 3240
   gagaggccat catttgccca gatattggtg tccttaaaca gaatgttaga ggagcgaaag 3300
   acctacgtga ataccacgct ttatgagaag tttacttatg caggaattga ctgttctgct 3360
   gaagaagcgg cctag 3375
<210> 10
   <211> 2409
   <212> DNA
   <213> Homo sapiens
<300>
<300>
   <302> beta5 integrin
   <310> X53002
<400> 10
   ncbsncvwra tgccgcgggc cccggcgccg ctgtacgcct gcctcctggg gctctgcgcg 60
   ctcctgcccc ggctcgcagg tctcaacata tgcactagtg gaagtgccac ctcatgtgaa 120
   gaatgtctgc taatccaccc aaaatgtgcc tggtgctcca aagaggactt cggaagccca 180
   cggtccatca cctctcggtg tgatctgagg gcaaaccttg tcaaaaatgg ctgtggaggt 240
   gagatagaga gcccagccag cagcttccat gtcctgagga gcctgcccct cagcagcaag 300
   ggttcgggct ctgcaggctg ggacgtcatt cagatgacac cacaggagat tgccgtgaac 360
   ctccggcccg gtgacaagac caccttccag ctacaggttc gccaggtgga ggactatcct 420
   gtggacctgt actacctgat ggacctctcc ctgtccatga aggatgactt ggacaatatc 480
   cggagcctgg gcaccaaact cgcggaggag atgaggaagc tcaccagcaa cttccggttg 540
   ggatttgggt cttttgttga taaggacatc tctcctttct cctacacggc accgaggtac 600
   cagaccaatc cgtgcattgg ttacaagttg tttccaaatt gcgtcccctc ctttgggttc 660
   cgccatctgc tgcctctcac agacagagtg gacagcttca atgaggaagt tcggaaacag 720
   agggtgtccc ggaaccgaga tgcccctgag gggggctttg atgcagtact ccaggcagcc 780
   gtctgcaagg agaagattgg ctggcgaaag gatgcactgc atttgctggt gttcacaaca 840
   gatgatgtgc cccacatcgc attggatgga aaattgggag gcctggtgca gccacacgat 900
   ggccagtgcc acctgaacga ggccaacgag tacacagcat ccaaccagat ggactatcca 960
   tcccttgcct tgcttggaga gaaattggca gagaacaaca tcaacctcat ctttgcagtg 1020
   acaaaaaacc attatatgct gtacaagaat tttacagccc tgatacctgg aacaacggtg 1080
   gagattttag atggagactc caaaaatatt attcaactga ttattaatgc atacaatagt 1140
   atccggtcta aagtggagtt gtcagtctgg gatcagcctg aggatcttaa tctcttcttt 1200
   actgctacct gccaagatgg ggtatcctat cctggtcaga ggaagtgtga gggtctgaag 1260
   attggggaca cggcatcttt tgaagtatca ttggaggccc gaagctgtcc cagcagacac 1320
   acggagcatg tgtttgccct gcggccggtg ggattccggg acagcctgga ggtgggggtc 1380
   acctacaact gcacgtgcgg ctgcagcgtg gggctggaac ccaacagcgc caggtgcaac 1440
   gggagcggga cctatgtctg cggcctgtgt gagtgcagcc ccggctacct gggcaccagg 1500
   tgcgagtgcc aggatgggga gaaccagagc gtgtaccaga acctgtgccg ggaggcagag 1560
   ggcaagccac tgtgcagcgg gcgtggggac tgcagctgca accagtgctc ctgcttcgag 1620
   agcgagtttg gcaagatcta tgggcctttc tgtgagtgcg acaacttctc ctgtgccagg 1680
   aacaagggag tcctctgctc aggccatggc gagtgtcact gcggggaatg caagtgccat 1740
   gcaggttaca tcggggacaa ctgtaactgc tcgacagaca tcagcacatg ccggggcaga 1800
   gatggccaga tctgcagcga gcgtgggcac tgtctctgtg ggcagtgcca atgcacggag 1860
   ccgggggcct ttggggagat gtgtgagaag tgccccacct gcccggatgc atgcagcacc 1920
   aagagagatt gcgtcgagtg cctgctgctc cactctggga aacctgacaa ccagacctgc 1980
   cacagcctat gcagggatga ggtgatcaca tgggtggaca ccatcgtgaa agatgaccag 2040
   gaggctgtgc tatgtttcta caaaaccgcc aaggactgcg tcatgatgtt cacctatgtg 2100
   gagctcccca gtgggaagtc caacctgacc gtcctcaggg agccagagtg tggaaacacc 2160
   cccaacgcca tgaccatcct cctggctgtg gtcggtagca tcctccttgt tgggcttgca 2220
   ctcctggcta tctggaagct gcttgtcacc atccacgacc ggagggagtt tgcaaagttt 2280
   cagagcgagc gatccagggc ccgctatgaa atggcttcaa atccattata cagaaagcct 2340
   atctccacgc acactgtgga cttcaccttc aacaagttca acaaatccta caatggcact 2400
   gtggactga                                                        2409
<210> 11
   <211> 2367
   <212> DNA
   <213> Homo sapiens
<300>
   <302> beta3 integrin
   <310> MM000212
<400> 11
   atgcgagcgc ggccgcggcc ccggccgctc tgggcgactg tgctggcgct gggggcgctg 60
   gcgggcgttg gcgtaggagg gcccaacatc tgtaccacgc gaggtgtgag ctcctgccag 120
   cagtgcctgg ctgtgagccc catgtgtgcc tggtgctctg atgaggccct gcctctgggc 180
   tcacctcgct gtgacctgaa ggagaatctg ctgaaggata actgtgcccc agaatccatc 240
   gagttcccag tgagtgaggc ccgagtacta gaggacaggc ccctcagcga caagggctct 300
   ggagacagct cccaggtcac tcaagtcagt ccccagagga ttgcactccg gctccggcca 360
   gatgattcga agaatttctc catccaagtg cggcaggtgg aggattaccc tgtggacatc 420
   tactacttga tggacctgtc ttactccatg aaggatgatc tgtggagcat ccagaacctg 480
   ggtaccaagc tggccaccca gatgcgaaag ctcaccagta acctgcggat tggcttcggg 540
   gcatttgtgg acaagcctgt gtcaccatac atgtatatct ccccaccaga ggccctcgaa 600
   aacccctgct atgatatgaa gaccacctgc ttgcccatgt ttggctacaa acacgtgctg 660
   acgctaactg accaggtgac ccgcttcaat gaggaagtga agaagcagag tgtgtcacgg 720
   aaccgagatg ccccagaggg tggctttgat gccatcatgc aggctacagt ctgtgatgaa 780
   aagattggct ggaggaatga tgcatcccac ttgctggtgt ttaccactga tgccaagact 840
   catatagcat tggacggaag gctggcaggc attgtccagc ctaatgacgg gcagtgtcat 900
   gttggtagtg acaatcatta ctctgcctcc actaccatgg attatccctc tttggggctg 960
   atgactgaga agctatccca gaaaaacatc aatttgatct ttgcagtgac tgaaaatgta 1020
   gtcaatctct atcagaacta tagtgagctc atcccaggga ccacagttgg ggttctgtcc 1080
   atggattcca gcaatgtcct ccagctcatt gttgatgctt atgggaaaat ccgttctaaa 1140
   gtagagctgg aagtgcgtga cctccctgaa gagttgtctc tatccttcaa tgccacctgc 1200
   ctcaacaatg aggtcatccc tggcctcaag tcttgtatgg gactcaagat tggagacacg 1260
   gtgagcttca gcattgaggc caaggtgcga ggctgtcccc aggagaagga gaagtccttt 1320
   accataaagc ccgtgggctt caaggacagc ctgatcgtcc aggtcacctt tgattgtgac 1380
   tgtgcctgcc aggcccaagc tgaacctaat agccatcgct gcaacaatgg caatgggacc 1440
   tttgagtgtg gggtatgccg ttgtgggcct ggctggctgg gatcccagtg tgagtgctca 1500
   gaggaggact atcgcccttc ccagcaggac gaatgcagcc cccgggaggg tcagcccgtc 1560
   tgcagccagc ggggcgagtg cctctgtggt caatgtgtct gccacagcag tgactttggc 1620
   aagatcacgg gcaagtactg cgagtgtgac gacttctcct gtgtccgcta caagggggag 1680
   atgtgctcag gccatggcca gtgcagctgt ggggactgcc tgtgtgactc cgactggacc 1740
   ggctactact gcaactgtac cacgcgtact gacacctgca tgtccagcaa tgggctgctg 1800
   tgcagcggcc gcggcaagtg tgaatgtggc agctgtgtct gtatccagcc gggctcctat 1860
   ggggacacct gtgagaagtg ccccacctgc ccagatgcct gcacctttaa gaaagaatgt 1920
   gtggagtgta agaagtttga ccgggagccc tacatgaccg aaaatacctg caaccgttac 1980
   tgccgtgacg agattgagtc agtgaaagag cttaaggaca ctggcaagga tgcagtgaat 2040
   tgtacctata agaatgagga tgactgtgtc gtcagattcc agtactatga agattctagt 2100
   ggaaagtcca tcctgtatgt ggtagaagag ccagagtgtc ccaagggccc tgacatcctg 2160
   gtggtcctgc tctcagtgat gggggccatt ctgctcattg gccttgccgc cctgctcatc 2220
   tggaaactcc tcatcaccat ccacgaccga aaagaattcg ctaaatttga ggaagaacgc 2280
   gccagagcaa aatgggacac agccaacaac ccactgtata aagaggccac gtctaccttc 2340
   accaatatca cgtaccgggg cacttaa                                     2367
<210> 12
   <211> 3147
   <212> DNA
   <213> Homo sapiens
<300>
   <302> alpha v intergrin
   <310> NM0022210
<400> 12
   atggcttttc cgccgcggcg acggctgcgc ctcggtcccc gcggcctccc gcttcttctc 60
   tcgggactcc tgctacctct gtgccgcgcc ttcaacctag acgtggacag tcctgccgag 120
   tactctggcc ccgagggaag ttacttcggc ttcgccgtgg atttcttcgt gcccagcgcg 180
   tcttcccgga tgtttcttct cgtgggagct cccaaagcaa acaccaccca gcctgggatt 240
   gtggaaggag ggcaggtcct caaatgtgac tggtcttcta cccgccggtg ccagccaatt 300
   gaatttgatg caacaggcaa tagagattat gccaaggatg atccattgga atttaagtcc 360
   catcagtggt ttggagcatc tgtgaggtcg aaacaggata aaattttggc ctgtgcccca 420
   ttgtaccatt ggagaactga gatgaaacag gagcgagagc ctgttggaac atgctttctt 480
   caagatggaa caaagactgt tgagtatgct ccatgtagat cacaagatat tgatgctgat 540
   ggacagggat tttgtcaagg aggattcagc attgatttta ctaaagctga cagagtactt 600
   cttggtggtc ctggtagctt ttattggcaa ggtcagctta tttcggatca agtggcagaa 660
   atcgtatcta aatacgaccc caatgtttac agcatcaagt ataataacca attagcaact 720
   cggactgcac aagctatttt tgatgacagc tatttgggtt attctgtggc tgtcggagat 780
   ttcaatggtg atggcataga tgactttgtt tcaggagttc caagagcagc aaggactttg 840
   ggaatggttt atatttatga tggcaagaac atgtcctcct tatacaattt tactggcgag 900
   cagatggctg catatttcgg attttctgta gctgccactg acattaatgg agatgattat 960
   gcagatgtgt ttattggagc acctctcttc atggatcgtg gctctgatgg caaactccaa 1020
   gaggtggggc aggtctcagt gtctctacag agagcttcag gagacttcca gacgacaaag 1080
   ctgaatggat ttgaggtctt tgcacggttt ggcagtgcca tagctccttt gggagatctg 1140
   gaccaggatg gtttcaatga tattgcaatt gctgctccat atgggggtga agataaaaaa 1200
   ggaattgttt atatcttcaa tggaagatca acaggcttga acgcagtccc atctcaaatc 1260
   cttgaagggc agtgggctgc tcgaagcatg ccaccaagct ttggctattc aatgaaagga 1320
   gccacagata tagacaaaaa tggatatcca gacttaattg taggagcttt tggtgtagat 1380
   cgagctatct tatacagggc cagaccagtt atcactgtaa atgctggtct tgaagtgtac 1440
   cctagcattt taaatcaaga caataaaacc tgctcactgc ctggaacagc tctcaaagtt 1500
   tcctgtttta atgttaggtt ctgcttaaag gcagatggca aaggagtact tcccaggaaa 1560
   cttaatttcc aggtggaact tcttttggat aaactcaagc aaaagggagc aattcgacga 1620
   gcactgtttc tctacagcag gtccccaagt cactccaaga acatgactat ttcaaggggg 1680
   ggactgatgc agtgtgagga attgatagcg tatctgcggg atgaatctga atttagagac 1740
   aaactcactc caattactat ttttatggaa tatcggttgg attatagaac agctgctgat 1800
   acaacaggct tgcaacccat tcttaaccag ttcacgcctg ctaacattag tcgacaggct 1860
   cacattctac ttgactgtgg tgaagacaat gtctgtaaac ccaagctgga agtttctgta 1920
   gatagtgatc aaaagaagat ctatattggg gatgacaacc ctctgacatt gattgttaag 1980
   gctcagaatc aaggagaagg tgcctacgaa gctgagctca tcgtttccat tccactgcag 2040
   gctgatttca tcggggttgt ccgaaacaat gaagccttag caagactttc ctgtgcattt 2100
   aagacagaaa accaaactcg ccaggtggta tgtgaccttg gaaacccaat gaaggctgga 2160
   actcaactct tagctggtct tcgtttcagt gtgcaccagc agtcagagat ggatacttct 2220
   gtgaaatttg acttacaaat ccaaagctca aatctatttg acaaagtaag cccagttgta 2280
   tctcacaaag ttgatcttgc tgttttagct gcagttgaga taagaggagt ctcgagtcct 2340
   gatcatatct ttcttccgat tccaaactgg gagcacaagg agaaccctga gactgaagaa 2400
   gatgttgggc cagttgttca gcacatctat gagctgagaa acaatggtcc aagttcattc 2460
   agcaaggcaa tgctccatct tcagtggcct tacaaatata ataataacac tctgttgtat 2520
   atccttcatt atgatattga tggaccaatg aactgcactt cagatatgga gatcaaccct 2580
   ttgagaatta agatctcatc tttgcaaaca actgaaaaga atgacacggt tgccgggcaa 2640
   ggtgagcggg accatctcat cactaagcgg gatcttgccc tcagtgaagg agatattcac 2700
   actttgggtt gtggagttgc tcagtgcttg aagattgtct gccaagttgg gagattagac 2760
   agaggaaaga gtgcaatctt gtacgtaaag tcattactgt ggactgagac ttttatgaat 2820
   aaagaaaatc agaatcattc ctattctctg aagtcgtctg cttcatttaa tgtcatagag 2880
   tttccttata agaatcttcc aattgaggat atcaccaact ccacattggt taccactaat 2940
   gtcacctggg gcattcagcc agcgcccatg cctgtgcctg tgtgggtgat cattttagca 3000
   gttctagcag gattgttgct actggctgtt ttggtatttg taatgtacag gatgggcttt 3060
   tttaaacggg tccggccacc tcaagaagaa caagaaaggg agcagcttca acctcatgaa 3120
   aatggtgaag gaaactcaga aacttaa 3147
<210> 13
   <211> 402
   <212> DNA
   <213> Homo sapiens
<300>
   <302> CaSm (cancer associated SM-like oncogene)
   <310> AF000177
<400> 13
   atgaactata tgcctggcac cgccagcctc atcgaggaca ttgacaaaaa gcacttggtt 60
   ctgcttcgag atggaaggac acttataggc tttttaagaa gcattgatca atttgcaaac 120
   ttagtgctac atcagactgt ggagcgtatt catgtgggca aaaaatacgg tgatattcct 180
   cgagggattt ttgtggtcag aggagaaaat gtggtcctac taggagaaat agacttggaa 240
   aaggagagtg acacacccct ccagcaagta tccattgaag aaattctaga agaacaaagg 300
   gtggaacagc agaccaagct ggaagcagag aagttgaaag tgcaggccct gaaggaccga 360
   ggtctttcca ttcctcgagc agatactctt gatgagtact aa 402
<210> 14
   <211> 1923
   <212> DNA
   <213> Homo sapiens
<300>
   <302> c-myb
   <310> NM005375
<400> 14
   atggcccgaa gaccccggca cagcatatat agcagtgacg aggatgatga ggactttgag 60
   atgtgtgacc atgactatga tgggctgctt cccaagtctg gaaagcgtca cttggggaaa 120
   acaaggtgga cccgggaaga ggatgaaaaa ctgaagaagc tggtggaaca gaatggaaca 180
   gatgactgga aagttattgc caattatctc ccgaatcgaa cagatgtgca gtgccagcac 240
   cgatggcaga aagtactaaa ccctgagctc atcaagggtc cttggaccaa agaagaagat 300
   cagagagtga tagagcttgt acagaaatac ggtccgaaac gttggtctgt tattgccaag 360
   cacttaaagg ggagaattgg aaaacaatgt agggagaggt ggcataacca cttgaatcca 420
   gaagttaaga aaacctcctg gacagaagag gaagacagaa ttatttacca ggcacacaag 480
   agactgggga acagatgggc agaaatcgca aagctactgc ctggacgaac tgataatgct 540
   atcaagaacc actggaattc tacaatgcgt cggaaggtcg aacaggaagg ttatctgcag 600
   gagtcttcaa aagccagcca gccagcagtg gccacaagct tccagaagaa cagtcatttg 660
   atgggttttg ctcaggctcc gcctacagct caactccctg ccactggcca gcccactgtt 720
   aacaacgact attcctatta ccacatttct gaagcacaaa atgtctccag tcatgttcca 780
   taccctgtag cgttacatgt aaatatagtc aatgtccctc agccagctgc cgcagccatt 840
   cagagacact ataatgatga agaccctgag aaggaaaagc gaataaagga attagaattg 900
   ctcctaatgt caaccgagaa tgagctaaaa ggacagcagg tgctaccaac acagaaccac 960
   acatgcagct accccgggtg gcacagcacc accattgccg accacaccag acctcatgga 1020
   gacagtgcac ctgtttcctg tttgggagaa caccactcca ctccatctct gccagcggat 1080
   cctggctccc tacctgaaga aagcgcctcg ccagcaaggt gcatgatcgt ccaccagggc 1140
   accattctgg ataatgttaa gaacctctta gaatttgcag aaacactcca atttatagat 1200
   tctttcttaa acacttccag taaccatgaa aactcagact tggaaatgcc ttctttaact 1260
   tccacccccc tcattggtca caaattgact gttacaacac catttcatag agaccagact 1320
   gtgaaaactc aaaaggaaaa tactgttttt agaaccccag ctatcaaaag gtcaatctta 1380
   gaaagctctc caagaactcc tacaccattc aaacatgcac ttgcagctca agaaattaaa 1440
   tacggtcccc tgaagatgct acctcagaca ccctctcatc tagtagaaga tctgcaggat 1500
   gtgatcaaac aggaatctga tgaatctgga tttgttgctg agtttcaaga aaatggacca 1560
   cccttactga agaaaatcaa acaagaggtg gaatctccaa ctgataaatc aggaaacttc 1620
   ttctgctcac accactggga aggggacagt ctgaataccc aactgttcac gcagacctcg 1680
   cctgtgcgag atgcaccgaa tattcttaca agctccgttt taatggcacc agcatcagaa 1740
   gatgaagaca atgttctcaa agcatttaca gtacctaaaa acaggtccct ggcgagcccc 1800
   ttgcagcctt gtagcagtac ctgggaacct gcatcctgtg gaaagatgga ggagcagatg 1860
   acatcttcca gtcaagctcg taaatacgtg aatgcattct cagcccggac gctggtcatg 1920
   tga 1923
<210> 15
   <211> 544
   <212> DNA
   <213> Homo sapiens
<300>
   <302> c-myc
   <310> J00120
<400> 15
   gacccccgag ctgtgctgct cgcggccgcc accgccgggc cccggccgtc cctggctccc 60
   ctcctgcctc gagaagggca gggcttctca gaggcttggc gggaaaaaga acggagggag 120
   ggatcgcgct gagtataaaa gccggttttc ggggctttat ctaactcgct gtagtaattc 180
   cagcgagagg cagagggagc gagcgggcgg ccggctaggg tggaagagcc gggcgagcag 240
   agctgcgctg cgggcgtcct gggaagggag atccggagcg aatagggggc ttcgcctctg 300
   gcccagccct cccgctgatc ccccagccag cggtccgcaa cccttgccgc atccacgaaa 360
   ctttgcccat agcagcgggc gggcactttg cactggaact tacaacaccc gagcaaggac 420
   gcgactctcc cgacgcgggg aggctattct gcccatttgg ggacacttcc ccgccgctgc 480
   caggacccgc ttctctgaaa ggctctcctt gcagctgctt agacgctgga tttttttcgg 540
   gtag                                                              544
<210> 16
   <211> 618
   <212> DNA
   <213> Homo sapiens
<300>
   <302> ephrin-A1
   <310> NM004428
<400> 16
   atggagttcc tctgggcccc tctcttgggt ctgtgctgca gtctggccgc tgctgatcgc 60
   cacaccgtct tctggaacag ttcaaatccc aagttccgga atgaggacta caccatacat 120
   gtgcagctga atgactacgt ggacatcatc tgtccgcact atgaagatca ctctgtggca 180
   gacgctgcca tggagcagta catactgtac ctggtggagc atgaggagta ccagctgtgc 240
   cagccccagt ccaaggacca agtccgctgg cagtgcaacc ggcccagtgc caagcatggc 300
   ccggagaagc tgtctgagaa gttccagcgc ttcacacctt tcaccctggg caaggagttc 360
   aaagaaggac acagctacta ctacatctcc aaacccatcc accagcatga agaccgctgc 420
   ttgaggttga aggtgactgt cagtggcaaa atcactcaca gtcctcaggc ccatgtcaat 480
   ccacaggaga agagacttgc agcagatgac ccagaggtgc gggttctaca tagcatcggt 540
   cacagtgctg ccccacgcct cttcccactt gcctggactg tgctgctcct tccacttctg 600
   ctgctgcaaa ccccgtga                                               618
<210> 17
   <211> 642
   <212> DNA
   <213> Homo sapiens
<400> 17
   atggcgcccg cgcagcgccc gctgctcccg ctgctgctcc tgctgttacc gctgccgccg 60
   ccgcccttcg cgcgcgccga ggacgccgcc cgcgccaact cggaccgcta cgccgtctac 120
   tggaaccgca gcaaccccag gttccacgca ggcgcggggg acgacggcgg gggctacacg 180
   gtggaggtga gcatcaatga ctacctggac atctactgcc cgcactatgg ggcgccgctg 240
   ccgccggccg agcgcatgga gcactacgtg ctgtacatgg tcaacggcga gggccacgcc 300
   tcctgcgacc accgccagcg cggcttcaag cgctgggagt gcaaccggcc cgcggcgccc 360
   ggggggccgc tcaagttctc ggagaagttc cagctcttca cgcccttctc cctgggcttc 420
   gagttccggc ccggccacga gtattactac atctctgcca cgcctcccaa tgctgtggac 480
   cggccctgcc tgcgactgaa ggtgtacgtg cggccgacca acgagaccct gtacgaggct 540
   cctgagccca tcttcaccag caataactcg tgtagcagcc cgggcggctg ccgcctcttc 600
   ctcagcacca tccccgtgct ctggaccctc ctgggttcct ag 642
<210> 18
   <211> 717
   <212> DNA
   <213> Homo sapiens
<300>
   <302> ephrin-A3
   <310> XM001787
<400> 18
   atggcggcgg ctccgctgct gctgctgctg ctgctcgtgc ccgtgccgct gctgccgctg 60
   ctggcccaag ggcccggagg ggcgctggga aaccggcatg cggtgtactg gaacagctcc 120
   aaccagcacc tgcggcgaga gggctacacc gtgcaggtga acgtgaacga ctatctggat 180
   atttactgcc cgcactacaa cagctcgggg gtgggccccg gggcgggacc ggggcccgga 240
   ggcggggcag agcagtacgt gctgtacatg gtgagccgca acggctaccg cacctgcaac 300
   gccagccagg gcttcaagcg ctgcgagtgc aaccggccgc acgccccgca cagccccatc 360
   aagttctcgg agaagttcca gcgctacagc gccttctctc tgggctacga gttccacgcc 420
   ggccacgagt actactacat ctccacgccc actcacaacc tgcactggaa gtgtctgagg 480
   atgaaggtgt tcgtctgctg cgcctccaca tcgcactccg ggagaagccc ggtccccact 540
   ctcccccagt tcaccatggg ccccaatatg aagatcaacg tgctggaaga ctttgaggga 600
   gagaaccctc aggtgcccaa gcttgagaag agcatcagcg ggaccagccc caaacgggaa 660
   cacctgcccc tggccgtggg catcgccttc ttcctcatga cgttcttggc ctcctag 717
<210> 19
   <211> 606
   <212> DNA
   <213> Homo sapiens
<300>
   <302> ephrin-A3
   <310> XM001784
<400> 19
   atgcggctgc tgcccctgct gcggactgtc ctctgggccg cgttcctcgg ctcccctctg 60
   cgcgggggct ccagcctccg ccacgtagtc tactggaact ccagtaaccc caggttgctt 120
   cgaggagacg ccgtggtgga gctgggcctc aacgattacc tagacattgt ctgcccccac 180
   tacgaaggcc cagggccccc tgagggcccc gagacgtttg ctttgtacat ggtggactgg 240
   ccaggctatg agtcctgcca ggcagagggc ccccgggcct acaagcgctg ggtgtgctcc 300
   ctgccctttg gccatgttca attctcagag aagattcagc gcttcacacc cttctccctc 360
   ggctttgagt tcttacctgg agagacttac tactacatct cggtgcccac tccagagagt 420
   tctggccagt gcttgaggct ccaggtgtct gtctgctgca aggagaggaa gtctgagtca 480
   gcccatcctg ttgggagccc tggagagagt ggcacatcag ggtggcgagg gggggacact 540
   cccagccccc tctgtctctt gctattactg ctgcttctga ttcttcgtct tctgcgaatt 600
   ctgtga 606
<210> 20
   <211> 687
   <212> DNA
   <213> Homo sapiens
<300>
   <302> ephrin-A5
   <310> NM001962
<400> 20
   atgttgcacg tggagatgtt gacgctggtg tttctggtgc tctggatgtg tgtgttcagc 60
   caggacccgg gctccaaggc cgtcgccgac cgctacgctg tctactggaa cagcagcaac 120
   cccagattcc agaggggtga ctaccatatt gatgtctgta tcaatgacta cctggatgtt 180
   ttctgccctc actatgagga ctccgtccca gaagataaga ctgagcgcta tgtcctctac 240
   atggtgaact ttgatggcta cagtgcctgc gaccacactt ccaaagggtt caagagatgg 300
   gaatgtaacc ggcctcactc tccaaatgga ccgctgaagt tctctgaaaa attccagctc 360
   ttcactccct tttctctagg atttgaattc aggccaggcc gagaatattt ctacatctcc 420
   tctgcaatcc cagataatgg aagaaggtcc tgtctaaagc tcaaagtctt tgtgagacca 480
   acaaatagct gtatgaaaac tataggtgtt catgatcgtg ttttcgatgt taacgacaaa 540
   gtagaaaatt cattagaacc agcagatgac accgtacatg agtcagccga gccatcccgc 600
   ggcgagaacg cggcacaaac accaaggata cccagccgcc ttttggcaat cctactgttc 660
   ctcctggcga tgcttttgac attatag                                      687
<210> 21
   <211> 2955
   <212> DNA
   <213> Homo sapiens
<400> 21
   atggccctgg attatctact actgctcctc ctggcatccg cagtggctgc gatggaagaa 60
   acgttaatgg acaccagaac ggctactgca gagctgggct ggacggccaa tcctgcgtcc 120
   gggtgggaag aagtcagtgg ctacgatgaa aacctgaaca ccatccgcac ctaccaggtg 180
   tgcaatgtct tcgagcccaa ccagaacaat tggctgctca ccaccttcat caaccggcgg 240
   ggggcccatc gcatctacac agagatgcgc ttcactgtga gagactgcag cagcctccct 300
   aatgtcccag gatcctgcaa ggagaccttc aacttgtatt actatgagac tgactctgtc 360
   attgccacca agaagtcagc cttctggtct gaggccccct acctcaaagt agacaccatt 420
   gctgcagatg agagcttctc ccaggtggac tttgggggaa ggctgatgaa ggtaaacaca 480
   gaagtcagga gctttgggcc tcttactcgg aatggttttt acctcgcttt tcaggattat 540
   ggagcctgta tgtctcttct ttctgtccgt gtcttcttca aaaagtgtcc cagcattgtg 600
   caaaattttg cagtgtttcc agagactatg acaggggcag agagcacatc tctggtgatt 660
   gctcggggca catgcatccc caacgcagag gaagtggacg tgcccatcaa actctactgc 720
   aacggggatg gggaatggat ggtgcctatt gggcgatgca cctgcaagcc tggctatgag 780
   cctgagaaca gcgtggcatg caaggcttgc cctgcaggga cattcaaggc cagccaggaa 840
   gctgaaggct gctcccactg cccctccaac agccgctccc ctgcagaggc gtctcccatc 900
   tgcacctgtc ggaccggtta ttaccgagcg gactttgacc ctccagaagt ggcatgcact 960
   agcgtcccat caggtccccg caatgttatc tccatcgtca atgagacgtc catcattctg 1020
   gagtggcacc ctccaaggga gacaggtggg cgggatgatg tgacctacaa catcatctgc 1080
   aaaaagtgcc gggcagaccg ccggagctgc tcccgctgtg acgacaatgt ggagtttgtg 1140
   cccaggcagc tgggcctgac ggagtgccgc gtctccatca gcagcctgtg ggcccacacc 1200
   ccctacacct ttgacatcca ggccatcaat ggagtctcca gcaagagtcc cttcccccca 1260
   cagcacgtct ctgtcaacat caccacaaac caagccgccc cctccaccgt tcccatcatg 1320
   caccaagtca gtgccactat gaggagcatc accttgtcat ggccacagcc ggagcagccc 1380
   aatggcatca tcctggacta tgagatccgg tactatgaga aggaacacaa tgagttcaac 1440
   tcctccatgg ccaggagtca gaccaacaca gcaaggattg atgggctgcg gcctggcatg 1500
   gtatatgtgg tacaggtgcg tgcccgcact gttgctggct acggcaagtt cagtggcaag 1560
   atgtgcttcc agactctgac tgacgatgat tacaagtcag agctgaggga gcagctgccc 1620
   ctgattgctg gctcggcagc ggccggggtc gtgttcgttg tgtccttggt ggccatctct 1680
   atcgtctgta gcaggaaacg ggcttatagc aaagaggctg tgtacagcga taagctccag 1740
   cattacagca caggccgagg ctccccaggg atgaagatct acattgaccc cttcacttat 1800
   gaggatccca acgaagctgt ccgggagttt gccaaggaga ttgatgtatc ttttgtgaaa 1860
   attgaagagg tcatcggagc aggggagttt ggagaagtgt acaaggggcg tttgaaactg 1920
   ccaggcaaga gggaaatcta cgtggccatc aagaccctga aggcagggta ctcggagaag 1980
   cagcgtcggg actttctgag tgaggcgagc atcatgggcc agttcgacca tcctaacatc 2040
   attcgcctgg agggtgtggt caccaagagt cggcctgtca tgatcatcac agagttcatg 2100
   gagaatggtg cattggattc tttcctcagg caaaatgacg ggcagttcac cgtgatccag 2160
   cttgtgggta tgctcagggg catcgctgct ggcatgaagt acctggctga gatgaattat 2220
   gtgcatcggg acctggctgc taggaacatt ctggtcaaca gtaacctggt gtgcaaggtg 2280
   tccgactttg gcctctcccg ctacctccag gatgacacct cagatcccac ctacaccagc 2340
   tccttgggag ggaagatccc tgtgagatgg acagctccag aggccatcgc ctaccgcaag 2400
   ttcacttcag ccagcgacgt ttggagctat gggatcgtca tgtgggaagt catgtcattt 2460
   ggagagagac cctattggga tatgtccaac caagatgtca tcaatgccat cgagcaggac 2520
   taccggctgc ccccacccat ggactgtcca gctgctctac accagctcat gctggactgt 2580
   tggcagaagg accggaacag ccggccccgg tttgcggaga ttgtcaacac cctagataag 2640
   atgatccgga acccggcaag tctcaagact gtggcaacca tcaccgccgt gccttcccag 2700
   cccctgctcg accgctccat cccagacttc acggccttta ccaccgtgga tgactggctc 2760
   agcgccatca aaatggtcca gtacagggac agcttcctca ctgctggctt cacctccctc 2820
   cagctggtca cccagatgac atcagaagac ctcctgagaa taggcatcac cttggcaggc 2880
   catcagaaga agatcctgaa cagcattcat tctatgaggg tccagataag tcagtcacca 2940
   acggcaatgg catga                                                  2955
<210> 22
   <211> 3168
   <212> DNA
   <213> Homo sapiens
<400> 22
   atggctctgc ggaggctggg ggccgcgctg ctgctgctgc cgctgctcgc cgccgtggaa 60
   gaaacgctaa tggactccac tacagcgact gctgagctgg gctggatggt gcatcctcca 120
   tcagggtggg aagaggtgag tggctacgat gagaacatga acacgatccg cacgtaccag 180
   gtgtgcaacg tgtttgagtc aagccagaac aactggctac ggaccaagtt tatccggcgc 240
   cgtggcgccc accgcatcca cgtggagatg aagttttcgg tgcgtgactg cagcagcatc 300
   cccagcgtgc ctggctcctg caaggagacc ttcaacctct attactatga ggctgacttt 360
   gactcggcca ccaagacctt ccccaactgg atggagaatc catgggtgaa ggtggatacc 420
   attgcagccg acgagagctt ctcccaggtg gacctgggtg gccgcgtcat gaaaatcaac 480
   accgaggtgc ggagcttcgg acctgtgtcc cgcagcggct tctacctggc cttccaggac 540
   tatggcggct gcatgtccct catcgccgtg cgtgtcttct accgcaagtg cccccgcatc 600
   atccagaatg gcgccatctt ccaggaaacc ctgtcggggg ctgagagcac atcgctggtg 660
   gctgcccggg gcagctgcat cgccaatgcg gaagaggtgg atgtacccat caagctctac 720
   tgtaacgggg acggcgagtg gctggtgccc atcgggcgct gcatgtgcaa agcaggcttc 780
   gaggccgttg agaatggcac cgtctgccga ggttgtccat ctgggacttt caaggccaac 840
   caaggggatg aggcctgtac ccactgtccc atcaacagcc ggaccacttc tgaaggggcc 900
   accaactgtg tctgccgcaa tggctactac agagcagacc tggaccccct ggacatgccc 960
   tgcacaacca tcccctccgc gccccaggct gtgatttcca gtgtcaatga gacctccctc 1020
   atgctggagt ggacccctcc ccgcgactcc ggaggccgag aggacctcgt ctacaacatc 1080
   atctgcaaga gctgtggctc gggccggggt gcctgcaccc gctgcgggga caatgtacag 1140
   tacgcaccac gccagctagg cctgaccgag ccacgcattt acatcagtga cctgctggcc 1200
   cacacccagt acaccttcga gatccaggct gtgaacggcg ttactgacca gagccccttc 1260
   tcgcctcagt tcgcctctgt gaacatcacc accaaccagg cagctccatc ggcagtgtcc 1320
   atcatgcatc aggtgagccg caccgtggac agcattaccc tgtcgtggtc ccagccagac 1380
   cagcccaatg gcgtgatcct ggactatgag ctgcagtact atgagaagga gctcagtgag 1440
   tacaacgcca cagccataaa aagccccacc aacacggtca ccgtgcaggg cctcaaagcc 1500
   ggcgccatct atgtcttcca ggtgcgggca cgcaccgtgg caggctacgg gcgctacagc 1560
   ggcaagatgt acttccagac catgacagaa gccgagtacc agacaagcat ccaggagaag 1620
   ttgccactca tcatcggctc ctcggccgct ggcctggtct tcctcattgc tgtggttgtc 1680
   atcgccatcg tgtgtaacag acgggggttt gagcgtgctg actcggagta cacggacaag 1740
   ctgcaacact acaccagtgg ccacatgacc ccaggcatga agatctacat cgatcctttc 1800
   acctacgagg accccaacga ggcagtgcgg gagtttgcca aggaaattga catctcctgt 1860
   gtcaaaattg agcaggtgat cggagcaggg gagtttggcg aggtctgcag tggccacctg 1920
   aagctgccag gcaagagaga gatctttgtg gccatcaaga cgctcaagtc gggctacacg 1980
   gagaagcagc gccgggactt cctgagcgaa gcctccatca tgggccagtt cgaccatccc 2040
   aacgtcatcc acctggaggg tgtcgtgacc aagagcacac ctgtgatgat catcaccgag 2100
   ttcatggaga atggctccct ggactccttt ctccggcaaa acgatgggca gttcacagtc 2160
   atccagctgg tgggcatgct tcggggcatc gcagctggca tgaagtacct ggcagacatg 2220
   aactatgttc accgtgacct ggctgcccgc aacatcctcg tcaacagcaa cctggtctgc 2280
   aaggtgtcgg actttgggct ctcacgcttt ctagaggacg atacctcaga ccccacctac 2340
   accagtgccc tgggcggaaa gatccccatc cgctggacag ccccggaagc catccagtac 2400
   cggaagttca cctcggccag tgatgtgtgg agctacggca ttgtcatgtg ggaggtgatg 2460
   tcctatgggg agcggcccta ctgggacatg accaaccagg atgtaatcaa tgccattgag 2520
   caggactatc ggctgccacc gcccatggac tgcccgagcg ccctgcacca actcatgctg 2580
   gactgttggc agaaggaccg caaccaccgg cccaagttcg gccaaattgt caacacgcta 2640
   gacaagatga tccgcaatcc caacagcctc aaagccatgg cgcccctctc ctctggcatc 2700
   aacctgccgc tgctggaccg cacgatcccc gactacacca gctttaacac ggtggacgag 2760
   tggctggagg ccatcaagat ggggcagtac aaggagagct tcgccaatgc cggcttcacc 2820
   tcctttgacg tcgtgtctca gatgatgatg gaggacattc tccgggttgg ggtcactttg 2880
   gctggccacc agaaaaaaat cctgaacagt atccaggtga tgcgggcgca gatgaaccag 2940
   attcagtctg tggagggcca gccactcgcc aggaggccac gggccacggg aagaaccaag 3000
   cggtgccagc cacgagacgt caccaagaaa acatgcaact caaacgacgg aaaaaaaaag 3060
   ggaatgggaa aaaagaaaac agatcctggg agggggcggg aaatacaagg aatatttttt 3120
   aaagaggatt ctcataagga aagcaatgac tgttcttgcg ggggataa 3168
<210> 23
   <211> 2997
   <212> DNA
   <213> Homo sapiens
<400> 23
   atggccagag cccgcccgcc gccgccgccg tcgccgccgc cggggcttct gccgctgctc 60
   cctccgctgc tgctgctgcc gctgctgctg ctgcccgccg gctgccgggc gctggaagag 120
   accctcatgg acacaaaatg ggtaacatct gagttggcgt ggacatctca tccagaaagt 180
   gggtgggaag aggtgagtgg ctacgatgag gccatgaatc ccatccgcac ataccaggtg 240
   tgtaatgtgc gcgagtcaag ccagaacaac tggcttcgca cggggttcat ctggcggcgg 300
   gatgtgcagc gggtctacgt ggagctcaag ttcactgtgc gtgactgcaa cagcatcccc 360
   aacatccccg gctcctgcaa ggagaccttc aacctcttct actacgaggc tgacagcgat 420
   gtggcctcag cctcctcccc cttctggatg gagaacccct acgtgaaagt ggacaccatt 480
   gcacccgatg agagcttctc gcggctggat gccggccgtg tcaacaccaa ggtgcgcagc 540
   tttgggccac tttccaaggc tggcttctac ctggccttcc aggaccaggg cgcctgcatg 600
   tcgctcatct ccgtgcgcgc cttctacaag aagtgtgcat ccaccaccgc aggcttcgca 660
   ctcttccccg agaccctcac tggggcggag cccacctcgc tggtcattgc tcctggcacc 720
   tgcatcccta acgccgtgga ggtgtcggtg ccactcaagc tctactgcaa cggcgatggg 780
   gagtggatgg tgcctgtggg tgcctgcacc tgtgccaccg gccatgagcc agctgccaag 840
   gagtcccagt gccgcccctg tccccctggg agctacaagg cgaagcaggg agaggggccc 900
   tgcctcccat gtccccccaa cagccgtacc acctccccag ccgccagcat ctgcacctgc 960
   cacaataact tctaccgtgc agactcggac tctgcggaca gtgcctgtac caccgtgcca 1020
   tctccacccc gaggtgtgat ctccaatgtg aatgaaacct cactgatcct cgagtggagt 1080
   gagccccggg acctgggtgt ccgggatgac ctcctgtaca atgtcatctg caagaagtgc 1140
   catggggctg gaggggcctc agcctgctca cgctgtgatg acaacgtgga gtttgtgcct 1200
   cggcagctgg gcctgtcgga gccccgggtc cacaccagcc atctgctggc ccacacgcgc 1260
   tacacctttg aggtgcaggc ggtcaacggt gtctcgggca agagccctct gccgcctcgt 1320
   tatgcggccg tgaatatcac cacaaaccag gctgccccgt ctgaagtgcc cacactacgc 1380
   ctgcacagca gctcaggcag cagcctcacc ctatcctggg cacccccaga gcggcccaac 1440
   ggagtcatcc tggactacga gatgaagtac tttgagaaga gcgagggcat cgcctccaca 1500
   gtgaccagcc agatgaactc cgtgcagctg gacgggcttc ggcctgacgc ccgctatgtg 1560
   gtccaggtcc gtgcccgcac agtagctggc tatgggcagt acagccgccc tgccgagttt 1620
   gagaccacaa gtgagagagg ctctggggcc cagcagctcc aggagcagct tcccctcatc 1680
   gtgggctccg ctacagctgg gcttgtcttc gtggtggctg tcgtggtcat cgctatcgtc 1740
   tgcctcagga agcagcgaca cggctctgat tcggagtaca cggagaagct gcagcagtac 1800
   attgctcctg gaatgaaggt ttatattgac ccttttacct acgaggaccc taatgaggct 1860
   gttcgggagt ttgccaagga gatcgacgtg tcctgcgtca agatcgagga ggtgatcgga 1920
   gctggggaat ttggggaagt gtgccgtggt cgactgaaac agcctggccg ccgagaggtg 1980
   tttgtggcca tcaagacgct gaaggtgggc tacaccgaga ggcagcggcg ggacttccta 2040
   agcgaggcct ccatcatggg tcagtttgat caccccaata taatccggct cgagggcgtg 2100
   gtcaccaaaa gtcggccagt tatgatcctc actgagttca tggaaaactg cgccctggac 2160
   tccttcctcc ggctcaacga tgggcagttc acggtcatcc agctggtggg catgttgcgg 2220
   ggcattgctg ccggcatgaa gtacctgtcc gagatgaact atgtgcaccg cgacctggct 2280
   gctcgcaaca tccttgtcaa cagcaacctg gtctgcaaag tctcagactt tggcctctcc 2340
   cgcttcctgg aggatgaccc ctccgatcct acctacacca gttccctggg cgggaagatc 2400
   cccatccgct ggactgcccc agaggccata gcctatcgga agttcacttc tgctagtgat 2460
   gtctggagct acggaattgt catgtgggag gtcatgagct atggagagcg accctactgg 2520
   gacatgagca accaggatgt catcaatgcc gtggagcagg attaccggct gccaccaccc 2580
   atggactgtc ccacagcact gcaccagctc atgctggact gctgggtgcg ggaccggaac 2640
   ctcaggccca aattctccca gattgtcaat accctggaca agctcatccg caatgctgcc 2700
   agcctcaagg tcattgccag cgctcagtct ggcatgtcac agcccctcct ggaccgcacg 2760
   gtcccagatt acacaacctt cacgacagtt ggtgattggc tggatgccat caagatgggg 2820
   cggtacaagg agagcttcgt cagtgcgggg tttgcatctt ttgacctggt ggcccagatg 2880
   acggcagaag acctgctccg tattggggtc accctggccg gccaccagaa gaagatcctg 2940
   agcagtatcc aggacatgcg gctgcagatg aaccagacgc tgcctgtgca ggtctga    2997
<210> 24
   <211> 2964
   <212> DNA
   <213> Homo sapiens
<400> 24
   atggagctcc gggtgctgct ctgctgggct tcgttggccg cagctttgga agagaccctg 60
   ctgaacacaa aattggaaac tgctgatctg aagtgggtga cattccctca ggtggacggg 120
   cagtgggagg aactgagcgg cctggatgag gaacagcaca gcgtgcgcac ctacgaagtg 180
   tgtgaagtgc agcgtgcccc gggccaggcc cactggcttc gcacaggttg ggtcccacgg 240
   cggggcgccg tccacgtgta cgccacgctg cgcttcacca tgctcgagtg cctgtccctg 300
   cctcgggctg ggcgctcctg caaggagacc ttcaccgtct tctactatga gagcgatgcg 360
   gacacggcca cggccctcac gccagcctgg atggagaacc cctacatcaa ggtggacacg 420
   gtggccgcgg agcatctcac ccggaagcgc cctggggccg aggccaccgg gaaggtgaat 480
   gtcaagacgc tgcgtctggg accgctcagc aaggctggct tctacctggc cttccaggac 540
   cagggtgcct gcatggccct gctatccctg cacctcttct acaaaaagtg cgcccagctg 600
   actgtgaacc tgactcgatt cccggagact gtgcctcggg agctggttgt gcccgtggcc 660
   ggtagctgcg tggtggatgc cgtccccgcc cctggcccca gccccagcct ctactgccgt 720
   gaggatggcc agtgggccga acagccggtc acgggctgca gctgtgctcc ggggttcgag 780
   gcagctgagg ggaacaccaa gtgccgagcc tgtgcccagg gcaccttcaa gcccctgtca 840
   ggagaagggt cctgccagcc atgcccagcc aatagccact ctaacaccat tggatctgcc 900
   gtctgccagt gccgcgtcgg ggacttccgg gcacgcacag acccccgggg tgcaccctgc 960
   accacccctc cttcggctcc gcggagcgtg gtttcccgcc tgaacggctc ctccctgcac 1020
   ctggaatgga gtgcccccct ggagtctggt ggccgagagg acctcaccta cgccctccgc 1080
   tgccgggagt gccgacccgg aggctcctgt gcgccctgcg ggggagacct gacttttgac 1140
   cccggccccc gggacctggt ggagccctgg gtggtggttc gagggctacg tccggacttc 1200
   acctatacct ttgaggtcac tgcattgaac ggggtatcct ccttagccac ggggcccgtc 1260
   ccatttgagc ctgtcaatgt caccactgac cgagaggtac ctcctgcagt gtctgacatc 1320
   cgggtgacgc ggtcctcacc cagcagcttg agcctggcct gggctgttcc ccgggcaccc 1380
   agtggggcgt ggctggacta cgaggtcaaa taccatgaga agggcgccga gggtcccagc 1440
   agcgtgcggt tcctgaagac gtcagaaaac cgggcagagc tgcgggggct gaagcgggga 1500
   gccagctacc tggtgcaggt acgggcgcgc tctgaggccg gctacgggcc cttcggccag 1560
   gaacatcaca gccagaccca actggatgag agcgagggct ggcgggagca gctggccctg 1620
   attgcgggca cggcagtcgt gggtgtggtc ctggtcctgg tggtcattgt ggtcgcagtt 1680
   ctctgcctca ggaagcagag caatgggaga gaagcagaat attcggacaa acacggacag 1740
   tatctcatcg gacatggtac taaggtctac atcgacccct tcacttatga agaccctaat 1800
   gaggctgtga gggaatttgc aaaagagatc gatgtctcct acgtcaagat tgaagaggtg 1860
   attggtgcag gtgagtttgg cgaggtgtgc cgggggcggc tcaaggcccc agggaagaag 1920
   gagagctgtg tggcaatcaa gaccctgaag ggtggctaca cggagcggca gcggcgtgag 1980
   tttctgagcg aggcctccat catgggccag ttcgagcacc ccaatatcat ccgcctggag 2040
   ggcgtggtca ccaacagcat gcccgtcatg attctcacag agttcatgga gaacggcgcc 2100
   ctggactcct tcctgcggct aaacgacgga cagttcacag tcatccagct cgtgggcatg 2160
   ctgcggggca tcgcctcggg catgcggtac cttgccgaga tgagctacgt ccaccgagac 2220
   ctggctgctc gcaacatcct agtcaacagc aacctcgtct gcaaagtgtc tgactttggc 2280
   ctttcccgat tcctggagga gaactcttcc gatcccacct acacgagctc cctgggagga 2340
   aagattccca tccgatggac tgccccggag gccattgcct tccggaagtt cacttccgcc 2400
   agtgatgcct ggagttacgg gattgtgatg tgggaggtga tgtcatttgg ggagaggccg 2460
   tactgggaca tgagcaatca ggacgtgatc aatgccattg aacaggacta ccggctgccc 2520
   ccgcccccag actgtcccac ctccctccac cagctcatgc tggactgttg gcagaaagac 2580
   cggaatgccc ggccccgctt cccccaggtg gtcagcgccc tggacaagat gatccggaac 2640
   cccgccagcc tcaaaatcgt ggcccgggag aatggcgggg cctcacaccc tctcctggac 2700
   cagcggcagc ctcactactc agcttttggc tctgtgggcg agtggcttcg ggccatcaaa 2760
   atgggaagat acgaagcccg tttcgcagcc gctggctttg gctccttcga gctggtcagc 2820
   cagatctctg ctgaggacct gctccgaatc ggagtcactc tggcgggaca ccagaagaaa 2880
   atcttggcca gtgtccagca catgaagtcc caggccaagc cgggaacccc gggtgggaca 2940
   ggaggaccgg ccccgcagta ctga                                        2964
<210> 25
   <211> 1041
   <212> DNA
   <213> Homo sapiens
<300>
   <302> ephrin-B1
   <310> NM004429
<400> 25
   atggctcggc ctgggcagcg ttggctcggc aagtggcttg tggcgatggt cgtgtgggcg 60
   ctgtgccggc tcgccacacc gctggccaag aacctggagc ccgtatcctg gagctccctc 120
   aaccccaagt tcctgagtgg gaagggcttg gtgatctatc cgaaaattgg agacaagctg 180
   gacatcatct gcccccgagc agaagcaggg cggccctatg agtactacaa gctgtacctg 240
   gtgcggcctg agcaggcagc tgcctgtagc acagttctcg accccaacgt gttggtcacc 300
   tgcaataggc cagagcagga aatacgcttt accatcaagt tccaggagtt cagccccaac 360
   tacatgggcc tggagttcaa gaagcaccat gattactaca ttacctcaac atccaatgga 420
   agcctggagg ggctggaaaa ccgggagggc ggtgtgtgcc gcacacgcac catgaagatc 480
   atcatgaagg ttgggcaaga tcccaatgct gtgacgcctg agcagctgac taccagcagg 540
   cccagcaagg aggcagacaa cactgtcaag atggccacac aggcccctgg tagtcggggc 600
   tccctgggtg actctgatgg caagcatgag actgtgaacc aggaagagaa gagtggccca 660
   ggtgcaagtg ggggcagcag cggggaccct gatggcttct tcaactccaa ggtggcattg 720
   ttcgcggctg tcggtgccgg ttgcgtcatc ttcctgctca tcatcatctt cctgacggtc 780
   ctactactga agctacgcaa gcggcaccgc aagcacacac agcagcgggc ggctgccctc 840
   tcgctcagta ccctggccag tcccaagggg ggcagtggca cagcgggcac cgagcccagc 900
   gacatcatca ttcccttacg gactacagag aacaactact gcccccacta tgagaaggtg 960
   agtggggact acgggcaccc tgtctacatc gtccaagaga tgccgcccca gagcccggcg 1020
   aacatctact acaaggtctg a 1041
<210> 26
   <211> 1002
   <212> DNA
   <213> Homo sapiens
<300>
<400> 26
   atggctgtga gaagggactc cgtgtggaag tactgctggg gtgttttgat ggttttatgc 60
   agaactgcga tttccaaatc gatagtttta gagcctatct attggaattc ctcgaactcc 120
   aaatttctac ctggacaagg actggtacta tacccacaga taggagacaa attggatatt 180
   atttgcccca aagtggactc taaaactgtt ggccagtatg aatattataa agtttatatg 240
   gttgataaag accaagcaga cagatgcact attaagaagg aaaatacccc tctcctcaac 300
   tgtgccaaac cagaccaaga tatcaaattc accatcaagt ttcaagaatt cagccctaac 360
   ctctggggtc tagaatttca gaagaacaaa gattattaca ttatatctac atcaaatggg 420
   tctttggagg gcctggataa ccaggaggga ggggtgtgcc agacaagagc catgaagatc 480
   ctcatgaaag ttggacaaga tgcaagttct gctggatcaa ccaggaataa agatccaaca 540
   agacgtccag aactagaagc tggtacaaat ggaagaagtt cgacaacaag tccctttgta 600
   aaaccaaatc caggttctag cacagacggc aacagcgccg gacattcggg gaacaacatc 660
   ctcggttccg aagtggcctt atttgcaggg attgcttcag gatgcatcat cttcatcgtc 720
   atcatcatca cgctggtggt cctcttgctg aagtaccgga ggagacacag gaagcactcg 780
   ccgcagcaca cgaccacgct gtcgctcagc acactggcca cacccaagcg cagcggcaac 840
   aacaacggct cagagcccag tgacattatc atcccgctaa ggactgcgga cagcgtcttc 900
   tgccctcact acgagaaggt cagcggcgac tacgggcacc cggtgtacat cgtccaggag 960
   atgcccccgc agagcccggc gaacatttac tacaaggtct ga 1002
<210> 27
   <211> 1023
   <212> DNA
   <213> Homo sapiens
<400> 27
   atggggcccc cccattctgg gccggggggc gtgcgagtcg gggccctgct gctgctgggg 60
   gttttggggc tggtgtctgg gctcagcctg gagcctgtct actggaactc ggcgaataag 120
   aggttccagg cagagggtgg ttatgtgctg taccctcaga tcggggaccg gctagacctg 180
   ctctgccccc gggcccggcc tcctggccct cactcctctc ctaattatga gttctacaag 240
   ctgtacctgg tagggggtgc tcagggccgg cgctgtgagg caccccctgc cccaaacctc 300
   cttctcactt gtgatcgccc agacctggat ctccgcttca ccatcaagtt ccaggagtat 360
   agccctaatc tctggggcca cgagttccgc tcgcaccacg attactacat cattgccaca 420
   tcggatggga cccgggaggg cctggagagc ctgcagggag gtgtgtgcct aaccagaggc 480
   atgaaggtgc ttctccgagt gggacaaagt ccccgaggag gggctgtccc ccgaaaacct 540
   gtgtctgaaa tgcccatgga aagagaccga ggggcagccc acagcctgga gcctgggaag 600
   gagaacctgc caggtgaccc caccagcaat gcaacctccc ggggtgctga aggccccctg 660
   ccccctccca gcatgcctgc agtggctggg gcagcagggg ggctggcgct gctcttgctg 720
   ggcgtggcag gggctggggg tgccatgtgt tggcggagac ggcgggccaa gccttcggag 780
   agtcgccacc ctggtcctgg ctccttcggg aggggagggt ctctgggcct ggggggtgga 840
   ggtgggatgg gacctcggga ggctgagcct ggggagctag ggatagctct gcggggtggc 900
   ggggctgcag atcccccctt ctgcccccac tatgagaagg tgagtggtga ctatgggcat 960
   cctgtgtata tcgtgcagga tgggcccccc cagagccctc caaacatcta ctacaaggta 1020
   tga 1023
<210> 28
   <211> 3399
   <212> DNA
   <213> Homo sapiens
<300>
   <302> telomerase reverse transcriptase
   <310> AF015950
<400> 28
<210> 29
   <211> 567
   <212> DNA
   <213> Homo sapiens
<300>
   <302> K-ras
   <310> M54968
<400> 29
   atgactgaat ataaacttgt ggtagttgga gcttgtggcg taggcaagag tgccttgacg 60
   atacagctaa ttcagaatca ttttgtggac gaatatgatc caacaataga ggattcctac 120
   aggaagcaag tagtaattga tggagaaacc tgtctcttgg atattctcga cacagcaggt 180
   caagaggagt acagtgcaat gagggaccag tacatgagga ctggggaggg ctttctttgt 240
   gtatttgcca taaataatac taaatcattt gaagatattc accattatag agaacaaatt 300
   aaaagagtta aggactctga agatgtacct atggtcctag taggaaataa atgtgatttg 360
   ccttctagaa cagtagacac aaaacaggct caggacttag caagaagtta tggaattcct 420
   tttattgaaa catcagcaaa gacaagacag ggtgttgatg atgccttcta tacattagtt 480
   cgagaaattc gaaaacataa agaaaagatg agcaaagatg gtaaaaagaa gaaaaagaag 540
   tcaaagacaa agtgtgtaat tatgtaa                                     567
<210> 30
   <211> 3840
   <212> DNA
   <213> Homo sapiens
<300>
   <302> mdr-1
   <310> AF016535
<400> 30
   atggatcttg aaggggaccg caatggagga gcaaagaaga agaacttttt taaactgaac 60
   aataaaagtg aaaaagataa gaaggaaaag aaaccaactg tcagtgtatt ttcaatgttt 120
   cgctattcaa attggcttga caagttgtat atggtggtgg gaactttggc tgccatcatc 180
   catggggctg gacttcctct catgatgctg gtgtttggag aaatgacaga tatctttgca 240
   aatgcaggaa atttagaaga tctgatgtca aacatcacta atagaagtga tatcaatgat 300
   acagggttct tcatgaatct ggaggaagac atgaccaggt atgcctatta ttacagtgga 360
   attggtgctg gggtgctggt tgctgcttac attcaggttt cattttggtg cctggcagct 420
   ggaagacaaa tacacaaaat tagaaaacag ttttttcatg ctataatgcg acaggagata 480
   ggctggtttg atgtgcacga tgttggggag cttaacaccc gacttacaga tgatgtctcc 540
   aagattaatg aaggaattgg tgacaaaatt ggaatgttct ttcagtcaat ggcaacattt 600
   ttcactgggt ttatagtagg atttacacgt ggttggaagc taacccttgt gattttggcc 660
   atcagtcctg ttcttggact gtcagctgct gtctgggcaa agatactatc ttcatttact 720
   gataaagaac tcttagcgta tgcaaaagct ggagcagtag ctgaagaggt cttggcagca 780
   attagaactg tgattgcatt tggaggacaa aagaaagaac ttgaaaggta caacaaaaat 840
   ttagaagaag ctaaaagaat tgggataaag aaagctatta cagccaatat ttctataggt 900
   gctgctttcc tgctgatcta tgcatcttat gctctggcct tctggtatgg gaccaccttg 960
   gtcctctcag gggaatattc tattggacaa gtactcactg tattttctgt attaattggg 1020
   gcttttagtg ttggacaggc atctccaagc attgaagcat ttgcaaatgc aagaggagca 1080
   gcttatgaaa tcttcaagat aattgataat aagccaagta ttgacagcta ttcgaagagt 1140
   gggcacaaac cagataatat taagggaaat ttggaattca gaaatgttca cttcagttac 1200
   ccatctcgaa aagaagttaa gatcttgaag ggtctgaacc tgaaggtgca gagtgggcag 1260
   acggtggccc tggttggaaa cagtggctgt gggaagagca caacagtcca gctgatgcag 1320
   aggctctatg accccacaga ggggatggtc agtgttgatg gacaggatat taggaccata 1380
   aatgtaaggt ttctacggga aatcattggt gtggtgagtc aggaacctgt attgtttgcc 1440
   accacgatag ctgaaaacat tcgctatggc cgtgaaaatg tcaccatgga tgagattgag 1500
   aaagctgtca aggaagccaa tgcctatgac tttatcatga aactgcctca taaatttgac 1560
   accctggttg gagagagagg ggcccagttg agtggtgggc agaagcagag gatcgccatt 1620
   gcacgtgccc tggttcgcaa ccccaagatc ctcctgctgg atgaggccac gtcagccttg 1680
   gacacagaaa gcgaagcagt ggttcaggtg gctctggata aggccagaaa aggtcggacc 1740
   accattgtga tagctcatcg tttgtctaca gttcgtaatg ctgacgtcat cgctggtttc 1800
   gatgatggag tcattgtgga gaaaggaaat catgatgaac tcatgaaaga gaaaggcatt 1860
   tacttcaaac ttgtcacaat gcagacagca ggaaatgaag ttgaattaga aaatgcagct 1920
   gatgaatcca aaagtgaaat tgatgccttg gaaatgtctt caaatgattc aagatccagt 1980
   ctaataagaa aaagatcaac tcgtaggagt gtccgtggat cacaagccca agacagaaag 2040
   cttagtacca aagaggctct ggatgaaagt atacctccag tttccttttg gaggattatg 2100
   aagctaaatt taactgaatg gccttatttt gttgttggtg tattttgtgc cattataaat 2160
   ggaggcctgc aaccagcatt tgcaataata ttttcaaaga ttataggggt ttttacaaga 2220
   attgatgatc ctgaaacaaa acgacagaat agtaacttgt tttcactatt gtttctagcc 2280
   cttggaatta tttcttttat tacatttttc cttcagggtt tcacatttgg caaagctgga 2340
   gagatcctca ccaagcggct ccgatacatg gttttccgat ccatgctcag acaggatgtg 2400
   agttggtttg atgaccctaa aaacaccact ggagcattga ctaccaggct cgccaatgat 2460
   gctgctcaag ttaaaggggc tataggttcc aggcttgctg taattaccca gaatatagca 2520
   aatcttggga caggaataat tatatccttc atctatggtt ggcaactaac actgttactc 2580
   ttagcaattg tacccatcat tgcaatagca ggagttgttg aaatgaaaat gttgtctgga 2640
   caagcactga aagataagaa agaactagaa ggtgctggga agatcgctac tgaagcaata 2700
   gaaaacttcc gaaccgttgt ttctttgact caggagcaga agtttgaaca tatgtatgct 2760
   cagagtttgc aggtaccata cagaaactct ttgaggaaag cacacatctt tggaattaca 2820
   ttttccttca cccaggcaat gatgtatttt tcctatgctg gatgtttccg gtttggagcc 2880
   tacttggtgg cacataaact catgagcttt gaggatgttc tgttagtatt ttcagctgtt 2940
   gtctttggtg ccatggccgt ggggcaagtc agttcatttg ctcctgacta tgccaaagcc 3000
   aaaatatcag cagcccacat catcatgatc attgaaaaaa cccctttgat tgacagctac 3060
   agcacggaag gcctaatgcc gaacacattg gaaggaaatg tcacatttgg tgaagttgta 3120
   ttcaactatc ccacccgacc ggacatccca gtgcttcagg gactgagcct ggaggtgaag 3180
   aagggccaga cgctggctct ggtgggcagc agtggctgtg ggaagagcac agtggtccag 3240
   ctcctggagc ggttctacga ccccttggca gggaaagtgc tgcttgatgg caaagaaata 3300
   aagcgactga atgttcagtg gctccgagca cacctgggca tcgtgtccca ggagcccatc 3360
   ctgtttgact gcagcattgc tgagaacatt gcctatggag acaacagccg ggtggtgtca 3420
   caggaagaga ttgtgagggc agcaaaggag gccaacatac atgccttcat cgagtcactg 3480
   cctaataaat atagcactaa agtaggagac aaaggaactc agctctctgg tggccagaaa 3540
   caacgcattg ccatagctcg tgcccttgtt agacagcctc atattttgct tttggatgaa 3600
   gccacgtcag ctctggatac agaaagtgaa aaggttgtcc aagaagccct ggacaaagcc 3660
   agagaaggcc gcacctgcat tgtgattgct caccgcctgt ccaccatcca gaatgcagac 3720
   ttaatagtgg tgtttcagaa tggcagagtc aaggagcatg gcacgcatca gcagctgctg 3780
   gcacagaaag gcatctattt ttcaatggtc agtgtccagg ctggaacaaa gcgccagtga 3840
<210> 31
   <211> 1318
   <212> DNA
   <213> Homo sapiens
<300>
   <302> UPAR (urokinase-type plasminogen activator receptor)
   <310> XM009232
<400> 31
   atgggtcacc cgccgctgct gccgctgctg ctgctgctcc acacctgcgt cccagcctct 60
   tggggcctgc ggtgcatgca gtgtaagacc aacggggatt gccgtgtgga agagtgcgcc 120
   ctgggacagg acctctgcag gaccacgatc gtgcgcttgt gggaagaagg agaagagctg 180
   gagctggtgg agaaaagctg tacccactca gagaagacca acaggaccct gagctatcgg 240
   actggcttga agatcaccag ccttaccgag gttgtgtgtg ggttagactt gtgcaaccag 300
   ggcaactctg gccgggctgt cacctattcc cgaagccgtt acctcgaatg catttcctgt 360
   ggctcatcag acatgagctg tgagaggggc cggcaccaga gcctgcagtg ccgcagccct 420
   gaagaacagt gcctggatgt ggtgacccac tggatccagg aaggtgaaga agggcgtcca 480
   aaggatgacc gccacctccg tggctgtggc taccttcccg gctgcccggg ctccaatggt 540
   ttccacaaca acgacacctt ccacttcctg aaatgctgca acaccaccaa atgcaacgag 600
   ggcccaatcc tggagcttga aaatctgccg cagaatggcc gccagtgtta cagctgcaag 660
   gggaacagca cccatggatg ctcctctgaa gagactttcc tcattgactg ccgaggcccc 720
   atgaatcaat gtctggtagc caccggcact cacgaaccga aaaaccaaag ctatatggta 780
   agaggctgtg caaccgcctc aatgtgccaa catgcccacc tgggtgacgc cttcagcatg 840
   aaccacattg atgtctcctg ctgtactaaa agtggctgta accacccaga cctggatgtc 900
   cagtaccgca gtggggctgc tcctcagcct ggccctgccc atctcagcct caccatcacc 960
   ctgctaatga ctgccagact gtggggaggc actctcctct ggacctaaac ctgaaatccc 1020
   cctctctgcc ctggctggat ccgggggacc cctttgccct tccctcggct cccagcccta 1080
   cagacttgct gtgtgacctc aggccagtgt gccgacctct ctgggcctca gttttcccag 1140
   ctatgaaaac agctatctca caaagttgtg tgaagcagaa gagaaaagct ggaggaaggc 1200
   cgtgggccaa tgggagagct cttgttatta ttaatattgt tgccgctgtt gtgttgttgt 1260
   tattaattaa tattcatatt atttatttta tacttacata aagattttgt accagtgg 1318
<210> 32
   <211> 636
   <212> DNA
   <213> Homo sapiens
<300>
   <302> Bak
   <310> U16811
<400> 32
   atggcttcgg ggcaaggccc aggtcctccc aggcaggagt gcggagagcc tgccctgccc 60
   tctgcttctg aggagcaggt agcccaggac acagaggagg ttttccgcag ctacgttttt 120
   taccgccatc agcaggaaca ggaggctgaa ggggtggctg cccctgccga cccagagatg 180
   gtcaccttac ctctgcaacc tagcagcacc atggggcagg tgggacggca gctcgccatc 240
   atcggggacg acatcaaccg acgctatgac tcagagttcc agaccatgtt gcagcacctg 300
   cagcccacgg cagagaatgc ctatgagtac ttcaccaaga ttgccaccag cctgtttgag 360
   agtggcatca attggggccg tgtggtggct cttctgggct tcggctaccg tctggcccta 420
   cacgtctacc agcatggcct gactggcttc ctaggccagg tgacccgctt cgtggtcgac 480
   ttcatgctgc atcactgcat tgcccggtgg attgcacaga ggggtggctg ggtggcagcc 540
   ctgaacttgg gcaatggtcc catcctgaac gtgctggtgg ttctgggtgt ggttctgttg 600
   ggccagtttg tggtacgaag attcttcaaa tcatga 636
<210> 33
   <211> 579
   <212> DNA
   <213> Homo sapiens
<300>
   <302> Bax alpha
   <310> L22473
<400> 33
   atggacgggt ccggggagca gcccagaggc ggggggccca ccagctctga gcagatcatg 60
   aagacagggg cccttttgct tcagggtttc atccaggatc gagcagggcg aatggggggg 120
   gaggcacccg agctggccct ggacccggtg cctcaggatg cgtccaccaa gaagctgagc 180
   gagtgtctca agcgcatcgg ggacgaactg gacagtaaca tggagctgca gaggatgatt 240
   gccgccgtgg acacagactc cccccgagag gtctttttcc gagtggcagc tgacatgttt 300
   tctgacggca acttcaactg gggccgggtt gtcgcccttt tctactttgc cagcaaactg 360
   gtgctcaagg ccctgtgcac caaggtgccg gaactgatca gaaccatcat gggctggaca 420
   ttggacttcc tccgggagcg gctgttgggc tggatccaag accagggtgg ttgggacggc 480
   ctcctctcct actttgggac gcccacgtgg cagaccgtga ccatctttgt ggcgggagtg 540
   ctcaccgcct cgctcaccat ctggaagaag atgggctga 579
<210> 34
   <211> 657
   <212> DNA
   <213> Homo sapiens
<300>
   <302> Bax beta
   <310> L22474
<400> 34
   atggacgggt ccggggagca gcccagaggc ggggggccca ccagctctga gcagatcatg 60
   aagacagggg cccttttgct tcagggtttc atccaggatc gagcagggcg aatggggggg 120
   gaggcacccg agctggccct ggacccggtg cctcaggatg cgtccaccaa gaagctgagc 180
   gagtgtctca agcgcatcgg ggacgaactg gacagtaaca tggagctgca gaggatgatt 240
   gccgccgtgg acacagactc cccccgagag gtctttttcc gagtggcagc tgacatgttt 300
   tctgacggca acttcaactg gggccgggtt gtcgcccttt tctactttgc cagcaaactg 360
   gtgctcaagg ccctgtgcac caaggtgccg gaactgatca gaaccatcat gggctggaca 420
   ttggacttcc tccgggagcg gctgttgggc tggatccaag accagggtgg ttgggtgaga 480
   ctcctcaagc ctcctcaccc ccaccaccgc gccctcacca ccgcccctgc cccaccgtcc 540
   ctgccccccg ccactcctct gggaccctgg gccttctgga gcaggtcaca gtggtgccct 600
   ctccccatct tcagatcatc agatgtggtc tataatgcgt tttccttacg tgtctga 657
<210> 35
   <211> 432
   <212> DNA
   <213> Homo sapiens
<300>
   <302> Bax delta
   <310> U19599
<400> 35
   atggacgggt ccggggagca gcccagaggc gggggcccca ccagctctga gcagatcatg 60
   aagacagggg cccttttgct tcaggggatg attgccgccg tggacacaga ctccccccga 120
   gaggtctttt tccgagtggc agctgacatg ttttctgacg gcaacttcaa ctggggccgg 180
   gttgtcgccc ttttctactt tgccagcaaa ctggtgctca aggccctgtg caccaaggtg 240
   ccggaactga tcagaaccat catgggctgg acattggact tcctccggga gcggctgttg 300
   ggctggatcc aagaccaggg tggttgggac ggcctcctct cctactttgg gacgcccacg 360
   tggcagaccg tgaccatctt tgtggcggga gtgctcaccg cctcgctcac catctggaag 420
   aagatgggct ga 432
<210> 36
   <211> 495
   <212> DNA
   <213> Homo sapiens
<300>
   <302> Bax epsolin
   <310> AF007826
<400> 36
   atggacgggt ccggggagca gcccagaggc gggggcccca ccagctctga gcagatcatg 60
   aagacagggg cccttttgct tcagggtttc atccaggatc gagcagggcg aatggggggg 120
   gaggcacccg agctggccct ggacccggtg cctcaggatg cgtccaccaa gaagctgagc 180
   gagtgtctca agcgcatcgg ggacgaactg gacagtaaca tggagctgca gaggatgatt 240
   gccgccgtgg acacagactc cccccgagag gtctttttcc gagtggcagc tgacatgttt 300
   tctgacggca acttcaactg gggccgggtt gtcgcccttt tctactttgc cagcaaactg 360
   gtgctcaagg ctggcgtgaa atggcgtgat ctgggctcac tgcaacctct gcctcctggg 420
   ttcaagcgat tcacctgcct cagcatccca aggagctggg attacaggcc ctgtgcacca 480
   aggtgccgga actga 495
<210> 37
   <211> 582
   <212> DNA
   <213> Homo sapiens
<300>
   <302> bcl-w
   <310> U59747
<400> 37
   atggcgaccc cagcctcggc cccagacaca cgggctctgg tggcagactt tgtaggttat 60
   aagctgaggc agaagggtta tgtctgtgga gctggccccg ggagggcccc agcagctgac 120
   ccgctgcacc aagccatgcg ggcagctgga gatgagttcg agacccgctt ccggcgcacc 180
   ttctctgatc tggcggctca gctgcatgtg accccaggct cagcccagca acgcttcacc 240
   caggtctccg acgaactttt tcaagggggc cccaactggg gccgccttgt agccttcttt 300
   gtctttgggg ctgcactgtg tgctgagagt gtcaacaagg agatggaacc actggtggga 360
   caagtgcagg agtggatggt ggcctacctg gagacgcggc tggctgactg gatccacagc 420
   agtgggggct gggcggagtt cacagctcta tacggggacg gggccctgga ggaggcgcgg 480
   cgtctgcggg aggggaactg ggcatcagtg aggacagtgc tgacgggggc cgtggcactg 540
   ggggccctgg taactgtagg ggcctttttt gctagcaagt ga 582
<210> 38
   <211> 2481
   <212> DNA
   <213> Homo sapiens
<300>
   <302> HIF-alpha
   <310> U22431
<400> 38
   atggagggcg ccggcggcgc gaacgacaag aaaaagataa gttctgaacg tcgaaaagaa 60
   aagtctcgag atgcagccag atctcggcga agtaaagaat ctgaagtttt ttatgagctt 120
   gctcatcagt tgccacttcc acataatgtg agttcgcatc ttgataaggc ctctgtgatg 180
   aggcttacca tcagctattt gcgtgtgagg aaacttctgg atgctggtga tttggatatt 240
   gaagatgaca tgaaagcaca gatgaattgc ttttatttga aagccttgga tggttttgtt 300
   atggttctca cagatgatgg tgacatgatt tacatttctg ataatgtgaa caaatacatg 360
   ggattaactc agtttgaact aactggacac agtgtgtttg attttactca tccatgtgac 420
   catgaggaaa tgagagaaat gcttacacac agaaatggcc ttgtgaaaaa gggtaaagaa 480
   caaaacacac agcgaagctt ttttctcaga atgaagtgta ccctaactag ccgaggaaga 540
   actatgaaca taaagtctgc aacatggaag gtattgcact gcacaggcca cattcacgta 600
   tatgatacca acagtaacca acctcagtgt gggtataaga aaccacctat gacctgcttg 660
   gtgctgattt gtgaacccat tcctcaccca tcaaatattg aaattccttt agatagcaag 720
   actttcctca gtcgacacag cctggatatg aaattttctt attgtgatga aagaattacc 780
   gaattgatgg gatatgagcc agaagaactt ttaggccgct caatttatga atattatcat 840
   gctttggact ctgatcatct gaccaaaact catcatgata tgtttactaa aggacaagtc 900
   accacaggac agtacaggat gcttgccaaa agaggtggat atgtctgggt tgaaactcaa 960
   gcaactgtca tatataacac caagaattct caaccacagt gcattgtatg tgtgaattac 1020
   gttgtgagtg gtattattca gcacgacttg attttctccc ttcaacaaac agaatgtgtc 1080
   cttaaaccgg ttgaatcttc agatatgaaa atgactcagc tattcaccaa agttgaatca 1140
   gaagatacaa gtagcctctt tgacaaactt aagaaggaac ctgatgcttt aactttgctg 1200
   gccccagccg ctggagacac aatcatatct ttagattttg gcagcaacga cacagaaact 1260
   gatgaccagc aacttgagga agtaccatta tataatgatg taatgctccc ctcacccaac 1320
   gaaaaattac agaatataaa tttggcaatg tctccattac ccaccgctga aacgccaaag 1380
   ccacttcgaa gtagtgctga ccctgcactc aatcaagaag ttgcattaaa attagaacca 1440
   aatccagagt cactggaact ttcttttacc atgccccaga ttcaggatca gacacctagt 1500
   ccttccgatg gaagcactag acaaagttca cctgagccta atagtcccag tgaatattgt 1560
   ttttatgtgg atagtgatat ggtcaatgaa ttcaagttgg aattggtaga aaaacttttt 1620
   gctgaagaca cagaagcaaa gaacccattt tctactcagg acacagattt agacttggag 1680
   atgttagctc cctatatccc aatggatgat gacttccagt tacgttcctt cgatcagttg 1740
   tcaccattag aaagcagttc cgcaagccct gaaagcgcaa gtcctcaaag cacagttaca 1800
   gtattccagc agactcaaat acaagaacct actgctaatg ccaccactac cactgccacc 1860
   actgatgaat taaaaacagt gacaaaagac cgtatggaag acattaaaat attgattgca 1920
   tctccatctc ctacccacat acataaagaa actactagtg ccacatcatc accatataga 1980
   gatactcaaa gtcggacagc ctcaccaaac agagcaggaa aaggagtcat agaacagaca 2040
   gaaaaatctc atccaagaag ccctaacgtg ttatctgtcg ctttgagtca aagaactaca 2100
   gttcctgagg aagaactaaa tccaaagata ctagctttgc agaatgctca gagaaagcga 2160
   aaaatggaac atgatggttc actttttcaa gcagtaggaa ttggaacatt attacagcag 2220
   ccagacgatc atgcagctac tacatcactt tcttggaaac gtgtaaaagg atgcaaatct 2280
   agtgaacaga atggaatgga gcaaaagaca attattttaa taccctctga tttagcatgt 2340
   agactgctgg ggcaatcaat ggatgaaagt ggattaccac agctgaccag ttatgattgt 2400
   gaagttaatg ctcctataca aggcagcaga aacctactgc agggtgaaga attactcaga 2460
   gctttggatc aagttaactg a                                            2481
<210> 39
   <211> 481
   <212> DNA
   <213> Homo sapiens
<300>
   <302> ID1
   <310> X77956
<400> 39
   atgaaagtcg ccagtggcag caccgccacc gccgccgcgg gccccagctg cgcgctgaag 60
   gccggcaaga cagcgagcgg tgcgggcgag gtggtgcgct gtctgtctga gcagagcgtg 120
   gccatctcgc gctgccgggg cgccggggcg cgcctgcctg ccctgctgga cgagcagcag 180
   gtaaacgtgc tgctctacga catgaacggc tgttactcac gcctcaagga gctggtgccc 240
   accctgcccc agaaccgcaa ggtgagcaag gtggagattc tccagcacgt catcgactac 300
   atcagggacc ttcagttgga gctgaactcg gaatccgaag ttgggacccc cgggggccga 360
   gggctgccgg tccgggctcc gctcagcacc ctcaacggcg agatcagcgc cctgacggcc 420
   gaggcggcat gcgttcctgc ggacgatcgc atcttgtgtc gctgaatggt gaaaaaaaaa 480
   a                                                                 481
<210> 40
   <211> 110
   <212> DNA
   <213> Homo sapiens
<300>
   <302> ID2B
   <310> M96843
<400> 40
   tgaaagcctt cagtcccgtg aggtccatta ggaaaaacag cctgttggac caccgcctgg 60
   gcatctccca gagcaaaacc ccggtggatg acctgatgag cctgctgtaa            110
<210> 41
   <211> 486
   <212> DNA
   <213> Homo sapiens
<300>
   <302> ID4
   <310> Y07958
<400> 41
   atgaaggcgg tgagcccggt gcgcccctcg ggccgcaagg cgccgtcggg ctgcggcggc 60
   ggggagctgg cgctgcgctg cctggccgag cacggccaca gcctgggtgg ctccgcagcc 120
   gcggcggcgg cggcggcggc agcgcgctgt aaggcggccg aggcggcggc cgacgagccg 180
   gcgctgtgcc tgcagtgcga tatgaacgac tgctatagcc gcctgcggag gctggtgccc 240
   accatcccgc ccaacaagaa agtcagcaaa gtggagatcc tgcagcacgt tatcgactac 300
   atcctggacc tgcagctggc gctggagacg cacccggccc tgctgaggca gccaccaccg 360
   cccgcgccgc cacaccaccc ggccgggacc tgtccagccg cgccgccgcg gaccccgctc 420
   actgcgctca acaccgaccc ggccggcgcg gtgaacaagc agggcgacag cattctgtgc 480
   cgctga                                                            486
<210> 42
   <211> 462
   <212> DNA
   <213> Homo sapiens
<300>
   <302> IGF1
   <310> NM000618
<400> 42
   atgggaaaaa tcagcagtct tccaacccaa ttatttaagt gctgcttttg tgatttcttg 60
   aaggtgaaga tgcacaccat gtcctcctcg catctcttct acctggcgct gtgcctgctc 120
   accttcacca gctctgccac ggctggaccg gagacgctct gcggggctga gctggtggat 180
   gctcttcagt tcgtgtgtgg agacaggggc ttttatttca acaagcccac agggtatggc 240
   tccagcagtc ggagggcgcc tcagacaggc atcgtggatg agtgctgctt ccggagctgt 300
   gatctaagga ggctggagat gtattgcgca cccctcaagc ctgccaagtc agctcgctct 360
   gtccgtgccc agcgccacac cgacatgccc aagacccaga aggaagtaca tttgaagaac 420
   gcaagtagag ggagtgcagg aaacaagaac tacaggatgt ag 462
<210> 43
   <211> 591
   <212> DNA
   <213> Homo sapiens
<300>
   <302> PDGFA
   <310> NM002607
<400> 43
   atgaggacct tggcttgcct gctgctcctc ggctgcggat acctcgccca tgttctggcc 60
   gaggaagccg agatcccccg cgaggtgatc gagaggctgg cccgcagtca gatccacagc 120
   atccgggacc tccagcgact cctggagata gactccgtag ggagtgagga ttctttggac 180
   accagcctga gagctcacgg ggtccacgcc actaagcatg tgcccgagaa gcggcccctg 240
   cccattcgga ggaagagaag catcgaggaa gctgtccccg ctgtctgcaa gaccaggacg 300
   gtcatttacg agattcctcg gagtcaggtc gaccccacgt ccgccaactt cctgatctgg 360
   cccccgtgcg tggaggtgaa acgctgcacc ggctgctgca acacgagcag tgtcaagtgc 420
   cagccctccc gcgtccacca ccgcagcgtc aaggtggcca aggtggaata cgtcaggaag 480
   aagccaaaat taaaagaagt ccaggtgagg ttagaggagc atttggagtg cgcctgcgcg 540
   accacaagcc tgaatccgga ttatcgggaa gaggacacgg atgtgaggtg a 591
<210> 44
   <211> 528
   <212> DNA
   <213> Homo sapiens
<300>
   <302> PDGFRA
   <310> XM003568
<400> 44
   atggccaagc ctgaccacgc taccagtgaa gtctacgaga tcatggtgaa atgctggaac 60
   agtgagccgg agaagagacc ctccttttac cacctgagtg agattgtgga gaatctgctg 120
   cctggacaat ataaaaagag ttatgaaaaa attcacctgg acttcctgaa gagtgaccat 180
   cctgctgtgg cacgcatgcg tgtggactca gacaatgcat acattggtgt cacctacaaa 240
   aacgaggaag acaagctgaa ggactgggag ggtggtctgg atgagcagag actgagcgct 300
   gacagtggct acatcattcc tctgcctgac attgaccctg tccctgagga ggaggacctg 360
   ggcaagagga acagacacag ctcgcagacc tctgaagaga gtgccattga gacgggttcc 420
   agcagttcca ccttcatcaa gagagaggac gagaccattg aagacatcga catgatggat 480
   gacatcggca tagactcttc agacctggtg gaagacagct tcctgtaa 528
<210> 45
   <211> 1911
   <212> DNA
   <213> Homo sapiens
<300>
   <302> PDGFRB
   <310> XM003790
<400> 45
   atgcggcttc cgggtgcgat gccagctctg gccctcaaag gcgagctgct gttgctgtct 60
   ctcctgttac ttctggaacc acagatctct cagggcctgg tcgtcacacc cccggggcca 120
   gagcttgtcc tcaatgtctc cagcaccttc gttctgacct gctcgggttc agctccggtg 180
   gtgtgggaac ggatgtccca ggagccccca caggaaatgg ccaaggccca ggatggcacc 240
   ttctccagcg tgctcacact gaccaacctc actgggctag acacgggaga atacttttgc 300
   acccacaatg actcccgtgg actggagacc gatgagcgga aacggctcta catctttgtg 360
   ccagatccca ccgtgggctt cctccctaat gatgccgagg aactattcat ctttctcacg 420
   gaaataactg agatcaccat tccatgccga gtaacagacc cacagctggt ggtgacactg 480
   cacgagaaga aaggggacgt tgcactgcct gtcccctatg atcaccaacg tggcttttct 540
   ggtatctttg aggacagaag ctacatctgc aaaaccacca ttggggacag ggaggtggat 600
   tctgatgcct actatgtcta cagactccag gtgtcatcca tcaacgtctc tgtgaacgca 660
   gtgcagactg tggtccgcca gggtgagaac atcaccctca tgtgcattgt gatcgggaat 720
   gaggtggtca acttcgagtg gacatacccc cgcaaagaaa gtgggcggct ggtggagccg 780
   gtgactgact tcctcttgga tatgccttac cacatccgct ccatcctgca catccccagt 840
   gccgagttag aagactcggg gacctacacc tgcaatgtga cggagagtgt gaatgaccat 900
   caggatgaaa aggccatcaa catcaccgtg gttgagagcg gctacgtgcg gctcctggga 960
   gaggtgggca cactacaatt tgctgagctg catcggagcc ggacactgca ggtagtgttc 1020
   gaggcctacc caccgcccac tgtcctgtgg ttcaaagaca accgcaccct gggcgactcc 1080
   agcgctggcg aaatcgccct gtccacgcgc aacgtgtcgg agacccggta tgtgtcagag 1140
   ctgacactgg ttcgcgtgaa ggtggcagag gctggccact acaccatgcg ggccttccat 1200
   gaggatgctg aggtccagct ctccttccag ctacagatca atgtccctgt ccgagtgctg 1260
   gagctaagtg agagccaccc tgacagtggg gaacagacag tccgctgtcg tggccggggc 1320
   atgccccagc cgaacatcat ctggtctgcc tgcagagacc tcaaaaggtg tccacgtgag 1380
   ctgccgccca cgctgctggg gaacagttcc gaagaggaga gccagctgga gactaacgtg 1440
   acgtactggg aggaggagca ggagtttgag gtggtgagca cactgcgtct gcagcacgtg 1500
   gatcggccac tgtcggtgcg ctgcacgctg cgcaacgctg tgggccagga cacgcaggag 1560
   gtcatcgtgg tgccacactc cttgcccttt aaggtggtgg tgatctcagc catcctggcc 1620
   ctggtggtgc tcaccatcat ctcccttatc atcctcatca tgctttggca gaagaagcca 1680
   cgttacgaga tccgatggaa ggtgattgag tctgtgagct ctgacggcca tgagtacatc 1740
   tacgtggacc ccatgcagct gccctatgac tccacgtggg agctgccgcg ggaccagctt 1800
   gtgctgggac gcaccctcgg ctctggggcc tttgggcagg tggtggaggc cacggttcat 1860
   ggcctgagcc attttcaagc cccaatgaaa gtggccgtca aaaatgctta a          1911
<210> 46
   <211> 1176
   <212> DNA
   <213> Homo sapiens
<300>
   <302> TGFbetal
   <310> NM000660
<400> 45
<210> 47
   <211> 1245
   <212> DNA
   <213> Homo sapiens
<300>
   <302> TGFbeta2
   <310> NM003238
<400> 47
   atgcactact gtgtgctgag cgcttttctg atcctgcatc tggtcacggt cgcgctcagc 60
   ctgtctacct gcagcacact cgatatggac cagttcatgc gcaagaggat cgaggcgatc 120
   cgcgggcaga tcctgagcaa gctgaagctc accagtcccc cagaagacta tcctgagccc 180
   gaggaagtcc ccccggaggt gatttccatc tacaacagca ccagggactt gctccaggag 240
   aaggcgagcc ggagggcggc cgcctgcgag cgcgagagga gcgacgaaga gtactacgcc 300
   aaggaggttt acaaaataga catgccgccc ttcttcccct ccgaaaatgc catcccgccc 360
   actttctaca gaccctactt cagaattgtt cgatttgacg tctcagcaat ggagaagaat 420
   gcttccaatt tggtgaaagc agagttcaga gtctttcgtt tgcagaaccc aaaagccaga 480
   gtgcctgaac aacggattga gctatatcag attctcaagt ccaaagattt aacatctcca 540
   acccagcgct acatcgacag caaagttgtg aaaacaagag cagaaggcga atggctctcc 600
   ttcgatgtaa ctgatgctgt tcatgaatgg cttcaccata aagacaggaa cctgggattt 660
   aaaataagct tacactgtcc ctgctgcact tttgtaccat ctaataatta catcatccca 720
   aataaaagtg aagaactaga agcaagattt gcaggtattg atggcacctc cacatatacc 780
   agtggtgatc agaaaactat aaagtccact aggaaaaaaa acagtgggaa gaccccacat 840
   ctcctgctaa tgttattgcc ctcctacaga cttgagtcac aacagaccaa ccggcggaag 900
   aagcgtgctt tggatgcggc ctattgcttt agaaatgtgc aggataattg ctgcctacgt 960
   ccactttaca ttgatttcaa gagggatcta gggtggaaat ggatacacga acccaaaggg 1020
   tacaatgcca acttctgtgc tggagcatgc ccgtatttat ggagttcaga cactcagcac 1080
   agcagggtcc tgagcttata taataccata aatccagaag catctgcttc tccttgctgc 1140
   gtgtcccaag atttagaacc tctaaccatt ctctactaca ttggcaaaac acccaagatt 1200
   gaacagcttt ctaatatgat tgtaaagtct tgcaaatgca gctaa                1245
<210> 48
   <211> 1239
   <212> DNA
   <213> Homo sapiens
<300>
   <302> TGFbeta3
   <310> XM007417
<400> 48
   atgaagatgc acttgcaaag ggctctggtg gtcctggccc tgctgaactt tgccacggtc 60
   agcctctctc tgtccacttg caccaccttg gacttcggcc acatcaagaa gaagagggtg 120
   gaagccatta ggggacagat cttgagcaag ctcaggctca ccagcccccc tgagccaacg 180
   gtgatgaccc acgtccccta tcaggtcctg gccctttaca acagcacccg ggagctgctg 240
   gaggagatgc atggggagag ggaggaaggc tgcacccagg aaaacaccga gtcggaatac 300
   tatgccaaag aaatccataa attcgacatg atccaggggc tggcggagca caacgaactg 360
   gctgtctgcc ctaaaggaat tacctccaag gttttccgct tcaatgtgtc ctcagtggag 420
   aaaaatagaa ccaacctatt ccgagcagaa ttccgggtct tgcgggtgcc caaccccagc 480
   tctaagcgga atgagcagag gatcgagctc ttccagatcc ttcggccaga tgagcacatt 540
   gccaaacagc gctatatcgg tggcaagaat ctgcccacac ggggcactgc cgagtggctg 600
   tcctttgatg tcactgacac tgtgcgtgag tggctgttga gaagagagtc caacttaggt 660
   ctagaaatca gcattcactg tccatgtcac acctttcagc ccaatggaga tatcctggaa 720
   aacattcacg aggtgatgga aatcaaattc aaaggcgtgg acaatgagga tgaccatggc 780
   cgtggagatc tggggcgcct caagaagcag aaggatcacc acaaccctca tctaatcctc 840
   atgatgattc ccccacaccg gctcgacaac ccgggccagg ggggtcagag gaagaagcgg 900
   gctttggaca ccaattactg cttccgcaac ttggaggaga actgctgtgt gcgccccctc 960
   tacattgact tccgacagga tctgggctgg aagtgggtcc atgaacctaa gggctactat 1020
   gccaacttct gctcaggccc ttgcccatac ctccgcagtg cagacacaac ccacagcacg 1080
   gtgctgggac tgtacaacac tctgaaccct gaagcatctg cctcgccttg ctgcgtgccc 1140
   caggacctgg agcccctgac catcctgtac tatgttggga ggacccccaa agtggagcag 1200
   ctctccaaca tggtggtgaa gtcttgtaaa tgtagctga 1239
<210> 49
   <211> 1704
   <212> DNA
   <213> Homo sapiens
<300>
   <302> TGFbetaR2
   <310> XM003094
<400> 49
   atgggtcggg ggctgctcag gggcctgtgg ccgctgcaca tcgtcctgtg gacgcgtatc 60
   gccagcacga tcccaccgca cgttcagaag tcggttaata acgacatgat agtcactgac 120
   aacaacggtg cagtcaagtt tccacaactg tgtaaatttt gtgatgtgag attttccacc 180
   tgtgacaacc agaaatcctg catgagcaac tgcagcatca cctccatctg tgagaagcca 240
   caggaagtct gtgtggctgt atggagaaag aatgacgaga acataacact agagacagtt 300
   tgccatgacc ccaagctccc ctaccatgac tttattctgg aagatgctgc ttctccaaag 360
   tgcattatga aggaaaaaaa aaagcctggt gagactttct tcatgtgttc ctgtagctct 420
   gatgagtgca atgacaacat catcttctca gaagaatata acaccagcaa tcctgacttg 480
   ttgctagtca tatttcaagt gacaggcatc agcctcctgc caccactggg agttgccata 540
   tctgtcatca tcatcttcta ctgctaccgc gttaaccggc agcagaagct gagttcaacc 600
   tgggaaaccg gcaagacgcg gaagctcatg gagttcagcg agcactgtgc catcatcctg 660
   gaagatgacc gctctgacat cagctccacg tgtgccaaca acatcaacca caacacagag 720
   ctgctgccca ttgagctgga caccctggtg gggaaaggtc gctttgctga ggtctataag 780
   gccaagctga agcagaacac ttcagagcag tttgagacag tggcagtcaa gatctttccc 840
   tatgaggagt atgcctcttg gaagacagag aaggacatct tctcagacat caatctgaag 900
   catgagaaca tactccagtt cctgacggct gaggagcgga agacggagtt ggggaaacaa 960
   tactggctga tcaccgcctt ccacgccaag ggcaacctac aggagtacct gacgcggcat 1020
   gtcatcagct gggaggacct gcgcaagctg ggcagctccc tcgcccgggg gattgctcac 1080
   ctccacagtg atcacactcc atgtgggagg cccaagatgc ccatcgtgca cagggacctc 1140
   aagagctcca atatcctcgt gaagaacgac ctaacctgct gcctgtgtga ctttgggctt 1200
   tccctgcgtc tggaccctac tctgtctgtg gatgacctgg ctaacagtgg gcaggtggga 1260
   actgcaagat acatggctcc agaagtccta gaatccagga tgaatttgga gaatgttgag 1320
   tccttcaagc agaccgatgt ctactccatg gctctggtgc tctgggaaat gacatctcgc 1380
   tgtaatgcag tgggagaagt aaaagattat gagcctccat ttggttccaa ggtgcgggag 1440
   cacccctgtg tcgaaagcat gaaggacaac gtgttgagag atcgagggcg accagaaatt 1500
   cccagcttct ggctcaacca ccagggcatc cagatggtgt gtgagacgtt gactgagtgc 1560
   tgggaccacg acccagaggc ccgtctcaca gcccagtgtg tggcagaacg cttcagtgag 1620
   ctggagcatc tggacaggct ctcgggaggg agctgctcgg aggagaagat tcctgaagac 1680
   ggctccctaa acactaccaa atag 1704
<210> 50
   <211> 609
   <212> DNA
   <213> Homo sapiens
<300>
   <302> TCFbeta3
   <310> XM001924
<400> 50
   atgtctcatt acaccattat tgagaatatt tgtcctaaag atgaatctgt gaaattctac 60
   agtcccaaga gagtgcactt tcctatcccg caagctgaca tggataagaa gcgattcagc 120
   tttgtcttca agcctgtctt caacacctca ctgctctttc tacagtgtga gctgacgctg 180
   tgtacgaaga tggagaagca cccccagaag ttgcctaagt gtgtgcctcc tgacgaagcc 240
   tgcacctcgc tggacgcctc gataatctgg gccatgatgc agaataagaa gacgttcact 300
   aagccccttg ctgtgatcca ccatgaagca gaatctaaag aaaaaggtcc aagcatgaag 360
   gaaccaaatc caatttctcc accaattttc catggtctgg acaccctaac cgtgatgggc 420
   attgcgtttg cagcctttgt gatcggagca ctcctgacgg gggccttgtg gtacatctat 480
   tctcacacag gggagacagc aggaaggcag caagtcccca cctccccgcc agcctcggaa 540
   aacagcagtg ctgcccacag catcggcagc acgcagagca cgccttgctc cagcagcagc 600
   acggcctag 609
<210> 51
   <211> 3633
   <212> DNA
   <213> Homo sapiens
<300>
   <302> EGFR
   <310> X00588
<400> 51
   atgcgaccct ccgggacggc cggggcagcg ctcctggcgc tgctggctgc gctctgcccg 60
   gcgagtcggg ctctggagga aaagaaagtt tgccaaggca cgagtaacaa gctcacgcag 120
   ttgggcactt ttgaagatca ttttctcagc ctccagagga tgttcaataa ctgtgaggtg 180
   gtccttggga atttggaaat tacctatgtg cagaggaatt atgatctttc cttcttaaag 240
   accatccagg aggtggctgg ttatgtcctc attgccctca acacagtgga gcgaattcct 300
   ttggaaaacc tgcagatcat cagaggaaat atgtactacg aaaattccta tgccttagca 360
   gtcttatcta actatgatgc aaataaaacc ggactgaagg agctgcccat gagaaattta 420
   caggaaatcc tgcatggcgc cgtgcggttc agcaacaacc ctgccctgtg caacgtggag 480
   agcatccagt ggcgggacat agtcagcagt gactttctca gcaacatgtc gatggacttc 540
   cagaaccacc tgggcagctg ccaaaagtgt gatccaagct gtcccaatgg gagctgctgg 600
   ggtgcaggag aggagaactg ccagaaactg accaaaatca tctgtgccca gcagtgctcc 660
   gggcgctgcc gtggcaagtc ccccagtgac tgctgccaca accagtgtgc tgcaggctgc 720
   acaggccccc gggagagcga ctgcctggtc tgccgcaaat tccgagacga agccacgtgc 780
   aaggacacct gccccccact catgctctac aaccccacca cgtaccagat ggatgtgaac 840
   cccgagggca aatacagctt tggtgccacc tgcgtgaaga agtgtccccg taattatgtg 900
   gtgacagatc acggctcgtg cgtccgagcc tgtggggccg acagctatga gatggaggaa 960
   gacggcgtcc gcaagtgtaa gaagtgcgaa gggccttgcc gcaaagtgtg taacggaata 1020
   ggtattggtg aatttaaaga ctcactctcc ataaatgcta cgaatattaa acacttcaaa 1080
   aactgcacct ccatcagtgg cgatctccac atcctgccgg tggcatttag gggtgactcc 1140
   ttcacacata ctcctcctct ggatccacag gaactggata ttctgaaaac cgtaaaggaa 1200
   atcacagggt ttttgctgat tcaggcttgg cctgaaaaca ggacggacct ccatgccttt 1260
   gagaacctag aaatcatacg cggcaggacc aagcaacatg gtcagttttc tcttgcagtc 1320
   gtcagcctga acataacatc cttgggatta cgctccctca aggagataag tgatggagat 1380
   gtgataattt caggaaacaa aaatttgtgc tatgcaaata caataaactg gaaaaaactg 1440
   tttgggacct ccggtcagaa aaccaaaatt ataagcaaca gaggtgaaaa cagctgcaag 1500
   gccacaggcc aggtctgcca tgccttgtgc tcccccgagg gctgctgggg cccggagccc 1560
   agggactgcg tctcttgccg gaatgtcagc cgaggcaggg aatgcgtgga caagtgcaag 1620
   cttctggagg gtgagccaag ggagtttgtg gagaactctg agtgcataca gtgccaccca 1680
   gagtgcctgc ctcaggccat gaacatcacc tgcacaggac ggggaccaga caactgtatc 1740
   cagtgtgccc actacattga cggcccccac tgcgtcaaga cctgcccggc aggagtcatg 1800
   ggagaaaaca acaccctggt ctggaagtac gcagacgccg gccatgtgtg ccacctgtgc 1860
   catccaaact gcacctacgg atgcactggg ccaggtcttg aaggctgtcc aacgaatggg 1920
   cctaagatcc cgtccatcgc cactgggatg gtgggggccc tcctcttgct gctggtggtg 1980
   gccctgggga tcggcctctt catgcgaagg cgccacatcg ttcggaagcg cacgctgcgg 2040
   aggctgctgc aggagaggga gcttgtggag cctcttacac ccagtggaga agctcccaac 2100
   caagctctct tgaggatctt gaaggaaact gaattcaaaa agatcaaagt gctgggctcc 2160
   ggtgcgttcg gcacggtgta taagggactc tggatcccag aaggtgagaa agttaaaatt 2220
   cccgtcgcta tcaaggaatt aagagaagca acatctccga aagccaacaa ggaaatcctc 2280
   gatgaagcct acgtgatggc cagcgtggac aacccccacg tgtgccgcct gctgggcatc 2340
   tgcctcacct ccaccgtgca actcatcacg cagctcatgc ccttcggctg cctcctggac 2400
   tatgtccggg aacacaaaga caatattggc tcccagtacc tgctcaactg gtgtgtgcag 2460
   atcgcaaagg gcatgaacta cttggaggac cgtcgcttgg tgcaccgcga cctggcagcc 2520
   aggaacgtac tggtgaaaac accgcagcat gtcaagatca cagattttgg gctggccaaa 2580
   ctgctgggtg cggaagagaa agaataccat gcagaaggag gcaaagtgcc tatcaagtgg 2640
   atggcattgg aatcaatttt acacagaatc tatacccacc agagtgatgt ctggagctac 2700
   ggggtgaccg tttgggagtt gatgaccttt ggatccaagc catatgacgg aatccctgcc 2760
   agcgagatct cctccatcct ggagaaagga gaacgcctcc ctcagccacc catatgtacc 2820
   atcgatgtct acatgatcat ggtcaagtgc tggatgatag acgcagatag tcgcccaaag 2880
   ttccgtgagt tgatcatcga attctccaaa atggcccgag acccccagcg ctaccttgtc 2940
   attcaggggg atgaaagaat gcatttgcca agtcctacag actccaactt ctaccgtgcc 3000
   ctgatggatg aagaagacat ggacgacgtg gtggatgccg acgagtacct catcccacag 3060
   cagggcttct tcagcagccc ctccacgtca cggactcccc tcctgagctc tctgagtgca 3120
   accagcaaca attccaccgt ggcttgcatt gatagaaatg ggctgcaaag ctgtcccatc 3180
   aaggaagaca gcttcttgca gcgatacagc tcagacccca caggcgcctt gactgaggac 3240
   agcatagacg acaccttcct cccagtgcct gaatacataa accagtccgt tcccaaaagg 3300
   cccgctggct ctgtgcagaa tcctgtctat cacaatcagc ctctgaaccc cgcgcccagc 3360
   agagacccac actaccagga cccccacagc actgcagtgg gcaaccccga gtatctcaac 3420
   actgtccagc ccacctgtgt caacagcaca ttcgacagcc ctgcccactg ggcccagaaa 3480
   ggcagccacc aaattagcct ggacaaccct gactaccagc aggacttctt tcccaaggaa 3540
   gccaagccaa atggcatctt taagggctcc acagctgaaa atgcagaata cctaagggtc 3600
   gcgccacaaa gcagtgaatt tattggagca tga 3633
<210> 52
   <211> 3768
   <212> DNA
   <213> Homo sapiens
<300>
   <302> ERBB2
   <310> NM004448
<400> 52
   atggagctgg cggccttgtg ccgctggggg ctcctcctcg ccctcttgcc ccccggagcc 60
   gcgagcaccc aagtgtgcac cggcacagac atgaagctgc ggctccctgc cagtcccgag 120
   acccacctgg acatgctccg ccacctctac cagggctgcc aggtggtgca gggaaacctg 180
   gaactcacct acctgcccac caatgccagc ctgtccttcc tgcaggatat ccaggaggtg 240
   cagggctacg tgctcatcgc tcacaaccaa gtgaggcagg tcccactgca gaggctgcgg 300
   attgtgcgag gcacccagct ctttgaggac aactatgccc tggccgtgct agacaatgga 360
   gacccgctga acaataccac ccctgtcaca ggggcctccc caggaggcct gcgggagctg 420
   cagcttcgaa gcctcacaga gatcttgaaa ggaggggtct tgatccagcg gaacccccag 480
   ctctgctacc aggacacgat tttgtggaag gacatcttcc acaagaacaa ccagctggct 540
   ctcacactga tagacaccaa ccgctctcgg gcctgccacc cctgttctcc gatgtgtaag 600
   ggctcccgct gctggggaga gagttctgag gattgtcaga gcctgacgcg cactgtctgt 660
   gccggtggct gtgcccgctg caagggccca ctgcccactg actgctgcca tgagcagtgt 720
   gctgccggct gcacgggccc caagcactct gactgcctgg cctgcctcca cttcaaccac 780
   agtggcatct gtgagctgca ctgcccagcc ctggtcacct acaacacaga cacgtttgag 840
   tccatgccca atcccgaggg ccggtataca ttcggcgcca gctgtgtgac tgcctgtccc 900
   tacaactacc tttctacgga cgtgggatcc tgcaccctcg tctgccccct gcacaaccaa 960
   gaggtgacag cagaggatgg aacacagcgg tgtgagaagt gcagcaagcc ctgtgcccga 1020
   gtgtgctatg gtctgggcat ggagcacttg cgagaggtga gggcagttac cagtgccaat 1080
   atccaggagt ttgctggctg caagaagatc tttgggagcc tggcatttct gccggagagc 1140
   tttgatgggg acccagcctc caacactgcc ccgctccagc cagagcagct ccaagtgttt 1200
   gagactctgg aagagatcac aggttaccta tacatctcag catggccgga cagcctgcct 1260
   gacctcagcg tcttccagaa cctgcaagta atccggggac gaattctgca caatggcgcc 1320
   tactcgctga ccctgcaagg gctgggcatc agctggctgg ggctgcgctc actgagggaa 1380
   ctgggcagtg gactggccct catccaccat aacacccacc tctgcttcgt gcacacggtg 1440
   ccctgggacc agctctttcg gaacccgcac caagctctgc tccacactgc caaccggcca 1500
   gaggacgagt gtgtgggcga gggcctggcc tgccaccagc tgtgcgcccg agggcactgc 1560
   tggggtccag ggcccaccca gtgtgtcaac tgcagccagt tccttcgggg ccaggagtgc 1620
   gtggaggaat gccgagtact gcaggggctc cccagggagt atgtgaatgc caggcactgt 1680
   ttgccgtgcc accctgagtg tcagccccag aatggctcag tgacctgttt tggaccggag 1740
   gctgaccagt gtgtggcctg tgcccactat aaggaccctc ccttctgcgt ggcccgctgc 1800
   cccagcggtg tgaaacctga cctctcctac atgcccatct ggaagtttcc agatgaggag 1860
   ggcgcatgcc agccttgccc catcaactgc acccactcct gtgtggacct ggatgacaag 1920
   ggctgccccg ccgagcagag agccagccct ctgacgtcca tcgtctctgc ggtggttggc 1980
   attctgctgg tcgtggtctt gggggtggtc tttgggatcc tcatcaagcg acggcagcag 2040
   aagatccgga agtacacgat gcggagactg ctgcaggaaa cggagctggt ggagccgctg 2100
   acacctagcg gagcgatgcc caaccaggcg cagatgcgga tcctgaaaga gacggagctg 2160
   aggaaggtga aggtgcttgg atctggcgct tttggcacag tctacaaggg catctggatc 2220
   cctgatgggg agaatgtgaa aattccagtg gccatcaaag tgttgaggga aaacacatcc 2280
   cccaaagcca acaaagaaat cttagacgaa gcatacgtga tggctggtgt gggctcccca 2340
   tatgtctccc gccttctggg catctgcctg acatccacgg tgcagctggt gacacagctt 2400
   atgccctatg gctgcctctt agaccatgtc cgggaaaacc gcggacgcct gggctcccag 2460
   gacctgctga actggtgtat gcagattgcc aaggggatga gctacctgga ggatgtgcgg 2520
   ctcgtacaca gggacttggc cgctcggaac gtgctggtca agagtcccaa ccatgtcaaa 2580
   attacagact tcgggctggc tcggctgctg gacattgacg agacagagta ccatgcagat 2640
   gggggcaagg tgcccatcaa gtggatggcg ctggagtcca ttctccgccg gcggttcacc 2700
   caccagagtg atgtgtggag ttatggtgtg actgtgtggg agctgatgac ttttggggcc 2760
   aaaccttacg atgggatccc agcccgggag atccctgacc tgctggaaaa gggggagcgg 2820
   ctgccccagc cccccatctg caccattgat gtctacatga tcatggtcaa atgttggatg 2880
   attgactctg aatgtcggcc aagattccgg gagttggtgt ctgaattctc ccgcatggcc 2940
   agggaccccc agcgctttgt ggtcatccag aatgaggact tgggcccagc cagtcccttg 3000
   gacagcacct tctaccgctc actgctggag gacgatgaca tgggggacct ggtggatgct 3060
   gaggagtatc tggtacccca gcagggcttc ttctgtccag accctgcccc gggcgctggg 3120
   ggcatggtcc accacaggca ccgcagctca tctaccagga gtggcggtgg ggacctgaca 3180
   ctagggctgg agccctctga agaggaggcc cccaggtctc cactggcacc ctccgaaggg 3240
   gctggctccg atgtatttga tggtgacctg ggaatggggg cagccaaggg gctgcaaagc 3300
   ctccccacac atgaccccag ccctctacag cggtacagtg aggaccccac agtacccctg 3360
   ccctctgaga ctgatggcta cgttgccccc ctgacctgca gcccccagcc tgaatatgtg 3420
   aaccagccag atgttcggcc ccagccccct tcgccccgag agggccctct gcctgctgcc 3480
   cgacctgctg gtgccactct ggaaagggcc aagactctct ccccagggaa gaatggggtc 3540
   gtcaaagacg tttttgcctt tgggggtgcc gtggagaacc ccgagtactt gacaccccag 3600
   ggaggagctg cccctcagcc ccaccctcct cctgccttca gcccagcctt cgacaacctc 3660
   tattactggg accaggaccc accagagcgg ggggctccac ccagcacctt caaagggaca 3720
   cctacggcag agaacccaga gtacctgggt ctggacgtgc cagtgtga              3768
<210> 53
   <211> 1986
   <212> DNA
   <213> Homo sapiens
<300>
   <302> ERBB3
   <310> XM006723
<400> 53
   atgcacaact tcagtgtttt ttccaatttg acaaccattg gaggcagaag cctctacaac 60
   cggggcttct cattgttgat catgaagaac ttgaatgtca catctctggg cttccgatcc 120
   ctgaaggaaa ttagtgctgg gcgtatctat ataagtgcca ataggcagct ctgctaccac 180
   cactctttga actggaccaa ggtgcttcgg gggcctacgg aagagcgact agacatcaag 240
   cataatcggc cgcgcagaga ctgcgtggca gagggcaaag tgtgtgaccc actgtgctcc 300
   tctgggggat gctggggccc aggccctggt cagtgcttgt cctgtcgaaa ttatagccga 360
   ggaggtgtct gtgtgaccca ctgcaacttt ctgaatgggg agcctcgaga atttgcccat 420
   gaggccgaat gcttctcctg ccacccggaa tgccaaccca tggagggcac tgccacatgc 480
   aatggctcgg gctctgatac ttgtgctcaa tgtgcccatt ttcgagatgg gccccactgt 540
   gtgagcagct gcccccatgg agtcctaggt gccaagggcc caatctacaa gtacccagat 600
   gttcagaatg aatgtcggcc ctgccatgag aactgcaccc aggggtgtaa aggaccagag 660
   cttcaagact gtttaggaca aacactggtg ctgatcggca aaacccatct gacaatggct 720
   ttgacagtga tagcaggatt ggtagtgatt ttcatgatgc tgggcggcac ttttctctac 780
   tggcgtgggc gccggattca gaataaaagg gctatgaggc gatacttgga acggggtgag 840
   agcatagagc ctctggaccc cagtgagaag gctaacaaag tcttggccag aatcttcaaa 900
   gagacagagc taaggaagct taaagtgctt ggctcgggtg tctttggaac tgtgcacaaa 960
   ggagtgtgga tccctgaggg tgaatcaatc aagattccag tctgcattaa agtcattgag 1020
   gacaagagtg gacggcagag ttttcaagct gtgacagatc atatgctggc cattggcagc 1080
   ctggaccatg cccacattgt aaggctgctg ggactatgcc cagggtcatc tctgcagctt 1140
   gtcactcaat atttgcctct gggttctctg ctggatcatg tgagacaaca ccggggggca 1200
   ctggggccac agctgctgct caactgggga gtacaaattg ccaagggaat gtactacctt 1260
   gaggaacatg gtatggtgca tagaaacctg gctgcccgaa acgtgctact caagtcaccc 1320
   agtcaggttc aggtggcaga ttttggtgtg gctgacctgc tgcctcctga tgataagcag 1380
   ctgctataca gtgaggccaa gactccaatt aagtggatgg cccttgagag tatccacttt 1440
   gggaaataca cacaccagag tgatgtctgg agctatggtg tgacagtttg ggagttgatg 1500
   accttcgggg cagagcccta tgcagggcta cgattggctg aagtaccaga cctgctagag 1560
   aagggggagc ggttggcaca gccccagatc tgcacaattg atgtctacat ggtgatggtc 1620
   aagtgttgga tgattgatga gaacattcgc ccaaccttta aagaactagc caatgagttc 1680
   accaggatgg cccgagaccc accacggtat ctggtcataa agagagagag tgggcctgga 1740
   atagcccctg ggccagagcc ccatggtctg acaaacaaga agctagagga agtagagctg 1800
   gagccagaac tagacctaga cctagacttg gaagcagagg aggacaacct ggcaaccacc 1860
   acactgggct ccgccctcag cctaccagtt ggaacactta atcggccacg tgggagccag 1920
   agccttttaa gtccatcatc tggatacatg cccatgaacc agggtaatct tggggttctt 1980
   ccttag                                                            1986
<210> 54
   <211> 1437
   <212> DNA
   <213> Homo sapiens
<300>
   <302> ERBB4
   <310> XM002260
<400> 54
   atgatgtacc tggaagaaag acgactcgtt catcgggatt tggcagcccg taatgtctta 60
   gtgaaatctc caaaccatgt gaaaatcaca gattttgggc tagccagact cttggaagga 120
   gatgaaaaag agtacaatgc tgatggagga aagatgccaa ttaaatggat ggctctggag 180
   tgtatacatt acaggaaatt cacccatcag agtgacgttt ggagctatgg agttactata 240
   tgggaactga tgacctttgg aggaaaaccc tatgatggaa ttccaacgcg agaaatccct 300
   gatttattag agaaaggaga acgtttgcct cagcctccca tctgcactat tgacgtttac 360
   atggtcatgg tcaaatgttg gatgattgat gctgacagta gacctaaatt taaggaactg 420
   gctgctgagt tttcaaggat ggctcgagac cctcaaagat acctagttat tcagggtgat 480
   gatcgtatga agcttcccag tccaaatgac agcaagttct ttcagaatct cttggatgaa 540
   gaggatttgg aagatatgat ggatgctgag gagtacttgg tccctcaggc tttcaacatc 600
   ccacctccca tctatacttc cagagcaaga attgactcga ataggagtga aattggacac 660
   agccctcctc ctgcctacac ccccatgtca ggaaaccagt ttgtataccg agatggaggt 720
   tttgctgctg aacaaggagt gtctgtgccc tacagagccc caactagcac aattccagaa 780
   gctcctgtgg cacagggtgc tactgctgag atttttgatg actcctgctg taatggcacc 840
   ctacgcaagc cagtggcacc ccatgtccaa gaggacagta gcacccagag gtacagtgct 900
   gaccccaccg tgtttgcccc agaacggagc ccacgaggag agctggatga ggaaggttac 960
   atgactccta tgcgagacaa acccaaacaa gaatacctga atccagtgga ggagaaccct 1020
   tttgtttctc ggagaaaaaa tggagacctt caagcattgg ataatcccga atatcacaat 1080
   gcatccaatg gtccacccaa ggccgaggat gagtatgtga atgagccact gtacctcaac 1140
   acctttgcca acaccttggg aaaagctgag tacctgaaga acaacatact gtcaatgcca 1200
   gagaaggcca agaaagcgtt tgacaacccc gactactgga accacagcct gccacctcgg 1260
   agcacccttc agcacccaga ctacctgcag gagtacagca caaaatattt ttataaacag 1320
   aatgggcgga tccggcctat tgtggcagag aatcctgaat acctctctga gttctccctg 1380
   aagccaggca ctgtgctgcc gcctccacct tacagacacc ggaatactgt ggtgtaa 1437
<210> 55
   <211> 627
   <212> DNA
   <213> Homo sapiens
<300>
   <302> FGF10
   <310> NM4004465
<400> 55
   atgtggaaat ggatactgac acattgtgcc tcagcctttc cccacctgcc cggctgctgc 60
   tgctgctgct ttttgttgct gttcttggtg tcttccgtcc ctgtcacctg ccaagccctt 120
   ggtcaggaca tggtgtcacc agaggccacc aactcttctt cctcctcctt ctcctctcct 180
   tccagcgcgg gaaggcatgt gcggagctac aatcaccttc aaggagatgt ccgctggaga 240
   aagctattct ctttcaccaa gtactttctc aagattgaga agaacgggaa ggtcagcggg 300
   accaagaagg agaactgccc gtacagcatc ctggagataa catcagtaga aatcggagtt 360
   gttgccgtca aagccattaa cagcaactat tacttagcca tgaacaagaa ggggaaactc 420
   tatggctcaa aagaatttaa caatgactgt aagctgaagg agaggataga ggaaaatgga 480
   tacaatacct atgcatcatt taactggcag cataatggga ggcaaatgta tgtggcattg 540
   aatggaaaag gagctccaag gagaggacag aaaacacgaa ggaaaaacac ctctgctcac 600
   tttcttccaa tggtggtaca ctcatag 627
<210> 56
   <211> 679
   <212> DNA
   <213> Homo sapiens
<300>
   <302> FGF11
   <310> XM008660
<400> 56
   aatggcggcg ctggccagta gcctgatccg gcagaagcgg gaggtccgcg agcccggggg 60
   cagccggccg gtgtcggcgc agcggcgcgt gtgtccccgc ggcaccaagt ccctttgcca 120
   gaagcagctc ctcatcctgc tgtccaaggt gcgactgtgc ggggggcggc ccgcgcggcc 180
   ggaccgcggc ccggagcctc agctcaaagg catcgtcacc aaactgttct gccgccaggg 240
   tttctacctc caggcgaatc ccgacggaag catccagggc accccagagg ataccagctc 300
   cttcacccac ttcaacctga tccctgtggg cctccgtgtg gtcaccatcc agagcgccaa 360
   gctgggtcac tacatggcca tgaatgctga gggactgctc tacagttcgc cgcatttcac 420
   agctgagtgt cgctttaagg agtgtgtctt tgagaattac tacgtcctgt acgcctctgc 480
   tctctaccgc cagcgtcgtt ctggccgggc ctggtacctc ggcctggaca acgagggcca 540
   ggtcatgaag ggaaaccgag ttaagaagac caaggcagct gcccactttc tgcccaagct 600
   cctggaggtg gccatgtacc aggagccttc tctccacagt gtccccgagg cctccccttc 660
   cagtccccct gccccctga 679
<210> 57
   <211> 732
   <212> DNA
   <213> Homo sapiens
<300>
   <302> FGF12
   <310> NM021032
<400> 57
   atggctgcgg cgatagccag ctccttgatc cggcagaagc ggcaggcgag ggagtccaac 60
   agcgaccgag tgtcggcctc caagcgccgc tccagcccca gcaaagacgg gcgctccctg 120
   tgcgagaggc acgtcctcgg ggtgttcagc aaagtgcgct tctgcagcgg ccgcaagagg 180
   ccggtgaggc ggagaccaga accccagctc aaagggattg tgacaaggtt attcagccag 240
   cagggatact tcctgcagat gcacccagat ggtaccattg atgggaccaa ggacgaaaac 300
   agcgactaca ctctcttcaa tctaattccc gtgggcctgc gtgtagtggc catccaagga 360
   gtgaaggcta gcctctatgt ggccatgaat ggtgaaggct atctctacag ttcagatgtt 420
   ttcactccag aatgcaaatt caaggaatct gtgtttgaaa actactatgt gatctattct 480
   tccacactgt accgccagca agaatcaggc cgagcttggt ttctgggact caataaagaa 540
   ggtcaaatta tgaaggggaa cagagtgaag aaaaccaagc cctcatcaca ttttgtaccg 600
   aaacctattg aagtgtgtat gtacagagaa ccatcgctac atgaaattgg agaaaaacaa 660
   gggcgttcaa ggaaaagttc tggaacacca accatgaatg gaggcaaagt tgtgaatcaa 720
   gattcaacat ag 732
<210> 58
   <211> 738
   <212> DNA
   <213> Homo sapiens
<300>
   <302> FGF13
   <310> XM010269
<400> 58
   atggcggcgg ctatcgccag ctcgctcatc cgtcagaaga ggcaagcccg cgagcgcgag 60
   aaatccaacg cctgcaagtg tgtcagcagc cccagcaaag gcaagaccag ctgcgacaaa 120
   aacaagttaa atgtcttttc ccgggtcaaa ctcttcggct ccaagaagag gcgcagaaga 180
   agaccagagc ctcagcttaa gggtatagtt accaagctat acagccgaca aggctaccac 240
   ttgcagctgc aggcggatgg aaccattgat ggcaccaaag atgaggacag cacttacact 300
   ctgtttaacc tcatccctgt gggtctgcga gtggtggcta tccaaggagt tcaaaccaag 360
   ctgtacttgg caatgaacag tgagggatac ttgtacacct cggaactttt cacacctgag 420
   tgcaaattca aagaatcagt gtttgaaaat tattatgtga catattcatc aatgatatac 480
   cgtcagcagc agtcaggccg agggtggtat ctgggtctga acaaagaagg agagatcatg 540
   aaaggcaacc atgtgaagaa gaacaagcct gcagctcatt ttctgcctaa accactgaaa 600
   gtggccatgt acaaggagcc atcactgcac gatctcacgg agttctcccg atctggaagc 660
   gggaccccaa ccaagagcag aagtgtctct ggcgtgctga acggaggcaa atccatgagc 720
   cacaatgaat caacgtag                                               738
<210> 59
   <211> 624
   <212> DNA
   <213> Homo sapiens
<300>
   <302> FGF16
   <310> NM003868
<400> 59
   atggcagagg tggggggcgt cttcgcctcc ttggactggg atctacacgg cttctcctcg 60
   tctctgggga acgtgccctt agctgactcc ccaggtttcc tgaacgagcg cctgggccaa 120
   atcgagggga agctgcagcg tggctcaccc acagacttcg cccacctgaa ggggatcctg 180
   cggcgccgcc agctctactg ccgcaccggc ttccacctgg agatcttccc caacggcacg 240
   gtgcacggga cccgccacga ccacagccgc ttcggaatcc tggagtttat cagcctggct 300
   gtggggctga tcagcatccg gggagtggac tctggcctgt acctaggaat gaatgagcga 360
   ggagaactct atgggtcgaa gaaactcaca cgtgaatgtg ttttccggga acagtttgaa 420
   gaaaactggt acaacaccta tgcctcaacc ttgtacaaac attcggactc agagagacag 480
   tattacgtgg ccctgaacaa agatggctca ccccgggagg gatacaggac taaacgacac 540
   cagaaattca ctcacttttt acccaggcct gtagatcctt ctaagttgcc ctccatgtcc 600
   agagacctct ttcactatag gtaa                                        624
<210> 60
   <211> 551
   <212> DNA
   <213> Homo sapiens
<300>
   <302> FGF17
   <310> XM005316
<400> 60
<210> 61
   <211> 624
   <212> DNA
   <213> Homo sapiens
<300>
   <302> FGF18
   <310> AF075292
<400> 61
   atgtattcag cgccctccgc ctgcacttgc ctgtgtttac acttcctgct gctgtgcttc 60
   caggtacagg tgctggttgc cgaggagaac gtggacttcc gcatccacgt ggagaaccag 120
   acgcgggctc gggacgatgt gagccgtaag cagctgcggc tgtaccagct ctacagccgg 180
   accagtggga aacacatcca ggtcctgggc cgcaggatca gtgcccgcgg cgaggatggg 240
   gacaagtatg cccagctcct agtggagaca gacaccttcg gtagtcaagt ccggatcaag 300
   ggcaaggaga cggaattcta cctgtgcatg aaccgcaaag gcaagctcgt ggggaagccc 360
   gatggcacca gcaaggagtg tgtgttcatc gagaaggttc tggagaacaa ctacacggcc 420
   ctgatgtcgg ctaagtactc cggctggtac gtgggcttca ccaagaaggg gcggccgcgg 480
   aagggcccca agacccggga gaaccagcag gacgtgcatt tcatgaagcg ctaccccaag 540
   gggcagccgg agcttcagaa gcccttcaag tacacgacgg tgaccaagag gtcccgtcgg 600
   atccggccca cacaccctgc ctag                                        624
<210> 62
   <211> 651
   <212> DNA
   <213> Homo sapiens
<300>
   <302> FGF19
   <310> AF110400
<400> 62
<210> 63
   <211> 468
   <212> DNA
   <213> Homo sapiens
<400> 63
   atggctgaag gggaaatcac caccttcaca gccctgaccg agaagtttaa tctgcctcca 60
   gggaattaca agaagcccaa actcctctac tgtagcaacg ggggccactt cctgaggatc 120
   cttccggatg gcacagtgga tgggacaagg gacaggagcg accagcacat tcagctgcag 180
   ctcagtgcgg aaagcgtggg ggaggtgtat ataaagagta ccgagactgg ccagtacttg 240
   gccatggaca ccgacgggct tttatacggc tcacagacac caaatgagga atgtttgttc 300
   ctggaaaggc tggaggagaa ccattacaac acctatatat ccaagaagca tgcagagaag 360
   aattggtttg ttggcctcaa gaagaatggg agctgcaaac gcggtcctcg gactcactat 420
   ggccagaaag caatcttgtt tctccccctg ccagtctctt ctgattaa 468
<210> 64
   <211> 636
   <212> DNA
   <213> Homo sapiens
<300>
   <302> FGF20
   <310> NM019851
<400> 64
   atggctccct tagccgaagt cgggggcttt ctgggcggcc tggagggctt gggccagcag 60
   gtgggttcgc atttcctgtt gcctcctgcc ggggagcggc cgccgctgct gggcgagcgc 120
   aggagcgcgg cggagcggag cgcccgcggc gggccggggg ctgcgcagct ggcgcacctg 180
   cacggcatcc tgcgccgccg gcagctctat tgccgcaccg gcttccacct gcagatcctg 240
   cccgacggca gcgtgcaggg cacccggcag gaccacagcc tcttcggtat cttggaattc 300
   atcagtgtgg cagtgggact ggtcagtatt agaggtgtgg acagtggtct ctatcttgga 360
   atgaatgaca aaggagaact ctatggatca gagaaactta cttccgaatg catctttagg 420
   gagcagtttg aagagaactg gtataacacc tattcatcta acatatataa acatggagac 480
   actggccgca ggtattttgt ggcacttaac aaagacggaa ctccaagaga tggcgccagg 540
   tccaagaggc atcagaaatt tacacatttc ttacctagac cagtggatcc agaaagagtt 600
   ccagaattgt acaaggacct actgatgtac acttga 636
<210> 65
   <211> 630
   <212> DNA
   <213> Homo sapiens
<300>
   <302> FGF21
   <310> XM009100
<400> 65
   atggactcgg acgagaccgg gttcgagcac tcaggactgt gggtttctgt gctggctggt 60
   cttctgctgg gagcctgcca ggcacacccc atccctgact ccagtcctct cctgcaattc 120
   gggggccaag tccggcagcg gtacctctac acagatgatg cccagcagac agaagcccac 180
   ctggagatca gggaggatgg gacggtgggg ggcgctgctg accagagccc cgaaagtctc 240
   ctgcagctga aagccttgaa gccgggagtt attcaaatct tgggagtcaa gacatccagg 300
   ttcctgtgcc agcggccaga tggggccctg tatggatcgc tccactttga ccctgaggcc 360
   tgcagcttcc gggagctgct tcttgaggac ggatacaatg tttaccagtc cgaagcccac 420
   ggcctcccgc tgcacctgcc agggaacaag tccccacacc gggaccctgc accccgagga 480
   ccagctcgct tcctgccact accaggcctg ccccccgcac tcccggagcc acccggaatc 540
   ctggcccccc agccccccga tgtgggctcc tcggaccctc tgagcatggt gggaccttcc 600
   cagggccgaa gccccagcta cgcttcctga                                   630
<210> 66
   <211> 513
   <212> DNA
   <213> Homo sapiens
<300>
   <302> FGF22
   <310> XM009271
<400> 66
   atgcgccgcc gcctgtggct gggcctggcc tggctgctgc tggcgcgggc gccggacgcc 60
   gcgggaaccc cgagcgcgtc gcggggaccg cgcagctacc cgcacctgga gggcgacgtg 120
   cgctggcggc gcctcttctc ctccactcac ttcttcctgc gcgtggatcc cggcggccgc 180
   gtgcagggca cccgctggcg ccacggccag gacagcatcc tggagatccg ctctgtacac 240
   gtgggcgtcg tggtcatcaa agcagtgtcc tcaggcttct acgtggccat gaaccgccgg 300
   ggccgcctct acgggtcgcg actctacacc gtggactgca ggttccggga gcgcatcgaa 360
   gagaacggcc acaacaccta cgcctcacag cgctggcgcc gccgcggcca gcccatgttc 420
   ctggcgctgg acaggagggg ggggccccgg ccaggcggcc ggacgcggcg gtaccacctg 480
   tccgcccact tcctgcccgt cctggtctcc tga                               513
<210> 67
   <211> 621
   <212> DNA
   <213> Homo sapiens
<300>
   <302> FGF4
   <310> NM002007
<400> 67
   atgtcggggc ccgggacggc cgcggtagcg ctgctcccgg cggtcctgct ggccttgctg 60
   gcgccctggg cgggccgagg gggcgccgcc gcacccactg cacccaacgg cacgctggag 120
   gccgagctgg agcgccgctg ggagagcctg gtggcgctct cgttggcgcg cctgccggtg 180
   gcagcgcagc ccaaggaggc ggccgtccag agcggcgccg gcgactacct gctgggcatc 240
   aagcggctgc ggcggctcta ctgcaacgtg ggcatcggct tccacctcca ggcgctcccc 300
   gacggccgca tcggcggcgc gcacgcggac acccgcgaca gcctgctgga gctctcgccc 360
   gtggagcggg gcgtggtgag catcttcggc gtggccagcc ggttcttcgt ggccatgagc 420
   agcaagggca agctctatgg ctcgcccttc ttcaccgatg agtgcacgtt caaggagatt 480
   ctccttccca acaactacaa cgcctacgag tcctacaagt accccggcat gttcatcgcc 540
   ctgagcaaga atgggaagac caagaagggg aaccgagtgt cgcccaccat gaaggtcacc 600
   cacttcctcc ccaggctgtg a                                            621
<210> 68
   <211> 597
   <212> DNA
   <213> Homo sapiens
<300>
   <302> FGF6
   <310> MM020996
<400> 68
   atgtcccggg gagcaggacg tctgcagggc acgctgtggg ctctcgtctt cctaggcatc 60
   ctagtgggca tggtggtgcc ctcgcctgca ggcacccgtg ccaacaacac gctgctggac 120
   tcgaggggct ggggcaccct gctgtccagg tctcgcgcgg ggctagctgg agagattgcc 180
   ggggtgaact gggaaagtgg ctatttggtg gggatcaagc ggcagcggag gctctactgc 240
   aacgtgggca tcggctttca cctccaggtg ctccccgacg gccggatcag cgggacccac 300
   gaggagaacc cctacagcct gctggaaatt tccactgtgg agcgaggcgt ggtgagtctc 360
   tttggagtga gaagtgccct cttcgttgcc atgaacagta aaggaagatt gtacgcaacg 420
   cccagcttcc aagaagaatg caagttcaga gaaaccctcc tgcccaacaa ttacaatgcc 480
   tacgagtcag acttgtacca agggacctac attgccctga gcaaatacgg acgggtaaag 540
   cggggcagca aggtgtcccc gatcatgact gtcactcatt tccttcccag gatctaa    597
<210> 69
   <212> 150
   <212> DNA
   <213> Homo sapiens
<300>
   <302> FGF7
   <310> XM007559
<400> 69
<210> 70
   <211> 628
   <212> DNA
   <213> Homo sapiens
<300>
   <302> FGF9
   <310> XM007105
<400> 70
   gatggctccc ttaggtgaag ttgggaacta tttcggtgtg caggatgcgg taccgtttgg 60
   gaatgtgccc gtgttgccgg tggacagccc ggttttgtta agtgaccacc tgggtcagtc 120
   cgaagcaggg gggctcccca ggggacccgc agtcacggac ttggatcatt taaaggggat 180
   tctcaggcgg aggcagctat actgcaggac tggatttcac ttagaaatct tccccaatgg 240
   tactatccag ggaaccagga aagaccacag ccgatttggc attctggaat ttatcagtat 300
   agcagtgggc ctggtcagca ttcgaggcgt ggacagtgga ctctacctcg ggatgaatga 360
   gaagggggag ctgtatggat cagaaaaact aacccaagag tgtgtattca gagaacagtt 420
   cgaagaaaac tggtataata cgtactcatc aaacctatat aagcacgtgg acactggaag 480
   gcgatactat gttgcattaa ataaagatgg gaccccgaga gaagggacta ggactaaacg 540
   gcaccagaaa ttcacacatt ttttacctag accagtggac cccgacaaag tacctgaact 600
   gtataaggat attctaagcc aaagttga 628
<210> 71
   <311> 2469
   <212> DNA
   <213>Homo sapiens
<300>
   <302> FGFR1
   <310> NM00604
<400> 71
<210> 72
   <211> 2409
   <212> DNA
   <213> Homo sapiens
<300>
   <302> FGFR4
   <310> XM003910
<400> 72
   atgcggctgc tgctggccct gttgggggtc ctgctgagtg tgcctgggcc tccagtcttg 60
   tccctggagg cctctgagga agtggagctt gagccctgcc tggctcccag cctggagcag 120
   caagagcagg agctgacagt agcccttggg cagcctgtgc ggctgtgctg tgggcgggct 180
   gagcgtggtg gccactggta caaggagggc agtcgcctgg cacctgctgg ccgtgtacgg 240
   ggctggaggg gccgcctaga gattgccagc ttcctacctg aggatgctgg ccgctacctc 300
   tgcctggcac gaggctccat gatcgtcctg cagaatctca ccttgattac aggtgactcc 360
   ttgacctcca gcaacgatga tgaggacccc aagtcccata gggacctctc gaataggcac 420
   agttaccccc agcaagcacc ctactggaca cacccccagc gcatggagaa gaaactgcat 480
   gcagtacctg cggggaacac cgtcaagttc cgctgtccag ctgcaggcaa ccccacgccc 540
   accatccgct ggcttaagga tggacaggcc tttcatgggg agaaccgcat tggaggcatt 600
   cggctgcgcc atcagcactg gagtctcgtg atggagagcg tggtgccctc ggaccgcggc 660
   acatacacct gcctggtaga gaacgctgtg ggcagcatcc gttataacta cctgctagat 720
   gtgctggagc ggtccccgca ccggcccatc ctgcaggccg ggctcccggc caacaccaca 780
   gccgtggtgg gcagcgacgt ggagctgctg tgcaaggtgt acagcgatgc ccagccccac 840
   atccagtggc tgaagcacat cgtcatcaac ggcagcagct tcggagccga cggtttcccc 900
   tatgtgcaag tcctaaagac tgcagacatc aatagctcag aggtggaggt cctgtacctg 960
   cggaacgtgt cagccgagga cgcaggcgag tacacctgcc tcgcaggcaa ttccatcggc 1020
   ctctcctacc agtctgcctg gctcacggtg ctgccagagg aggaccccac atggaccgca 1080
   gcagcgcccg aggccaggta tacggacatc atcctgtacg cgtcgggctc cctggccttg 1140
   gctgtgctcc tgctgctggc caggctgtat cgagggcagg cgctccacgg ccggcacccc 1200
   cgcccgcccg ccactgtgca gaagctctcc cgcttccctc tggcccgaca gttctccctg 1260
   gagtcaggct cttccggcaa gtcaagctca tccctggtac gaggcgtgcg tctctcctcc 1320
   agcggccccg ccttgctcgc cggcctcgtg agtctagatc tacctctcga cccactatgg 1380
   gagttccccc gggacaggct ggtgcttggg aagcccctag gcgagggctg ctttggccag 1440
   gtagtacgtg cagaggcctt tggcatggac cctgcccggc ctgaccaagc cagcactgtg 1500
   gccgtcaaga tgctcaaaga caacgcctct gacaaggacc tggccgacct ggtctcggag 1560
   atggaggtga tgaagctgat cggccgacac aagaacatca tcaacctgct tggtgtctgc 1620
   acccaggaag ggcccctgta cgtgatcgtg gagtgcgccg ccaagggaaa cctgcgggag 1680
   ttcctgcggg cccggcgccc cccaggcccc gacctcagcc ccgacggtcc tcggagcagt 1740
   gaggggccgc tctccttccc agtcctggtc tcctgcgcct accaggtggc ccgaggcatg 1800
   cagtatctgg agtcccggaa gtgtatccac cgggacctgg ctgcccgcaa tgtgctggtg 1860
   actgaggaca atgtgatgaa gattgctgac tttgggctgg cccgcggcgt ccaccacatt 1920
   gactactata agaaaaccag caacggccgc ctgcctgtga agtggatggc gcccgaggcc 1980
   ttgtttgacc gggtgtacac acaccagagt gacgtgtggt cttttgggat cctgctatgg 2040
   gagatcttca ccctcggggg ctccccgtat cctggcatcc cggtggagga gctgttctcg 2100
   ctgctgcggg agggacatcg gatggaccga cccccacact gccccccaga gctgtacggg 2160
   ctgatgcgtg agtgctggca cgcagcgccc tcccagaggc ctaccttcaa gcagctggtg 2220
   gaggcgctgg acaaggtcct gctggccgtc tctgaggagt acctcgacct ccgcctgacc 2280
   ttcggaccct attccccctc tggtggggac gccagcagca cctgctcctc cagcgattct 2340
   gtcttcagcc acgaccccct gccattggga tccagctcct tccccttcgg gtctggggtg 2400
   cagacatga 2409
<210> 73
   <212> 1695
   <212> DNA
   <213> Homo sapiens
<300>
   <302> MT2MMP
   <310> D86331
<400> 73
<210> 74
   <211> 1824
   <212> DNA
   <213> Homo sapiens
<300>
   <302> MT3MMP
   <310> D85511
<400> 74
   atgatcttac tcacattcag cactggaaga cggttggatt tcgtgcatca ttcgggggtg 60
   tttttcttgc aaaccttgct ttggatttta tgtgctacag tctgcggaac ggagcagtat 120
   ttcaatgtgg aggtttggtt acaaaagtac ggctaccttc caccgactga ccccagaatg 180
   tcagtgctgc gctctgcaga gaccatgcag tctgccctag ctgccatgca gcagttctat 240
   ggcattaaca tgacaggaaa agtggacaga aacacaattg actggatgaa gaagccccga 300
   tgcggtgtac ctgaccagac aagaggtagc tccaaatttc atattcgtcg aaagcgatat 360
   gcattgacag gacagaaatg gcagcacaag cacatcactt acagtataaa gaacgtaact 420
   ccaaaagtag gagaccctga gactcgtaaa gctattcgcc gtgcctttga tgtgtggcag 480
   aatgtaactc ctctgacatt tgaagaagtt ccctacagtg aattagaaaa tggcaaacgt 540
   gatgtggata taaccattat ttttgcatct ggtttccatg gggacagctc tccctttgat 600
   ggagagggag gatttttggc acatgcctac ttccctggac caggaattgg aggagatacc 660
   cattttgact cagatgagcc atggacacta ggaaatccta atcatgatgg aaatgactta 720
   tttcttgtag cagtccatga actgggacat gctctgggat tggagcattc caatgacccc 780
   actgccatca tggctccatt ttaccagtac atggaaacag acaacttcaa actacctaat 840
   gatgatttac agggcatcca gaagatatat ggtccacctg acaagattcc tccacctaca 900
   agacctctac cgacagtgcc cccacaccgc tctattcctc cggctgaccc aaggaaaaat 960
   gacaggccaa aacctcctcg gcctccaacc ggcagaccct cctatcccgg agccaaaccc 1020
   aacatctgtg atgggaactt taacactcta gctattcttc gtcgtgagat gtttgttttc 1080
   aaggaccagt ggttttggcg agtgagaaac aacagggtga tggatggata cccaatgcaa 1140
   attacttact tctggcgggg cttgcctcct agtatcgatg cagtttatga aaatagcgac 1200
   gggaattttg tgttctttaa aggtaacaaa tattgggtgt tcaaggatac aactcttcaa 1260
   cctggttacc ctcatgactt gataaccctt ggaagtggaa ttccccctca tggtattgat 1320
   tcagccattt ggtgggagga cgtcgggaaa acctatttct tcaagggaga cagatattgg 1380
   agatatagtg aagaaatgaa aacaatggac cctggctatc ccaagccaat cacagtctgg 1440
   aaagggatcc ctgaatctcc tcagggagca tttgtacaca aagaaaatgg ctttacgtat 1500
   ttctacaaag gaaacgagta ttggaaattc aacaaccaga tactcaaggt agaacctgga 1560
   tatccaagat ccatcctcaa ggattttatg ggctgtgatg gaccaacaga cagagttaaa 1620
   gaaggacaca gcccaccaga tgatgtagac attgtcatca aactggacaa cacagccagc 1680
   actgtgaaag ccatagctat tgtcattccc tgcatcttgg ccttatgcct ccttgtattg 1740
   gtttacactg tgttccagtt caagaggaaa ggaacacccc gccacatact gtactgtaaa 1800
   cgctctatgc aagagtgggt gtga 1824
<210> 75
   <211> 1818
   <212> DNA
   <213> Homo sapiens
<300>
   <302> MT4MMP
   <310> AB021225
<400> 75
   atgcggcgcc gcgcagcccg gggacccggc ccgccgcccc cagggcccgg actctcgcgg 60
   ctgccgctgc tgccgctgcc gctgctgctg ctgctggcgc tggggacccg cgggggctgc 120
   gccgcgccgg aacccgcgcg gcgcgccgag gacctcagcc tgggagtgga gtggctaagc 180
   aggttcggtt acctgccccc ggctgacccc acaacagggc agctgcagac gcaagaggag 240
   ctgtctaagg ccatcacagc catgcagcag tttggtggcc tggaggccac cggcatcctg 300
   gacgaggcca ccctggccct gatgaaaacc ccacgctgct ccctgccaga cctccctgtc 360
   ctgacccagg ctcgcaggag acgccaggct ccagccccca ccaagtggaa caagaggaac 420
   ctgtcgtgga gggtccggac gttcccacgg gactcaccac tggggcacga cacggtgcgt 480
   gcactcatgt actacgccct caaggtctgg agcgacattg cgcccctgaa cttccacgag 540
   gtggcgggca gcaccgccga catccagatc gacttctcca aggccgacca taacgacggc 600
   taccccttcg acgcccggcg gcaccgtgcc cacgccttct tccccggcca ccaccacacc 660
   gccgggtaca cccactttaa cgatgacgag gcctggacct tccgctcctc ggatgcccac 720
   gggatggacc tgtttgcagt ggctgtccac gagtttggcc acgccattgg gttaagccat 780
   gtggccgctg cacactccat catgcggccg tactaccagg gcccggtggg tgacccgctg 840
   cgctacgggc tcccctacga ggacaaggtg cgcgtctggc agctgtacgg tgtgcgggag 900
   tctgtgtctc ccacggcgca gcccgaggag cctcccctgc tgccggagcc cccagacaac 960
   cggtccagcg ccccgcccag gaaggacgtg ccccacagat gcagcactca ctttgacgcg 1020
   gtggcccaga tccggggtga agctttcttc ttcaaaggca agtacttctg gcggctgacg 1080
   cgggaccggc acctggtgtc cctgcagccg gcacagatgc accgcttctg gcggggcctg 1140
   ccgctgcacc tggacagcgt ggacgccgtg tacgagcgca ccagcgacca caagatcgtc 1200
   ttctttaaag gagacaggta ctgggtgttc aaggacaata acgtagagga aggatacccg 1260
   cgccccgtct ccgacttcag cctcccgcct ggcggcatcg acgctgcctt ctcctgggcc 1320
   cacaatgaca ggacttattt ctttaaggac cagctgtact ggcgctacga tgaccacacg 1380
   aggcacatgg accccggcta ccccgcccag agccccctgt ggaggggtgt ccccagcacg 1440
   ctggacgacg ccatgcgctg gtccgacggt gcctcctact tcttccgtgg ccaggagtac 1500
   tggaaagtgc tggatggcga gctggaggtg gcacccgggt acccacagtc cacggcccgg 1560
   gactggctgg tgtgtggaga ctcacaggcc gatggatctg tggctgcggg cgtggacgcg 1620
   gcagaggggc cccgcgcccc tccaggacaa catgaccaga gccgctcgga ggacggttac 1680
   gaggtctgct catgcacctc tggggcatcc tctcccccgg gggccccagg cccactggtg 1740
   gctgccacca tgctgctgct gctgccgcca ctgtcaccag gcgccctgtg gacagcggcc 1800
   caggccctga cgctatga                                               1818
<210> 76
   <211> 1938
   <212> DNA
   <213> Homo sapiens
<300>
   <302> MT5MMP
   <310> AB021227
<400> 76
   atgccgagga gccggggcgg ccgcgccgcg ccggggccgc cgccgccgcc gccgccgccg 60
   ggccaggccc cgcgctggag ccgctggcgg gtccctgggc ggctgctgct gctgctgctg 120
   cccgcgctct gctgcctccc gggcgccgcg cgggcggcgg cggcggcggc gggggcaggg 180
   aaccgggcag cggtggcggt ggcggtggcg cgggcggacg aggcggaggc gcccttcgcc 240
   gggcagaact ggttaaagtc ctatggctat ctgcttccct atgactcacg ggcatctgcg 300
   ctgcactcag cgaaggcctt gcagtcggca gtctccacta tgcagcagtt ttacgggatc 360
   ccggtcaccg gtgtgttgga tcagacaacg atcgagtgga tgaagaaacc ccgatgtggt 420
   gtccctgatc acccccactt aagccgtagg cggagaaaca agcgctatgc cctgactgga 480
   cagaagtgga ggcaaaaaca catcacctac agcattcaca actatacccc aaaagtgggt 540
   gagctagaca cgcggaaagc tattcgccag gctttcgatg tgtggcagaa ggtgacccca 600
   ctgacctttg aagaggtgcc ataccatgag atcaaaagtg accggaagga ggcagacatc 660
   atgatctttt ttgcttctgg tttccatggc gacagctccc catttgatgg agaacgggga 720
   ttcctggccc atgcctactt ccctggccca gggattggag gagacaccca ctttgactcc 780
   gatgagccat ggacgctagg aaacgccaac catgacggga acgacctctt cctggtggct 840
   gtgcatgagc tgggccacgc gctgggactg gagcactcca gcgaccccag cgccatcatg 900
   gcgcccttct accagtacat ggagacgcac aacttcaagc tgccccagga cgatctccag 960
   ggcatccaga agatctatgg acccccagcc gagcctctgg agcccacaag gccactccct 1020
   acactccccg tccgcaggat ccactcacca tcggagagga aacacgagcg ccagcccagg 1080
   ccccctcggc cgcccctcgg ggaccggcca tccacaccag gcaccaaacc caacatctgt 1140
   gacggcaact tcaacacagt ggccctcttc cggggcgaga tgtttgtctt taaggatcgc 1200
   tggttctggc gtctgcgcaa taaccgagtg caggagggct accccatgca gatcgagcag 1260
   ttctggaagg gcctgcctgc ccgcatcgac gcagcctatg aaagggccga tgggagattt 1320
   gtcttcttca aaggtgacaa gtattgggtg tttaaggagg tgacggtgga gcctgggtac 1380
   ccccacagcc tgggggagct gggcagctgt ttgccccgtg aaggcattga cacagctctg 1440
   cgctgggaac ctgtgggcaa gacctacttt ttcaaaggcg agcggtactg gcgctacagc 1500
   gaggagcggc gggccacgga ccctggctac cctaagccca tcaccgtgtg gaagggcatc 1560
   ccacaggctc cccaaggagc cttcatcagc aaggaaggat attacaccta tttctacaag 1620
   ggccgggact actggaagtt tgacaaccag aaactgagcg tggagccagg ctacccgcgc 1680
   aacatcctgc gtgactggat gggctgcaac cagaaggagg tggagcggcg gaaggagcgg 1740
   cggctgcccc aggacgacgt ggacatcatg gtgaccatca acgatgtgcc gggctccgtg 1800
   aacgccgtgg ccgtggtcat cccctgcatc ctgtccctct gcatcctggt gctggtctac 1860
   accatcttcc agttcaagaa caagacaggc cctcagcctg tcacctacta taagcggcca 1920
   gtccaggaat gggtgtga                                                 1938
<210> 77
   <211> 1689
   <212> DNA
   <213> Homo sapiens
<300>
   <302> MT6MMP
   <310> AJ27137
<400> 77
   atgcggctgc ggctccggct tctggcgctg ctgcttctgc tgctggcacc gcccgcgcgc 60
   gccccgaagc cctcggcgca ggacgtgagc ctgggcgtgg actggctgac tcgctatggt 120
   tacctgccgc caccccaccc tgcccaggcc cagctgcaga gccctgagaa gttgcgcgat 180
   gccatcaaag tcatgcagag gttcgcgggg ctgccggaga ccggccgcat ggacccaggg 240
   acagtggcca ccatgcgtaa gccccgctgc tccctgcctg acgtgctggg ggtggcgggg 300
   ctggtcaggc ggcgtcgccg gtacgctctg agcggcagcg tgtggaagaa gcgaaccctg 360
   acatggaggg tacgttcctt cccccagagc tcccagctga gccaggagac cgtgcgggtc 420
   ctcatgagct atgccctgat ggcctggggc atggagtcag gcctcacatt tcatgaggtg 480
   gattcccccc agggccagga gcccgacatc ctcatcgact ttgcccgcgc cttccaccag 540
   gacagctacc ccttcgacgg gttggggggc accctagccc atgccttctt ccctggggag 600
   caccccatct ccggggacac tcactttgac gatgaggaga cctggacttt tgggtcaaaa 660
   gacggcgagg ggaccgacct gtttgccgtg gctgtccatg agtttggcca cgccctgggc 720
   ctgggccact cctcagcccc caactccatt atgaggccct tctaccaggg tccggtgggc 780
   gaccctgaca agtaccgcct gtctcaggat gaccgcgatg gcctgcagca actctatggg 840
   aaggcgcccc aaaccccata tgacaagccc acaaggaaac ccctggctcc tccgccccag 900
   cccccggcct cgcccacaca cagcccatcc ttccccatcc ctgatcgatg tgagggcaat 960
   tttgacgcca tcgccaacat ccgaggggaa actttcttct tcaaaggccc ctggttctgg 1020
   cgcctccagc cctccggaca gctggtgtcc ccgcgacccg cacggctgca ccgcttctgg 1080
   gaggggctgc ccgcccaggt gagggtggtg caggccgcct atgctcggca ccgagacggc 1140
   cgaatcctcc tctttagcgg gccccagttc tgggtgttcc aggaccggca gctggagggc 1200
   ggggcgcggc cgctcacgga gctggggctg cccccgggag aggaggtgga cgccgtgttc 1260
   tcgtggccac agaacgggaa gacctacctg gtccgcggcc ggcagtactg gcgctacgac 1320
   gaggcggcgg cgcgcccgga ccccggctac cctcgcgacc tgagcctctg ggaaggcgcg 1380
   cccccctccc ctgacgatgt caccgtcagc aacgcaggtg acacctactt cttcaagggc 1440
   gcccactact ggcgcttccc caagaacagc atcaagaccg agccggacgc cccccagccc 1500
   atggggccca actggctgga ctgccccgcc ccgagctctg gtccccgcgc ccccaggccc 1560
   cccaaagcga cccccgtgtc cgaaacctgc gattgtcagt gcgagctcaa ccaggccgca 1620
   ggacgttggc ctgctcccat cccgctgctc ctcttgcccc tgctggtggg gggtgtagcc 1680
   tcccgctga 1689
<210> 78
   <212> 1749
   <212> DNA
   <213> Homo sapiens
<300>
   <302> MTMMP
   <310> X90925
<400> 78
<210> 79
   <211> 744
   <212> DNA
   <213> Homo sapiens
<300>
   <302> FGF1
   <310> XM003647
<400> 79
   atggccgcgg ccatcgctag cggcttgatc cgccagaagc ggcaggcgcg ggagcagcac 60
   tgggaccggc cgtctgccag caggaggcgg agcagcccca gcaagaaccg cgggctctgc 120
   aacggcaacc tggtggatat cttctccaaa gtgcgcatct tcggcctcaa gaagcgcagg 180
   ttgcggcgcc aagatcccca gctcaagggt atagtgacca ggttatattg caggcaaggc 240
   tactacttgc aaatgcaccc cgatggagct ctcgatggaa ccaaggatga cagcactaat 300
   tctacactct tcaacctcat accagtggga ctacgtgttg ttgccatcca gggagtgaaa 360
   acagggttgt atatagccat gaatggagaa ggttacctct acccatcaga actttttacc 420
   cctgaatgca agtttaaaga atctgttttt gaaaattatt atgtaatcta ctcatccatg 480
   ttgtacagac aacaggaatc tggtagagcc tggtttttgg gattaaataa ggaagggcaa 540
   gctatgaaag ggaacagagt aaagaaaacc aaaccagcag ctcattttct acccaagcca 600
   ttggaagttg ccatgtaccg agaaccatct ttgcatgatg ttggggaaac ggtcccgaag 660
   cctggggtga cgccaagtaa aagcacaagt gcgtctgcaa taatgaatgg aggcaaacca 720
   gtcaacaaga gtaagacaac atag                                        744
<210> 80
   <211> 468
   <212> DNA
   <213> Homo sapiens
<300>
   <302> FGF2
   <310> NM002006
<400> 80
   atggcagccg ggagcatcac cacgctgccc gccttgcccg aggatggcgg cagcggcgcc 60
   ttcccgcccg gccacttcaa ggaccccaag cggctgtact gcaaaaacgg gggcttcttc 120
   ctgcgcatcc accccgacgg ccgagttgac ggggtccggg agaagagcga ccctcacatc 180
   aagctacaac ttcaagcaga agagagagga gttgtgtcta tcaaaggagt gtgtgctaac 240
   cgttacctgg ctatgaagga agatggaaga ttactggctt ctaaatgtgt tacggatgag 300
   tgtttctttt ttgaacgatt ggaatctaat aactacaata cttaccggtc aaggaaatac 360
   accagttggt atgtggcact gaaacgaact gggcagtata aacttggatc caaaacagga 420
   cctgggcaga aagctatact ttttcttcca atgtctgcta agagctga              468
<210> 81
   <211> 756
   <212> DNA
   <213> Homo sapiens
<300>
   <302> FGF23
   <310> NM020638
<400> 81
   atgttggggg cccgcctcag gctctgggtc tgtgccttgt gcagcgtctg cagcatgagc 60
   gtcctcagag cctatcccaa tgcctcccca ctgctcggct ccagctgggg tggcctgatc 120
   cacctgtaca cagccacagc caggaacagc taccacctgc agatccacaa gaatggccat 180
   gtggatggcg caccccatca gaccatctac agtgccctga tgatcagatc agaggatgct 240
   ggctttgtgg tgattacagg tgtgatgagc agaagatacc tctgcatgga tttcagaggc 300
   aacatttttg gatcacacta tttcgacccg gagaactgca ggttccaaca ccagacgctg 360
   gaaaacgggt acgacgtcta ccactctcct cagtatcact tcctggtcag tctgggccgg 420
   gcgaagagag ccttcctgcc aggcatgaac ccacccccgt actcccagtt cctgtcccgg 480
   aggaacgaga tccccctaat tcacttcaac acccccatac cacggcggca cacccggagc 540
   gccgaggacg actcggagcg ggaccccctg aacgtgctga agccccgggc ccggatgacc 600
   ccggccccgg cctcctgttc acaggagctc ccgagcgccg aggacaacag cccgatggcc 660
   agtgacccat taggggtggt caggggcggt cgagtgaaca cgcacgctgg gggaacgggc 720
   ccggaaggct gccgcccctt cgccaagttc atctag                           756
<210> 82
   <211> 720
   <212> DNA
   <213> Homo sapiens
<300>
   <302> FGF3
   <310> NM005247
<400> 82
   atgggcctaa tctggctgct actgctcagc ctgctggagc ccggctggcc cgcagcgggc 60
   cctggggcgc ggttgcggcg cgatgcgggc ggccgtggcg gcgtctacga gcaccttggc 120
   ggggcgcccc ggcgccgcaa gctctactgc gccacgaagt accacctcca gctgcacccg 180
   agcggccgcg tcaacggcag cctggagaac agcgcctaca gtattttgga gataacggca 240
   gtggaggtgg gcattgtggc catcaggggt ctcttctccg ggcggtacct ggccatgaac 300
   aagagcggac gactctatgc ttcggagcac tacagcgccg agtgcgagtt tgtggagcgg 360
   atccacgagc tgggctataa tacgtatgcc tcccggctgt accggacggt gtctagtacg 420
   cctggggccc gccggcagcc cagcgccgag agactgtggt acgtgtctgt gaacggcaag 480
   ggccggcccc gcaggggctt caagacccgc cgcacacaga agtcctccct gttcctgccc 540
   cgcgtgctgg accacaggga ccacgagatg gtgcggcagc tacagagtgg gctgcccaga 600
   ccccctggta agggggtcca gccccgacgg cggcggcaga agcagagccc ggataacctg 660
   gagccctctc acgttcaggc ttcgagactg ggctcccagc tggaggccag tgcgcactag 720
<210> 83
   <211> 807
   <212> DNA
   <213> Homo sapiens
<300>
   <302> FGF5
   <310> NM004464
<400> 83
   atgagcttgt ccttcctcct cctcctcttc ttcagccacc tgatcctcag cgcctgggct 60
   cacggggaga agcgtctcgc ccccaaaggg caacccggac ccgctgccac tgataggaac 120
   cctataggct ccagcagcag acagagcagc agtagcgcta tgtcttcctc ttctgcctcc 180
   tcctcccccg cagcttctct gggcagccaa ggaagtggct tggagcagag cagtttccag 240
   tggagcccct cggggcgccg gaccggcagc ctctactgca gagtgggcat cggtttccat 300
   ctgcagatct acccggatgg caaagtcaat ggatcccacg aagccaatat gttaagtgtt 360
   ttggaaatat ttgctgtgtc tcaggggatt gtaggaatac gaggagtttt cagcaacaaa 420
   tttttagcga tgtcaaaaaa aggaaaactc catgcaagtg ccaagttcac agatgactgc 480
   aagttcaggg agcgttttca agaaaatagc tataatacct atgcctcagc aatacataga 540
   actgaaaaaa cagggcggga gtggtatgtt gccctgaata aaagaggaaa agccaaacga 600
   gggtgcagcc cccgggttaa accccagcat atctctaccc attttcttcc aagattcaag 660
   cagtcggagc agccagaact ttctttcacg gttactgttc ctgaaaagaa aaatccacct 720
   agccctatca agtcaaagat tcccctttct gcacctcgga aaaataccaa ctcagtgaaa 780
   tacagactca agtttcgctt tggataa 807
<210> 84
   <211> 649
   <212> DNA
   <213> Homo sapiens
<300>
   <302> FGF8
   <310> NM006119
<400> 84
   atgggcagcc cccgctccgc gctgagctgc ctgctgttgc acttgctggt cctctgcctc 60
   caagcccagg taactgttca gtcctcacct aattttacac agcatgtgag ggagcagagc 120
   ctggtgacgg atcagctcag ccgccgcctc atccggacct accaactcta cagccgcacc 180
   agcgggaagc acgtgcaggt cctggccaac aagcgcatca acgccatggc agaggacggc 240
   gaccccttcg caaagctcat cgtggagacg gacacctttg gaagcagagt tcgagtccga 300
   ggagccgaga cgggcctcta catctgcatg aacaagaagg ggaagctgat cgccaagagc 360
   aacggcaaag gcaaggactg cgtcttcacg gagattgtgc tggagaacaa ctacacagcg 420
   ctgcagaatg ccaagtacga gggctggtac atggccttca cccgcaaggg ccggccccgc 480
   aagggctcca agacgcggca gcaccagcgt gaggtccact tcatgaagcg gctgccccgg 540
   ggccaccaca ccaccgagca gagcctgcgc ttcgagttcc tcaactaccc gcccttcacg 600
   cgcagcctgc gcggcagcca gaggacttgg gccccggaac cccgatagg 649
<210> 85
   <211> 2466
   <212> DNA
   <213> Homo sapiens
<300>
   <302> FGFR2
   <310> NM000141
<400> 85
   atggtcagct ggggtcgttt catctgcctg gtcgtggtca ccatggcaac cttgtccctg 60
   gcccggccct ccttcagttt agttgaggat accacattag agccagaaga gccaccaacc 120
   aaataccaaa tctctcaacc agaagtgtac gtggctgcgc caggggagtc gctagaggtg 180
   cgctgcctgt tgaaagatgc cgccgtgatc agttggacta aggatggggt gcacttgggg 240
   cccaacaata ggacagtgct tattggggag tacttgcaga taaagggcgc cacgcctaga 300
   gactccggcc tctatgcttg tactgccagt aggactgtag acagtgaaac ttggtacttc 360
   atggtgaatg tcacagatgc catctcatcc ggagatgatg aggatgacac cgatggtgcg 420
   gaagattttg tcagtgagaa cagtaacaac aagagagcac catactggac caacacagaa 480
   aagatggaaa agcggctcca tgctgtgcct gcggccaaca ctgtcaagtt tcgctgccca 540
   gccgggggga acccaatgcc aaccatgcgg tggctgaaaa acgggaagga gtttaagcag 600
   gagcatcgca ttggaggcta caaggtacga aaccagcact ggagcctcat tatggaaagt 660
   gtggtcccat ctgacaaggg aaattatacc tgtgtggtgg agaatgaata cgggtccatc 720
   aatcacacgt accacctgga tgttgtggag cgatcgcctc accggcccat cctccaagcc 780
   ggactgccgg caaatgcctc cacagtggtc ggaggagacg tagagtttgt ctgcaaggtt 840
   tacagtgatg cccagcccca catccagtgg atcaagcacg tggaaaagaa cggcagtaaa 900
   tacgggcccg acgggctgcc ctacctcaag gttctcaagg ccgccggtgt taacaccacg 960
   gacaaagaga ttgaggttct ctatattcgg aatgtaactt ttgaggacgc tggggaatat 1020
   acgtgcttgg cgggtaattc tattgggata tcctttcact ctgcatggtt gacagttctg 1080
   ccagcgcctg gaagagaaaa ggagattaca gcttccccag actacctgga gatagccatt 1140
   tactgcatag gggtcttctt aatcgcctgt atggtggtaa cagtcatcct gtgccgaatg 1200
   aagaacacga ccaagaagcc agacttcagc agccagccgg ctgtgcacaa gctgaccaaa 1260
   cgtatccccc tgcggagaca ggtaacagtt tcggctgagt ccagctcctc catgaactcc 1320
   aacaccccgc tggtgaggat aacaacacgc ctctcttcaa cggcagacac ccccatgctg 1380
   gcaggggtct ccgagtatga acttccagag gacccaaaat gggagtttcc aagagataag 1440
   ctgacactgg gcaagcccct gggagaaggt tgctttgggc aagtggtcat ggcggaagca 1500
   gtgggaattg acaaagacaa gcccaaggag gcggtcaccg tggccgtgaa gatgttgaaa 1560
   gatgatgcca cagagaaaga cctttctgat ctggtgtcag agatggagat gatgaagatg 1620
   attgggaaac acaagaatat cataaatctt cttggagcct gcacacagga tgggcctctc 1680
   tatgtcatag ttgagtatgc ctctaaaggc aacctccgag aatacctccg agcccggagg 1740
   ccacccggga tggagtactc ctatgacatt aaccgtgttc ctgaggagca gatgaccttc 1800
   aaggacttgg tgtcatgcac ctaccagctg gccagaggca tggagtactt ggcttcccaa 1860
   aaatgtattc atcgagattt agcagccaga aatgttttgg taacagaaaa caatgtgatg 1920
   aaaatagcag actttggact cgccagagat atcaacaata tagactatta caaaaagacc 1980
   accaatgggc ggcttccagt caagtggatg gctccagaag ccctgtttga tagagtatac 2040
   actcatcaga gtgatgtctg gtccttcggg gtgttaatgt gggagatctt cactttaggg 2100
   ggctcgccct acccagggat tcccgtggag gaacttttta agctgctgaa ggaaggacac 2160
   agaatggata agccagccaa ctgcaccaac gaactgtaca tgatgatgag ggactgttgg 2220
   catgcagtgc cctcccagag accaacgttc aagcagttgg tagaagactt ggatcgaatt 2280
   ctcactctca caaccaatga ggaatacttg gacctcagcc aacctctcga acagtattca 2340
   cctagttacc ctgacacaag aagttcttgt tcttcaggag atgattctgt tttttctcca 2400
   gaccccatgc cttacgaacc atgccttccc cagtatccac acataaacgg cagtgttaaa 2460
   acatga                                                            2466
<210> 86
   <211> 2421
   <212> DNA
   <213> Homo sapiens
<300>
   <302> FGFR3
   <310> NM000142
<400> 86
   atgggcgccc ctgcctgcgc cctcgcgctc tgcgtggccg tggccatcgt ggccggcgcc 60
   tcctcggagt ccttggggac ggagcagcgc gtcgtggggc gagcggcaga agtcccgggc 120
   ccagagcccg gccagcagga gcagttggtc ttcggcagcg gggatgctgt ggagctgagc 180
   tgtcccccgc ccgggggtgg tcccatgggg cccactgtct gggtcaagga tggcacaggg 240
   ctggtgccct cggagcgtgt cctggtgggg ccccagcggc tgcaggtgct gaatgcctcc 300
   cacgaggact ccggggccta cagctgccgg cagcggctca cgcagcgcgt actgtgccac 360
   ttcagtgtgc gggtgacaga cgctccatcc tcgggagatg acgaagacgg ggaggacgag 420
   gctgaggaca caggtgtgga cacaggggcc ccttactgga cacggcccga gcggatggac 480
   aagaagctgc tggccgtgcc ggccgccaac accgtccgct tccgctgccc agccgctggc 540
   aaccccactc cctccatctc ctggctgaag aacggcaggg agttccgcgg cgagcaccgc 600
   attggaggca tcaagctgcg gcatcagcag tggagcctgg tcatggaaag cgtggtgccc 660
   tcggaccgcg gcaactacac ctgcgtcgtg gagaacaagt ttggcagcat ccggcagacg 720
   tacacgctgg acgtgctgga gcgctccccg caccggccca tcctgcaggc ggggctgccg 780
   gccaaccaga cggcggtgct gggcagcgac gtggagttcc actgcaaggt gtacagtgac 840
   gcacagcccc acatccagtg gctcaagcac gtggaggtga acggcagcaa ggtgggcccg 900
   gacggcacac cctacgttac cgtgctcaag acggcgggcg ctaacaccac cgacaaggag 960
   ctagaggttc tctccttgca caacgtcacc tttgaggacg ccggggagta cacctgcctg 1020
   gcgggcaatt ctattgggtt ttctcatcac tctgcgtggc tggtggtgct gccagccgag 1080
   gaggagctgg tggaggctga cgaggcgggc agtgtgtatg caggcatcct cagctacggg 1140
   gtgggcttct tcctgttcat cctggtggtg gcggctgtga cgctctgccg cctgcgcagc 1200
   ccccccaaga aaggcctggg ctcccccacc gtgcacaaga tctcccgctt cccgctcaag 1260
   cgacaggtgt ccctggagtc caacgcgtcc atgagctcca acacaccact ggtgcgcatc 1320
   gcaaggctgt cctcagggga gggccccacg ctggccaatg tctccgagct cgagctgcct 1380
   gccgacccca aatgggagct gtctcgggcc cggctgaccc tgggcaagcc ccttggggag 1440
   ggctgcttcg gccaggtggt catggcggag gccatcggca ttgacaagga ccgggccgcc 1500
   aagcctgtca ccgtagccgt gaagatgctg aaagacgatg ccactgacaa ggacctgtcg 1560
   gacctggtgt ctgagatgga gatgatgaag atgatcggga aacacaaaaa catcatcaac 1620
   ctgctgggcg cctgcacgca gggcgggccc ctgtacgtgc tggtggagta cgcggccaag 1680
   ggtaacctgc gggagtttct gcgggcgcgg cggcccccgg gcctggacta ctccttcgac 1740
   acctgcaagc cgcccgagga gcagctcacc ttcaaggacc tggtgtcctg tgcctaccag 1800
   gtggcccggg gcatggagta cttggcctcc cagaagtgca tccacaggga cctggctgcc 1860
   cgcaatgtgc tggtgaccga ggacaacgtg atgaagatcg cagacttcgg gctggcccgg 1920
   gacgtgcaca acctcgacta ctacaagaag acaaccaacg gccggctgcc cgtgaagtgg 1980
   atggcgcctg aggccttgtt tgaccgagtc tacactcacc agagtgacgt ctggtccttt 2040
   ggggtcctgc tctgggagat cttcacgctg gggggctccc cgtaccccgg catccctgtg 2100
   gaggagctct tcaagctgct gaaggagggc caccgcatgg acaagcccgc caactgcaca 2160
   cacgacctgt acatgatcat gcgggagtgc tggcatgccg cgccctccca gaggcccacc 2220
   ttcaagcagc tggtggagga cctggaccgt gtccttaccg tgacgtccac cgacgagtac 2280
   ctggacctgt cggcgccttt cgagcagtac tccccgggtg gccaggacac ccccagctcc 2340
   agctcctcag gggacgactc cgtgtttgcc cacgacctgc tgcccccggc cccacccagc 2400
   agtgggggct cgcggacgtg a 2421
<210> 87
   <211> 2102
   <212> DNA
   <213> Homo sapiens
<300>
   <302> HGF
   <310> E08541
<400> 87
   atgcagaggg acaaaggaaa agaagaaata caattcatga attcaaaaaa tcagcaaaga 60
   ctaccctaat caaaatagat ccagcactga agataaaaac caaaaaagtg aatactgcag 120
   accaatgtgc taatagatgt actaggaata aaggacttcc attcacttgc aaggcttttg 180
   tttttgataa agcaagaaaa caatgcctct ggttcccctt caatagcatg tcaagtggag 240
   tgaaaaaaga atttggccat gaatttgacc tctatgaaaa caaagactac attagaaact 300
   gcatcattgg taaaggacgc agctacaagg gaacagtatc tatcactaag agtggcatca 360
   aatgtcagcc ctggagttcc atgataccac acgaacacag ctttttgcct tcgagctatc 420
   ggggtaaaga cctacaggaa aactactgtc gaaatcctcg aggggaagaa gggggaccct 480
   ggtgtttcac aagcaatcca gaggtacgct acgaagtctg tgacattcct cagtgttcag 540
   aagttgaatg catgacctgc aatggggaga gttatcgagg tctcatggat catacagaat 600
   caggcaagat ttgtcagcgc tgggatcatc agacaccaca ccggcacaaa ttcttgcctg 660
   aaagatatcc cgacaagggc tttgatgata attattgccg caatcccgat ggccagccga 720
   ggccatggtg ctatactctt gaccctcaca cccgctggga gtactgtgca attaaaacat 780
   gcgctgacaa tactatgaat gacactgatg ttcctttgga aacaactgaa tgcatccaag 840
   gtcaaggaga aggctacagg ggcactgtca ataccatttg gaatggaatt ccatgtcagc 900
   gttgggattc tcagtatcct cacgagcatg acatgactcc tgaaaatttc aagtgcaagg 960
   acctacgaga aaattactgc cgaaatccag atgggtctga atcaccctgg tgttttacca 1020
   ctgatccaaa catccgagtt ggctactgct cccaaattcc aaactgtgat atgtcacatg 1080
   gacaagattg ttatcgtggg aatggcaaaa attatatggg caacttatcc caaacaagat 1140
   ctggactaac atgttcaatg tgggacaaga acatggaaga cttacatcgt catatcttct 1200
   gggaaccaga tgcaagtaag ctgaatgaga attactgccg aaatccagat gatgatgctc 1260
   atggaccctg gtgctacacg ggaaatccac tcattccttg ggattattgc cctatttctc 1320
   gttgtgaagg tgataccaca cctacaatag tcaatttaga ccatcccgta atatcttgtg 1380
   ccaaaaggaa acaattgcga gttgtaaatg ggattccaac acgaacaaac ataggatgga 1440
   tggttagttt gagatacaga aataaacata tctgcggagg atcattgata aaggagagtt 1500
   gggttcttac tgcacgacag tgtttccctt ctcgagactt gaaagattat gaagcttggc 1560
   ttggaattca tgatgtccac ggaagaggag atgagaaatg caaacaggtt ctcaatgttt 1620
   cccagctggt atatggccct gaaggatcag atctggtttt aatgaagctt gccaggcctg 1680
   ctgtcctgga tgattttgtt agtacgattg atttacctaa ttatggatgc acaattcctg 1740
   aaaagaccag ttgcagtgtt tatggctggg gctacactgg attgatcaac tatgatggcc 1800
   tattacgagt ggcacatctc tatataatgg gaaatgagaa atgcagccag catcatcgag 1860
   ggaaggtgac tctgaatgag tctgaaatat gtgctggggc tgaaaagatt ggatcaggac 1920
   catgtgaggg ggattatggt ggcccacttg tttgtgagca acataaaatg agaatggttc 1980
   ttggtgtcat tgttcctggt cgtggatgtg ccattccaaa tcgtcctggt atttttgtcc 2040
   gagtagcata ttatgcaaaa tggatacaca aaattatttt aacatataag gtaccacagt 2100
   ca                                                                2102
<210> 88
   <211> 360
   <212> DNA
   <213> Homo sapiens
<300>
   <302> ID3
   <310> XM001539
<400> 88
   atgaaggcgc tgagcccggt gcgcggctgc tacgaggcgg tgtgctgcct gtcggaacgc 60
   agtctggcca tcgcccgggg ccgagggaag ggcccggcag ctgaggagcc gctgagcttg 120
   ctggacgaca tgaaccactg ctactcccgc ctgcgggaac tggtacccgg agtcccgaga 180
   ggcactcagc ttagccaggt ggaaatccta cagcgcgtca tcgactacat tctcgacctg 240
   caggtagtcc tggccgagcc agcccctgga ccccctgatg gcccccacct tcccatccag 300
   acagccgagc tcactccgga acttgtcatc tccaacgaca aaaggagctt ttgccactga 360
<210> 89
   <211> 743
   <212> DNA
   <313> Homo sapiens
<300>
   <302> IGF2
   <310> NM000612
<400> 89
   atgggaatcc caatgcggaa gtcgatgctg gtgcttctca ccttcttggc cttcgcctcg 60
   tgctgcattg ctgcttaccg ccccagtgag accctgtgcg gcggggagct ggtcgacacc 120
   ctccagttcg tctgtgggga ccgcggcttc tacttcagca ggcccgcaag ccgtgtgagc 180
   cgtcgcagcc gtggcatcgt tgaggagtgc tgtttccgca gctgtgacct ggccctcctg 240
   gagacgtact gtgctacccc cgccaagtcc gagagggacg tgtcgacccc tccgaccgtg 300
   cttccggaca acttccccag ataccccgtg ggcaagttct tccaatatga cacctggaag 360
   cagtccaccc agcgcctgcg caggggcctg cctgccctcc tgcgtgcccg ccggggtcac 420
   gtgctcgcca aggagctcga ggcgttcagg gaggccaaac gtcaccgtcc cctgattgct 480
   ctacccaccc aagaccccgc ccacgggggc gcccccccag agatggccag caatcggaag 540
   tgagcaaaac tgccgcaagt ctgcagcccg gcgccaccat cctgcagcct cctcctgacc 600
   acggacgttt ccatcaggtt ccatcccgaa aatctctcgg ttccacgtcc ccctggggct 660
   tctcctgacc cagtccccgt gccccgcctc cccgaaacag gctactctcc tcggccccct 720
   ccatcgggct gaggaagcac agc 743
<210> 90
   <211> 7476
   <212> DNA
   <213> Homo sapiens
<300>
   <302> IGF2R
   <310> NM000876
<400> 90
   atgggggccg ccgccggccg gagcccccac ctggggcccg cgcccgcccg ccgcccgcag 60
   cgctctctgc tcctgctgca gctgctgctg ctcgtcgctg ccccggggtc cacgcaggcc 120
   caggccgccc cgttccccga gctgtgcagt tatacatggg aagctgttga taccaaaaat 180
   aatgtacttt ataaaatcaa catctgtgga agtgtggata ttgtccagtg cgggccatca 240
   agtgctgttt gtatgcacga cttgaagaca cgcacttatc attcagtggg tgactctgtt 300
   ttgagaagtg caaccagatc tctcctggaa ttcaacacaa cagtgagctg tgaccagcaa 360
   ggcacaaatc acagagtcca gagcagcatt gccttcctgt gtgggaaaac cctgggaact 420
   cctgaatttg taactgcaac agaatgtgtg cactactttg agtggaggac cactgcagcc 480
   tgcaagaaag acatatttaa agcaaataag gaggtgccat gctatgtgtt tgatgaagag 540
   ttgaggaagc atgatctcaa tcctctgatc aagcttagtg gtgcctactt ggtggatgac 600
   tccgatccgg acacttctct attcatcaat gtttgtagag acatagacac actacgagac 660
   ccaggttcac agctgcgggc ctgtcccccc ggcactgccg cctgcctggt aagaggacac 720
   caggcgtttg atgttggcca gccccgggac ggactgaagc tggtgcgcaa ggacaggctt 780
   gtcctgagtt acgtgaggga agaggcagga aagctagact tttgtgatgg tcacagccct 840
   gcggtgacta ttacatttgt ttgcccgtcg gagcggagag agggcaccat tcccaaactc 900
   acagctaaat ccaactgccg ctatgaaatt gagtggatta ctgagtatgc ctgccacaga 960
   gattacctgg aaagtaaaac ttgttctctg agcggcgagc agcaggatgt ctccatagac 1020
   ctcacaccac ttgcccagag cggaggttca tcctatattt cagatggaaa agaatatttg 1080
   ttttatttga atgtctgtgg agaaactgaa atacagttct gtaataaaaa acaagctgca 1140
   gtttgccaag tgaaaaagag cgatacctct caagtcaaag cagcaggaag ataccacaat 1200
   cagaccctcc gatattcgga tggagacctc accttgatat attttggagg tgatgaatgc 1260
   agctcagggt ttcagcggat gagcgtcata aactttgagt gcaataaaac cgcaggtaac 1320
   gatgggaaag gaactcctgt attcacaggg gaggttgact gcacctactt cttcacatgg 1380
   gacacggaat acgcctgtgt taaggagaag gaagacctcc tctgcggtgc caccgacggg 1440
   aagaagcgct atgacctgtc cgcgctggtc cgccatgcag aaccagagca gaattgggaa 1500
   gctgtggatg gcagtcagac ggaaacagag aagaagcatt ttttcattaa tatttgtcac 1560
   agagtgctgc aggaaggcaa ggcacgaggg tgtcccgagg acgcggcagt gtgtgcagtg 1620
   gataaaaatg gaagtaaaaa tctgggaaaa tttatttcct ctcccatgaa agagaaagga 1680
   aacattcaac tctcttattc agatggtgat gattgtggtc atggcaagaa aattaaaact 1740
   aatatcacac ttgtatgcaa gccaggtgat ctggaaagtg caccagtgtt gagaacttct 1800
   ggggaaggcg gttgctttta tgagtttgag tggcgcacag ctgcggcctg tgtgctgtct 1860
   aagacagaag gggagaactg cacggtcttt gactcccagg cagggttttc ttttgactta 1920
   tcacctctca caaagaaaaa tggtgcctat aaagttgaga caaagaagta tgacttttat 1980
   ataaatgtgt gtggcccggt gtctgtgagc ccctgtcagc cagactcagg agcctgccag 2040
   gtggcaaaaa gtgatgagaa gacttggaac ttgggtctga gtaatgcgaa gctttcatat 2100
   tatgatggga tgatccaact gaactacaga ggcggcacac cctataacaa tgaaagacac 2160
   acaccgagag ctacgctcat cacctttctc tgtgatcgag acgcgggagt gggcttccct 2220
   gaatatcagg aagaggataa ctccacctac aacttccggt ggtacaccag ctatgcctgc 2280
   ccggaggagc ccctggaatg cgtagtgacc gacccctcca cgctggagca gtacgacctc 2340
   tccagtctgg caaaatctga aggtggcctt ggaggaaact ggtatgccat ggacaactca 2400
   ggggaacatg tcacgtggag gaaatactac attaacgtgt gtcggcctct gaatccagtg 2460
   ccgggctgca accgatatgc atcggcttgc cagatgaagt atgaaaaaga tcagggctcc 2520
   ttcactgaag tggtttccat cagtaacttg ggaatggcaa agaccggccc ggtggttgag 2580
   gacagcggca gcctccttct ggaatacgtg aatgggtcgg cctgcaccac cagcgatggc 2640
   agacagacca catataccac gaggatccat ctcgtctgct ccaggggcag gctgaacagc 2700
   caccccatct tttctctcaa ctgggagtgt gtggtcagtt tcctgtggaa cacagaggct 2760
   gcctgtccca ttcagacaac gacggataca gaccaggctt gctctataag ggatcccaac 2820
   agtggatttg tgtttaatct taatccgcta aacagttcgc aaggatataa cgtctctggc 2880
   attgggaaga tttttatgtt taatgtctgc ggcacaatgc ctgtctgtgg gaccatcctg 2940
   ggaaaacctg cttctggctg tgaggcagaa acccaaactg aagagctcaa gaattggaag 3000
   ccagcaaggc cagtcggaat tgagaaaagc ctccagctgt ccacagaggg cttcatcact 3060
   ctgacctaca aagggcctct ctctgccaaa ggtaccgctg atgcttttat cgtccgcttt 3120
   gtttgcaatg atgatgttta ctcagggccc ctcaaattcc tgcatcaaga tatcgactct 3180
   gggcaaggga tccgaaacac ttactttgag tttgaaaccg cgttggcctg tgttccttct 3240
   ccagtggact gccaagtcac cgacctggct ggaaatgagt acgacctgac tggcctaagc 3300
   acagtcagga aaccttggac ggctgttgac acctctgtcg atgggagaaa gaggactttc 3360
   tatttgagcg tttgcaatcc tctcccttac attcctggat gccagggcag cgcagtgggg 3420
   tcttgcttag tgtcagaagg caatagctgg aatctgggtg tggtgcagat gagtccccaa 3480
   gccgcggcga atggatcttt gagcatcatg tatgtcaacg gtgacaagtg tgggaaccag 3540
   cgcttctcca ccaggatcac gtttgagtgt gctcagatat cgggctcacc agcatttcag 3600
   cttcaggatg gttgtgagta cgtgtttatc tggagaactg tggaagcctg tcccgttgtc 3660
   agagtggaag gggacaactg tgaggtgaaa gacccaaggc atggcaactt gtatgacctg 3720
   aagcccctgg gcctcaacga caccatcgtg agcgctggcg aatacactta ttacttccgg 3780
   gtctgtggga agctttcctc agacgtctgc cccacaagtg acaagtccaa ggtggtctcc 3840
   tcatgtcagg aaaagcggga accgcaggga tttcacaaag tggcaggtct cctgactcag 3900
   aagctaactt atgaaaatgg cttgttaaaa atgaacttca cgggggggga cacttgccat 3960
   aaggtttatc agcgctccac agccatcttc ttctactgtg accgcggcac ccagcggcca 4020
   gtatttctaa aggagacttc agattgttcc tacttgtttg agtggcgaac gcagtatgcc 4080
   tgcccacctt tcgatctgac tgaatgttca ttcaaagatg gggctggcaa ctccttcgac 4140
   ctctcgtccc tgtcaaggta cagtgacaac tgggaagcca tcactgggac gggggacccg 4200
   gagcactacc tcatcaatgt ctgcaagtct ctggccccgc aggctggcac tgagccgtgc 4260
   cctccagaag cagccgcgtg tctgctgggt ggctccaagc ccgtgaacct cggcagggta 4320
   agggacggac ctcagtggag agatggcata attgtcctga aatacgttga tggcgactta 4380
   tgtccagatg ggattcggaa aaagtcaacc accatccgat tcacctgcag cgagagccaa 4440
   gtgaactcca ggcccatgtt catcagcgcc gtggaggact gtgagtacac ctttgcctgg 4500
   cccacagcca cagcctgtcc catgaagagc aacgagcatg atgactgcca ggtcaccaac 4560
   ccaagcacag gacacctgtt tgatctgagc tccttaagtg gcagggcggg attcacagct 4620
   gcttacagcg agaaggggtt ggtttacatg agcatctgtg gggagaatga aaactgccct 4680
   cctggcgtgg gggcctgctt tggacagacc aggattagcg tgggcaaggc caacaagagg 4740
   ctgagatacg tggaccaggt cctgcagctg gtgtacaagg atgggtcccc ttgtccctcc 4800
   aaatccggcc tgagctataa gagtgtgatc agtttcgtgt gcaggcctga ggccgggcca 4860
   accaataggc ccatgctcat ctccctggac aagcagacat gcactctctt cttctcctgg 4920
   cacacgccgc tggcctgcga gcaagcgacc gaatgttccg tgaggaatgg aagctctatt 4980
   gttgacttgt ctccccttat tcatcgcact ggtggttatg aggcttatga tgagagtgag 5040
   gatgatgcct ccgataccaa ccctgatttc tacatcaata tttgtcagcc actaaatccc 5100
   atgcacgcag tgccctgtcc tgccggagcc gctgtgtgca aagttcctat tgatggtccc 5160
   cccatagata tcggccgggt agcaggacca ccaatactca atccaatagc aaatgagatt 5220
   tacttgaatt ttgaaagcag tactccttgc ttagcggaca agcatttcaa ctacacctcg 5280
   ctcatcgcgt ttcactgtaa gagaggtgtg agcatgggaa cgcctaagct gttaaggacc 5340
   agcgagtgcg actttgtgtt cgaatgggag actcctgtcg tctgtcctga tgaagtgagg 5400
   atggatggct gtaccctgac agatgagcag ctcctctaca gcttcaactt gtccagcctt 5460
   tccacgagca cctttaaggt gactcgcgac tcgcgcacct acagcgttgg ggtgtgcacc 5520
   tttgcagtcg ggccagaaca aggaggctgt aaggacggag gagtctgtct gctctcaggc 5580
   accaaggggg catcctttgg acggctgcaa tcaatgaaac tggattacag gcaccaggat 5640
   gaagcggtcg ttttaagtta cgtgaatggt gatcgttgcc ctccagaaac cgatgacggc 5700
   gtcccctgtg tcttcccctt catattcaat gggaagagct acgaggagtg catcatagag 5760
   agcagggcga agctgtggtg tagcacaact gcggactacg acagagacca cgagtggggc 5820
   ttctgcagac actcaaacag ctaccggaca tccagcatca tatttaagtg tgatgaagat 5880
   gaggacattg ggaggccaca agtcttcagt gaagtgcgtg ggtgtgatgt gacatttgag 5940
   tggaaaacaa aagttgtctg ccctccaaag aagttggagt gcaaattcgt ccagaaacac 6000
   aaaacctacg acctgcggct gctctcctct ctcaccgggt cctggtccct ggtccacaac 6060
   ggagtctcgt actatataaa tctgtgccag aaaatatata aagggcccct gggctgctct 6120
   gaaagggcca gcatttgcag aaggaccaca actggtgacg tccaggtcct gggactcgtt 6180
   cacacgcaga agctgggtgt cataggtgac aaagttgttg tcacgtactc caaaggttat 6240
   ccgtgtggtg gaaataagac cgcatcctcc gtgatagaat tgacctgtac aaagacggtg 6300
   ggcagacctg cattcaagag gtttgatatc gacagctgca cttactactt cagctgggac 6360
   tcccgggctg cctgcgccgt gaagcctcag gaggtgcaga tggtgaatgg gaccatcacc 6420
   aaccctataa atggcaagag cttcagcctc ggagatattt attttaagct gttcagagcc 6480
   tctggggaca tgaggaccaa tggggacaac tacctgtatg agatccaact ttcctccatc 6540
   acaagctcca gaaacccggc gtgctctgga gccaacatat gccaggtgaa gcccaacgat 6600
   cagcacttca gtcggaaagt tggaacctct gacaagacca agtactacct tcaagacggc 6660
   gatctcgatg tcgtgtttgc ctcttcctct aagtgcggaa aggataagac caagtctgtt 6720
   tcttccacca tcttcttcca ctgtgaccct ctggtggagg acgggatccc cgagttcagt 6780
   cacgagactg ccgactgcca gtacctcttc tcttggtaca cctcagccgt gtgtcctctg 6840
   ggggtgggct ttgacagcga gaatcccggg gacgacgggc agatgcacaa ggggctgtca 6900
   gaacggagcc aggcagtcgg cgcggtgctc agcctgctgc tggtggcgct cacctgctgc 6960
   ctgctggccc tgttgctcta caagaaggag aggagggaaa cagtgataag taagctgacc 7020
   acttgctgta ggagaagttc caacgtgtcc tacaaatact caaaggtgaa taaggaagaa 7080
   gagacagatg agaatgaaac agagtggctg atggaagaga tccagctgcc tcctccacgg 7140
   cagggaaagg aagggcagga gaacggccat attaccacca agtcagtgaa agccctcagc 7200
   tccctgcatg ggatgaccag ggacagtgag gatgaggttc tgaccatccc agaggtgaaa 7260
   gttcactcgg gcaggggagc tggggcagag agctcccacc cagtgagaaa cgcacagagc 7320
   aatgcccttc aggagcgtga ggacgatagg gtggggctgg tcaggggtga gaaggcgagg 7380
   aaagggaagt ccagctctgc acagcagaag acagtgagct ccaccaagct ggtgtccttc 7440
   catgacgaca gcgacgagga cctcttacac atctga                            7476
<210> 91
   <211> 4104
   <212> DNA
   <213> Homo sapiens
<300>
   <302> IGF1R
   <310> NM000875
<400> 91
   atgaagtctg gctccggagg agggtccccg acctcgctgt gggggctcct gtttctctcc 60
   gccgcgctct cgctctggcc gacgagtgga gaaatctgcg ggccaggcat cgacatccgc 120
   aacgactatc agcagctgaa gcgcctggag aactgcacgg tgatcgaggg ctacctccac 180
   atcctgctca tctccaaggc cgaggactac cgcagctacc gcttccccaa gctcacggtc 240
   attaccgagt acttgctgct gttccgagtg gctggcctcg agagcctcgg agacctcttc 300
   cccaacctca cggtcatccg cggctggaaa ctcttctaca actacgccct ggtcatcttc 360
   gagatgacca atctcaagga tattgggctt tacaacctga ggaacattac tcggggggcc 420
   atcaggattg agaaaaatgc tgacctctgt tacctctcca ctgtggactg gtccctgatc 480
   ctggatgcgg tgtccaataa ctacattgtg gggaataagc ccccaaagga atgtggggac 540
   ctgtgtccag ggaccatgga ggagaagccg atgtgtgaga agaccaccat caacaatgag 600
   tacaactacc gctgctggac cacaaaccgc tgccagaaaa tgtgcccaag cacgtgtggg 660
   aagcgggcgt gcaccgagaa caatgagtgc tgccaccccg agtgcctggg cagctgcagc 720
   gcgcctgaca acgacacggc ctgtgtagct tgccgccact actactatgc cggtgtctgt 780
   gtgcctgcct gcccgcccaa cacctacagg tttgagggct ggcgctgtgt ggaccgtgac 840
   ttctgcgcca acatcctcag cgccgagagc agcgactccg aggggtttgt gatccacgac 900
   ggcgagtgca tgcaggagtg cccctcgggc ttcatccgca acggcagcca gagcatgtac 960
   tgcatccctt gtgaaggtcc ttgcccgaag gtctgtgagg aagaaaagaa aacaaagacc 1020
   attgattctg ttacttctgc tcagatgctc caaggatgca ccatcttcaa gggcaatttg 1080
   ctcattaaca tccgacgggg gaataacatt gcttcagagc tggagaactt catggggctc 1140
   atcgaggtgg tgacgggcta cgtgaagatc cgccattctc atgccttggt ctccttgtcc 1200
   ttcctaaaaa accttcgcct catcctagga gaggagcagc tagaagggaa ttactccttc 1260
   tacgtcctcg acaaccagaa cttgcagcaa ctgtgggact gggaccaccg caacctgacc 1320
   atcaaagcag ggaaaatgta ctttgctttc aatcccaaat tatgtgtttc cgaaatttac 1380
   cgcatggagg aagtgacggg gactaaaggg cgccaaagca aaggggacat aaacaccagg 1440
   aacaacgggg agagagcctc ctgtgaaagt gacgtcctgc atttcacctc caccaccacg 1500
   tcgaagaatc gcatcatcat aacctggcac cggtaccggc cccctgacta cagggatctc 1560
   atcagcttca ccgtttacta caaggaagca ccctttaaga atgtcacaga gtatgatggg 1620
   caggatgcct gcggctccaa cagctggaac atggtggacg tggacctccc gcccaacaag 1680
   gacgtggagc ccggcatctt actacatggg ctgaagccct ggactcagta cgccgtttac 1740
   gtcaaggctg tgaccctcac catggtggag aacgaccata tccgtggggc caagagtgag 1800
   atcttgtaca ttcgcaccaa tgcttcagtt ccttccattc ccttggacgt tctttcagca 1860
   tcgaactcct cttctcagtt aatcgtgaag tggaaccctc cctctctgcc caacggcaac 1920
   ctgagttact acattgtgcg ctggcagcgg cagcctcagg acggctacct ttaccggcac 1980
   aattactgct ccaaagacaa aatccccatc aggaagtatg ccgacggcac catcgacatt 2040
   gaggaggtca cagagaaccc caagactgag gtgtgtggtg gggagaaagg gccttgctgc 2100
   gcctgcccca aaactgaagc cgagaagcag gccgagaagg aggaggctga ataccgcaaa 2160
   gtctttgaga atttcctgca caactccatc ttcgtgccca gacctgaaag gaagcggaga 2220
   gatgtcatgc aagtggccaa caccaccatg tccagccgaa gcaggaacac cacggccgca 2280
   gacacctaca acatcaccga cccggaagag ctggagacag agtacccttt ctttgagagc 2340
   agagtggata acaaggagag aactgtcatt tctaaccttc ggcctttcac attgtaccgc 2400
   atcgatatcc acagctgcaa ccacgaggct gagaagctgg gctgcagcgc ctccaacttc 2460
   gtctttgcaa ggactatgcc cgcagaagga gcagatgaca ttcctgggcc agtgacctgg 2520
   gagccaaggc ctgaaaactc catcttttta aagtggccgg aacctgagaa tcccaatgga 2580
   ttgattctaa tgtatgaaat aaaatacgga tcacaagttg aggatcagcg agaatgtgtg 2640
   tccagacagg aatacaggaa gtatggaggg gccaagctaa accggctaaa cccggggaac 2700
   tacacagccc ggattcaggc cacatctctc tctgggaatg ggtcgtggac agatcctgtg 2760
   ttcttctatg tccaggccaa aacaggatat gaaaacttca tccatctgat catcgctctg 2820
   cccgtcgctg tcctgttgat cgtgggaggg ttggtgatta tgctgtacgt cttccataga 2880
   aagagaaata acagcaggct ggggaatgga gtgctgtatg cctctgtgaa cccggagtac 2940
   ttcagcgctg ctgatgtgta cgttcctgat gagtgggagg tggctcggga gaagatcacc 3000
   atgagccggg aacttgggca ggggtcgttt gggatggtct atgaaggagt tgccaagggt 3060
   gtggtgaaag atgaacctga aaccagagtg gccattaaaa cagtgaacga ggccgcaagc 3120
   atgcgtgaga ggattgagtt tctcaacgaa gcttctgtga tgaaggagtt caattgtcac 3180
   catgtggtgc gattgctggg tgtggtgtcc caaggccagc caacactggt catcatggaa 3240
   ctgatgacac ggggcgatct caaaagttat ctccggtctc tgaggccaga aatggagaat 3300
   aatccagtcc tagcacctcc aagcctgagc aagatgattc agatggccgg agagattgca 3360
   gacggcatgg catacctcaa cgccaataag ttcgtccaca gagaccttgc tgcccggaat 3420
   tgcatggtag ccgaagattt cacagtcaaa atcggagatt ttggtatgac gcgagatatc 3480
   tatgagacag actattaccg gaaaggaggc aaagggctgc tgcccgtgcg ctggatgtct 3540
   cctgagtccc tcaaggatgg agtcttcacc acttactcgg acgtctggtc cttcggggtc 3600
   gtcctctggg agatcgccac actggccgag cagccctacc agggcttgtc caacgagcaa 3660
   gtccttcgct tcgtcatgga gggcggcctt ctggacaagc cagacaactg tcctgacatg 3720
   ctgtttgaac tgatgcgcat gtgctggcag tataacccca agatgaggcc ttccttcctg 3780
   gagatcatca gcagcatcaa agaggagatg gagcctggct tccgggaggt ctccttctac 3840
   tacagcgagg agaacaagct gcccgagccg gaggagctgg acctggagcc agagaacatg 3900
   gagagcgtcc ccctggaccc ctcggcctcc tcgtcctccc tgccactgcc cgacagacac 3960
   tcaggacaca aggccgagaa cggccccggc cctggggtgc tggtcctccg cgccagcttc 4020
   gacgagagac agccttacgc ccacatgaac gggggccgca agaacgagcg ggccttgccg 4080
   ctgccccagt cttcgacctg ctga                                       4104
<210> 92
   <211> 726
   <212> DNA
   <213> Homo sapiens
<300>
   <302> PDGFB
   <310> NM002608
<400> 92
   atgaatcgct gctgggcgct cttcctgtct ctctgctgct acctgcgtct ggtcagcgcc 60
   gagggggacc ccattcccga ggagctttat gagatgctga gtgaccactc gatccgctcc 120
   tttgatgatc tccaacgcct gctgcacgga gaccccggag aggaagatgg ggccgagttg 180
   gacctgaaca tgacccgctc ccactctgga ggcgagctgg agagcttggc tcgtggaaga 240
   aggagcctgg gttccctgac cattgctgag cccgccatga tcgccgagtg caagacgcgc 300
   accgaggtgt tcgagatctc ccggcgcctc atagaccgca ccaacgccaa cttcctggtg 360
   tggccgccct gtgtggaggt gcagcgctgc tccggctgct gcaacaaccg caacgtgcag 420
   tgccgcccca cccaggtgca gctgcgacct gtccaggtga gaaagatcga gattgtgcgg 480
   aagaagccaa tctttaagaa ggccacggtg acgctggaag accacctggc atgcaagtgt 540
   gagacagtgg cagctgcacg gcctgtgacc cgaagcccgg ggggttccca cgagcagcga 600
   gccaaaacgc cccaaactcg ggtgaccatt cggacggtgc gagtccgccg gccccccaag 660
   ggcaagcacc ggaaattcaa gcacacgcat gacaagacgg cactgaagga gacccttgga 720
   gcctag                                                           726
<210> 93
   <212> 1512
   <212> DNA
   <213> Homo sapiens
<300>
   <302> TGFbetaR1
   <310> NM004612
<400> 93
<210> 94
   <211> 4044
   <212> DNA
   <213> Homo sapiens
<300>
   <302> Flk1
   <310> AF035121
<400> 94
   atgcagagca aggtgctgct ggccgtcgcc ctgtggctct gcgtggagac ccgggccgcc 60
   tctgtgggtt tgcctagtgt ttctcttgat ctgcccaggc tcagcataca aaaagacata 120
   cttacaatta aggctaatac aactcttcaa attacttgca ggggacagag ggacttggac 180
   tggctttggc ccaataatca gagtggcagt gagcaaaggg tggaggtgac tgagtgcagc 240
   gatggcctct tctgtaagac actcacaatt ccaaaagtga tcggaaatga cactggagcc 300
   tacaagtgct tctaccggga aactgacttg gcctcggtca tttatgtcta tgttcaagat 360
   tacagatctc catttattgc ttctgttagt gaccaacatg gagtcgtgta cattactgag 420
   aacaaaaaca aaactgtggt gattccatgt ctcgggtcca tttcaaatct caacgtgtca 480
   ctttgtgcaa gatacccaga aaagagattt gttcctgatg gtaacagaat ttcctgggac 540
   agcaagaagg gctttactat tcccagctac atgatcagct atgctggcat ggtcttctgt 600
   gaagcaaaaa ttaatgatga aagttaccag tctattatgt acatagttgt cgttgtaggg 660
   tataggattt atgatgtggt tctgagtccg tctcatggaa ttgaactatc tgttggagaa 720
   aagcttgtct taaattgtac agcaagaact gaactaaatg tggggattga cttcaactgg 780
   gaataccctt cttcgaagca tcagcataac aaacttgtaa accgagacct aaaaacccag 840
   tctgggagtg agatgaagaa atttttgagc accttaacta tagatggtgt aacccggagt 900
   gaccaaggat tgtacacctg tgcagcatcc agtgggctga tgaccaagaa gaacagcaca 960
   tttgtcaggg tccatgaaaa accttttgtt gcttttggaa gtggcatgga atctctggtg 1020
   gaagccacgg tgggggagcg tgtcagaatc cctgcgaagt accttggtta cccaccccca 1080
   gaaataaaat ggtataaaaa tggaataccc cttgagtcca atcacacaat taaagcgggg 1140
   catgtactga cgattatgga agtgagtgaa agagacacag gaaattacac tgtcatcctt 1200
   accaatccca tttcaaagga gaagcagagc catgtggtct ctctggttgt gtatgtccca 1260
   ccccagattg gtgagaaatc tctaatctct cctgtggatt cctaccagta cggcaccact 1320
   caaacgctga catgtacggt ctatgccatt cctcccccgc atcacatcca ctggtattgg 1380
   cagttggagg aagagtgcgc caacgagccc agccaagctg tctcagtgac aaacccatac 1440
   ccttgtgaag aatggagaag tgtggaggac ttccagggag gaaataaaat tgaagttaat 1500
   aaaaatcaat ttgctctaat tgaaggaaaa aacaaaactg taagtaccct tgttatccaa 1560
   gcggcaaatg tgtcagcttt gtacaaatgt gaagcggtca acaaagtcgg gagaggagag 1620
   agggtgatct ccttccacgt gaccaggggt cctgaaatta ctttgcaacc tgacatgcag 1680
   cccactgagc aggagagcgt gtctttgtgg tgcactgcag acagatctac gtttgagaac 1740
   ctcacatggt acaagcttgg cccacagcct ctgccaatcc atgtgggaga gttgcccaca 1800
   cctgtttgca agaacttgga tactctttgg aaattgaatg ccaccatgtt ctctaatagc 1860
   acaaatgaca ttttgatcat ggagcttaag aatgcatcct tgcaggacca aggagactat 1920
   gtctgccttg ctcaagacag gaagaccaag aaaagacatt gcgtggtcag gcagctcaca 1980
   gtcctagagc gtgtggcacc cacgatcaca ggaaacctgg agaatcagac gacaagtatt 2040
   ggggaaagca tcgaagtctc atgcacggca tctgggaatc cccctccaca gatcatgtgg 2100
   tttaaagata atgagaccct tgtagaagac tcaggcattg tattgaagga tgggaaccgg 2160
   aacctcacta tccgcagagt gaggaaggag gacgaaggcc tctacacctg ccaggcatgc 2220
   agtgttcttg gctgtgcaaa agtggaggca tttttcataa tagaaggtgc ccaggaaaag 2280
   acgaacttgg aaatcattat tctagtaggc acggcggtga ttgccatgtt cttctggcta 2340
   cttcttgtca tcatcctacg gaccgttaag cgggccaatg gaggggaact gaagacaggc 2400
   tacttgtcca tcgtcatgga tccagatgaa ctcccattgg atgaacattg tgaacgactg 2460
   ccttatgatg ccagcaaatg ggaattcccc agagaccggc tgaagctagg taagcctctt 2520
   ggccgtggtg cctttggcca agtgattgaa gcagatgcct ttggaattga caagacagca 2580
   acttgcagga cagtagcagt caaaatgttg aaagaaggag caacacacag tgagcatcga 2640
   gctctcatgt ctgaactcaa gatcctcatt catattggtc accatctcaa tgtggtcaac 2700
   cttctaggtg cctgtaccaa gccaggaggg ccactcatgg tgattgtgga attctgcaaa 2760
   tttggaaacc tgtccactta cctgaggagc aagagaaatg aatttgtccc ctacaagacc 2820
   aaaggggcac gattccgtca agggaaagac tacgttggag caatccctgt ggatctgaaa 2880
   cggcgcttgg acagcatcac cagtagccag agctcagcca gctctggatt tgtggaggag 2940
   aagtccctca gtgatgtaga agaagaggaa gctcctgaag atctgtataa ggacttcctg 3000
   accttggagc atctcatctg ttacagcttc caagtggcta agggcatgga gttcttggca 3060
   tcgcgaaagt gtatccacag ggacctggcg gcacgaaata tcctcttatc ggagaagaac 3120
   gtggttaaaa tctgtgactt tggcttggcc cgggatattt ataaagatcc agattatgtc 3180
   agaaaaggag atgctcgcct ccctttgaaa tggatggccc cagaaacaat tttgacagaa 3240
   gtgtacacaa tccagagtga cgtctggtct tttggtgttt tgctgtggga aatattttcc 3300
   ttaggtgctt ctccatatcc tggggtaaag attgatgaag aattttgtag gcgattgaaa 3360
   gaaggaacta gaatgagggc ccctgattat actacaccag aaatgtacca gaccatgctg 3420
   gactgctggc acggggagcc cagtcagaga cccacgtttt cagagttggt ggaacatttg 3480
   ggaaatctct tgcaagctaa tgctcagcag gatggcaaag actacattgt tcttccgata 3540
   tcagagactt tgagcatgga agaggattct ggactctctc tgcctacctc acctgtttcc 3600
   tgtatggagg aggaggaagt atgtgacccc aaattccatt atgacaacac agcaggaatc 3660
   agtcagtatc tgcagaacag taagcgaaag agccggcctg tgagtgtaaa aacatttgaa 3720
   gatatcccgt tagaagaacc agaagtaaaa gtaatcccag atgacaacca gacggacagt 3780
   ggtatggttc ttgcctcaga agagctgaaa actttggaag acagaaccaa attatctcca 3840
   tcttttggtg gaatggtgcc cagcaaaagc agggagtctg tggcatctga aggctcaaac 3900
   cagacaagcg gctaccagtc cggatatcac tccgatgaca cagacaccac cgtgtactcc 3960
   agtgaggaag cagaactttt aaagctgata gagattggag tgcaaaccgg tagcacagcc 4020
   cagattctcc agcctgactc gggg                                        4044
<210> 95
   <222> 4017
   <212> DNA
   <213> Homo sapiens
<300>
   <302> Flt1
   <310> AF063657
<400> 95
<210> 96
   <211> 3897
   <212> DNA
   <213> Homo sapiens
<300>
   <302> Flt4
   <310> XM003852
<400> 96
   atgcagcggg gcgccgcgct gtgcctgcga ctgtggctct gcctgggact cctggacggc 60
   ctggtgagtg gctactccat gacccccccg accttgaaca tcacggagga gtcacacgtc 120
   atcgacaccg gtgacagcct gtccatctcc tgcaggggac agcaccccct cgagtgggct 180
   tggccaggag ctcaggaggc gccagccacc ggagacaagg acagcgagga cacgggggtg 240
   gtgcgagact gcgagggcac agacgccagg ccctactgca aggtgttgct gctgcacgag 300
   gtacatgcca acgacacagg cagctacgtc tgctactaca agtacatcaa ggcacgcatc 360
   gagggcacca cggccgccag ctcctacgtg ttcgtgagag actttgagca gccattcatc 420
   aacaagcctg acacgctctt ggtcaacagg aaggacgcca tgtgggtgcc ctgtctggtg 480
   tccatccccg gcctcaatgt cacgctgcgc tcgcaaagct cggtgctgtg gccagacggg 540
   caggaggtgg tgtgggatga ccggcggggc atgctcgtgt ccacgccact gctgcacgat 600
   gccctgtacc tgcagtgcga gaccacctgg ggagaccagg acttcctttc caaccccttc 660
   ctggtgcaca tcacaggcaa cgagctctat gacatccagc tgttgcccag gaagtcgctg 720
   gagctgctgg taggggagaa gctggtcctg aactgcaccg tgtgggctga gtttaactca 780
   ggtgtcacct ttgactggga ctacccaggg aagcaggcag agcggggtaa gtgggtgccc 840
   gagcgacgct cccagcagac ccacacagaa ctctccagca tcctgaccat ccacaacgtc 900
   agccagcacg acctgggctc gtatgtgtgc aaggccaaca acggcatcca gcgatttcgg 960
   gagagcaccg aggtcattgt gcatgaaaat cccttcatca gcgtcgagtg gctcaaagga 1020
   cccatcctgg aggccacggc aggagacgag ctggtgaagc tgcccgtgaa gctggcagcg 1080
   taccccccgc ccgagttcca gtggtacaag gatggaaagg cactgtccgg gcgccacagt 1140
   ccacatgccc tggtgctcaa ggaggtgaca gaggccagca caggcaccta caccctcgcc 1200
   ctgtggaact ccgctgctgg cctgaggcgc aacatcagcc tggagctggt ggtgaatgtg 1260
   cccccccaga tacatgagaa ggaggcctcc tcccccagca tctactcgcg tcacagccgc 1320
   caggccctca cctgcacggc ctacggggtg cccctgcctc tcagcatcca gtggcactgg 1380
   cggccctgga caccctgcaa gatgtttgcc cagcgtagtc tccggcggcg gcagcagcaa 1440
   gacctcatgc cacagtgccg tgactggagg gcggtgaccg cgcaggatgc cgtgaacccc 1500
   atcgagagcc tggacacctg gaccgagttt gtggagggaa agaataagac tgtgagcaag 1560
   ctggtgatcc agaatgccaa cgtgtctgcc atgtacaagt gtgtggtctc caacaaggtg 1620
   ggccaggatg agcggctcat ctacttctat gtgaccacca tccccgacgg cttcaccatc 1680
   gaatccaagc catccgagga gctactagag ggccagccgg tgctcctgag ctgccaagcc 1740
   gacagctaca agtacgagca tctgcgctgg taccgcctca acctgtccac gctgcacgat 1800
   gcgcacggga acccgcttct gctcgactgc aagaacgtgc atctgttcgc cacccctctg 1860
   gccgccagcc tggaggaggt ggcacctggg gcgcgccacg ccacgctcag cctgagtatc 1920
   ccccgcgtcg cgcccgagca cgagggccac tatgtgtgcg aagtgcaaga ccggcgcagc 1980
   catgacaagc actgccacaa gaagtacctg tcggtgcagg ccctggaagc ccctcggctc 2040
   acgcagaact tgaccgacct cctggtgaac gtgagcgact cgctggagat gcagtgcttg 2100
   gtggccggag cgcacgcgcc cagcatcgtg tggtacaaag acgagaggct gctggaggaa 2160
   aagtctggag tcgacttggc ggactccaac cagaagctga gcatccagcg cgtgcgcgag 2220
   gaggatgcgg gacgctatct gtgcagcgtg tgcaacgcca agggctgcgt caactcctcc 2280
   gccagcgtgg ccgtggaagg ctccgaggat aagggcagca tggagatcgt gatccttgtc 2340
   ggtaccggcg tcatcgctgt cttcttctgg gtcctcctcc tcctcatctt ctgtaacatg 2400
   aggaggccgg cccacgcaga catcaagacg ggctacctgt ccatcatcat ggaccccggg 2460
   gaggtgcctc tggaggagca atgcgaatac ctgtcctacg atgccagcca gtgggaattc 2520
   ccccgagagc ggctgcacct ggggagagtg ctcggctacg gcgccttcgg gaaggtggtg 2580
   gaagcctccg ctttcggcat ccacaagggc agcagctgtg acaccgtggc cgtgaaaatg 2640
   ctgaaagagg gcgccacggc cagcgagcag cgcgcgctga tgtcggagct caagatcctc 2700
   attcacatcg gcaaccacct caacgtggtc aacctcctcg gggcgtgcac caagccgcag 2760
   ggccccctca tggtgatcgt ggagttctgc aagtacggca acctctccaa cttcctgcgc 2820
   gccaagcggg acgccttcag cccctgcgcg gagaagtctc ccgagcagcg cggacgcttc 2880
   cgcgccatgg tggagctcgc caggctggat cggaggcggc cggggagcag cgacagggtc 2940
   ctcttcgcgc ggttctcgaa gaccgagggc ggagcgaggc gggcttctcc agaccaagaa 3000
   gctgaggacc tgtggctgag cccgctgacc atggaagatc ttgtctgcta cagcttccag 3060
   gtggccagag ggatggagtt cctggcttcc cgaaagtgca tccacagaga cctggctgct 3120
   cggaacattc tgctgtcgga aagcgacgtg gtgaagatct gtgactttgg ccttgcccgg 3180
   gacatctaca aagaccccga ctacgtccgc aagggcagtg cccggctgcc cctgaagtgg 3240
   atggcccctg aaagcatctt cgacaaggtg tacaccacgc agagtgacgt gtggtccttt 3300
   ggggtgcttc tctgggagat cttctctctg gggcctcccc cgtaccctgg ggtgcagatc 3360
   aatgaggagt tctgccagcg gctgagagac ggcacaagga tgagggcccc ggagctggcc 3420
   actcccgcca tacgccgcat catgctgaac tgctggtccg gagaccccaa ggcgagacct 3480
   gcattctcgg agctggtgga gatcctgggg gacctgctcc agggcagggg cctgcaagag 3540
   gaagaggagg tctgcatggc cccgcgcagc tctcagagct cagaagaggg cagcttctcg 3600
   caggtgtcca ccatggccct acacatcgcc caggctgacg ctgaggacag cccgccaagc 3660
   ctgcagcgcc acagcctggc cgccaggtat tacaactggg tgtcctttcc cgggtgcctg 3720
   gccagagggg ctgagacccg tggttcctcc aggatgaaga catttgagga attccccatg 3780
   accccaacga cctacaaagg ctctgtggac aaccagacag acagtgggat ggtgctggcc 3840
   tcggaggagt ttgagcagat agagagcagg catagacaag aaagcggctt caggtag 3897
<210> 97
   <211> 4071
   <212> DNA
<213> Homo sapiens
<300>
   <302> KDR
   <310> AF063658
<400> 97
   atggagagca aggtgctgct ggccgtcgcc ctgtggctct gcgtggagac ccgggccgcc 60
   tctgtgggtt tgcctagtgt ttctcttgat ctgcccaggc tcagcataca aaaagacata 120
   cttacaatta aggctaatac aactcttcaa attacttgca ggggacagag ggacttggac 180
   tggctttggc ccaataatca gagtggcagt gagcaaaggg tggaggtgac tgagtgcagc 240
   gatggcctct tctgtaagac actcacaatt ccaaaagtga tcggaaatga cactggagcc 300
   tacaagtgct tctaccggga aactgacttg gcctcggtca tttatgtcta tgttcaagat 360
   tacagatctc catttattgc ttctgttagt gaccaacatg gagtcgtgta cattactgag 420
   aacaaaaaca aaactgtggt gattccatgt ctcgggtcca tttcaaatct caacgtgtca 480
   ctttgtgcaa gatacccaga aaagagattt gttcctgatg gtaacagaat ttcctgggac 540
   agcaagaagg gctttactat tcccagctac atgatcagct atgctggcat ggtcttctgt 600
   gaagcaaaaa ttaatgatga aagttaccag tctattatgt acatagttgt cgttgtaggg 660
   tataggattt atgatgtggt tctgagtccg tctcatggaa ttgaactatc tgttggagaa 720
   aagcttgtct taaattgtac agcaagaact gaactaaatg tggggattga cttcaactgg 780
   gaataccctt cttcgaagca tcagcataag aaacttgtaa accgagacct aaaaacccag 840
   tctgggagtg agatgaagaa atttttgagc accttaacta tagatggtgt aacccggagt 900
   gaccaaggat tgtacacctg tgcagcatcc agtgggctga tgaccaagaa gaacagcaca 960
   tttgtcaggg tccatgaaaa accttttgtt gcttttggaa gtggcatgga atctctggtg 1020
   gaagccacgg tgggggagcg tgtcagaatc cctgcgaagt accttggtta cccaccccca 1080
   gaaataaaat ggtataaaaa tggaataccc cttgagtcca atcacacaat taaagcgggg 1140
   catgtactga cgattatgga agtgagtgaa agagacacag gaaattacac tgtcatcctt 1200
   accaatccca tttcaaagga gaagcagagc catgtggtct ctctggttgt gtatgtccca 1260
   ccccagattg gtgagaaatc tctaatctct cctgtggatt cctaccagta cggcaccact 1320
   caaacgctga catgtacggt ctatgccatt cctcccccgc atcacatcca ctggtattgg 1380
   cagttggagg aagagtgcgc caacgagccc agccaagctg tctcagtgac aaacccatac 1440
   ccttgtgaag aatggagaag tgtggaggac ttccagggag gaaataaaat tgaagttaat 1500
   aaaaatcaat ttgctctaat tgaaggaaaa aacaaaactg taagtaccct tgttatccaa 1560
   gcggcaaatg tgtcagcttt gtacaaatgt gaagcggtca acaaagtcgg gagaggagag 1620
   agggtgatct ccttccacgt gaccaggggt cctgaaatta ctttgcaacc tgacatgcag 1680
   cccactgagc aggagagcgt gtctttgtgg tgcactgcag acagatctac gtttgagaac 1740
   ctcacatggt acaagcttgg cccacagcct ctgccaatcc atgtgggaga gttgcccaca 1800
   cctgtttgca agaacttgga tactctttgg aaattgaatg ccaccatgtt ctctaatagc 1860
   acaaatgaca ttttgatcat ggagcttaag aatgcatcct tgcaggacca aggagactat 1920
   gtctgccttg ctcaagacag gaagaccaag aaaagacatt gcgtggtcag gcagctcaca 1980
   gtcctagagc gtgtggcacc cacgatcaca ggaaacctgg agaatcagac gacaagtatt 2040
   ggggaaagca tcgaagtctc atgcacggca tctgggaatc cccctccaca gatcatgtgg 2100
   tttaaagata atgagaccct tgtagaagac tcaggcattg tattgaagga tgggaaccgg 2160
   aacctcacta tccgcagagt gaggaaggag gacgaaggcc tctacacctg ccaggcatgc 2220
   agtgttcttg gctgtgcaaa agtggaggca tttttcataa tagaaggtgc ccaggaaaag 2280
   acgaacttgg aaatcattat tctagtaggc acggcggtga ttgccatgtt cttctggcta 2340
   cttcttgtca tcatcctacg gaccgttaag cgggccaatg gaggggaact gaagacaggc 2400
   tacttgtcca tcgtcatgga tccagatgaa ctcccattgg atgaacattg tgaacgactg 2460
   ccttatgatg ccagcaaatg ggaattcccc agagaccggc tgaagctagg taagcctctt 2520
   ggccgtggtg cctttggcca agtgattgaa gcagatgcct ttggaattga caagacagca 2580
   acttgcagga cagtagcagt caaaatgttg aaagaaggag caacacacag tgagcatcga 2640
   gctctcatgt ctgaactcaa gatcctcatt catattggtc accatctcaa tgtggtcaac 2700
   cttctaggtg cctgtaccaa gccaggaggg ccactcatgg tgattgtgga attctgcaaa 2760
   tttggaaacc tgtccactta cctgaggagc aagagaaatg aatttgtccc ctacaagacc 2820
   aaaggggcac gattccgtca agggaaagac tacgttggag caatccctgt ggatctgaaa 2880
   cggcgcttgg acagcatcac cagtagccag agctcagcca gctctggatt tgtggaggag 2940
   aagtccctca gtgatgtaga agaagaggaa gctcctgaag atctgtataa ggacttcctg 3000
   accttggagc atctcatctg ttacagcttc caagtggcta agggcatgga gttcttggca 3060
   tcgcgaaagt gtatccacag ggacctggcg gcacgaaata tcctcttatc ggagaagaac 3120
   gtggttaaaa tctgtgactt tggcttggcc cgggatattt ataaagatcc agattatgtc 3180
   agaaaaggag atgctcgcct ccctttgaaa tggatggccc cagaaacaat ttttgacaga 3240
   gtgtacacaa tccagagtga cgtctggtct tttggtgttt tgctgtggga aatattttcc 3300
   ttaggtgctt ctccatatcc tggggtaaag attgatgaag aattttgtag gcgattgaaa 3360
   gaaggaacta gaatgagggc ccctgattat actacaccag aaatgtacca gaccatgctg 3420
   gactgctggc acggggagcc cagtcagaga cccacgtttt cagagttggt ggaacatttg 3480
   ggaaatctct tgcaagctaa tgctcagcag gatggcaaag actacattgt tcttccgata 3540
   tcagagactt tgagcatgga agaggattct ggactctctc tgcctacctc acctgtttcc 3600
   tgtatggagg aggaggaagt atgtgacccc aaattccatt atgacaacac agcaggaatc 3660
   agtcagtatc tgcagaacag taagcgaaag agccggcctg tgagtgtaaa aacatttgaa 3720
   gatatcccgt tagaagaacc agaagtaaaa gtaatcccag atgacaacca gacggacagt 3780
   ggtatggttc ttgcctcaga agagctgaaa actttggaag acagaaccaa attatctcca 3840
   tcttttggtg gaatggtgcc cagcaaaagc agggagtctg tggcatctga aggctcaaac 3900
   cagacaagcg gctaccagtc cggatatcac tccgatgaca cagacaccac cgtgtactcc 3960
   agtgaggaag cagaactttt aaagctgata gagattggag tgcaaaccgg tagcacagcc 4020
   cagattctcc agcctgactc ggggaccaca ctgagctctc ctcctgttta a          4071
<210> 98
   <211> 1410
   <212> DNA
   <213> Homo sapiens
<300>
   <302> MMP1
   <310> M13509
<400> 98
   atgcacagct ttcctccact gctgctgctg ctgttctggg gtgtggtgtc tcacagcttc   60
   ccagcgactc tagaaacaca agagcaagat gtggacttag tccagaaata cctggaaaaa  120
   tactacaacc tgaagaatga tgggaggcaa gttgaaaagc ggagaaatag tggcccagtg  180
   gttgaaaaat tgaagcaaat gcaggaattc tttgggctga aagtgactgg gaaaccagat  240
   gctgaaaccc tgaaggtgat gaagcagccc agatgtggag tgcctgatgt ggctcagttt  300
   gtcctcactg agggaaaccc tcgctgggag caaacacatc tgaggtacag gattgaaaat  360
   tacacgccag atttgccaag agcagatgtg gaccatgcca ttgagaaagc cttccaactc  420
   tggagtaatg tcacacctct gacattcacc aaggtctctg agggtcaagc agacatcatg  480
   atatcttttg tcaggggaga tcatcgggac aactctcctt ttgatggacc tggaggaaat  540
   cttgctcatg cttttcaacc aggcccaggt attggagggg atgctcattt tgatgaagat  600
   gaaaggtgga ccaacaattt cagagagtac aacttacatc gtgttgcggc tcatgaactc  660
   ggccattctc ttggactctc ccattctact gatatcgggg ctttgatgta ccctagctac  720
   accttcagtg gtgatgttca gctagctcag gatgacattg atggcatcca agccatatat  780
   ggacgttccc aaaatcctgt ccagcccatc ggcccacaaa ccccaaaagc gtgtgacagt  840
   aagctaacct ttgatgctat aactacgatt cggggagaag tgatgttctt taaagacaga  900
   ttctacatgc gcacaaatcc cttctacccg gaagttgagc tcaatttcat ttctgttttc  960
   tggccacaac tgccaaatgg gcttgaagct gcttacgaat ttgccgacag agatgaagtc 1020
   cggtttttca aagggaataa gtactgggct gttcagggac agaatgtgct acacggatac 1080
   cccaaggaca tctacagctc ctttggcttc cctagaactg tgaagcatat cgatgctgct 1140
   ctttctgagg aaaacactgg aaaaacctac ttctttgttg ctaacaaata ctggaggtat 1200
   gatgaatata aacgatctat ggatccaagt tatcccaaaa tgatagcaca tgactttcct 1260
   ggaattggcc acaaagttga tgcagttttc atgaaagatg gatttttcta tttctttcat 1320
   ggaacaagac aatacaaatt tgatcctaaa acgaagagaa ttttgactct ccagaaagct 1380
   aatagctggt tcaactgcag gaaaaattga                                  1410
<210> 99
   <211> 1743
   <212> DNA
   <213> Homo sapiens
<300>
   <302> MMP10
   <310> XM006269
<400> 99
   aaagaaggta agggcagtga gaatgatgca tcttgcattc cttgtgctgt tgtgtctgcc 60
   agtctgctct gcctatcctc tgagtggggc agcaaaagag gaggactcca acaaggatct 120
   tgcccagcaa tacctagaaa agtactacaa cctcgaaaag gatgtgaaac agtttagaag 180
   aaaggacagt aatctcattg ttaaaaaaat ccaaggaatg cagaagttcc ttgggttgga 240
   ggtgacaggg aagctagaca ctgacactct ggaggtgatg cgcaagccca ggtgtggagt 300
   tcctgacgtt ggtcacttca gctcctttcc tggcatgccg aagtggagga aaacccacct 360
   tacatacagg attgtgaatt atacaccaga tttgccaaga gatgctgttg attctgccat 420
   tgagaaagct ctgaaagtct gggaagaggt gactccactc acattctcca ggctgtatga 480
   aggagaggct gatataatga tctcttttgc agttaaagaa catggagact tttactcttt 540
   tgatggccca ggacacagtt tggctcatgc ctacccacct ggacctgggc tttatggaga 600
   tattcacttt gatgatgatg aaaaatggac agaagatgca tcaggcacca atttattcct 660
   cgttgctgct catgaacttg gccactccct ggggctcttt cactcagcca acactgaagc 720
   tttgatgtac ccactctaca actcattcac agagctcgcc cagttccgcc tttcgcaaga 780
   tgatgtgaat ggcattcagt ctctctacgg acctccccct gcctctactg aggaacccct 840
   ggtgcccaca aaatctgttc cttcgggatc tgagatgcca gccaagtgtg atcctgcttt 900
   gtccttcgat gccatcagca ctctgagggg agaatatctg ttctttaaag acagatattt 960
   ttggcgaaga tcccactgga accctgaacc tgaatttcat ttgatttctg cattttggcc 1020
   ctctcttcca tcatatttgg atgctgcata tgaagttaac agcagggaca ccgtttttat 1080
   ttttaaagga aatgagttct gggccatcag aggaaatgag gtacaagcag gttatccaag 1140
   aggcatccat accctgggtt ttcctccaac cataaggaaa attgatgcag ctgtttctga 1200
   caaggaaaag aagaaaacat acttctttgc agcggacaaa tactggagat ttgatgaaaa 1260
   tagccagtcc atggagcaag gcttccctag actaatagct gatgactttc caggagttga 1320
   gcctaaggtt gatgctgtat tacaggcatt tggatttttc tacttcttca gtggatcatc 1380
   acagtttgag tttgacccca atgccaggat ggtgacacac atattaaaga gtaacagctg 1440
   gttacattgc taggcgagat agggggaaga cagatatggg tgtttttaat aaatctaata 1500
   attattcatc taatgtatta tgagccaaaa tggttaattt ttcctgcatg ttctgtgact 1560
   gaagaagatg agccttgcag atatctgcat gtgtcatgaa gaatgtttct ggaattcttc 1620
   acttgctttt gaattgcact gaacagaatt aagaaatact catgtgcaat aggtgagaga 1680
   atgtattttc atagatgtgt tattacttcc tcaataaaaa gttttatttt gggcctgttc 1740
   ctt                                                               1743
<210> 100
   <211> 1467
   <212> DNA
   <213> Homo sapiens
<300>
   <302> MMP11
   <310> XM009873
<400> 100
   atggctccgg ccgcctggct ccgcagcgcg gccgcgcgcg ccctcctgcc cccgatgctg 60
   ctgctgctgc tccagccgcc gccgctgctg gcccgggctc tgccgccgga cgcccaccac 120
   ctccatgccg agaggagggg gccacagccc tggcatgcag ccctgcccag tagcccggca 180
   cctgcccctg ccacgcagga agccccccgg cctgccagca gcctcaggcc tccccgctgt 240
   ggcgtgcccg acccatctga tgggctgagt gcccgcaacc gacagaagag gttcgtgctt 300
   tctggcgggc gctgggagaa gacggacctc acctacagga tccttcggtt cccatggcag 360
   ttggtgcagg agcaggtgcg gcagacgatg gcagaggccc taaaggtatg gagcgatgtg 420
   acgccactca cctttactga ggtgcacgag ggccgtgctg acatcatgat cgacttcgcc 480
   aggtactggc atggggacga cctgccgttt gatgggcctg ggggcatcct ggcccatgcc 540
   ttcttcccca agactcaccg agaaggggat gtccacttcg actatgatga gacctggact 600
   atcggggatg accagggcac agacctgctg caggtggcag cccatgaatt tggccacgtg 660
   ctggggctgc agcacacaac agcagccaag gccctgatgt ccgccttcta cacctttcgc 720
   tacccactga gtctcagccc agatgactgc aggggcgttc aacacctata tggccagccc 780
   tggcccactg tcacctccag gaccccagcc ctgggccccc aggctgggat agacaccaat 840
   gagattgcac cgctggagcc agacgccccg ccagatgcct gtgaggcctc ctttgacgcg 900
   gtctccacca tccgaggcga gctctttttc ttcaaagcgg gctttgtgtg gcgcctccgt 960
   gggggccagc tgcagcccgg ctacccagca ttggcctctc gccactggca gggactgccc 1020
   agccctgtgg acgctgcctt cgaggatgcc cagggccaca tttggttctt ccaaggtgct 1080
   cagtactggg tgtacgacgg tgaaaagcca gtcctgggcc ccgcacccct caccgagctg 1140
   ggcctggtga ggttcccggt ccatgctgcc ttggtctggg gtcccgagaa gaacaagatc 1200
   tacttcttcc gaggcaggga ctactggcgt ttccacccca gcacccggcg tgtagacagt 1260
   cccgtgcccc gcagggccac tgactggaga ggggtgccct ctgagatcga cgctgccttc 1320
   caggatgctg atggctatgc ctacttcctg cgcggccgcc tctactggaa gtttgaccct 1380
   gtgaaggtga aggctctgga aggcttcccc cgtctcgtgg gtcctgactt ctttggctgt 1440
   gccgagcctg ccaacacttt cctctga                                    1467
<210> 101
   <211> 1653
   <212> DNA
   <213> Homo sapiens
<300>
   <302> MMP12
   <310> XM006272
<400> 101
   atgaagtttc ttctaatact gctcctgcag gccactgctt ctggagctct tcccctgaac 60
   agctctacaa gcctggaaaa aaataatgtg ctatttggtg agagatactt agaaaaattt 120
   tatggccttg agataaacaa acttccagtg acaaaaatga aatatagtgg aaacttaatg 180
   aaggaaaaaa tccaagaaat gcagcacttc ttgggtctga aagtgaccgg gcaactggac 240
   acatctaccc tggagatgat gcacgcacct cgatgtggag tccccgatgt ccatcatttc 300
   agggaaatgc caggggggcc cgtatggagg aaacattata tcacctacag aatcaataat 360
   tacacacctg acatgaaccg tgaggatgtt gactacgcaa tccggaaagc tttccaagta 420
   tggagtaatg ttaccccctt gaaattcagc aagattaaca caggcatggc tgacattttg 480
   gtggtttttg cccgtggagc tcatggagac ttccatgctt ttgatggcaa aggtggaatc 540
   ctagcccatg cttttggacc tggatctggc attggagggg atgcacattt cgatgaggac 600
   gaattctgga ctacacattc aggagnnnnn nnnnnnnnnn nnnnnnnnnn nnnnnnnnnn 660
   nnnnnnnnnn nnnnnnnnnn nnnnnnnnnn nnnnnnnnnn nnnnnnnnnn nnnnnnnnnn 720
   nnnnnnnnnn nnnnnnnnnn nnnnnnnnnn nnnnnnnnnn nnnnnnnnnn nnnnnnnnnn 780
   nnnnnnnnnn nnnnnnnnnn nnnnnnnnnn nnnnnnnnnn nnnnnnnnnn nnnnnnnnnn 840
   nnnnnnnnnn nnnnnnnnnn nnnnnnnnnn nnnnnnnnnn nnnnnnnnnn nnnnnnnnnn 900
   nnnnnnnnnn nnnnnnnnnn nnnnngagag gatccaaagg ccgtaatgtt ccccacctac 960
   aaatatgttg acatcaacac atttcgcctc tctgctgatg acatacgtgg cattcagtcc 1020
   ctgtatggag acccaaaaga gaaccaacgc ttgccaaatc ctgacaattc agraccagct 1080
   ctctgtgacc ccaatttgag ttttgatgct gtcactaccg tgggaaataa gatctttttc 1140
   ttcaaagaca ggttcttctg gctgaaggtt tctgagagac caaagaccag tgttaattta 1200
   atttcttcct tatggccaac cttgccatct ggcattgaag ctgcttatga aattgaagcc 1260
   agaaatcaag tttttctttt taaagatgac aaatactggt taattagcaa tttaagacca 1320
   gagccaaatt atcccaagag catacattct tttggttttc ctaactttgt gaaaaaaatt 1380
   gatgcagctg tttttaaccc acgtttttat aggacctact tctttgtaga taaccagtat 1440
   tggaggtatg atgaaaggag acagatgatg gaccctggtt atcccaaact gattaccaag 1500
   aacttccaag gaatcgggcc taaaattgat gcagtcttct actctaaaaa caaatactac 1560
   tatttcttcc aaggatctaa ccaatttgaa tatgacttcc tactccaacg tatcaccaaa 1620
   acactgaaaa gcaatagctg gtttggttgt tag 1653
<210> 102
   <211> 1416
   <212> DNA
   <213> Homo sapiens
<400> 102
   atgcatccag gggtcctggc tgccttcctc ttcttgagct ggactcattg tcgggccctg 60
   ccccttccca gtggtggtga tgaagatgat ttgtctgagg aagacctcca gtttgcagag 120
   cgctacctga gatcatacta ccatcctaca aatctcgcgg gaatcctgaa ggagaatgca 180
   gcaagctcca tgactgagag gctccgagaa atgcagtctt tcttcggctt agaggtgact 240
   ggcaaacttg acgataacac cttagatgtc atgaaaaagc caagatgcgg ggttcctgat 300
   gtgggtgaat acaatgtttt ccctcgaact cttaaatggt ccaaaatgaa tttaacctac 360
   agaattgtga attacacccc tgatatgact cattctgaag tcgaaaaggc attcaaaaaa 420
   gccttcaaag tttggtccga tgtaactcct ctgaatttta ccagacttca cgatggcatt 480
   gctgacatca tgatctcttt tggaattaag gagcatggcg acttctaccc atttgatggg 540
   ccctctggcc tgctggctca tgcttttcct cctgggccaa attatggagg agatgcccat 600
   tttgatgatg atgaaacctg gacaagtagt tccaaaggct acaacttgtt tcttgttgct 660
   gcgcatgagt tcggccactc cttaggtctt gaccactcca aggaccctgg agcactcatg 720
   tttcctatct acacctacac cggcaaaagc cactttatgc ttcctgatga cgatgtacaa 780
   gggatccagt ctctctatgg tccaggagat gaagacccca accctaaaca tccaaaaacg 840
   ccagacaaat gtgacccttc cttatccctt gatgccatta ccagtctccg aggagaaaca 900
   atgatcttta aagacagatt cttctggcgc ctgcatcctc agcaggttga tgcggagctg 960
   tttttaacga aatcattttg gccagaactt cccaaccgta ttgatgctgc atatgagcac 1020
   ccttctcatg acctcatctt catcttcaga ggtagaaaat tttgggctct taatggttat 1080
   gacattctgg aaggttatcc caaaaaaata tctgaactgg gtcttccaaa agaagttaag 1140
   aagataagtg cagctgttca ctttgaggat acaggcaaga ctctcctgtt ctcaggaaac 1200
   caggtctgga gatatgatga tactaaccat attatggata aagactatcc gagactaata 1260
   gaagaagact tcccaggaat tggtgataaa gtagatgctg tctatgagaa aaatggttat 1320
   atctattttt tcaacggacc catacagttt gaatacagca tctggagtaa ccgtattgtt 1380
   cgcgtcatgc cagcaaattc cattttgtgg tgttaa                           1416
<210> 103
   <211> 1749
   <212> DNA
   <213> Homo sapiens
<300>
   <302> MMP14
   <310> NM004995
<400> 103
   atgtctcccg ccccaagacc cccccgttgt ctcctgctcc ccctgctcac gctcggcacc 60
   gcgctcgcct ccctcggctc ggcccaaagc agcagcttca gccccgaagc ctggctacag 120
   caatatggct acctgcctcc cggggaccta cgtacccaca cacagcgctc accccagtca 180
   ctctcagcgg ccatcgctgc catgcagaag ttttacggct tgcaagtaac aggcaaagct 240
   gatgcagaca ccatgaaggc catgaggcgc ccccgatgtg gtgttccaga caagtttggg 300
   gctgagatca aggccaatgt tcgaaggaag cgctacgcca tccagggtct caaatggcaa 360
   cataatgaaa tcactttctg catccagaat tacaccccca aggtgggcga gtatgccaca 420
   tacgaggcca ttcgcaaggc gttccgcgtg tgggagagtg ccacaccact gcgcttccgc 480
   gaggtgccct atgcctacat ccgtgagggc catgagaagc aggccgacat catgatcttc 540
   tttgccgagg gcttccatgg cgacagcacg cccttcgatg gtgagggcgg cttcctggcc 600
   catgcctact tcccaggccc caacattgga ggagacaccc actttgactc tgccgagcct 660
   tggactgtca ggaatgagga tctgaatgga aatgacatct tcctggtggc tgtgcacgag 720
   ctgggccatg ccctggggct cgagcattcc agtgacccct cggccatcat ggcacccttt 780
   taccagtgga tggacacgga gaattttgtg ctgcccgatg atgaccgccg gggcatccag 840
   caactttatg ggggtgagtc agggttcccc accaagatgc cccctcaacc caggactacc 900
   tcccggcctt ctgttcctga taaacccaaa aaccccacct atgggcccaa catctgtgac 960
   gggaactttg acaccgtggc catgctccga ggggagatgt ttgtcttcaa ggagcgctgg 1020
   ttctggcggg tgaggaataa ccaagtgatg gatggatacc caatgcccat tggccagttc 1080
   tggcggggcc tgcctgcgtc catcaacact gcctacgaga ggaaggatgg caaattcgtc 1140
   ttcttcaaag gagacaagca ttgggtgttt gatgaggcgt ccctggaacc tggctacccc 1200
   aagcacatta aggagctggg ccgagggctg cctaccgaca agattgatgc tgctctcttc 1260
   tggatgccca atggaaagac ctacttcttc cgtggaaaca agtactaccg tttcaacgaa 1320
   gagctcaggg cagtggatag cgagtacccc aagaacatca aagtctggga agggatccct 1380
   gagtctccca gagggtcatt catgggcagc gatgaagtct tcacttactt ctacaagggg 1440
   aacaaatact ggaaattcaa caaccagaag ctgaaggtag aaccgggcta ccccaagtca 1500
   gccctgaggg actggatggg ctgcccatcg ggaggccggc cggatgaggg gactgaggag 1560
   gagacggagg tgatcatcat tgaggtggac gaggagggcg gcggggcggt gagcgcggct 1620
   gccgtggtgc tgcccgtgct gctgctgctc ctggtgctgg cggtgggcct tgcagtcttc 1680
   ttcttcagac gccatgggac ccccaggcga ctgctctact gccagcgttc cctgctggac 1740
   aaggtctga                                                         1749
<210> 104
   <211> 2010
   <212> DNA
   <213> Homo sapiens
<300>
   <302> MMP15
   <310> NM002428
<400> 104
   atgggcagcg acccgagcgc gcccggacgg ccgggctgga cgggcagcct cctcggcgac 60
   cgggaggagg cggcgcggcc gcgactgctg ccgctgctcc tggtgcttct gggctgcctg 120
   ggccttggcg tagcggccga agacgcggag gtccatgccg agaactggct gcggctttat 180
   ggctacctgc ctcagcccag ccgccatatg tccaccatgc gttccgccca gatcttggcc 240
   tcggcccttg cagagatgca gcgcttctac gggatcccag tcaccggtgt gctcgacgaa 300
   gagaccaagg agtggatgaa gcggccccgc tgtggggtgc cagaccagtt cggggtacga 360
   gtgaaagcca acctgcggcg gcgtcggaag cgctacgccc tcaccgggag gaagtggaac 420
   aaccaccatc tgacctttag catccagaac tacacggaga agttgggctg gtaccactcg 480
   atggaggcgg tgcgcagggc cttccgcgtg tgggagcagg ccacgcccct ggtcttccag 540
   gaggtgccct atgaggacat ccggctgcgg cgacagaagg aggccgacat catggtactc 600
   tttgcctctg gcttccacgg cgacagctcg ccgtttgatg gcaccggtgg cttcctggcc 660
   cacgcctatt tccctggccc cggcctaggc ggggacaccc attttgacgc agatgagccc 720
   tggaccttct ccagcactga cctgcatgga aacaacctct tcctggtggc agtgcatgag 780
   ctgggccacg cgctggggct ggagcactcc agcaacccca atgccatcat ggcgccgttc 840
   taccagtgga aggacgttga caacttcaag ctgcccgagg acgatctccg tggcatccag 900
   cagctctacg gtaccccaga cggtcagcca cagcctaccc agcctctccc cactgtgacg 960
   ccacggcggc caggccggcc tgaccaccgg ccgccccggc ctccccagcc accaccccca 1020
   ggtgggaagc cagagcggcc cccaaagccg ggccccccag tccagccccg agccacagag 1080
   cggcccgacc agtatggccc caacatctgc gacggggact ttgacacagt ggccatgctt 1140
   cgcggggaga tgttcgtgtt caaggcccgc tggttctggc gagtccggca caaccgcgtc 1200
   ctggacaact atcccatgcc catcgggcac ttctggcgtg gtctgcccgg tgacatcagt 1260
   gctgcctacg agcgccaaga cggtcgtttt gtctttttca aaggtgaccg ctactggctc 1320
   tttcgagaag cgaacctgga gcccggctac ccacagccgc tgaccagcta tggcctgggc 1380
   atcccctatg accgcattga cacggccatc tggtgggagc ccacaggcca caccttcttc 1440
   ttccaagagg acaggtactg gcgcttcaac gaggagacac agcgtggaga ccctgggtac 1500
   cccaagccca tcagtgtctg gcaggggatc cctgcctccc ctaaaggggc cttcctgagc 1560
   aatgacgcag cctacaccta cttctacaag ggcaccaaat actggaaatt cgacaatgag 1620
   cgcctgcgga tggagcccgg ctaccccaag tccatcctgc gggacttcat gggctgccag 1680
   gagcacgtgg agccaggccc ccgatggccc gacgtggccc ggccgccctt caacccccac 1740
   gggggtgcag agcccggggc ggacagcgca gagggcgacg tgggggatgg ggatggggac 1800
   tttggggccg gggtcaacaa ggacgggggc agccgcgtgg tggtgcagat ggaggaggtg 1860
   gcacggacgg tgaacgtggt gatggtgctg gtgccactgc tgctgctgct ctgcgtcctg 1920
   ggcctcacct acgcgctggt gcagatgcag cgcaagggtg cgccacgtgt cctgctttac 1980
   tgcaagcgct cgctgcagga gtgggtctga                                 2010
<210> 105
   <211> 1824
   <212> DNA
   <213> Homo sapiens
<300>
   <302> MMP16
   <310> NM005941
<400> 105
   atgatcttac tcacattcag cactggaaga cggttggatt tcgtgcatca ttcgggggtg 60
   tttttcttgc aaaccttgct ttggatttta tgtgctacag tctgcggaac ggagcagtat 120
   ttcaatgtgg aggtttggtt acaaaagtac ggctaccttc caccgactga ccccagaatg 180
   tcagtgctgc gctctgcaga gaccatgcag tctgccctag ctgccatgca gcagttctat 240
   ggcattaaca tgacaggaaa agtggacaga aacacaattg actggatgaa gaagccccga 300
   tgcggtgtac ctgaccagac aagaggtagc tccaaatttc atattcgtcg aaagcgatat 360
   gcattgacag gacagaaatg gcagcacaag cacatcactt acagtataaa gaacgtaact 420
   ccaaaagtag gagaccctga gactcgtaaa gctattcgcc gtgcctttga tgtgtggcag 480
   aatgtaactc ctctgacatt tgaagaagtt ccctacagtg aattagaaaa tggcaaacgt 540
   gatgtggata taaccattat ttttgcatct ggtttccatg gggacagctc tccctttgat 600
   ggagagggag gatttttggc acatgcctac ttccctggac caggaattgg aggagatacc 660
   cattttgact cagatgagcc atggacacta ggaaatccta atcatgatgg aaatgactta 720
   tttcttgtag cagtccatga actgggacat gctctgggat tggagcattc caatgacccc 780
   actgccatca tggctccatt ttaccagtac atggaaacag acaacttcaa actacctaat 840
   gatgatttac agggcatcca gaaaatatat ggtccacctg acaagattcc tccacctaca 900
   agacctctac cgacagtgcc cccacaccgc tctattcctc cggctgaccc aaggaaaaat 960
   gacaggccaa aacctcctcg gcctccaacc ggcagaccct cctatcccgg agccaaaccc 1020
   aacatctgtg atgggaactt taacactcta gctattcttc gtcgtgagat gtttgttttc 1080
   aaggaccagt ggttttggcg agtgagaaac aacagggtga tggatggata cccaatgcaa 1140
   attacttact tctggcgggg cttgcctcct agtatcgatg cagtttatga aaatagcgac 1200
   gggaattttg tgttctttaa aggtaacaaa tattgggtgt tcaaggatac aactcttcaa 1260
   cctggttacc ctcatgactt gataaccctt ggaagtggaa ttccccctca tggtattgat 1320
   tcagccattt ggtgggagga cgtcgggaaa acctatttct tcaagggaga cagatattgg 1380
   agatatagtg aagaaatgaa aacaatggac cctggctatc ccaagccaat cacagtctgg 1440
   aaagggatcc ctgaatctcc tcagggagca tttgtacaca aagaaaatgg ctttacgtat 1500
   ttctacaaag gaaaggagta ttggaaattc aacaaccaga tactcaaggt agaacctgga 1560
   catccaagat ccatcctcaa ggattttatg ggctgtgatg gaccaacaga cagagttaaa 1620
   gaaggacaca gcccaccaga tgatgtagac attgtcatca aactggacaa cacagccagc 1680
   actgtgaaag ccatagctat tgtcattccc tgcatcttgg ccttatgcct ccttgtattg 1740
   gtttacactg tgttccagtt caagaggaaa ggaacacccc gccacatact gtactgtaaa 1800
   cgctctatgc aagagtgggt gtga                                        1824
<210> 106
   <211> 1550
   <212> DNA
   <213> Homo sapiens
<300>
   <302> MMP17
   <310> NM004141
<400> 106
<210> 107
   <211> 1983
   <212> DNA
   <213> Homo sapiens
<300>
   <302> MMP2
   <310> NM004530
<400> 107
   atggaggcgc taatggcccg gggcgcgctc acgggtcccc tgagggcgct ctgtctcctg 60
   ggctgcctgc tgagccacgc cgccgccgcg ccgtcgccca tcatcaagtt ccccggcgat 120
   gtcgccccca aaacggacaa agagttggca gtgcaatacc tgaacacctt ctatggctgc 180
   cccaaggaga gctgcaacct gtttgtgctg aaggacacac taaagaagat gcagaagttc 240
   tttggactgc cccagacagg tgatcttgac cagaatacca tcgagaccat gcggaagcca 300
   cgctgcggca acccagatgt ggccaactac aacttcttcc ctcgcaagcc caagtgggac 360
   aagaaccaga tcacatacag gatcattggc tacacacctg atctggaccc agagacagtg 420
   gatgatgcct ttgctcgtgc cttccaagtc tggagcgatg tgaccccact gcggttttct 480
   cgaatccatg atggagaggc agacatcatg atcaactttg gccgctggga gcatggcgat 540
   ggatacccct ttgacggtaa ggacggactc ctggctcatg ccttcgcccc aggcactggt 600
   gttgggggag actcccattt tgatgacgat gagctatgga ccttgggaga aggccaagtg 660
   gtccgtgtga agtatggcaa cgccgatggg gagtactgca agttcccctt cttgttcaat 720
   ggcaaggagt acaacagctg cactgatact ggccgcagcg atggcttcct ctggtgctcc 780
   accacctaca actttgagaa ggatggcaag tacggcttct gtccccatga agccctgttc 840
   accatgggcg gcaacgctga aggacagccc tgcaagtttc cattccgctt ccagggcaca 900
   tcctatgaca gctgcaccac tgagggccgc acggatggct accgctggtg cggcaccact 960
   gaggactacg accgcgacaa gaagtatggc ttctgccctg agaccgccat gtccactgtt 1020
   ggtgggaact cagaaggtgc cccctgtgtc ttccccttca ctttcctggg caacaaatat 1080
   gagagctgca ccagcgccgg ccgcagtgac ggaaagatgt ggtgtgcgac cacagccaac 1140
   tacgatgacg accgcaagtg gggcttctgc cctgaccaag ggtacagcct gttcctcgtg 1200
   gcagcccacg agtttggcca cgccatgggg ctggagcact cccaagaccc tggggccctg 1260
   atggcaccca tttacaccta caccaagaac ttccgtctgt cccaggatga catcaagggc 1320
   attcaggagc tctatggggc ctctcctgac attgaccttg gcaccggccc cacccccaca 1380
   ctgggccctg tcactcctga gatctgcaaa caggacattg tatttgatgg catcgctcag 1440
   atccgtggtg agatcttctt cttcaaggac cggttcattt ggcggactgt gacgccacgt 1500
   gacaagccca tggggcccct gctggtggcc acattctggc ctgagctccc ggaaaagatt 1560
   gatgcggtat acgaggcccc acaggaggag aaggctgtgt tctttgcagg gaatgaatac 1620
   tggatctact cagccagcac cctggagcga gggtacccca agccactgac cagcctggga 1680
   ctgccccctg atgtccagcg agtggatgcc gcctttaact ggagcaaaaa caagaagaca 1740
   tacatctttg ctggagacaa attctggaga tacaatgagg tgaagaagaa aatggatcct 1800
   ggctttccca agctcatcgc agatgcctgg aatgccatcc ccgataacct ggatgccgtc 1860
   gtggacctgc agggcggcgg tcacagctac ttcttcaagg gtgcctatta cctgaagctg 1920
   gagaaccaaa gtctgaagag cgtgaagttt ggaagcatca aatccgactg gctaggctgc 1980
   tga                                                               1983
<210> 108
   <211> 1434
   <212> DNA
   <213> Homo sapiens
<300>
   <302> MMP2
   <310> XM006271
<300>
   <302> MMP3
   <310> XM006271
<400> 108
   atgaagagtc ttccaatcct actgttgctg tgcgtggcag tttgctcagc ctatccattg 60
   gatggagctg caaggggtga ggacaccagc atgaaccttg ttcagaaata tctagaaaac 120
   tactacgacc tcgaaaaaga tgtgaaacag tttgttagga gaaaggacag tggtcctgtt 180
   gttaaaaaaa tccgagaaat gcagaagttc cttggattgg aggtgacggg gaagctggac 240
   tccgacactc tggaggtgat gcgcaagccc aggtgtggag ttcctgacgt tggtcacttc 300
   agaacctttc ctggcatccc gaagtggagg aaaacccacc ttacatacag gattgtgaat 360
   tatacaccag atttgccaaa agatgctgtt gattctgctg ttgagaaagc tctgaaagtc 420
   tgggaagagg tgactccact cacattctcc aggctgtatg aaggagaggc tgatataatg 480
   atctcttttg cagttagaga acatggagac ttttaccctt ttgatggacc tggaaatgtt 540
   ttggcccatg cctatgcccc tgggccaggg attaatggag atgcccactt tgatgatgat 600
   gaacaatgga caaaggatac aacagggacc aatttatttc tcgttgctgc tcatgaaatt 660
   ggccactccc tgggtctctt tcactcagcc aacactgaag ctttgatgta cccactctat 720
   cactcactca cagacctgac tcggttccgc ctgtctcaag atgatataaa tggcattcag 780
   tccctctatg gacctccccc tgactcccct gagacccccc tggtacccac ggaacctgtc 840
   cctccagaac ctgggacgcc agccaactgt gatcctgctt tgtcctttga tgctgtcagc 900
   actctgaggg gagaaatcct gatctttaaa gacaggcact tttggcgcaa atccctcagg 960
   aagcttgaac ctgaattgca tttgatctct tcattttggc catctcttcc ttcaggcgtg 1020
   gatgccgcat atgaagttac tagcaaggac ctcgttttca tttttaaagg aaatcaattc 1080
   tgggccatca gaggaaatga ggtacgagct ggatacccaa gaggcatcca caccctaggt 1140
   ttccctccaa ccgtgaggaa aatcgatgca gccatttctg ataaggaaaa gaacaaaaca 1200
   tatttctttg tagaggacaa atactggaga tttgatgaga agagaaattc catggagcca 1260
   ggctttccca agcaaatagc tgaagacttt ccagggattg actcaaagat tgatgctgtt 1320
   tttgaagaat ttgggttctt ttatttcttt actggatctt cacagttgga gtttgaccca 1380
   aatgcaaaga aagtgacaca cactttgaag agtaacagct ggcttaattg ttga 1434
<210> 109
   <211> 1404
   <212> DNA
   <213> Homo sapiens
<300>
   <302> MMP8
   <310> NM002424
<400> 109
   atgttctccc tgaagacgct tccatttctg ctcttactcc atgtgcagat ttccaaggcc 60
   tttcctgtat cttctaaaga gaaaaataca aaaactgttc aggactacct ggaaaagttc 120
   taccaattac caagcaacca gtatcagtct acaaggaaga atggcactaa tgtgatcgtt 180
   gaaaagctta aagaaatgca gcgatttttt gggttgaatg tgacggggaa gccaaatgag 240
   gaaactctgg acatgatgaa aaagcctcgc tgtggagtgc ctgacagtgg tggttttatg 300
   ttaaccccag gaaaccccaa gtgggaacgc actaacttga cctacaggat tcgaaactat 360
   accccacagc tgtcagaggc tgaggtagaa agagctatca aggatgcctt tgaactctgg 420
   agtgttgcat cacctctcat cttcaccagg atctcacagg gagaggcaga tatcaacatt 480
   gctttttacc aaagagatca cggtgacaat tctccatttg atggacccaa tggaatcctt 540
   gctcatgcct ttcagccagg ccaaggtatt ggaggagatg ctcattttga tgccgaagaa 600
   acatggacca acacctccgc aaattacaac ttgtttcttg ttgctgctca tgaatttggc 660
   cattctttgg ggctcgctca ctcctctgac cctggtgcct tgatgtatcc caactatgct 720
   ttcagggaaa ccagcaacta ctcactccct caagatgaca tcgatggcat tcaggccatc 780
   tatggacttt caagcaaccc tatccaacct actggaccaa gcacacccaa accctgtgac 840
   cccagtttga catttgatgc tatcaccaca ctccgtggag aaatactttt ctttaaagac 900
   aggtacttct ggagaaggca tcctcagcta caaagagtcg aaatgaattt tatttctcta 960
   ttctggccat cccttccaac tggtatacag gctgcttatg aagattttga cagagacctc 1020
   attttcctat ttaaaggcaa ccaatactgg gctctgagtg gctatgatat tctgcaaggt 1080
   tatcccaagg atatatcaaa ctatggcttc cccagcagcg tccaagcaat tgacgcagct 1140
   gttttctaca gaagtaaaac atacttcttt gtaaatgacc aattctggag atatgataac 1200
   caaagacaat tcatggagcc aggttatccc aaaagcatat caggtgcctt tccaggaata 1260
   gagagtaaag ttgatgcagt ttttcagcaa gaacatttct tccatgtctt cagtggacca 1320
   agatattacg catttgatct tattgctcag agagttacca gagttgcaag aggcaataaa 1380
   tggcttaact gtagatatgg ctga 1404
<210> 110
   <211> 2124
   <212> DNA
   <213> Homo sapiens
<300>
   <302> MMP9
   <310> XM009491
<400> 110
   atgagcctct ggcagcccct ggtcctggtg ctcctggtgc tgggctgctg ctttgctgcc 60
   cccagacagc gccagtccac ccttgtgctc ttccctggag acctgagaac caatctcacc 120
   gacaggcagc tggcagagga atacctgtac cgctatggtt acactcgggt ggcagagatg 180
   cgtggagagt cgaaatctct ggggcctgcg ctgctgcttc tccagaagca actgtccctg 240
   cccgagaccg gtgagctgga tagcgccacg ctgaaggcca tgcgaacccc acggtgcggg 300
   gtcccagacc tgggcagatt ccaaaccttt gagggcgacc tcaagtggca ccaccacaac 360
   atcacctatt ggatccaaaa ctactcggaa gacttgccgc gggcggtgat tgacgacgcc 420
   tttgcccgcg ccttcgcact gtggagcgcg gtgacgccgc tcaccttcac tcgcgtgtac 480
   agccgggacg cagacatcgt catccagttt ggtgtcgcgg agcacggaga cgggtatccc 540
   ttcgacggga aggacgggct cctggcacac gcctttcctc ctggccccgg cattcaggga 600
   gacgcccatt tcgacgatga cgagttgtgg tccctgggca agggcgtcgt ggttccaact 660
   cggtttggaa acgcagatgg cgcggcctgc cacttcccct tcatcttcga gggccgctcc 720
   tactctgcct gcaccaccga cggtcgctcc gacggcttgc cctggtgcag taccacggcc 780
   aactacgaca ccgacgaccg gtttggcttc tgccccagcg agagactcta cacccaggac 840
   ggcaatgctg atgggaaacc ctgccagttt ccattcatct tccaaggcca atcctactcc 900
   gcctgcacca cggacggtcg ctccgacggc taccgctggt gcgccaccac cgccaactac 960
   gaccgggaca agctcttcgg cttctgcccg acccgagctg actcgacggt gatggggggc 1020
   aactcggcgg gggagctgtg cgtcttcccc ttcactttcc tgggtaagga gtactcgacc 1080
   tgtaccagcg agggccgcgg agatgggcgc ctctggtgcg ctaccacctc gaactttgac 1140
   agcgacaaga agtggggctt ctgcccggac caaggataca gtttgttcct cgtggcggcg 1200
   catgagttcg gccacgcgct gggcttagat cattcctcag tgccggaggc gctcatgtac 1260
   cctatgtacc gcttcactga ggggcccccc ttgcataagg acgacgtgaa tggcatccgg 1320
   cacctctatg gtcctcgccc tgaacctgag ccacggcctc caaccaccac cacaccgcag 1380
   cccacggctc ccccgacggt ctgccccacc ggacccccca ctgtccaccc ctcagagcgc 1440
   cccacagctg gccccacagg tcccccctca gctggcccca caggtccccc cactgctggc 1500
   ccttctacgg ccactactgt gcctttgagt ccggtggacg atgcctgcaa cgtgaacatc 1560
   ttcgacgcca tcgcggagat tgggaaccag ctgtatttgt tcaaggatgg gaagtactgg 1620
   cgattctctg agggcagggg gagccggccg cagggcccct tccttatcgc cgacaagtgg 1680
   cccgcgctgc cccgcaagct ggactcggtc tttgaggagc ggctctccaa gaagcttttc 1740
   ttcttctctg ggcgccaggt gtgggtgtac acaggcgcgt cggtgctggg cccgaggcgt 1800
   ctggacaagc tgggcctggg agccgacgtg gcccaggtga ccggggccct ccggagtggc 1860
   agggggaaga tgctgctgtt cagcgggcgg cgcctctgga ggttcgacgt gaaggcgcag 1920
   atggtggatc cccggagcgc cagcgaggtg gaccggatgt tccccggggt gcctttggac 1980
   acgcacgacg tcttccagta ccgagagaaa gcctatttct gccaggaccg cttctactgg 2040
   cgcgtgagtt cccggagtga gttgaaccag gtggaccaag tgggctacgt gacctatgac 2100
   atcctgcagt gccctgagga ctag                                        2124
<210> 111
   <211> 2019
   <212> DNA
   <213> Homo sapiens
<300>
   <302> PKC alpha
   <310> NM002737
<400> 111
   atggctgacg ttttcccggg caacgactcc acggcgtctc aggacgtggc caaccgcttc 60
   gcccgcaaag gggcgctgag gcagaagaac gtgcacgagg tgaaggacca caaattcatc 120
   gcgcgcttct tcaagcagcc caccttctgc agccactgca ccgacttcat ctgggggttt 180
   gggaaacaag gcttccagtg ccaagtttgc tgttttgtgg tccacaagag gtgccatgaa 240
   tttgttactt tttcttgtcc gggtgcggat aagggacccg acactgatga ccccaggagc 300
   aagcacaagt tcaaaatcca cacttacgga agccccacct tctgcgatca ctgtgggtca 360
   ctgctctatg gacttatcca tcaagggatg aaatgtgaca cctgcgatat gaacgttcac 420
   aagcaatgcg tcatcaatgt ccccagcctc tgcggaatgg atcacactga gaagaggggg 480
   cggatttacc taaaggctga ggttgctgat gaaaagctcc atgtcacagt acgagatgca 540
   aaaaatctaa tccctatgga tccaaacggg ctttcagatc cttatgtgaa gctgaaactt 600
   attcctgatc ccaagaatga aagcaagcaa aaaaccaaaa ccatccgctc cacactaaat 660
   ccgcagtgga atgagtcctt tacattcaaa ttgaaacctt cagacaaaga ccgacgactg 720
   tctgtagaaa tctgggactg ggatcgaaca acaaggaatg acttcatggg atccctttcc 780
   tttggagttt cggagctgat gaagatgccg gccagtggat ggtacaagtt gcttaaccaa 840
   gaagaaggtg agtactacaa cgtacccatt ccggaagggg acgaggaagg aaacatggaa 900
   ctcaggcaga aattcgagaa agccaaactt ggccctgctg gcaacaaagt catcagtccc 960
   tctgaagaca ggaaacaacc ttccaacaac cttgaccgag tgaaactcac ggacttcaat 1020
   ttcctcatgg tgttgggaaa ggggagtttt ggaaaggtga tgcttgccga caggaagggc 1080
   acagaagaac tgtatgcaat caaaatcctg aagaaggatg tggtgattca ggatgatgac 1140
   gtggagtgca ccatggtaga aaagcgagtc ttggccctgc ttgacaaacc cccgttcttg 1200
   acgcagctgc actcctgctt ccagacagtg gatcggctgt acttcgtcat ggaatatgtc 1260
   aacggtgggg acctcatgta ccacattcag caagtaggaa aatttaagga accacaagca 1320
   gtattctatg cggcagagat ttccatcgga ttgttctttc ttcataaaag aggaatcatt 1380
   tatagggatc tgaagttaga taacgtcatg ttggattcag aaggacatat caaaattgct 1440
   gactttggga tgtgcaagga acacatgatg gatggagtca cgaccaggac cttctgtggg 1500
   actccagatt atatcgcccc agagataatc gcttatcagc cgtatggaaa atctgtggac 1560
   tggtgggcct atggcgtcct gttgtatgaa atgcttgccg ggcagcctcc atttgatggt 1620
   gaagatgaag acgagctatt tcagtctatc atggagcaca acgtttccta tccaaaatcc 1680
   ttgtccaagg aggctgtttc tatctgcaaa ggactgatga ccaaacaccc agccaagcgg 1740
   ctgggctgtg ggcctgaggg ggagacggac gtgagagagc atgccttctt ccggaggatc 1800
   gactgggaaa aactggagaa cacggagatc cagccaccat tcaagcccaa agtgtgtggc 1860
   aaaggagcag agaactttga caagttcttc acacgaggac agcccgtctt aacaccacct 1920
   gatcagctgg ttattgctaa catagaccag tctgattttg aagggttctc gtatgtcaac 1980
   ccccagtttg tgcaccccat cttacagagt gcagtatga 2019
<210> 112
   <211> 2022
   <212> DNA
   <213> Homo sapiens
<300>
   <302> PKC beta
   <310> X07109
<400> 112
   atggctgacc cggctgcggg gccgccgccg agcgagggcg aggagagcac cgtgcgcttc 60
   gcccgcaaag gcgccctccg gcagaagaac gtgcatgagg tcaagaacca caaattcacc 120
   gcccgcttct tcaagcagcc caccttctgc agccactgca ccgacttcat ctggggcttc 180
   gggaagcagg gattccagtg ccaagtttgc tgctttgtgg tgcacaagcg gtgccatgaa 240
   tttgtcacat tctcctgccc tggcgctgac aagggtccag cctccgatga cccccgcagc 300
   aaacacaagt ttaagatcca cacgtactcc agccccacgt tttgtgacca ctgtgggtca 360
   ctgctgtatg gactcatcca ccaggggatg aaatgtgaca cctgcatgat gaatgtgcac 420
   aagcgctgcg tgatgaatgt tcccagcctg tgtggcacgg accacacgga gcgccgcggc 480
   cgcatctaca tccaggccca catcgacagg gacgtcctca ttgtcctcgt aagagatgct 540
   aaaaaccttg tacctatgga ccccaatggc ctgtcagatc cctacgtaaa actgaaactg 600
   attcccgatc ccaaaagtga gagcaaacag aagaccaaaa ccatcaaatg ctccctcaac 660
   cctgagtgga atgagacatt tagatttcag ctgaaagaat cggacaaaga cagaagactg 720
   tcagtagaga tttgggattg ggatttgacc agcaggaatg acttcatggg atctttgtcc 780
   tttgggattt ctgaacttca gaaggccagt gttgatggct ggtttaagtt actgagccag 840
   gaggaaggcg agtacttcaa tgtgcctgtg ccaccagaag gaagtgaggc caatgaagaa 900
   ctgcggcaga aatttgagag ggccaagatc agtcagggaa ccaaggtccc ggaagaaaag 960
   acgaccaaca ctgtctccaa atttgacaac aatggcaaca gagaccggat gaaactgacc 1020
   gattttaact tcctaatggt gctggggaaa ggcagctttg gcaaggtcat gctttcagaa 1080
   cgaaaaggca cagatgagct ctatgctgtg aagatcctga agaaggacgt tgtgatccaa 1140
   gatgatgacg tggagtgcac tatggtggag aagcgggtgt tggccctgcc tgggaagccg 1200
   cccttcctga cccagctcca ctcctgcttc cagaccatgg accgcctgta ctttgtgatg 1260
   gagtacgtga atgggggcga cctcatgtat cacatccagc aagtcggccg gttcaaggag 1320
   ccccatgctg tattttacgc tgcagaaatt gccatcggtc tgttcttctt acagagtaag 1380
   ggcatcattt accgtgacct aaaacttgac aacgtgatgc tcgattctga gggacacatc 1440
   aagattgccg attttggcat gtgtaaggaa aacatctggg atggggtgac aaccaagaca 1500
   ttctgtggca ctccagacta catcgccccc gagataattg cttatcagcc ctatgggaag 1560
   tccgtggatt ggtgggcatt tggagtcctg ctgtatgaaa tgttggctgg gcaggcaccc 1620
   tttgaagggg aggatgaaga tgaactcttc caatccatca tggaacacaa cgtagcctat 1680
   cccaagtcta tgtccaagga agctgtggcc atctgcaaag ggctgatgac caaacaccca 1740
   ggcaaacgtc tgggttgtgg acctgaaggc gaacgtgata tcaaagagca tgcatttttc 1800
   cggtatattg attgggagaa acttgaacgc aaagagatcc agccccctta taagccaaaa 1860
   gcttgtgggc gaaatgctga aaacttcgac cgatttttca cccgccatcc accagtccta 1920
   acacctcccg accaggaagt catcaggaat attgaccaat cagaattcga aggattttcc 1980
   tttgttaact ctgaattttt aaaacccgaa gtcaagagct aa 2022
<210> 113
   <211> 2031
   <212> DNA
   <213> Homo sapiens
<300>
   <302> PKC delta
   <310> NM006254
<400> 113
   atggcgccgt tcctgcgcat cgccttcaac tcctatgagc tgggctccct gcaggccgag 60
   gacgaggcga accagccctt ctgtgccgtg aagatgaagg aggcgctcag cacagagcgt 120
   gggaaaacac tggtgcagaa gaagccgacc atgtatcctg agtggaagtc gacgttcgat 180
   gcccacatct atcaggggcg cgtcatccag attgtgctaa tgcgggcagc agaggagcca 240
   gtgtctgagg tgaccgtggg tgtgtcggtg ctggccgagc gctgcaagaa gaacaatggc 300
   aaggctgagt tctggctgga cctgcagcct caggccaagg tgttgatgtc tgttcagtat 360
   ttcctggagg acgtggattg caaacaatct atgcgcagtg aggacgaggc caagttccca 420
   acgatgaacc gccgcggagc catcaaacag gccaaaatcc actacatcaa gaaccatgag 480
   tttatcgcca ccttctttgg gcaacccacc ttctgttctg tgtgcaaaga ctttgtctgg 540
   ggcctcaaca agcaaggcta caaatgcagg caatgtaacg ctgccatcca caagaaatgc 600
   atcgacaaga tcatcggcag atgcactggc accgcggcca acagccggga cactatattc 660
   cagaaagaac gcttcaacat cgacatgccg caccgcttca aggttcacaa ctacatgagc 720
   cccaccttct gtgaccactg cggcagcctg ctctggggac tggtgaagca gggattaaag 780
   tgtgaagact gcggcatgaa tgtgcaccat aaatgccggg agaaggtggc caacctctgc 840
   ggcatcaacc agaagctttt ggctgaggcc ttgaaccaag tcacccagag agcctcccgg 900
   agatcagact cagcctcctc agagcctgtt gggatatatc agggtttcga gaagaagacc 960
   ggagttgctg gggaggacat gcaagacaac agtgggacct acggcaagat ctgggagggc 1020
   agcagcaagt gcaacatcaa caacttcatc ttccacaagg tcctgggcaa aggcagcttc 1080
   gggaaggtgc tgcttggaga gctgaagggc agaggagagt actctgccat caaggccctc 1140
   aagaaggatg tggtcctgat cgacgacgac gtggagtgca ccatggttga gaagcgggtg 1200
   ctgacacttg ccgcagagaa tccctttctc acccacctca tctgcacctt ccagaccaag 1260
   gaccacctgt tctttgtgat ggagttcctc aacggggggg acctgatgta ccacatccag 1320
   gacaaaggcc gctttgaact ctaccgtgcc acgttttatg ccgctgagat aatgtgtgga 1380
   ctgcagtttc tacacagcaa gggcatcatt tacagggacc tcaaactgga caatgtgctg 1440
   ttggaccggg atggccacat caagattgcc gactttggga tgtgcaaaga gaacatattc 1500
   ggggagagcc gggccagcac cttctgcggc acccctgact atatcgcccc tgagatccta 1560
   cagggcctga agtacacatt ctctgtggac tggtggtctt tcggggtcct tctgtacgag 1620
   atgctcattg gccagtcccc cttccatggt gatgatgagg atgaactctt cgagtccatc 1680
   cgtgtggaca cgccacatta tccccgctgg atcaccaagg agtccaagga catcctggag 1740
   aagctctttg aaagggaacc aaccaagagg ctgggaatga cgggaaacat caaaatccac 1800
   cccttcttca agaccataaa ctggactctg ctggaaaagc ggaggttgga gccacccttc 1860
   aggcccaaag tgaagtcacc cagagactac agtaactttg accaggagtt cctgaacgag 1920
   aaggcgcgcc tctcctacag cgacaagaac ctcatcgact ccatggacca gtctgcattc 1980
   gctggcttct cctttgtgaa ccccaaattc gagcacctcc tggaagattg a 2031
<210> 114
   <211> 2049
   <212> DNA
   <213> Homo sapiens
<300>
   <302> PKC eta
   <310> NM006255
<400> 114
   atgtcgtctg gcaccatgaa gttcaatggc tatttgaggg tccgcatcgg tgaggcagtg 60
   gggctgcagc ccacccgctg gtccctgcgc cactcgctct tcaagaaggg ccaccagctg 120
   ctggacccct atctgacggt gagcgtggac caggtgcgcg tgggccagac cagcaccaag 180
   cagaagacca acaaacccac gtacaacgag gagttttgcg ctaacgtcac cgacggcggc 240
   cacctcgagt tggccgtctt ccacgagacc cccctgggct acgacttcgt ggccaactgc 300
   accctgcagt tccaggagct cgtcggcacg accggcgcct cggacacctt cgagggttgg 360
   gtggatctcg agccagaggg gaaagtattt gtggtaataa cccttaccgg gagtttcact 420
   gaagctactc tccagagaga ccggatcttc aaacatttta ccaggaagcg ccaaagggct 480
   atgcgaaggc gagtccacca gatcaatgga cacaagttca tggccacgta tctgaggcag 540
   cccacctact gctctcactg cagggagttt atctggggag tgtttgggaa acagggttat 600
   cagtgccaag tgtgcacctg tgtcgtccat aaacgctgcc atcatctaat tgttacagcc 660
   tgtacttgcc aaaacaatat taacaaagtg gattcaaaga ttgcagaaca gaggttcggg 720
   atcaacatcc cacacaagtt cagcatccac aactacaaag tgccaacatt ctgcgatcac 780
   tgtggctcac tgctctgggg aataatgcga caaggacttc agtgtaaaat atgtaaaatg 840
   aatgtgcata ttcgatgtca agcgaacgtg gcccctaact gtggggtaaa tgcggtggaa 900
   cttgccaaga ccctggcagg gatgggtctc caacccggaa atatttctcc aacctcgaaa 960
   ctcgtttcca gatcgaccct aagacgacag ggaaaggaga gcagcaaaga aggaaatggg 1020
   attggggtta attcttccaa ccgacttggt atcgacaact ttgagttcat ccgagtgttg 1080
   gggaagggga gttttgggaa ggtgatgctt gcaagagtaa aagaaacagg agacctctat 1140
   gctgtgaagg tgctgaagaa ggacgtgatt ctgctggatg atgatgtgga atgcaccatg 1200
   accgagaaaa ggatcctgtc tctggcccgc aatcacccct tcctcactca gttgttctgc 1260
   tgctttcaga cccccgatcg tctgtttttt gtgatggagt ttgtgaatgg gggtgacttg 1320
   atgttccaca ttcagaagtc tcgtcgtttt gatgaagcac gagctcgctt ctatgctgca 1380
   gaaatcattt cggctctcat gttcctccat gataaaggaa tcatctatag agatctgaaa 1440
   ctggacaatg tcctgttgga ccacgagggt cactgtaaac tggcagactt cggaatgtgc 1500
   aaggagggga tttgcaatgg tgtcaccacg gccacattct gtggcacgcc agactatatc 1560
   gctccagaga tcctccagga aatgctgtac gggcctgcag tagactggtg ggcaatgggc 1620
   gtgttgctct atgagatgct ctgtggtcac gcgccttttg aggcagagaa tgaagatgac 1680
   ctctttgagg ccatactgaa tgatgaggtg gtctacccta cctggctcca tgaagatgcc 1740
   acagggatcc taaaatcttt catgaccaag aaccccacca tgcgcttggg cagcctgact 1800
   cagggaggcg agcacgccat cttgagacat ccttttttta aggaaatcga ctgggcccag 1860
   ctgaaccatc gccaaataga accgcctttc agacccagaa tcaaatcccg agaagatgtc 1920
   agtaattttg accctgactt cataaaggaa gagccagttt taactccaat tgatgaggga 1980
   catcttccaa tgattaacca ggatgagttt agaaactttt cctatgtgtc tccagaattg 2040
   caaccatag 2049
<210> 115
   <211> 948
   <212> DNA
   <213> Homo sapiens
<300>
   <302> PKC epsilon
   <310> XM002370
<400> 115
   atgttggcag aactcaaggg caaagatgaa gtatatgctg tgaaggtctt aaagaaggac 60
   gtcatccttc aggatgatga cgtggactgc acaatgacag agaagaggat tttggctctg 120
   gcacggaaac acccgtacct tacccaactc tactgctgct tccagaccaa ggaccgcctc 180
   tttttcgtca tggaatatgt aaatggtgga gacctcatgt ttcagattca gcgctcccga 240
   aaattcgacg agcctcgttc acggttctat gctgcagagg tcacatcggc cctcatgttc 300
   ctccaccagc atggagtcat ctacagggat ttgaaactgg acaacatcct tctggatgca 360
   gaaggtcact gcaagctggc tgacttcggg atgtgcaagg aagggattct gaatggtgtg 420
   acgaccacca cgttctgtgg gactcctgac tacatagctc ctgagatcct gcaggagttg 480
   gagtatggcc cctccgtgga ctggtgggcc ctgggggtgc tgatgtacga gatgatggct 540
   ggacagcctc cctttgaggc cgacaatgag gacgacctat ttgagtccat cctccatgac 600
   gacgtgctgt acccagtctg gctcagcaag gaggctgtca gcatcttgaa agctttcatg 660
   acgaagaatc cccacaagcg cctgggctgt gtggcatcgc agaatggcga ggacgccatc 720
   aagcagcacc cattcttcaa agagattgac tgggtgctcc tggagcagaa gaagatcaag 780
   ccacccttca aaccacgcat taaaaccaaa agagacgtca ataattttga ccaagacttt 840
   acccgggaag agccggtact cacccttgtg gacgaagcaa ttgtaaagca gatcaaccag 900
   gaggaattca aaggtttctc ctactttggt gaagacctga tgccctga 948
<210> 116
   <211> 1764
   <212> DNA
   <213> Homo sapiens
<300>
   <302> PKC iota
   <310> NM002740
<400> 116
   atgtcccaca cggtcgcagg cggcggcagc ggggaccatt cccaccaggt ccgggtgaaa 60
   gcctactacc gcggggatat catgataaca cattttgaac cttccatctc ctttgagggc 120
   ctttgcaatg aggttcgaga catgtgttct tttgacaacg aacagctctt caccatgaaa 180
   tggatagatg aggaaggaga cccgtgtaca gtatcatctc agttggagtt agaagaagcc 240
   tttagacttt atgagctaaa caaggattct gaactcttga ttcatgtgtt cccttgtgta 300
   ccagaacgtc ctgggatgcc ttgtccagga gaagataaat ccatctaccg tagaggtgca 360
   cgccgctgga gaaagcttta ttgtgccaat ggccacactt tccaagccaa gcgtttcaac 420
   aggcgtgctc actgtgccat ctgcacagac cgaatatggg gacttggacg ccaaggatat 480
   aagtgcatca actgcaaact cttggttcat aagaagtgcc ataaactcgt cacaattgaa 540
   tgtgggcggc attctttgcc acaggaacca gtgatgccca tggatcagtc atccatgcat 600
   tctgaccatg cacagacagt aattccatat aatccttcaa gtcatgagag tttggatcaa 660
   gttggtgaag aaaaagaggc aatgaacacc agggaaagtg gcaaagcttc atccagtcta 720
   ggtcttcagg attttgattt gctccgggta ataggaagag gaagttatgc caaagtactg 780
   ttggttcgat taaaaaaaac agatcgtatt tatgcaatga aagttgtgaa aaaagagctt 840
   gttaatgatg atgaggatat tgattgggta cagacagaga agcatgtgtt tgagcaggca 900
   tccaatcatc ctttccttgt tgggctgcat tcttgctttc agacagaaag cagattgttc 960
   tttgttatag agtatgtaaa tggaggagac ctaatgtttc atatgcagcg acaaagaaaa 1020
   cttcctgaag aacatgccag attttactct gcagaaatca gtctagcatt aaattatctt 1080
   catgagcgag ggataattta tagagatttg aaactggaca atgtattact ggactctgaa 1140
   ggccacatta aactcactga ctacggcatg tgtaaggaag gattacggcc aggagataca 1200
   accagcactt tctgtggtac tcctaattac attgctcctg aaattttaag aggagaagat 1260
   tatggtttca gtgttgactg gtgggctctt ggagtgctca tgtttgagat gatggcagga 1320
   aggtctccat ttgatattgt tgggagctcc gataaccctg accagaacac agaggattat 1380
   ctcttccaag ttattttgga aaaacaaatt cgcataccac gttctctgtc tgtaaaagct 1440
   gcaagtgttc tgaagagttt tcttaataag gaccctaagg aacgattggg ttgtcatcct 1500
   caaacaggat ttgctgatat tcagggacac ccgttcttcc gaaatgttga ttgggatatg 1560
   atggagcaaa aacaggtggt acctcccttt aaaccaaata tttctgggga atttggtttg 1620
   gacaactttg attctcagtt tactaatgaa cctgtccagc tcactccaga tgacgatgac 1680
   attgtgagga agattgatca gtctgaattt gaaggttttg agtatatcaa tcctcttttg 1740
   atgtctgcag aagaatgtgt ctga 1764
<210> 117
   <211> 2451
   <212> DNA
   <213> Homo sapiens
<300>
   <302> PKC mu
   <310> XM007234
<400> 117
   atgtatgata agatcctgct ttttcgccat gaccctacct ctgaaaacat ccttcagctg 60
   gtgaaagcgg ccagtgatat ccaggaaggc gatcttattg aagtggtctt gtcagcttcc 120
   gccacctttg aagactttca gattcgtccc cacgctctct ttgttcattc atacagagct 180
   ccagctttct gtgatcactg tggagaaatg ctgtgggggc tggtacgtca aggtcttaaa 240
   tgtgaagggt gtggtctgaa ttaccataag agatgtgcat ttaaaatacc caacaattgc 300
   agcggtgtga ggcggagaag gctctcaaac gtttccctca ctggggtcag caccatccgc 360
   acatcatctg ctgaactctc tacaagtgcc cctgatgagc cccttctgca aaaatcacca 420
   tcagagtcgt ttattggtcg agagaagagg tcaaattctc aatcatacat tggacgacca 480
   attcaccttg acaagatttt gatgtctaaa gttaaagtgc cgcacacatt tgtcatccac 540
   tcctacaccc ggcccacagt gtgccagtac tgcaagaagc ttctgaaggg gcttttcagg 600
   cagggcttgc agtgcaaaga ttgcagattc aactgccata aacgttgtgc accgaaagta 660
   ccaaacaact gccttggcga agtgaccatt aatggagatt tgcttagccc tggggcagag 720
   tctgatgtgg tcatggaaga agggagtgat gacaatgata gtgaaaggaa cagtgggctc 780
   atggatgata tggaagaagc aatggtccaa gatgcagaga tggcaatggc agagtgccag 840
   aacgacagtg gcgagatgca agatccagac ccagaccacg aggacgccaa cagaaccatc 900
   agtccatcaa caagcaacaa tatcccactc atgagggtag tgcagtctgt caaacacacg 960
   aagaggaaaa gcagcacagt catgaaagaa ggatggatgg tccactacac cagcaaggac 1020
   acgctgcgga aacggcacta ttggagattg gatagcaaat gtattaccct ctttcagaat 1080
   gacacaggaa gcaggtacta caaggaaatt cctttatctg aaattttgtc tctggaacca 1140
   gtaaaaactt cagctttaat tcctaatggg gccaatcctc attgtttcga aatcactacg 1200
   gcaaatgtag tgtattatgt gggagaaaat gtggtcaatc cttccagccc atcaccaaat 1260
   aacagtgctc tcaccagtgg cgttggtgca gatgtggcca ggatgtggga gatagccatc 1320
   cagcatgccc ttatgcccgt cattcccaag ggctcctccg tgggtacagg aaccaacttg 1380
   cacagagata tctctgtgag tatttcagta tcaaattgcc agattcaaga aaatgtggac 1440
   atcagcacag tatatcagat ttttcctgat gaagtactgg gttctggaca gtttggaatt 1500
   gtttatggag gaaaacatcg taaaacagga agagatgtag ctattaaaat cattgacaaa 1560
   ttacgatttc caacaaaaca agaaagccag cttcgtaatg aggttgcaat tctacagaac 1620
   cttcatcacc ctggtgttgt aaatttggag tgtatgtttg agacgcctga aagagtgttt 1680
   gttgttatgg aaaaactcca tggagacatg ctggaaatga tcttgtcaag tgaaaagggc 1740
   aggttgccag agcacataac gaagttttta attactcaga tactcgtggc tttgcggcac 1800
   cttcatttta aaaatatcgt tcactgtgac ctcaaaccag aaaatgtgtt gctagcctca 1860
   gctgatcctt ttcctcaggt gaaactttgt gattttggtt ttgcccggat cattggagag 1920
   aagtctttcc ggaggtcagt ggtgggtacc cccgcttacc tggctcctga ggtcctaagg 1980
   aacaagggct acaatcgctc tctagacatg tggtctgttg gggtcatcat ctatgtaagc 2040
   ctaagcggca cattcccatt taatgaagat gaagacatac acgaccaaat tcagaatgca 2100
   gctttcatgt atccaccaaa tccctggaag gaaatatctc atgaagccat tgatcttatc 2160
   aacaatttgc tgcaagtaaa aatgagaaag cgctacagtg tggataagac cttgagccac 2220
   ccttggctac aggactatca gacctggtta gatttgcgag agctggaatg caaaatcggg 2280
   gagcgctaca tcacccatga aagtgatgac ctgaggtggg agaagtatgc aggcgagcag 2340
   gggctgcagt accccacaca cctgatcaat ccaagtgcta gccacagtga cactcctgag 2400
   actgaagaaa cagaaatgaa agccctcggt gagcgtgtca gcatcctatg a 2451
<210> 118
   <211> 2673
   <212> DNA
   <213> Homo sapiens
<300>
   <302> PKC nu
   <310> NM005813
<400> 118
   atgtctgcaa ataattcccc tccatcagcc cagaagtctg tattacccac agctattcct 60
   gctgtgcttc cagctgcttc tccgtgttca agtcctaaga cgggactctc tgcccgactc 120
   tctaatggaa gcttcagtgc accatcactc accaactcca gaggctcagt gcatacagtt 180
   tcatttctac tgcaaattgg cctcacacgg gagagtgtta ccattgaagc ccaggaactg 240
   tctttatctg ctgtcaagga tcttgtgtgc tccatagttt atcaaaagtt tccagagtgt 300
   ggattctttg gcatgtatga caaaattctt ctctttcgcc atgacatgaa ctcagaaaac 360
   attttgcagc tgattacctc agcagatgaa atacatgaag gagacctagt ggaagtggtt 420
   ctttcagctt tagccacagt agaagacttc cagattcgtc cacatactct ctatgtacat 480
   tcttacaaag ctcctacttt ctgtgattac tgtggtgaga tgctgtgggg attggtacgt 540
   caaggactga aatgtgaagg ctgtggatta aattaccata aacgatgtgc cttcaagatt 600
   ccaaataact gtagtggagt aagaaagaga cgtctgtcaa atgtatcttt accaggaccc 660
   ggcctctcag ttccaagacc cctacagcct gaatatgtag cccttcccag tgaagagtca 720
   catgtccacc aggaaccaag taagagaatt ccttcttgga gtggtcgccc aatctggatg 780
   gaaaagatgg taatgtgcag agtgaaagtt ccacacacat ttgctgttca ctcttacacc 840
   cgtcccacga tatgtcagta ctgcaagcgg ttactgaaag gcctctttcg ccaaggaatg 900
   cagtgtaaag attgcaaatt caactgccat aaacgctgtg catcaaaagt accaagagac 960
   tgccttggag aggttacttt caatggagaa ccttccagtc tgggaacaga tacagatata 1020
   ccaatggata ttgacaataa tgacataaat agtgatagta gtcggggttt ggatgacaca 1080
   gaagagccat cacccccaga agataagatg ttcttcttgg atccatctga tctcgatgtg 1140
   gaaagagatg aagaagccgt taaaacaatc agtccatcaa caagcaataa tattccgcta 1200
   atgagggttg tacaatccat caagcacaca aagaggaaga gcagcacaat ggtgaaggaa 1260
   gggtggatgg tccattacac cagcagggat aacctgagaa agaggcatta ttggagactt 1320
   gacagcaaat gtctaacatt atttcagaat gaatctggat caaagtatta taaggaaatt 1380
   ccactttcag aaattctccg catatcttca ccacgagatt tcacaaacat ttcacaaggc 1440
   agcaatccac actgttttga aatcattact gatactatgg tatacttcgt tggtgagaac 1500
   aatggggaca gctctcataa tcctgttctt gctgccactg gagttggact tgatgtagca 1560
   cagagctggg aaaaagcaat tcgccaagcc ctcatgcctg ttactcctca agcaagtgtt 1620
   tgcacttctc cagggcaagg gaaagatcac aaagatttgt ctacaagtat ctctgtatct 1680
   aattgtcaga ttcaggagaa tgtggatatc agtactgttt accagatctt tgcagatgag 1740
   gtgcttggtt caggccagtt tggcatcgtt tatggaggaa aacatagaaa gactgggagg 1800
   gatgtggcta ttaaagtaat tgataagatg agattcccca caaaacaaga aagtcaactc 1860
   cgtaatgaag tggctatttt acagaatttg caccatcctg ggattgtaaa cctggaatgt 1920
   atgtttgaaa ccccagaacg agtctttgta gtaatggaaa agctgcatgg agatatgttg 1980
   gaaatgattc tatccagtga gaaaagtcgg cttccagaac gaattactaa attcatggtc 2040
   acacagatac ttgttgcttt gaggaatctg cattttaaga atattgtgca ctgtgattta 2100
   aagccagaaa atgtgctgct tgcatcagca gagccatttc ctcaggtgaa gctgtgtgac 2160
   tttggatttg cacgcatcat tggtgaaaag tcattcagga gatctgtggt aggaactcca 2220
   gcatacttag cccctgaagt tctccggagc aaaggttaca accgttccct agatatgtgg 2280
   tcagtgggag ttatcatcca tgtgagcctc agtggcacat ttccttttaa tgaggatgaa 2340
   gatataaatg accaaatcca aaatgctgca tttatgtacc caccaaatcc atggagagaa 2400
   atttctggtg aagcaattga tctgataaac aatctgcttc aagtgaagat gagaaaacgt 2460
   tacagtgttg acaaatctct tagtcatccc tggctacagg actatcagac ttggcttgac 2520
   cttagagaat ttgaaactcg cattggagaa cgttacatta cacatgaaag tgatgatgct 2580
   cgctgggaaa tacatgcata cacacataac cttgtatacc caaagcactt cattatggct 2640
   cctaatccag atgatatgga agaagatcct taa 2673
<210> 119
   <211> 2121
   <212> DNA
   <213> Homo sapiens
<300>
   <302> PKC tau
   <310> NM006257
<400> 119
   atgtcgccat ttcttcggat tggcttgtcc aactttgact gcgggtcctg ccagtcttgt 60
   cagggcgagg ctgttaaccc ttactgtgct gtgctcgtca aagagtatgt cgaatcagag 120
   aacgggcaga tgtatatcca gaaaaagcct accatgtacc caccctggga cagcactttt 180
   gatgcccata tcaacaaggg aagagtcatg cagatcattg tgaaaggcaa aaacgtggac 240
   ctcatctctg aaaccaccgt ggagctctac tcgctggctg agaggtgcag gaagaacaac 300
   gggaagacag aaatatggtt agagctgaaa cctcaaggcc gaatgctaat gaatgcaaga 360
   tactttctgg aaatgagtga cacaaaggac atgaatgaat ttgagacgga aggcttcttt 420
   gctttgcatc agcgccgggg tgccatcaag caggcaaagg tccaccacgt caagtgccac 480
   gagttcactg ccaccttctt cccacagccc acattttgct ctgtctgcca cgagtttgtc 540
   tggggcctga acaaacaggg ctaccagtgc cgacaatgca atgcagcaat tcacaagaag 600
   tgtattgata aagttatagc aaagtgcaca ggatcagcta tcaatagccg agaaaccatg 660
   ttccacaagg agagattcaa aattgacatg ccacacagat ttaaagtcta caattacaag 720
   agcccgacct tctgtgaaca ctgtgggacc ctgctgtggg gactggcacg gcaaggactc 780
   aagtgtgatg catgtggcat gaatgtgcat catagatgcc agacaaaggt ggccaacctt 840
   tgtggcataa accagaagct aatggctgaa gcgctggcca tgattgagag cactcaacag 900
   gctcgctgct taagagatac tgaacagatc ttcagagaag gtccggttga aattggtctc 960
   ccatgctcca tcaaaaatga agcaaggccg ccatgtttac cgacaccggg aaaaagagag 1020
   cctcagggca tttcctggga gtctccgttg gatgaggtgg ataaaatgtg ccatcttcca 1080
   gaacctgaac tgaacaaaga aagaccatct ctgcagatta aactaaaaat tgaggatttt 1140
   atcttgcaca aaatgttggg gaaaggaagt tttggcaagg tcttcctggc agaattcaag 1200
   aaaaccaatc aatttttcgc aataaaggcc ttaaagaaag atgtggtctt gatggacgat 1260
   gatgttgagt gcacgatggt agagaagaga gttctttcct tggcctggga gcatccgttt 1320
   ctgacgcaca tgttttgtac attccagacc aaggaaaacc tcttttttgt gatggagtac 1380
   ctcaacggag gggacttaat gtaccacatc caaagctgcc acaagttcga cctttccaga 1440
   gcgacgtttt atgctgctga aatcattctt ggtctgcagt tccttcattc caaaggaata 1500
   gtctacaggg acctgaagct agataacatc ctgttagaca aagatggaca tatcaagatc 1560
   gcggattttg gaatgtgcaa ggagaacatg ttaggagatg ccaagacgaa taccttctgt 1620
   gggacacctg actacatcgc cccagagatc ttgctgggtc agaaatacaa ccactctgtg 1680
   gactggtggt ccttcggggt tctcctttat gaaatgctga ttggtcagtc gcctttccac 1740
   gggcaggatg aggaggagct cttccactcc atccgcatgg acaatccctt ttacccacgg 1800
   tggctggaga aggaagcaaa ggaccttctg gtgaagctct tcgtgcgaga acctgagaag 1860
   aggctgggcg tgaggggaga catccgccag caccctttgt ttcgggagat caactgggag 1920
   gaacttgaac ggaaggagat tgacccaccg ttccggccga aagtgaaatc accatttgac 1980
   tgcagcaatt tcgacaaaga attcttaaac gagaagcccc ggctgtcatt tgccgacaga 2040
   gcactgatca acagcatgga ccagaatatg ttcaggaact tttccttcat gaaccccggg 2100
   atggagcggc tgatatcctg a 2121
<210> 120
   <211> 1779
   <212> DNA
   <213> Homo sapiens
<300>
   <302> PKC zeta
   <310> NM2744
<400> 120
   atgcccagca ggaccgaccc caagatggaa gggagcggcg gccgcgtccg cctcaaggcg 60
   cattacgggg gggacatctt catcaccagc gtggacgccg ccacgacctt cgaggagctc 120
   tgtgaggaag tgagagacat gtgtcgtctg caccagcagc acccgctcac cctcaagtgg 180
   gtggacagcg aaggtgaccc ttgcacggtg tcctcccaga tggagctgga agaggctttc 240
   cgcctggccc gtcagtgcag ggatgaaggc ctcatcattc atgtttcccc gagcacccct 300
   gagcagcctg gcctgccatg tccgggagaa gacaaatcta tctaccgccg gggagccaga 360
   agatggagga agctgtaccg tgccaacggc cacctcttcc aagccaagcg ctttaacagg 420
   agagcgtact gcggtcagtg cagcgagagg atatggggcc tcgcgaggca aggctacagg 480
   tgcatcaact gcaaactgct ggtccataag cgctgccacg gcctcgtccc gctgacctgc 540
   aggaagcata tggattctgt catgccttcc caagagcctc cagtagacga caagaacgag 600
   gacgccgacc ttccttccga ggagacagat ggaattgctt acatttcctc atcccggaag 660
   catgacagca ttaaagacga ctcggaggac cttaagccag ttatcgatgg gatggatgga 720
   atcaaaatct ctcaggggct tgggctgcag gactttgacc taatcagagt catcgggcgc 780
   gggagctacg ccaaggttct cctggtgcgg ttgaagaaga atgaccaaat ttacgccatg 840
   aaagtggtga agaaagagct ggtgcatgat gacgaggata ttgactgggt acagacagag 900
   aagcacgtgt ttgagcaggc atccagcaac cccttcctgg tcggattaca ctcctgcttc 960
   cagacgacaa gtcggttgtt cctggtcatt gagtacgtca acggcgggga cctgatgttc 1020
   cacatgcaga ggcagaggaa gctccctgag gagcacgcca ggttctacgc ggccgagatc 1080
   tgcatcgccc tcaacttcct gcacgagagg gggatcatct acagggacct gaagctggac 1140
   aacgtcctcc tggatgcgga cgggcacatc aagctcacag actacggcat gtgcaaggaa 1200
   ggcctgggcc ctggtgacac aacgagcact ttctgcggaa ccccgaatta catcgccccc 1260
   gaaatcctgc ggggagagga gtacgggttc agcgtggact ggtgggcgct gggagtcctc 1320
   atgtttgaga tgatggccgg gcgctccccg ttcgacatca tcaccgacaa cccggacatg 1380
   aacacagagg actacctttt ccaagtgatc ctggagaagc ccatccggat cccccggttc 1440
   ctgtccgtca aagcctccca tgttttaaaa ggatttttaa ataaggaccc caaagagagg 1500
   ctcggctgcc ggccacagac tggattttct gacatcaagt cccacgcgtt cttccgcagc 1560
   atagactggg acttgctgga gaagaagcag gcgctccctc cattccagcc acagatcaca 1620
   gacgactacg gtctggacaa ctttgacaca cagttcacca gcgagcccgt gcagctgacc 1680
   ccagacgatg aggatgccat aaagaggatc gaccagtcag agttcgaagg ctttgagtat 1740
   atcaacccat tattgctgtc caccgaggag tcggtgtga 1779
<210> 121
   <211> 576
   <212> DNA
   <213> Homo sapiens
<300>
   <302> VEGF
   <310> NM003376
<400> 121
   atgaactttc tgctgtcttg ggtgcattgg agccttgcct tgctgctcta cctccaccat 60
   gccaagtggt cccaggctgc acccatggca gaaggaggag ggcagaatca tcacgaagtg 120
   gtgaagttca tggatgtcta tcagcgcagc tactgccatc caatcgagac cctggtggac 180
   atcttccagg agtaccctga tgagatcgag tacatcttca agccatcctg tgtgcccctg 240
   atgcgatgcg ggggctgctg caatgacgag ggcctggagt gtgtgcccac tgaggagtcc 300
   aacatcacca tgcagattat gcggatcaaa cctcaccaag gccagcacat aggagagatg 360
   agcttcctac agcacaacaa atgtgaatgc agaccaaaga aagatagagc aagacaagaa 420
   aatccctgtg ggccttgctc agagcggaga aagcatttgt ttgtacaaga tccgcagacg 480
   tgtaaatgtt cctgcaaaaa cacagactcg cgttgcaagg cgaggcagct tgagttaaac 540
   gaacgtactt gcagatgtga caagccgagg cggtga 576
<210> 122
   <211> 624
   <212> DNA
   <213> Homo sapiens
<300>
   <302> VEGF B
   <310> NM003377
<400> 122
   atgagccctc tgctccgccg cctgctgctc gccgcactcc tgcagctggc ccccgcccag 60
   gcccctgtct cccagcctga tgcccctggc caccagagga aagtggtgtc atggatagat 120
   gtgtatactc gcgctacctg ccagccccgg gaggtggtgg tgcccttgac tgtggagctc 180
   atgggcaccg tggccaaaca gctggtgccc agctgcgtga ctgtgcagcg ctgtggtggc 240
   tgctgccctg acgatggcct ggagtgtgtg cccactgggc agcaccaagt ccggatgcag 300
   atcctcatga tccggtaccc gagcagtcag ctgggggaga tgtccctgga agaacacagc 360
   cagtgtgaat gcagacctaa aaaaaaggac agtgctgtga agccagacag ggctgccact 420
   ccccaccacc gtccccagcc ccgttctgtt ccgggctggg actctgcccc cggagcaccc 480
   tccccagctg acatcaccca tcccactcca gccccaggcc cctctgccca cgctgcaccc 540
   agcaccacca gcgccctgac ccccggacct gccgccgccg ctgccgacgc cgcagcttcc 600
   tccgttgcca agggcggggc ttag                                        624
<210> 123
   <211> 1260
   <212> DNA
   <213> Homo sapiens
<300>
   <302> VEGP C
   <310> NM005429
<400> 123
   atgcacttgc tgggcttctt ctctgtggcg tgttctctgc tcgccgctgc gctgctcccg 60
   ggtcctcgcg aggcgcccgc cgccgccgcc gccttcgagt ccggactcga cctctcggac 120
   gcggagcccg acgcgggcga ggccacggct tatgcaagca aagatctgga ggagcagtta 180
   cggtctgtgt ccagtgtaga tgaactcatg actgtactct acccagaata ttggaaaatg 240
   tacaagtgtc agctaaggaa aggaggctgg caacataaca gagaacaggc caacctcaac 300
   tcaaggacag aagagactat aaaatttgct gcagcacatt ataatacaga gatcttgaaa 360
   agtattgata atgagtggag aaagactcaa tgcatgccac gggaggtgtg tatagatgtg 420
   gggaaggagt ttggagtcgc gacaaacacc ttctttaaac ctccatgtgt gtccgtctac 480
   agatgtgggg gttgctgcaa tagtgagggg ctgcagtgca tgaacaccag cacgagctac 540
   ctcagcaaga cgttatttga aattacagtg cctctctctc aaggccccaa accagtaaca 600
   atcagttttg ccaatcacac ttcctgccga tgcatgtcta aactggatgt ttacagacaa 660
   gttcattcca ttattagacg ttccctgcca gcaacactac cacagtgtca ggcagcgaac 720
   aagacctgcc ccaccaatta catgtggaat aatcacatct gcagatgcct ggctcaggaa 780
   gattttatgt tttcctcgga tgctggagat gactcaacag atggattcca tgacatctgt 840
   ggaccaaaca aggagctgga tgaagagacc tgtcagtgtg tctgcagagc ggggcttcgg 900
   cctgccagct gtggacccca caaagaacta gacagaaact catgccagtg tgtctgtaaa 960
   aacaaactct tccccagcca atgtggggcc aaccgagaat ttgatgaaaa cacatgccag 1020
   tgtgtatgta aaagaacctg ccccagaaat caacccctaa atcctggaaa atgtgcctgt 1080
   gaatgtacag aaagtccaca gaaatgcttg ttaaaaggaa agaagttcca ccaccaaaca 1140
   tgcagctgtt acagacggcc atgtacgaac cgccagaagg cttgtgagcc aggattttca 1200
   tatagtgaag aagtgtgtcg ttgtgtccct tcatattgga aaagaccaca aatgagctaa 1260
<210> 124
   <212> 1074
   <212> DNA
   <213> Homo sapiens
<300>
   <302> VEGF D
   <310> AJ000135
<400> 124
<210> 125
   <211> 1314
   <212> DNA
   <213> Homo sapiens
<300>
   <302> E2F
   <310> M96577
<400> 125
   atggccttgg ccggggcccc tgcgggcggc ccatgcgcgc cggcgctgga ggccctgctc 60
   ggggccggcg cgctgcggct gctcgactcc tcgcagatcg tcatcatctc cgccgcgcag 120
   gacgccagcg ccccgccggc tcccaccggc cccgcggcgc ccgccgccgg cccctgcgac 180
   cctgacctgc tgctcttcgc cacaccgcag gcgccccggc ccacacccag tgcgccgcgg 240
   cccgcgctcg gccgcccgcc ggtgaagcgg aggctggacc tggaaactga ccatcagtac 300
   ctggccgaga gcagtgggcc agctcggggc agaggccgcc atccaggaaa aggtgtgaaa 360
   tccccggggg agaagtcacg ctatgagacc tcactgaatc tgaccaccaa gcgcttcctg 420
   gagctgctga gccactcggc tgacggtgtc gtcgacctga actgggctgc cgaggtgctg 480
   aaggtgcaga agcggcgcat ctatgacatc accaacgtcc ttgagggcat ccagctcatt 540
   gccaagaagt ccaagaacca catccagtgg ctgggcagcc acaccacagt gggcgtcggc 600
   ggacggcttg aggggttgac ccaggacctc cgacagctgc aggagagcga gcagcagctg 660
   gaccacctga tgaatatctg tactacgcag ctgcgcctgc tctccgagga cactgacagc 720
   cagcgcctgg cctacgtgac gtgtcaggac cttcgtagca ttgcagaccc tgcagagcag 780
   atggttatgg tgatcaaagc ccctcctgag acccagctcc aagccgtgga ctcttcggag 840
   aactttcaga tctcccttaa gagcaaacaa ggcccgatcg atgttttcct gtgccctgag 900
   gagaccgtag gtgggatcag ccctgggaag accccatccc aggaggtcac ttctgaggag 960
   gagaacaggg ccactgactc tgccaccata gtgtcaccac caccatcatc tcccccctca 1020
   tccctcacca cagatcccag ccagtctcta ctcagcctgg agcaagaacc gctgttgtcc 1080
   cggatgggca gcctgcgggc tcccgtggac gaggaccgcc tgtccccgct ggtggcggcc 1140
   gactcgctcc tggagcatgt gcgggaggac ttctccggcc tcctccctga ggagttcatc 1200
   agcctttccc caccccacga ggccctcgac taccacttcg gcctcgagga gggcgagggc 1260
   atcagagacc tcttcgactg tgactttggg gacctcaccc ccctggattt ctga       1314
<210> 126
   <211> 166
   <212> DNA
   <213> Human papillomavirus
<300>
   <302> EBER-1
   <310> Jo2078
<400> 126
   ggacctacgc tgccctagag gttttgctag ggaggagacg tgtgtggctg tagccacccg 60
   tcccgggtac aagtcccggg tggtgaggac ggtgtctgtg gttgtcttcc cagactctgc 120
   tttctgccgt cttcggtcaa gtaccagctg gtggtccgca tgtttt 166
<210> 127
   <211> 172
   <212> DNA
   <213> Hepatitis C virus
<300>
   <302> EBER-2
   <310> J02078
<400> 127
   ggacagccgt tgccctagtg gtttcggaca caccgccaac gctcagtgcg gtgctaccga 60
   cccgaggtca agtcccgggg gaggagaaga gaggcttccc gcctagagca tttgcaagtc 120
   aggattctct aatccctctg ggagaagggt attcggcttg tccgctattt tt 172
<210> 128
   <211> 651
   <212> DNA
   <213> Hepatitis C virus
<300>
   <302> NS2
   <310> AJ238799
<400> 128
   atggaccggg agatggcagc atcgtgcgga ggcgcggttt tcgtaggtct gatactcttg 60
   accttgtcac cgcactataa gctgttcctc gctaggctca tatggtggtt acaatatttt 120
   atcaccaggg ccgaggcaca cttgcaagtg tggatccccc ccctcaacgt tcgggggggc 180
   cgcgatgccg tcatcctcct cacgtgcgcg atccacccag agctaatctt taccatcacc 240
   aaaatcttgc tcgccatact cggtccactc atggtgctcc aggctggtat aaccaaagtg 300
   ccgtacttcg tgcgcgcaca cgggctcatt cgtgcatgca tgctggtgcg gaaggttgct 360
   gggggtcatt atgtccaaat ggctctcatg aagttggccg cactgacagg tacgtacgtt 420
   tatgaccatc tcaccccact gcgggactgg gcccacgcgg gcctacgaga ccttgcggtg 480
   gcagttgagc ccgtcgtctt ctctgatatg gagaccaagg ttatcacctg gggggcagac 540
   accgcggcgt gtggggacat catcttgggc ctgcccgtct ccgcccgcag ggggagggag 600
   atacatctgg gaccggcaga cagccttgaa gggcaggggt ggcgactcct c 651
<210> 129
   <211> 161
   <212> DNA
   <213> Hepatitis C virus
<300>
   <302> NS4A
   <310> AJ235799
<400> 129
   gcacctgggt gctggtaggc ggagtcctag cagctctggc cgcgtattgc ctgacaacag 60
   gcagcgtggt cattgtgggc aggatcatct tgtccggaaa gccggccatc attcccgaca 120
   gggaagtcct ttaccgggag ttcgatgaga tggaagagtg c                     161
<210> 130
   <211> 783
   <212> DNA
   <213> Hepatitis C virus
<300>
   <302> NS4B
   <310> AJ238799
<400> 130
   gcctcacacc tcccttacat cgaacaggga atgcagctcg ccgaacaatt caaacagaag 60
   gcaatcgggt tgctgcaaac agccaccaag caagcggagg ctgctgctcc cgtggtggaa 120
   tccaagtggc ggaccctcga agccttctgg gcgaagcata tgtggaattt catcagcggg 180
   atacaatatt tagcaggctt gtccactctg cctggcaacc ccgcgatagc atcactgatg 240
   gcattcacag cctctatcac cagcccgctc accacccaac ataccctcct gtttaacatc 300
   ctggggggat gggtggccgc ccaacttgct cctcccagcg ctgcttctgc tttcgtaggc 360
   gccggcatcg ctggagcggc tgttggcagc ataggccttg ggaaggtgct tgtggatatt 420
   ttggcaggtt atggagcagg ggtggcaggc gcgctcgtgg cctttaaggt catgagcggc 480
   gagatgccct ccaccgagga cctggttaac ctactccctg ctatcctctc ccctggcgcc 540
   ctagtcgtcg gggtcgtgtg cgcagcgata ctgcgtcggc acgtgggccc aggggagggg 600
   gctgtgcagt ggatgaaccg gctgatagcg ttcgcttcgc ggggtaacca cgtctccccc 660
   acgcactatg tgcctgagag cgacgctgca gcacgtgtca ctcagatcct ctctagtctt 720
   accatcactc agctgctgaa gaggcttcac cagtggatca acgaggactg ctccacgcca 780
   tgc                                                                783
<210> 131
   <211> 1341
   <212> DNA
   <213> Hepatitis C virus
<300>
   <302> NS5A
   <310> AJ238799
<400> 131
   tccggctcgt ggctaagaga tgtttgggat tggatatgca cggtgttgac tgatttcaag 60
   acctggctcc agtccaagct cctgccgcga ttgccgggag tccccttctt ctcatgtcaa 120
   cgtgggtaca agggagtctg gcggggcgac ggcatcatgc aaaccacctg cccatgtgga 180
   gcacagatca ccggacatgt gaaaaacggt tccatgagga tcgtggggcc taggacctgt 240
   agtaacacgt ggcatggaac attccccatt aacgcgtaca ccacgggccc ctgcacgccc 300
   tccccggcgc caaattattc tagggcgctg tggcgggtgg ctgctgagga gtacgtggag 360
   gttacgcggg tgggggattt ccactacgtg acgggcatga ccactgacaa cgtaaagtgc 420
   ccgtgtcagg ttccggcccc cgaattcttc acagaagtgg atggggtgcg gttgcacagg 480
   tacgctccag cgtgcaaacc cctcctacgg gaggaggtca cattcctggt cgggctcaat 540
   caatacctgg ttgggtcaca gctcccatgc gagcccgaac cggacgtagc agtgctcact 600
   tccatgctca ccgacccctc ccacattacg gcggagacgg ctaagcgtag gctggccagg 660
   ggatctcccc cctccttggc cagctcatca gctagccagc tgtctgcgcc ttccttgaag 720
   gcaacatgca ctacccgtca tgactccccg gacgctgacc tcatcgaggc caacctcctg 780
   tggcggcagg agatgggcgg gaacatcacc cgcgtggagt cagaaaataa ggtagtaatt 840
   ttggactctt tcgagccgct ccaagcggag gaggatgaga gggaagtatc cgttccggcg 900
   gagatcctgc ggaggtccag gaaattccct cgagcgatgc ccatatgggc acgcccggat 960
   tacaaccctc cactgttaga gtcctggaag gacccggact acgtccctcc agtggtacac 1020
   gggtgtccat tgccgcctgc caaggcccct ccgataccac ctccacggag gaagaggacg 1080
   gttgtcctgt cagaatctac cgtgtcttct gccttggcgg agctcgccac aaagaccttc 1140
   ggcagctccg aatcgtcggc cgtcgacagc ggcacggcaa cggcctctcc tgaccagccc 1200
   tccgacgacg gcgacgcggg atccgacgtt gagtcgtact cctccatgcc cccccttgag 1260
   ggggagccgg gggatcccga tctcagcgac gggtcttggt ctaccgtaag cgaggaggct 1320
   agtgaggacg tcgtctgctg c                                          1341
<210> 132
   <211> 1772
   <212> DNA
   <213> Hepatitis C virus
<300>
   <302> NS5B
   <310> AJ238799
<400> 132
   tcgatgtcct acacatggac aggcgccctg atcacgccat gcgctgcgga ggaaaccaag 60
   ctgcccatca atgcactgag caactctttg ctccgtcacc acaacttggt ctatgctaca 120
   acatctcgca gcgcaagcct gcggcagaag aaggtcacct ttgacagact gcaggtcctg 180
   gacgaccact accgggacgt gctcaaggag atgaaggcga aggcgtccac agttaaggct 240
   aaacttctat ccgtggagga agcctgtaag ctgacgcccc cacattcggc cagatctaaa 300
   tttggctatg gggcaaagga cgtccggaac ctatccagca aggccgttaa ccacatccgc 360
   tccgtgtgga aggacttgct ggaagacact gagacaccaa ttgacaccac catcatggca 420
   aaaaatgagg ttttctgcgt ccaaccagag aaggggggcc gcaagccagc tcgccttatc 480
   gtattcccag atttgggggt tcgtgtgtgc gagaaaatgg ccctttacga tgtggtctcc 540
   accctccctc aggccgtgat gggctcttca tacggattcc aatactctcc tggacagcgg 600
   gtcgagttcc tggtgaatgc ctggaaagcg aagaaatgcc ctatgggctt cgcatatgac 660
   acccgctgtt ttgactcaac ggtcactgag aatgacatcc gtgttgagga gtcaatctac 720
   caatgttgtg acttggcccc cgaagccaga caggccataa ggtcgctcac agagcggctt 780
   tacatcgggg gccccctgac taattctaaa gggcagaact gcggctatcg ccggtgccgc 840
   gcgagcggtg tactgacgac cagctgcggt aataccctca catgttactt gaaggccgct 900
   gcggcctgtc gagctgcgaa gctccaggac tgcacgatgc tcgtatgcgg agacgacctt 960
   gtcgttatct gtgaaagcgc ggggacccaa gaggacgagg cgagcctacg ggccttcacg 1020
   gaggctatga ctagatactc tgccccccct ggggacccgc ccaaaccaga atacgacttg 1080
   gagttgataa catcatgctc ctccaatgtg tcagtcgcgc acgatgcatc tggcaaaagg 1140
   gtgtactatc tcacccgtga ccccaccacc ccccttgcgc gggctgcgtg ggagacagct 1200
   agacacactc cagtcaattc ctggctaggc aacatcatca tgtatgcgcc caccttgtgg 1260
   gcaaggatga tcctgatgac tcatttcttc tccatccttc tagctcagga acaacttgaa 1320
   aaagccctag attgtcagat ctacggggcc tgttactcca ttgagccact tgacctacct 1380
   cagatcattc aacgactcca tggccttagc gcattttcac tccatagtta ctctccaggt 1440
   gagatcaata gggtggcttc atgcctcagg aaacttgggg taccgccctt gcgagtctgg 1500
   agacatcggg ccagaagtgt ccgcgctagg ctactgtccc agggggggag ggctgccact 1560
   tgtggcaagt acctcttcaa ctgggcagta aggaccaagc tcaaactcac tccaatcccg 1620
   gctgcgtccc agttggattt atccagctgg ttcgttgctg gttacagcgg gggagacata 1680
   tatcacagcc tgtctcgtgc ccgaccccgc tggttcatgt ggtgcctact cctactttct 1740
   gtaggggtag gcatctatct actccccaac cg 1772
<210> 133
   <211> 1892
   <212> DNA
   <213> Hepatitis C virus
<300>
   <302> NS3
   <310> AJ238799
<400> 133
   cgcctattac ggcctactcc caacagacgc gaggcctact tggctgcatc atcactagcc 60
   tcacaggccg ggacaggaac caggtcgagg gggaggtcca agtggtctcc accgcaacac 120
   aatctttcct ggcgacctgc gtcaatggcg tgtgttggac tgtctatcat ggtgccggct 180
   caaagaccct tgccggccca aagggcccaa tcacccaaat gtacaccaat gtggaccagg 240
   acctcgtcgg ctggcaagcg ccccccgggg cgcgttcctt gacaccatgc acctgcggca 300
   gctcggacct ttacttggtc acgaggcatg ccgatgtcat tccggtgcgc cggcggggcg 360
   acagcagggg gagcctactc tcccccaggc ccgtctccta cttgaagggc tcttcgggcg 420
   gtccactgct ctgcccctcg gggcacgctg tgggcatctt tcgggctgcc gtgtgcaccc 480
   gaggggttgc gaaggcggtg gactttgtac ccgtcgagtc tatggaaacc actatgcggt 540
   ccccggtctt cacggacaac tcgtcccctc cggccgtacc gcagacattc caggtggccc 600
   atctacacgc ccctactggt agcggcaaga gcactaaggt gccggctgcg tatgcagccc 660
   aagggtataa ggtgcttgtc ctgaacccgt ccgtcgccgc caccctaggt ttcggggcgt 720
   atatgtctaa ggcacatggt atcgacccta acatcagaac cggggtaagg accatcacca 780
   cgggtgcccc catcacgtac tccacctatg gcaagtttct tgccgacggt ggttgctctg 840
   ggggcgccta tgacatcata atatgtgatg agtgccactc aactgactcg accactatcc 900
   tgggcatcgg cacagtcctg gaccaagcgg agacggctgg agcgcgactc gtcgtgctcg 960
   ccaccgctac gcctccggga tcggtcaccg tgccacatcc aaacatcgag gaggtggctc 1020
   tgtccagcac tggagaaatc cccttttatg gcaaagccat ccccatcgag accatcaagg 1080
   gggggaggca cctcattttc tgccattcca agaagaaatg tgatgagctc gccgcgaagc 1140
   tgtccggcct cggactcaat gctgtagcat attaccgggg ccttgatgta tccgtcatac 1200
   caactagcgg agacgtcatt gtcgtagcaa cggacgctct aatgacgggc tttaccggcg 1260
   atttcgactc agtgatcgac tgcaatacat gtgtcaccca gacagtcgac ttcagcctgg 1320
   acccgacctt caccattgag acgacgaccg tgccacaaga cgcggtgtca cgctcgcagc 1380
   ggcgaggcag gactggtagg ggcaggatgg gcatttacag gtttgtgact ccaggagaac 1440
   ggccctcggg catgttcgat tcctcggttc tgtgcgagtg ctatgacgcg ggctgtgctt 1500
   ggtacgagct cacgcccgcc gagacctcag ttaggttgcg ggcttaccta aacacaccag 1560
   ggttgcccgt ctgccaggac catctggagt tctgggagag cgtctttaca ggcctcaccc 1620
   acatagacgc ccatttcttg tcccagacta agcaggcagg agacaacttc ccctacctgg 1680
   tagcatacca ggctacggtg tgcgccaggg ctcaggctcc acctccatcg tgggaccaaa 1740
   tgtggaagtg tctcatacgg ctaaagccta cgctgcacgg gccaacgccc ctgctgtata 1800
   ggctgggagc cgttcaaaac gaggttacta ccacacaccc cataaccaaa tacatcatgg 1860
   catgcatgtc ggctgacctg gaggtcgtca cg 1892
<210> 134
   <211> 822
   <212> DNA
   <213> Homo sapiens
<300>
   <302> stmn cell factor
   <310> M59964
<400> 134
   atgaagaaga cacaaacttg gattctcact tgcatttatc ttcagctgct cctatttaat 60
   cctctcgtca aaactgaagg gatctgcagg aatcgtgtga ctaataatgt aaaagacgtc 120
   actaaattgg tggcaaatct tccaaaagac tacatgataa ccctcaaata tgtccccggg 180
   atggatgttt tgccaagtca ttgttggata agcgagatgg tagtacaatt gtcagacagc 240
   ttgactgatc ttctggacaa gttttcaaat atttctgaag gcttgagtaa ttattccatc 300
   atagacaaac ttgtgaatat agtcgatgac cttgtggagt gcgtcaaaga aaactcatct 360
   aaggatctaa aaaaatcatt caagagccca gaacccaggc tctttactcc tgaagaattc 420
   tttagaattt ttaatagatc cattgatgcc ttcaaggact ttgtagtggc atctgaaact 480
   agtgattgtg tggtttcttc aacattaagt cctgagaaag attccagagt cagtgtcaca 540
   aaaccattta tgttaccccc tgttgcagcc agctccctta ggaatgacag cagtagcagt 600
   aataggaagg ccaaaaatcc ccctggagac tccagcctac actgggcagc catggcattg 660
   ccagcattgt tttctcttat aattggcttt gcttttggag ccttatactg gaagaagaga 720
   cagccaagtc ttacaagggc agttgaaaat atacaaatta atgaagagga taatgagata 780
   agtatgttgc aagagaaaga gagagagttt caagaagtgt aa                     822
<210> 135
   <211> 483
   <212> DNA
   <213> Homo sapiens
<300>
   <302> TGFalpha
   <310> AF123238
<400> 135
   atggtcccct cggctggaca gctcgccctg ttcgctctgg gtattgtgtt ggctgcgtgc 60
   caggccctgg agaacagcac gtccccgctg agtgcagacc cgcccgtggc tgcagcagtg 120
   gtgtcccatt ttaatgactg cccagattcc cacactcagt tctgcttcca tggaacctgc 180
   aggtttttgg tgcaggagga caagccagca tgtgtctgcc attctgggta cgttggtgca 240
   cgctgtgagc atgcggacct cctggccgtg gtggctgcca gccagaagaa gcaggccatc 300
   accgccttgg tggtggtctc catcgtggcc ctggctgtcc ttatcatcac atgtgtgctg 360
   atacactgct gccaggtccg aaaacactgt gagtggtgcc gggccctcat ctgccggcac 420
   gagaagccca gcgccctcct gaagggaaga accgcttgct gccactcaga aacagtggtc 480
   tga                                                               483
<210> 136
   <211> 1071
   <212> DNA
   <213> Homo sapiens
<300>
   <302> GD3 synthase
   <310> NM003034
<400> 136
   atgagcccct gcgggcgggc ccggcgacaa acgtccagag gggccatggc tgtactggcg 60
   tggaagttcc cgcggacccg gctgcccatg ggagccagtg ccctctgtgt cgtggtcctc 120
   tgttggctct acatcttccc cgtctaccgg ctgcccaacg agaaagagat cgtgcagggg 180
   gtgctgcaac agggcacggc gtggaggagg aaccagaccg cggccagagc gttcaggaaa 240
   caaatggaag actgctgcga ccctgcccat ctctttgcta tgactaaaat gaattcccct 300
   atggggaaga gcatgtggta tgacggggag tttttatact cattcaccat tgacaattca 360
   acttactctc tcttcccaca ggcaacccca ttccagctgc cattgaagaa atgcgcggtg 420
   gtgggaaatg gtgggattct gaagaagagt ggctgtggcc gtcaaataga tgaagcaaat 480
   tttgtcatgc gatgcaatct ccctcctttg tcaagtgaat acactaagga tgttggatcc 540
   aaaagtcagt tagtgacagc taatcccagc ataattcggc aaaggtttca gaaccttctg 600
   tggtccagaa agacatttgt ggacaacatg aaaatctata accacagtta catctacatg 660
   cctgcctttt ctatgaagac aggaacagag ccatctttga gggtttatta tacactgtca 720
   gatgttggtg ccaatcaaac agtgctgttt gccaacccca actttctgcg tagcattgga 780
   aagttctgga aaagtagagg aatccatgcc aagcgcctgt ccacaggact ttttctggtg 840
   agcgcagctc tgggtctctg tgaagaggtg gccatctatg gcttctggcc cttctctgtg 900
   aatatgcatg agcagcccat cagccaccac tactatgaca acgtcttacc cttttctggc 960
   ttccatgcca tgcccgagga atttctccaa ctctggtatc ttcataaaat cggtgcactg 1020
   agaatgcagc tggacccatg tgaagatacc tcactccagc ccacttccta g 1071
<210> 137
   <211> 744
   <212> DNA
   <213> Homo sapiens
<300>
   <302> FGF14
   <310> NM004115
<400> 137
   atggccgcgg ccatcgctag cggcttgatc cgccagaagc ggcaggcgcg ggagcagcac 60
   tgggaccggc cgtctgccag caggaggcgg agcagcccca gcaagaaccg cgggctctgc 120
   aacggcaacc tggtggatat cttctccaaa gtgcgcatct tcggcctcaa gaagcgcagg 180
   ttgcggcgcc aagatcccca gctcaagggt atagtgacca ggttatattg caggcaaggc 240
   tactacttgc aaatgcaccc cgatggagct ctcgatggaa ccaaggatga cagcactaat 300
   tctacactct tcaacctcat accagtggga ctacgtgttg ttgccatcca gggagtgaaa 360
   acagggttgt atatagccat gaatggagaa ggttacctct acccatcaga actttttacc 420
   cctgaatgca agtttaaaga atctgttttt gaaaattatt atgtaatcta ctcatccatg 480
   ttgtacagac aacaggaatc tggtagagcc tggtttttgg gattaaataa ggaagggcaa 540
   gctatgaaag ggaacagagt aaagaaaacc aaaccagcag ctcattttct acccaagcca 600
   ttggaagttg ccatgtaccg agaaccatct ttgcatgatg ttggggaaac ggtcccgaag 660
   cctggggtga cgccaagtaa aagcacaagt gcgtctgcaa taatgaatgg aggcaaacca 720
   gtcaacaaga gtaagacaac atag 744
<210> 138
   <211> 1503
   <212> DNA
   <213> Human immunodeficiency virus
<300>
   <302> gag (HIV)
   <310> NC001802
<400> 138
   atgggtgcga gagcgtcagt attaagcggg ggagaattag atcgatggga aaaaattcgg 60
   ttaaggccag ggggaaagaa aaaatataaa ttaaaacata tagtatgggc aagcagggag 120
   ctagaacgat tcgcagttaa tcctggcctg ttagaaacat cagaaggctg tagacaaata 180
   ctgggacagc tacaaccatc ccttcagaca ggatcagaag aacttagatc attatataat 240
   acagtagcaa ccctctattg tgtgcatcaa aggatagaga taaaagacac caaggaagct 300
   ttagacaaga tagaggaaga gcaaaacaaa agtaagaaaa aagcacagca agcagcagct 360
   gacacaggac acagcaatca ggtcagccaa aattacccta tagtgcagaa catccagggg 420
   caaatggtac atcaggccat atcacctaga actttaaatg catgggtaaa agtagtagaa 480
   gagaaggctt tcagcccaga agtgataccc atgttttcag cattatcaga aggagccacc 540
   ccacaagatt taaacaccat gctaaacaca gtggggggac atcaagcagc catgcaaatg 600
   ttaaaagaga ccatcaatga ggaagctgca gaatgggata gagtgcatcc agtgcatgca 660
   gggcctattg caccaggcca gatgagagaa ccaaggggaa gtgacatagc aggaactact 720
   agtacccttc aggaacaaat aggatggatg acaaataatc cacctatccc agtaggagaa 780
   atttataaaa gatggataat cctgggatta aataaaatag taagaatgta tagccctacc 840
   agcattctgg acataagaca aggaccaaag gaacccttta gagactatgt agaccggttc 900
   tataaaactc taagagccga gcaagcttca caggaggtaa aaaattggat gacagaaacc 960
   ttgttggtcc aaaatgcgaa cccagattgt aagactattt taaaagcatt gggaccagcg 1020
   gctacactag aagaaatgat gacagcatgt cagggagtag gaggacccgg ccataaggca 1080
   agagttttgg ctgaagcaat gagccaagta acaaattcag ctaccataat gatgcagaga 1140
   ggcaatttta ggaaccaaag aaagattgtt aagtgtttca attgtggcaa agaagggcac 1200
   acagccagaa attgcagggc ccctaggaaa aagggctgtt ggaaatgtgg aaaggaagga 1260
   caccaaatga aagattgtac tgagagacag gctaattttt tagggaagat ctggccttcc 1320
   tacaagggaa ggccagggaa ttttcttcag agcagaccag agccaacagc cccaccagaa 1380
   gagagcttca ggtctggggt agagacaaca actccccctc agaagcagga gccgatagac 1440
   aaggaactgt atcctttaac ttccctcagg tcactctttg gcaacgaccc ctcgtcacaa 1500
   taa 1503
<210> 139
   <211> 1101
   <212> DNA
   <213> Human immunodeficiency virus
<300>
   <302> TARBP2
   <310> NM004178
<400> 139
   atgagtgaag aggagcaagg ctccggcact accacgggct gcgggctgcc tagtatagag 60
   caaatgctgg ccgccaaccc aggcaagacc ccgatcagcc ttctgcagga gtatgggacc 120
   agaataggga agacgcctgt gtacgacctt ctcaaagccg agggccaagc ccaccagcct 180
   aatttcacct tccgggtcac cgttggcgac accagctgca ctggtcaggg ccccagcaag 240
   aaggcagcca agcacaaggc agctgaggtg gccctcaaac acctcaaagg ggggagcatg 300
   ctggagccgg ccctggagga cagcagttct ttttctcccc tagactcttc actgcctgag 360
   gacattccgg tttttactgc tgcagcagct gctaccccag ttccatctgt agtcctaacc 420
   aggagccccc ccatggaact gcagccccct gtctcccctc agcagtctga gtgcaacccc 480
   gttggtgctc tgcaggagct ggtggtgcag aaaggctggc ggttgccgga gtacacagtg 540
   acccaggagt ctgggccagc ccaccgcaaa gaattcacca tgacctgtcg agtggagcgt 600
   ttcattgaga ttgggagtgg cacttccaaa aaattggcaa agcggaatgc ggcggccaaa 660
   atgctgcttc gagtgcacac ggtgcctctg gatgcccggg atggcaatga ggtggagcct 720
   gatgatgacc acttctccat tggtgtgggc ttccgcctgg atggtcttcg aaaccggggc 780
   ccaggttgca cctgggattc tctacgaaat tcagtaggag agaagatcct gtccctccgc 840
   agttgctccc tgggctccct gggtgccctg ggccctgcct gctgccgtgt cctcagtgag 900
   ctctctgagg agcaggcctt tcacgtcagc tacctggata ttgaggagct gagcctgagt 960
   ggactctgcc agtgcctggt ggaactgtcc acccagccgg ccactgtgtg tcatggctct 1020
   gcaaccacca gggaggcagc ccgtggtgag gctgcccgcc gtgccctgca gtacctcaag 1080
   atcatggcag gcagcaagtg a 1101
<210> 140
   <211> 219
   <212> DNA
   <213> Human immunodeficiency virus
<300>
   <302> TAT (HIV)
   <310> U44023
<400> 140
   atggagccag tagatcctag cctagagccc tggaagcatc caggaagtca gcctaagact 60
   gcttgtacca cttgctattg taaagagtgt tgctttcatt gccaagtttg tttcataaca 120
   aaaggcttag gcatctccta tggcaggaag aagcggagac agcgacgaag aactcctcaa 180
   ggtcatcaga ctaatcaagt ttctctatca aagcagtaa 219
<210> 141
   <211> 22
   <212> RNA
   <213> Künstliche Sequenz
<220>
   <223> Beschreibung der künstlichen Sequenz: Sense-Strang (R1A) einer dsRNA, die homolog zur MDR-1-Sequenz ist
<400> 141
   ccaucucgaa aagaaguuaa ga 22
<210> 142
   <211> 24
   <212> RNA
   <213> Künstliche Sequenz
<220>
   <223> Beschreibung der künstlichen Sequenz: antisense-Strang (R1B) einer dsRNA, die komplementär zur MDR-1-Sequenz ist
<400> 142
   ucuuaacuuc uuuucgagau gggu 24
<210> 143
   <211> 22
   <222> RNA
   <213> Künszliche Sequenz
<220>
   <223> Beschreibung der künstlichen Sequenz: sense-Strang (R2A) einer dsRNA, die homolog zur MDR-1- Sequenz ist
<400> 143
   uauagguucc aggcuugcug ua 22
<210> 144
   <211> 22
   <212> RNA
   <213> Künstliche Sequenz
<220>
   <223> Beschreibung der künstlichen Sequenz: sense-Strang (R3A) einer dsRNA, die homolog zur Sequenz des MDR 1-Gens ist
<400> 144
   ccagagaagg ccgcaccugc au 22
<210> 145
   <211> 24
   <212> RNA
   <213> Künstliche Sequenz
<220>
   <223> Beschreibung der künstlichen Sequenz: antisense-Strang (R3B) einer dsRNA, die komplementär zur MDR-1-Sequenz ist
<400> 145
   augcaggugc ggccuucucu ggcu 24
<210> 146
   <211> 21
   <212> RNA
   <213> Künstliche Sequenz
<220>
   <223> Beschreibung der künstlichen Sequenz: sense-Strang (R4A) einer dsRNA, die homolog zur MDR-1-Sequenz ist
<400> 146
   ccaucucgaa aagaaguuaa g 21
<210> 147
   <211> 21
   <212> RNA
   <213> Künstliche Sequenz
<220>
   <223> Beschreibung der künstlichen Sequenz: antisense-Strang (R4B) einer dsRNA, die komplementär zur MD3-1-Sequenz ist
<400> 147
   uaacuucuuu ucgagauggg u 21
<210> 148
   <211> 22
   <212> RNA
   <213> Künstliche Sequenz
<220>
   <223> Beschreibung der künstlichen Sequenz: sense-Strang (S1A) einer dsRNA, die homolog zur YFP- bzw. GFP-Sequenz ist
<400> 148
   ccacaugaag cagcacgacu uc 22
<210> 149
   <211> 22
   <212> RNA
   <213> Künstliche Sequenz
<220>
   <223> Beschreibung der künstlichen Sequenz: antisense-Strang (S1B) einer dsRNA, die komplementär zur YFP- bzw. GFP-Sequenz ist
<400> 149
   gaagucgugc ugcuucaugu gg 22
<210> 150
   <211> 21
   <212> RNA
   <213> Künstliche Sequenz
<220>
   <223> Beschreibung der künstlichen Sequenz: antisense-Strang (S7A) einer dsRNA, die homolog zur YFP- bzw. GFP-Sequenz ist
<400> 150
   ccacaugaag cagcacgacu u 21
<210> 151
   <211> 21
   <212> RNA
   <213> Künstliche Sequenz
<220>
   <223> Beschreibung der künstlichen Sequenz: antisense-Strang (S7B) einer dsRNA, die komplementär zur YFP- bzw. GFP-Sequenz ist
<400> 151
   gucgugcugc uucauguggu c 21
<210> 152
   <211> 24
   <212> RNA
   <213> Künstliche Sequenz
<220>
   <223> Beschreibung der künstlichen Sequenz: antisense-Strang (R2B) einer dsRNA, die komplementär zur MDR-1-Sequenz ist
<400> 152
   uacagcaagc cuggaaccua uagc 24
<210> 153
   <211> 22
   <212> RNA
   <213> Künstliche Sequenz
<220>
   <223> Beschreibung der künstlichen Sequenz: sense-Strang (K1A) einer dsRNA, die homolog zur 5'-UTR der Neomycin-Sequenz ist
<400> 153
   acaggaugag gaucguuucg ca 22
<210> 154
   <211> 22
   <212> RNA
   <213> Künstliche Sequenz
<220>
   <223> Beschreibung der künstlichen Sequenz: antisense-Strang (K1B) einer dsRNA, die komplementär zur 5'-UTR der Neomycin-Sequenz ist
<400> 154
   ugcgaaacga uccucauccu gu 22
<210> 155
   <211> 21
   <212> RNA
   <213> Künstliche Sequenz
<220>
   <223> Beschreibung der künstlichen Sequenz: sense-Strang (K3A) einer dsRNA, die homolog zur 5'-UTR der Neomycin-Sequenz ist
<400> 155
   gaugaggauc guuucgcaug a 21
<210> 156
   <211> 21
   <212> RNA
   <213> Künstliche Sequenz
<220>
   <223> Beschreibung der künstlichen Sequenz: antisense-Strang (K3B) einer dsRNA, die komplementär zur 5'-UTR der Neomycin-Sequenz ist
<400> 156
   augcgaaacg auccucaucc u 21
<210> 157
   <211> 24
   <212> RNA
   <213> Künstliche Sequenz
<220>
   <223> Beschreibung der künstlichen Sequenz: sense-Strang (K2A) einer dsRNA, die homolog zur 5'-UTR der Neomycin-Sequenz ist
<400> 157
   acaggaugag gaucguuucg caug 24
<210> 158
   <211> 24
   <212> RNA
   <213> Künstliche Sequenz
<220>
   <223> Beschreibung der künstlichen Sequenz: antisense-Strang (K2B) einer dsRNA, die komplementär zur 5'-UTR der Neomycin-Sequenz ist
<400> 158
   ugcgaaacga uccucauccu gucu 24
<210> 159
   <211> 24
   <212> RNA
   <213> Künstliche Sequenz
<220>
   <223> Beschreibung der künstlichen Sequenz: antisense-Strang (S4B) einer dsRNA, die komplementär zur YFP-bzw. GFP-Sequenz ist
<400> 159
   gaagucgugc ugcuucaugu gguc 24
<210> 160
   <211> 24
   <212> RNA
   <213> Künstliche Sequenz
<220>
   <223> Beschreibung der künstlichen Sequenz: sense-Strang (PKC1 A) einer dsRNA, die homolog zur Proteinkinase C-Sequenz ist
<400> 160
   cuucuccgcc ucacaccgcu gcaa 24
<210> 161
   <211> 22
   <212> RNA
   <213> Künstliche Sequenz
<220>
   <223> Beschreibung der künstlichen Sequenz: antisense-Strang (PKC2 B) einer dsRNA, die komplementär zur Proteinkinase C-Sequenz ist
<400> 161
   gcagcggugu gaggcggaga ag 22
<210> 162
   <211> 21
   <212> RNA
   <213> Künstliche Sequenz
<220>
   <223> Beschreibung der künstlichen Sequenz: antisense-Strang (S12B) einer dsRNA, die komplementär zur YFP- bzw. GFP-Sequenz ist
<400> 162
   aagucgugcu gcuucaugug g 21
<210> 163
   <211> 23
   <212> RNA
   <213> Künstliche Sequenz
<220>
   <223> Beschreibung der künstlichen Sequenz: antisense-Strang (S11B) einer dsRNA, die komplementär zur YFP- bzw. GFP-Sequenz ist
<400> 163
   aagucgugcu gcuucaugug guc 23
<210> 164
   <211> 20
   <212> RNA
   <213> Künstliche Sequenz
<220>
   <223> Beschreibung der künstlichen Sequenz: sense-Strang (S13A) einer dsRNA, die homolog zur YFP- bzw. GFP-Sequenz ist
<400> 164
   ccacaugaag cagcacgacu 20
<210> 165
   <211> 22
   <212> RNA
   <213> Künstliche Sequenz
<220>
   <223> Beschreibung der künstlichen Sequenz: antisense-Strang (S13B) einer dsRNA, die komplementär zur YFP- bzw. GFP-Sequenz ist
<400> 155
<210> 166
   <211> 20
   <212> RNA
   <213> Künstliche Sequenz
<220>
   <223> Beschreibung der künstlichen Sequenz: antisense-Strang (S14B) einer dsRNA, die komplementär zur YFP- bzw. GFP-Sequenz ist
<400> 166
   agucgugcug cuucaugugg 20
<210> 167
   <211> 24
   <212> RNA
   <213> Künstliche Sequenz
<220>
   <223> Beschreibung der künstlichen Sequenz: sense-Strang (S4A) einer dsRNA, die homolog zur YFP- bzw. GFP-Sequenz ist
<400> 167
   ccacaugaag cagcacgacu ucuu 24
<210> 168
   <211> 21
   <212> RNA
   <213> Künstliche Sequenz
<220>
   <223> Beschreibung der künstlichen Sequenz: sense-Strang (ES-7A) einer dsRNA, die homolog zur humanen EGFR-Sequenz ist
<400> 168
   aacaccgcag caugucaaga u 21
<210> 169
   <211> 21
   <212> RNA
   <213> Künstliche Sequenz
<220>
   <223> Beschreibung der künstlichen Sequenz: antisense-Strang (ES-7B) einer dsRNA, die komplementär zur humanen EGFR-Sequenz ist
<400> 169
   cuugacaugc ugcgguguuu u 21
<210> 170
   <211> 22
   <212> RNA
   <213> Künstliche Sequenz
<220>
   <223> Beschreibung der künstlichen Sequenz: sense-Strang (ES-8A) einer dsRNA, die homolog zur humanen EGFR-Sequenz ist
<400> 170
   aaguuaaaau ucccgucgcu au 22
<210> 171
   <211> 22
   <212> RNA
   <213> Künstliche Sequenz
<220>
   <223> Beschreibung der künstlichen Sequenz: antisense-Strang (ES-8B) einer dsRNA, die komplementär zur humanen EGFR-Sequenz ist
<400> 171
   ugauagcgac gggaauuuua ac 22
<210> 172
   <211> 22
   <212> RNA
   <213> Künstliche Sequenz
<220>
   <223> Beschreibung der künstlichen Sequenz: sense-Strang (ES-2A) einer dsRNA, die homolog zur humanen EGFR-Sequenz ist
<400> 172
   agugugaucc aagcuguccc aa 22
<210> 173
   <211> 24
   <212> RNA
   <213> Künstliche Sequenz
<220>
   <223> Beschreibung der künstlichen Sequenz: antisense-Strang (ES-5B) einer dsRNA, die komplementär zur humanen EGFR-Sequenz ist
<400> 173
   uugggacagc uuggaucaca cuuu                                                24